# EUROPEAN PATENT APPLICATION

(11) **EP 4 527 406 A1**
(43) Date of publication of application: **26.03.2025**
(21) Application number: 23807697.0
(22) Date of filing: 18.05.2023
(51) Int. Cl.: A61K 39/00, A61P 35/00, A61P 37/04, C12N 5/0784, A61K 38/08, A61K 38/10, C07K 14/725

(54) **CANCER VACCINE USING COMMON CANCER ANTIGEN COCKTAIL, TCR/CAR-T CELL THERAPEUTIC, COMPANION DIAGNOSTIC METHOD, AND METHOD FOR DIAGNOSING CANCER ONSET RISK BY BLOOD-CIRCULATING CANCER CELL DETECTION**

(30) Priority: 18.05.2022 US 202263343252 P
(71) Applicant: National Cancer Center, Tokyo 104-0045 (JP)
(72) Inventor: NAKATSURA Tetsuya, Kashiwa-shi, Chiba 277-8577 (JP); TAKENOUCHI Kazumasa, Kashiwa-shi, Chiba 277-8577 (JP); TSUKAMOTO Nobuo, Kashiwa-shi, Chiba 277-8577 (JP); SHIMOMURA Manami, Kashiwa-shi, Chiba 277-8577 (JP); SUZUKI Toshihiro, Kashiwa-shi, Chiba 277-8577 (JP)
(74) Representative: Godemeyer Blum Lenze Patentanwälte Partnerschaft mbB - werkpatent
(86) International application number: PCT/JP2023/018618
(87) International publication number: WO 2023/224096

(57) **Abstract**

An object of the present invention is to provide a cancer vaccine with use of a common cancer antigen cocktail, a TCR/CAR-T cell therapeutic, a companion diagnostic method, and a method for diagnosing risk of cancer onset by detecting circulating tumor cells. The present invention provides a cancer vaccine comprising: (1) common cancer antigens comprising three or more selected from GPC3, ROBO1, EPHB4, CLDN1, and LAT1; (2) partial peptides of the three or more common cancer antigens with CTL inducibility; (3) a dendritic cell stimulated with the partial peptides; or (4) mRNAs encoding the common cancer antigens or the partial peptides.

## Description

### Technical Field

The present invention relates to a cancer vaccine with use of a common cancer antigen cocktail. The present invention further relates to a TCR/CAR-T cell therapeutic, a companion diagnostic method, and a method for diagnosing risk of cancer onset by detecting circulating tumor cells.

### Background Art

Many patients diagnosed with cancer are not able to survive for 10 years or more even if they receive radical surgical excision. One million individuals are affected by cancer and 400,000 individuals are killed thereby every year in Japan. If a cancer vaccine, a TCR/CAR-T cell therapeutic, a companion diagnostic method, and a method for diagnosing risk of cancer onset by detecting circulating tumor cells are developed for cancer, 1,000,000 affected individuals are expected to be targeted every year. If diagnosis of risk of cancer onset by blood collection and application of a cancer prevention vaccine to individuals with onset risk become available, all the citizens can be targeted.

However, previous developments of vaccine therapy for cancer have all ended in failure. No TCR/CAR-T cell treatment promising as a therapeutic method for solid cancer has appeared yet. Furthermore, there is no CTC-capturing technique that can be regarded as completed one, and thus there is no technique that assures with certainty that cells suspected to be CTCs are cancer cells and contributes to diagnosis of risk of recurrence and/or onset of cancer. The spotlighted techniques, vaccination with neoantigens and TCR-T cell treatment, are personalized treatments, and difficult to implement because of development cost and regulation.

### Summary of Invention

### Object to be Solved by the Invention

To develop a cancer vaccine and therapy using T cells having a TCR gene introduced therein that can be applied to many individuals, antigens that are expressed on target cancer cells are the key. Neoantigens, which need different handlings for different individuals, are not suitable as such antigens, and antigens that are expressed in common cancers and not expressed in most of the normal organs, what is called common cancer antigens, have significance. For CAR-T cell therapy or antibody treatment, or for capturing circulating tumor cells, common cancer antigens that are expressed on cell membranes, in particular, are needed. Disadvantageously, most of the famous cancer antigens including cancer-testis antigens as previously reported are not expressed on cell membranes, have small expression frequency, and are expressed also in normal tissues.

An object of the present invention to achieve is to provide a cancer vaccine with use of a common cancer antigen cocktail, a TCR/CAR-T cell therapeutic, a companion diagnostic method, and a method for diagnosing risk of cancer onset by detecting circulating tumor cells.

### Means for Solving the Object

The present inventors have diligently examined to achieve the object, and identified ten common cancer antigens that are highly frequently expressed in various solid cancers and hardly expressed in normal tissues. Five of the ten are membrane protein antigens, and at least one or more of the five antigens are expressed in most of the previously examined cases of solid cancer such as head and neck cancer, lung cancer, liver cancer, biliary cancer, pancreatic cancer, and colorectal cancer; thus, common cancer antigen cocktails of them have been proved to be able to cover all types of solid cancer. The present invention has been completed on the basis of the findings.

Specifically, the present invention provides the followings.
<1> A cancer vaccine comprising:
   (1) common cancer antigens comprising three or more selected from GPC3, ROBO1, EPHB4, CLDN1, and LAT1;
   (2) partial peptides of the three or more common cancer antigens with CTL inducibility;
   (3) a dendritic cell stimulated with the partial peptides; or
   (4) mRNAs encoding the common cancer antigens or the partial peptides.
<2> The cancer vaccine according to <1>, wherein the common cancer antigens further comprise one or more of AFP, TGFBI, SPARC, HSP105α, and FOXM1.
<3> The cancer vaccine according to <1>, wherein the common cancer antigens comprise all of GPC3, ROBO1, EPHB4, CLDN1, and LAT1.
<4> The cancer vaccine according to <1>, wherein the common cancer antigens comprise all of GPC3, ROBO1, EPHB4, CLDN1, LAT1, AFP, TGFBI, SPARC, HSP105α, and FOXM1.
<5> The cancer vaccine according to any one of <1> to <4>, wherein the partial peptides are each a peptide having an amino acid sequence set forth in any of SEQ ID NOs: 1 to 80.
<6> A cancer vaccine comprising:
   (1) common cancer antigens comprising three or more selected from GPC3, ROBO1, EPHB4, CLDN1, LAT1, AFP, TGFBI, SPARC, HSP105α, and FOXM1;
   (2) partial peptides of the three or more common cancer antigens with CTL inducibility;
   (3) a dendritic cell stimulated with the partial peptides; or
   (4) mRNAs encoding the common cancer antigens or the partial peptides.
<7> A peptide having an amino acid sequence set forth in any of SEQ ID NOs: 5, 7 to 10, 14 to 22, 24 to 38, 40, 42, 48, 49, and 52 to 80.
<8> A CAR-T cell therapy agent comprising a mixture of T cells with chimeric antigen receptors (CARs) for common cancer antigens comprising three or more selected from GPC3, ROBO1, EPHB4, CLDN1, and LAT1.
<9> The CAR-T cell therapy agent according to <8>, wherein the common cancer antigens further comprise one or more of AFP, TGFBI, SPARC, HSP105α, and FOXM1.
<10> The CAR-T cell therapy agent according to <8>, wherein the common cancer antigens comprise all of GPC3, ROBO1, EPHB4, CLDN1, and LAT1.
<11> The CAR-T cell therapy agent according to <8>, wherein the common cancer antigens comprise all of GPC3, ROBO1, EPHB4, CLDN1, LAT1, AFP, TGFBI, SPARC, HSP105α, and FOXM1.
<12> A CAR-T cell therapy agent comprising a mixture of T cells with chimeric antigen receptors (CARs) for common cancer antigens comprising three or more selected from GPC3, ROBO1, EPHB4, CLDN1, LAT1, AFP, TGFBI, SPARC, HSP105α, and FOXM1.
<13> A TCR-T cell therapy drug comprising a mixture of T cells with T-cell receptors (TCRs) capable of recognizing MHC class I-binding antigen peptides derived from common cancer antigens comprising three or more of GPC3, ROBO1, EPHB4, CLDN1, and LAT1.
<14> The TCR-T cell therapy agent according to <13>, wherein the common cancer antigens further comprise one or more of AFP, TGFBI, SPARC, HSP105α, and FOXM1.
<15> The TCR-T cell therapy agent according to <13>, wherein the common cancer antigens comprise all of GPC3, ROBO1, EPHB4, CLDN1, and LAT1.
<16> The TCR-T cell therapy agent according to <13>, wherein the common cancer antigens comprise all of GPC3, ROBO1, EPHB4, CLDN1, LAT1, AFP, TGFBI, SPARC, HSP105α, and FOXM1.
<17> A TCR-T cell therapy drug comprising a mixture of T cells with T-cell receptors (TCRs) capable of recognizing MHC class I-binding antigen peptides derived from common cancer antigens comprising three or more of GPC3, ROBO1, EPHB4, CLDN1, LAT1, AFP, TGFBI, SPARC, HSP105α, and FOXM1.
<18> The TCR-T cell therapy agent according to any one of <13> to <17>, wherein each T-cell receptor (TCR) is any of:
   a heterodimer consisting of a combination of a protein of SEQ ID NO: 103 and a protein of SEQ ID NO: 104;
   a heterodimer consisting of a combination of a protein of SEQ ID NO: 105 and a protein of SEQ ID NO: 106;
   a heterodimer consisting of a combination of a protein of SEQ ID NO: 107 and a protein of SEQ ID NO: 108;
   a heterodimer consisting of a combination of a protein of SEQ ID NO: 109 and a protein of SEQ ID NO: 110;
   a heterodimer consisting of a combination of a protein of SEQ ID NO: 111 and a protein of SEQ ID NO: 112;
   a heterodimer consisting of a combination of a protein of SEQ ID NO: 123 and a protein of SEQ ID NO: 124;
   a heterodimer consisting of a combination of a protein of SEQ ID NO: 125 and a protein of SEQ ID NO: 126;
   a heterodimer consisting of a combination of a protein of SEQ ID NO: 127 and a protein of SEQ ID NO: 128;
   a heterodimer consisting of a combination of a protein of SEQ ID NO: 129 or 130 and a protein of SEQ ID NO: 131;
   a heterodimer consisting of a combination of a protein of SEQ ID NO: 132 and a protein of SEQ ID NO: 133;
   a heterodimer consisting of a combination of a protein of SEQ ID NO: 134 and a protein of SEQ ID NO: 135;
   a heterodimer consisting of a combination of a protein of SEQ ID NO: 136 and a protein of SEQ ID NO: 137;
   a heterodimer consisting of a combination of a protein of SEQ ID NO: 138 or 139 and a protein of SEQ ID NO: 140;
   a heterodimer consisting of a combination of a protein of SEQ ID NO: 141 and a protein of SEQ ID NO: 142;
   a heterodimer consisting of a combination of a protein of SEQ ID NO: 143 and a protein of SEQ ID NO: 144;
   a heterodimer consisting of a combination of a protein of SEQ ID NO: 145 and a protein of SEQ ID NO: 146;
   a heterodimer consisting of a combination of a protein of SEQ ID NO: 147 and a protein of SEQ ID NO: 148;
   a heterodimer consisting of a combination of a protein of SEQ ID NO: 177 and a protein of SEQ ID NO: 178;
   a heterodimer consisting of a combination of a protein of SEQ ID NO: 179 and a protein of SEQ ID NO: 180;
   a heterodimer consisting of a combination of a protein of SEQ ID NO: 181 and a protein of SEQ ID NO: 182;
   a heterodimer consisting of a combination of a protein of SEQ ID NO: 183 and a protein of SEQ ID NO: 184;
   a heterodimer consisting of a combination of a protein of SEQ ID NO: 193 and a protein of SEQ ID NO: 194;
   a heterodimer consisting of a combination of a protein of SEQ ID NO: 195 and a protein of SEQ ID NO: 196;
   a heterodimer consisting of a combination of a protein of SEQ ID NO: 197 and a protein of SEQ ID NO: 198;
   a heterodimer consisting of a combination of a protein of SEQ ID NO: 199 or 200 and a protein of SEQ ID NO: 201;
   a heterodimer consisting of a combination of a protein of SEQ ID NO: 202 and a protein of SEQ ID NO: 203;
   a heterodimer consisting of a combination of a protein of SEQ ID NO: 216 and a protein of SEQ ID NO: 217; and
   a heterodimer consisting of a combination of a protein of SEQ ID NO: 218 and a protein of SEQ ID NO: 219.
<19> A protein having an amino acid sequence set forth in any of SEQ ID NOs: 103 to 112, 123 to 148, 177 to 184, 193 to 203, and 215 to 219.
<20> A T-cell receptor (TCR) being any of:
   a heterodimer consisting of a combination of a protein of SEQ ID NO: 103 and a protein of SEQ ID NO: 104;
   a heterodimer consisting of a combination of a protein of SEQ ID NO: 105 and a protein of SEQ ID NO: 106;
   a heterodimer consisting of a combination of a protein of SEQ ID NO: 107 and a protein of SEQ ID NO: 108;
   a heterodimer consisting of a combination of a protein of SEQ ID NO: 109 and a protein of SEQ ID NO: 110;
   a heterodimer consisting of a combination of a protein of SEQ ID NO: 111 and a protein of SEQ ID NO: 112;
   a heterodimer consisting of a combination of a protein of SEQ ID NO: 123 and a protein of SEQ ID NO: 124;
   a heterodimer consisting of a combination of a protein of SEQ ID NO: 125 and a protein of SEQ ID NO: 126;
   a heterodimer consisting of a combination of a protein of SEQ ID NO: 127 and a protein of SEQ ID NO: 128;
   a heterodimer consisting of a combination of a protein of SEQ ID NO: 129 or 130 and a protein of SEQ ID NO: 131;
   a heterodimer consisting of a combination of a protein of SEQ ID NO: 132 and a protein of SEQ ID NO: 133;
   a heterodimer consisting of a combination of a protein of SEQ ID NO: 134 and a protein of SEQ ID NO: 135;
   a heterodimer consisting of a combination of a protein of SEQ ID NO: 136 and a protein of SEQ ID NO: 137;
   a heterodimer consisting of a combination of a protein of SEQ ID NO: 138 or 139 and a protein of SEQ ID NO: 140;
   a heterodimer consisting of a combination of a protein of SEQ ID NO: 141 and a protein of SEQ ID NO: 142;
   a heterodimer consisting of a combination of a protein of SEQ ID NO: 143 and a protein of SEQ ID NO: 144;
   a heterodimer consisting of a combination of a protein of SEQ ID NO: 145 and a protein of SEQ ID NO: 146;
   a heterodimer consisting of a combination of a protein of SEQ ID NO: 147 and a protein of SEQ ID NO: 148;
   a heterodimer consisting of a combination of a protein of SEQ ID NO: 177 and a protein of SEQ ID NO: 178;
   a heterodimer consisting of a combination of a protein of SEQ ID NO: 179 and a protein of SEQ ID NO: 180;
   a heterodimer consisting of a combination of a protein of SEQ ID NO: 181 and a protein of SEQ ID NO: 182;
   a heterodimer consisting of a combination of a protein of SEQ ID NO: 183 and a protein of SEQ ID NO: 184;
   a heterodimer consisting of a combination of a protein of SEQ ID NO: 193 and a protein of SEQ ID NO: 194;
   a heterodimer consisting of a combination of a protein of SEQ ID NO: 195 and a protein of SEQ ID NO: 196;
   a heterodimer consisting of a combination of a protein of SEQ ID NO: 197 and a protein of SEQ ID NO: 198;
   a heterodimer consisting of a combination of a protein of SEQ ID NO: 199 or 200 and a protein of SEQ ID NO: 201;
   a heterodimer consisting of a combination of a protein of SEQ ID NO: 202 and a protein of SEQ ID NO: 203;
   a heterodimer consisting of a combination of a protein of SEQ ID NO: 216 and a protein of SEQ ID NO: 217; and
   a heterodimer consisting of a combination of a protein of SEQ ID NO: 218 and a protein of SEQ ID NO: 219.
<21> A gene having a nucleotide sequence of any of SEQ ID NOs: 113 to 122, 149 to 176, 185 to 192, 204 to 214, and 220 to 224.
<22> A companion diagnostic method comprising: step 1 of simultaneously measuring the presence or absence of expressions of three or more of GPC3, ROBO1, EPHB4, CLDN1, and LAT1 and cell membrane expression of HLA class I in a sample derived from a subject by multiple immunofluorescence staining; and step 2 of determining indication for cancer immunotherapy on the basis of the presence or absence of the expressions.
<23> The companion diagnostic method according to <22>, wherein, in step 1, the presence or absence of expressions of one or more of AFP, TGFBI, SPARC, HSP105α, and FOXM1 is measured.
<24> The companion diagnostic method according to <22>, wherein, in step 1, the presence or absence of expressions of all of GPC3, ROBO1, EPHB4, CLDN1, and LAT1 is measured.
<25> The companion diagnostic method according to <22>, wherein, in step 1, the presence or absence of expressions of all of GPC3, ROBO1, EPHB4, CLDN1, LAT1, AFP, TGFBI, SPARC, HSP105α, and FOXM1 is measured.
<26> A method for diagnosing risk of cancer onset, comprising analyzing expressions of three or more of GPC3, ROBO1, EPHB4, CLDN1, and LAT1 in cells in a blood sample derived from a patient.
<27> The method for diagnosing risk of cancer onset according to <26>, further comprising analyzing expressions of one or more of AFP, TGFBI, SPARC, HSP105α, and FOXM1.
<28> The method for diagnosing risk of cancer onset according to <26>, wherein expressions of all of GPC3, ROBO1, EPHB4, CLDN1, and LAT1 are analyzed.
<29> The method for diagnosing risk of cancer onset according to <26>, wherein expressions of all of GPC3, ROBO1, EPHB4, CLDN1, LAT1, AFP, TGFBI, SPARC, HSP105α, and FOXM1 are analyzed.

### Advantageous Effects of Invention

The present invention provides a cancer vaccine with use of a common cancer antigen cocktail with use of a common cancer antigen cocktail, a TCR/CAR-T cell therapeutic, a companion diagnostic method, and a method for diagnosing risk of cancer onset by detecting circulating tumor cells.

### Brief Description of Drawings

[Fig. 1] Fig. 1 shows results of immunohistochemical staining of paraffin-embedded tissue sections to confirm expressions of ten common cancer antigens in hepatocellular carcinoma and other cancers.
[Fig. 2] Fig. 2 shows expressions of the cell membrane-expressed common cancer antigens GPC3, EPHB4, CLDN1, LAT1, and ROBO1 in different cancer types.
[Fig. 3] Fig. 3 shows expressions of common cancer antigens and expression of HLA-Class I in neuroblastoma, nephroblastoma, and choriocarcinoma, which are each childhood cancer.
[Fig. 4] Fig. 4 shows results of confirming expression of each common cancer antigen in non-cancer parts of a hepatocellular carcinoma human clinical specimen.
[Fig. 5] Fig. 5 is a table showing expressions of the cell membrane-expressed common cancer antigens GPC3, EPHB4, CLDN1, LAT1, and ROBO1 in 31 normal tissues with use of a human normal tissue microarray.
[Fig. 6] Fig. 6 shows the concept of indication diagnosis system development using multiple immunofluorescence staining to determine which common cancer antigen is effective when being targeted in immunotherapy such as TCR-T, CAR-T, and a cancer vaccine.
[Fig. 7] Fig. 7 shows results of multiple immunofluorescence staining for the cell membrane-expressed common cancer antigens GPC3, EPHB4, CLDN1, LAT1, and ROBO1 and HLA-Class I with samples derived from hepatocellular carcinoma and lung cancer patients as PDXs and samples derived from hepatocellular carcinoma, intrahepatic bile duct cancer, and oropharyngeal cancer (HPV-) patients as human clinical specimens.
[Fig. 8] Fig. 8 shows gene expressions for different cancer antigens in hepatocellular carcinoma and primary colorectal cancer.
[Fig. 9] Fig. 9 shows gene expressions for different cancer antigens in hepatocellular carcinoma and colorectal cancer liver metastasis for which antigen prediction was performed.
[Fig. 10] Fig. 10 shows a prediction pipeline for cancer antigen-derived epitope peptides.
[Fig. 11] Fig. 11 shows the distribution of SCOREadj of different cancer antigen-derived peptides in hepatocellular carcinoma.
[Fig. 12] Fig. 12 shows the total numbers of cancer antigen peptides predicted in seven hepatocellular carcinoma cases.
[Fig. 13] Fig. 13 shows a method for identifying a common cancer antigen-derived epitope peptide that is capable of inducing antigen-specific T cells. From ten common cancer antigen amino acid sequences, 72 HLA-A*24:02-restricted epitope peptides and 73 HLA-A*02:01-restricted epitope peptides were predicted by using an algorithm established in collaboration with BrightPath Biotherapeutics Co., Ltd.; Fig. 13 shows results of evaluation of antigen-specific productions of IFN-y with an ELISpot method in transgenic mice vaccinated with synthesized peptides.
[Fig. 14] Fig. 14 shows identification of multiple peptides each capable of inducing CD8-positive killer T cells (CTLs) reactive with the peptide in splenocytes of mice systemically expressing human HLA-A*02:01 in their cells by screening, wherein a peptide vaccine of peptides predicted to be presented by HLA-A*02:01 and synthesized from the amino acid sequences of ten common cancer antigen full-length proteins was administered once per week, three times in total.
[Fig. 15] Fig. 15 shows identification of multiple peptides each capable of inducing CD8-positive killer T cells (CTLs) reactive with the peptide of splenocytes in mice systemically expressing human HLA-A*24:02 in their cells by screening, wherein a peptide vaccine of peptides predicted to be presented by HLA-A*24:02 and synthesized from the amino acid sequences of ten common cancer antigen full-length proteins was administered once per week, three times in total.
[Fig. 16] Fig. 16 shows a method of evaluation on whether antigen-specific T cells are induced through vaccination of transgenic mice with dendritic cells (DCs) sensitized with a common cancer antigen-derived short peptide found to induce CTLs through the use of transgenic mice with different HLA genes introduced therein.
[Fig. 17] Fig. 17 shows results of evaluation on whether antigen-specific T cells are induced through vaccination of HLA-A*24:02 transgenic mice with dendritic cells (DCs) sensitized with a common cancer antigen-derived short peptide found to induce CTLs through the use of transgenic mice with the HLA gene introduced therein.
[Fig. 18] Fig. 18 shows results of evaluation on whether antigen-specific T cells are induced through vaccination of HLA-A*02:01 transgenic mice with dendritic cells (DCs) sensitized with a common cancer antigen-derived short peptide found to induce CTLs through the use of transgenic mice with the HLA gene introduced therein.
[Fig. 19] Fig. 19 shows results of validation with HLA-A*02:01 Tgm or HLA-A*24:02 Tgm to check whether a peptide is actually intracellularly processed and presented on HLA by using long peptides each containing the sequence of a common cancer antigen-derived short peptide found to induce CTLs.
[Fig. 20] Fig. 20 shows an administration method by triple administration of LNP-Covid-19 Spike mRNA.
[Fig. 21] Fig. 21 shows results of Spike antigen-specific IFN-γ production in spleen cells of HLA-A*02:01 Tg mice to which LNP-Covid-19 Spike mRNA was administered three times.
[Fig. 22] Fig. 22 shows results of Spike antigen-specific IFN-γ production in spleen cells of HLA-A*02:01 Tg mice to which LNP-eGFP mRNA was administered three times.
[Fig. 23] Fig. 23 shows results of Spike antigen-specific IFN-γ production in inguinal lymph node cells of HLA-A*02:01 Tg mice to which LNP-Covid-19 Spike mRNA or LNP-eGFP mRNA was administered three times.
[Fig. 24] Fig. 24 shows results of Spike antigen-specific IFN-γ production in spleen cells of HLA-A*24:02 Tg mice to which LNP-Covid-19 Spike mRNA was administered three times.
[Fig. 25] Fig. 25 shows results of Spike antigen-specific IFN-γ production in spleen cells of HLA-A*24:02 Tg mice to which LNP-eGFP mRNA was administered three times.
[Fig. 26] Fig. 26 shows results of Spike antigen-specific IFN-γ production in inguinal lymph node cells of HLA-A*24:02 Tg mice to which LNP-Covid-19 Spike mRNA or LNP-eGFP mRNA was administered three times.
[Fig. 27] Fig. 27 shows an administration method by double administration of LNP-Covid-19 Spike mRNA.
[Fig. 28] Fig. 28 shows results of Spike antigen-specific IFN-γ production in spleen cells of HLA-A*02:01 Tg mice to which LNP-Covid-19 Spike mRNA was administered twice.
[Fig. 29] Fig. 29 shows results of Spike antigen-specific IFN-γ production in spleen cells of HLA-A*02:01 Tg mice to which LNP-eGFP mRNA was administered twice.
[Fig. 30] Fig. 30 shows results of Spike antigen-specific IFN-γ production in inguinal lymph node cells of HLA-A*02:01 Tg mice to which LNP-Covid-19 Spike mRNA or LNP-eGFP mRNA was administered twice.
[Fig. 31] Fig. 31 shows results of Spike antigen-specific IFN-γ production in spleen cells of HLA-A*24:02 Tg mice to which LNP-Covid-19 Spike mRNA was administered twice.
[Fig. 32] Fig. 32 shows results of Spike antigen-specific IFN-γ production in spleen cells of HLA-A*24:02 Tg mice to which LNP-eGFP mRNA was administered twice.
[Fig. 33] Fig. 33 shows results of Spike antigen-specific IFN-γ production in inguinal lymph node cells of HLA-A*24:02 Tg mice to which LNP-Covid-19 Spike mRNA or LNP-eGFP mRNA was administered twice.
[Fig. 34] Fig. 34 shows results of antibody measurement for Spike protein.
[Fig. 35] Fig. 35 shows an administration method by triple administration of LNP-hGPC3 mRNA.
[Fig. 36] Fig. 36 shows results of evaluation of antigen-specific IFN-γ production in spleen cells of HLA-A*02:01 Tg mice with an ELISPOT method after administering LNP-hGPC3 mRNA to the mice.
[Fig. 37] Fig. 37 shows results of evaluation of antigen-specific IFN-γ production in spleen cells of HLA-A*02:01 Tg mice with an ELISPOT method after administering LNP-Luciferase mRNA to the mice.
[Fig. 38] Fig. 38 shows results of evaluation of antigen-specific IFN-γ production in spleen cells of HLA-A*24:02 Tg mice with an ELISPOT method after administering LNP-hGPC3 mRNA to the mice.
[Fig. 39] Fig. 39 shows results of evaluation of antigen-specific IFN-γ production in spleen cells of HLA-A*24:02 Tg mice with an ELISPOT method after administering LNP-Luciferase mRNA to the mice.
[Fig. 40] Fig. 40 shows an administration method by triple administration of LNP-hROBO1 mRNA.
[Fig. 41] Fig. 41 shows results of evaluation of antigen-specific IFN-γ production in spleen cells of HLA-A*02:01 Tg mice with an ELISPOT method after administering LNP-hROBO1 mRNA or LNP-eGFP mRNA to the mice.
[Fig. 42] Fig. 42 shows results of evaluation of antigen-specific IFN-γ production in spleen cells of HLA-A*24:02 Tg mice with an ELISPOT method after administering LNP-hROBO1 mRNA or LNP-eGFP mRNA to the mice.
[Fig. 43] Fig. 43 shows an administration method by triple administration of LNP-hCLDN1 mRNA or LNP-hEPHB4 mRNA.
[Fig. 44] Fig. 44 shows results of evaluation of antigen-specific IFN-γ production in spleen cells of HLA-A*02:01 Tg mice with an ELISPOT method after administering LNP-hCLDN1 mRNA, LNP-hEPHB4 mRNA, or LNP-eGFP mRNA to the mice.
[Fig. 45] Fig. 45 shows results of evaluation of antigen-specific IFN-γ production in spleen cells of HLA-A*24:02 Tg mice with an ELISPOT method after administering LNP-hCLDN1 mRNA, LNP-hEPHB4 mRNA, or LNP-eGFP mRNA to the mice.
[Fig. 46] Fig. 46 shows an administration method by triple administration of LNP-hTGFBI mRNA or LNP-hSPARC mRNA.
[Fig. 47] Fig. 47 shows results of evaluation of antigen-specific IFN-γ production in spleen cells of HLA-A*02:01 Tg mice with an ELISPOT method after administering LNP-hTGFBI mRNA, LNP-hSPARC mRNA, or LNP-eGFP mRNA to the mice.
[Fig. 48] Fig. 48 shows results of evaluation of antigen-specific IFN-γ production in spleen cells of HLA-A*24:02 Tg mice with an ELISPOT method after administering LNP-hTGFBI mRNA, LNP-hSPARC mRNA, or LNP-eGFP mRNA to the mice.
[Fig. 49] Fig. 49 shows an administration method by triple administration of LNP-hAFP mRNA or LNP-hHSP105α mRNA.
[Fig. 50] Fig. 50 shows results of evaluation of antigen-specific IFN-γ production in spleen cells of HLA-A*02:01 Tg mice with an ELISPOT method after administering LNP-hAFP mRNA, LNP-hHSP105α mRNA, or LNP-eGFP mRNA to the mice.
[Fig. 51] Fig. 51 shows results of evaluation of antigen-specific IFN-γ production in spleen cells of HLA-A*24:02 Tg mice with an ELISPOT method after administering LNP-hAFP mRNA, LNP-hHSP105α mRNA, or LNP-eGFP mRNA to the mice.
[Fig. 52] Fig. 52 shows an administration method by triple administration of LNP-(hCLDN1+hEPHB4) mRNA.
[Fig. 53] Fig. 53 shows results of evaluation of antigen-specific IFN-γ production in spleen cells of HLA-A*02:01 Tg mice with an ELISPOT method after administering LNP-(hCLDN1+hEPHB4) mRNA or LNP-Luciferase mRNA to the mice.
[Fig. 54] Fig. 54 shows results of evaluation of antigen-specific IFN-γ production in inguinal lymph nodes of HLA-A*02:01 Tg mice with an ELISPOT method after administering LNP-(hCLDN1+hEPHB4) mRNA or LNP-Luciferase mRNA to the mice.
[Fig. 55] Fig. 55 shows results of evaluation of antigen-specific IFN-γ production in spleen cells of HLA-A*24:02 Tg mice with an ELISPOT method after administering LNP-(hCLDN1+hEPHB4) mRNA or LNP-Luciferase mRNA to the mice.
[Fig. 56] Fig. 56 shows results of evaluation of antigen-specific IFN-γ production in inguinal lymph nodes of HLA-A*24:02 Tg mice with an ELISPOT method after administering LNP-(hCLDN1+hEPHB4) mRNA or LNP-Luciferase mRNA to the mice.
[Fig. 57] Fig. 57 shows a method for selecting high-affinity T cell clones through inhibition of binding of CD8 molecules to MHC.
[Fig. 58] Fig. 58 shows establishment of peptide-specific CTL clones from the peripheral blood of a patient inoculated with a GPC3 peptide vaccine.
[Fig. 59] Fig. 59 shows establishment of peptide-specific CTL clones from the peripheral blood of a patient inoculated with a FOXM1 peptide vaccine.
[Fig. 60] Fig. 60 shows establishment of peptide-specific CTL clones from the peripheral blood of a patient inoculated with an HSP105 peptide vaccine.
[Fig. 61] Fig. 61 shows identification of peptide-reactive TCR genes by using mice with human HLA genes introduced therein.
[Fig. 62] Fig. 62 shows preparation of TCR-T cells that recognize a common cancer antigen (FOXM1) by an electroporation method.
[Fig. 63] Fig. 63 shows a development scheme for cocktail TCR-T cells to overcome the diversity of cancers.
[Fig. 64] Fig. 64 shows *in vitro* cytotoxicity evaluation for CAR-T cells.
[Fig. 65] Fig. 65 shows *in vivo* cytotoxicity evaluation for CAR-T cells.
[Fig. 66] Fig. 66 shows development of cocktail CAR-T treatment.
[Fig. 67] Fig. 67 shows results of expression analysis for membrane protein common cancer antigens in various cancer cell lines.
[Fig. 68] Fig. 68 shows results of expression analysis for membrane protein common cancer antigens in various cancer cell lines.
[Fig. 69] Fig. 69 shows results of detection of CTC-like cells with expression of any of the membrane protein common cancer antigens GPC3, ROBO1, CLDN1, and EPHB4 on cell membranes in a blood collection specimen from a case of hepatocellular carcinoma.

### Embodiment of Carrying out the Invention

Hereinafter, the present invention will be described in more detail.

The present inventors have identified ten common cancer antigens that are highly frequently expressed in various solid cancers and hardly expressed in normal tissues through immunohistochemical analysis. Five of the ten are membrane protein antigens, and at least one or more of the five antigens are expressed in most of the previously examined cases of solid cancer such as head and neck cancer, lung cancer, liver cancer, biliary cancer, pancreatic cancer, and colorectal cancer; thus, common cancer antigen cocktails of them have been proved to be able to cover all types of solid cancer. These results have revealed that cancer vaccines with use of such a common cancer antigen cocktail, TCR-T cell treatment, CAR-T cell treatment, companion diagnosis for the common cancer antigens, and diagnosis of risk of cancer onset by detecting circulating tumor cells are achievable.

Eighty-five percents of Japanese people, and 50% of the world population possess HLA-A24 or -A2. In the present invention, peptides predicted to be presented by HLA-A24 and peptides predicted to be presented by HLA-A2 were synthesized from the amino acid sequences of ten common cancer antigen full-length proteins, and peptide vaccines thereof were administered to mice systemically expressing human HLA-A24 or -A2 in their cells once per week, three times in total, and the peptide vaccines were proved to induce CD8-positive killer T cells (CTLs) reactive with the peptides in splenocytes. These peptides are superior in CTL inducibility, and applicable as a peptide vaccine or a dendritic cell vaccine with such a peptide added thereto to treatment of, prevention of, recurrence of, or prevention of cancer. The ten common cancer antigens contain many peptide sequences capable of inducing CTLs, making application of mRNA vaccines possible.

Moreover, cloning of T-cell receptors (TCRs) that have been induced as a result of administration of a peptide vaccine derived from any of the ten common cancer antigens, exist on the cell surfaces of CTLs specifically reactive with any of the peptides, and recognize a complex of HLA and any of the peptides on cancer cell surfaces allows development of T cell therapy with T cells transfected with such TCRs (TCR-T treatment). Enhancing the repertoire of TCRs reactive with each common cancer antigen peptide enables cocktail TCR-T treatment optimum to individual patients or applicable to various cancers. Some of the 10 membrane protein common cancer antigens are expressed in most cancer tissues. This fact means that cocktail antibody therapy or cocktail CAR-T treatment against them can be used as a treatment method for most solid cancers.

### <Common cancer antigens>

GPC3, ROBO1, EPHB4, CLDN1, LAT1, AFP, TGFBI, SPARC, HSP105α, and FOXM1 are known proteins. In Examples shown later, proteins represented as the amino acid sequences shown as hGPC3_isoform_2_NP_004475
hROBO1_isoform_X5_XP_006713340 (hROBO1 of intracytoplasmic type)
hROBO1_isoform_a_NP_002932 (hROBO1 of membrane-delimited type)
hEPHB4_NP_004435
hCLDN1_NP_066924
hSLC7A5_NP_003477
hAFP_isoform_1_NP_001125
hTGFBI_NP_000349
hSPARC_isoform_1_NP_003109
hFOXM1_isoform_2_NP_068772
hHSPH1_isoform_1_NP_006635, isoforms thereof, or homologs thereof can be used. Examples of the isoforms include variants including splicing variants and SNP based on individual differences. Specific examples include: a protein consisting of an amino acid sequence having a sequence identity preferably of 95% or more, more preferably of 98% or more with any of those amino acid sequences shown in Examples; and (2) a protein consisting of an amino acid sequence given by substitution, deletion, addition, or insertion of one or more, preferably one to several, more preferably one to ten, one to five, one to three, or one or two amino acids in any one of those amino acid sequences shown in Examples.

### <Peptides and proteins>

Peptides and proteins in the present invention can be each produced with an expression vector, a cloning vector, or the like through a common gene engineering procedure including operations of DNA cloning with reference to nucleotide sequence information on a gene encoding the peptide or protein, plasmid construction, transfection into a host, culture of the transformant, and collection of a protein from the culture.

A recombinant vector can be produced by incorporating a polynucleotide into an appropriate vector. A vector according to the type of host and the intended use can be appropriately selected. Examples of vectors include vectors derived from a chromosome, an episome, or a virus. Specific examples include vectors derived from a bacterial plasmid, a bacteriophage, a transposon, a yeast episome, an insertion element, a yeast chromosome element, or a virus (e.g., a baculovirus, a papovavirus, an SV40, a vaccinia virus, an adenovirus, and a retrovirus), and vectors obtained by combining any of them, and vectors derived from a genetic element of a plasmid or a bacteriophage (e.g., a cosmid and a phagemid).

A recombinant vector containing a polynucleotide can be obtained by inserting a polynucleotide into a vector with a known method.

A transformant with a recombinant vector introduced therein can be obtained by introducing a recombinant vector with a polynucleotide introduced therein into a known host such as a bacterium such as *Escherichia coli* and a Bacillus bacterium, a yeast, and an insect cell or animal cell (e.g., a COS-7 cell, a Vero cell, a CHO cell) with a known method.

Transfection can be performed with a method known to those skilled in the art. Specific examples include calcium phosphate transfection, DEAE-dextran-mediated transfection, microinjection, cationic lipid-mediated transfection, electroporation, transduction, and infection.

### <Cancer vaccine>

The cancer vaccine of the present invention comprises:
(1) common cancer antigens comprising three or more selected from GPC3, ROBO1, EPHB4, CLDN1, and LAT1;
(2) partial peptides of the three or more common cancer antigens with CTL inducibility;
(3) a dendritic cell stimulated with the partial peptides; or
(4) mRNAs encoding the common cancer antigens or the partial peptides.

It is sufficient for the common cancer antigens to include three or more selected from GPC3, ROBO1, EPHB4, CLDN1, and LAT1, and the common cancer antigens may include three, four, or five selected from GPC3, ROBO1, EPHB4, CLDN1, and LAT1. Preferably, the common cancer antigens may include all of GPC3, ROBO1, EPHB4, CLDN1, and LAT1.

The common cancer antigens may further include one or more of AFP, TGFBI, SPARC, HSP105α, and FOXM1 in addition to those selected from GPC3, ROBO1, EPHB4, CLDN1, and LAT1. That is, the common cancer antigens may further include one, two, three, four, or five of AFP, TGFBI, SPARC, HSP105α, and FOXM1.

Preferably, the common cancer antigens may include all of GPC3, ROBO1, EPHB4, CLDN1, LAT1, AFP, TGFBI, SPARC, HSP105α, and FOXM1.

In the present invention, partial peptides of the three or more common cancer antigens with CTL inducibility can be used as a cancer vaccine. It follows that at least three or more such partial peptides are used as the partial peptides.

Specifically, it is sufficient for the partial peptides to include three or more selected from a partial peptide of GPC3, a partial peptide of ROBO1, a partial peptide of EPHB4, a partial peptide of CLDN1, and a partial peptide of LAT1, and the partial peptides may include three, four, or five of a partial peptide of GPC3, a partial peptide of ROBO1, a partial peptide of EPHB4, a partial peptide of CLDN1, and a partial peptide of LAT1. Preferably, the partial peptides may include all of a partial peptide of GPC3, a partial peptide of ROBO1, a partial peptide of EPHB4, a partial peptide of CLDN1, and a partial peptide of LAT1.

The partial peptides may further include one or more of a partial peptide of AFP, a partial peptide of TGFBI, a partial peptide of SPARC, a partial peptide of HSP105α, and a partial peptide of FOXM1 in addition to a partial peptide of GPC3, a partial peptide of ROBO1, a partial peptide of EPHB4, a partial peptide of CLDN1, and a partial peptide of LAT1. That is, the common cancer antigens may further include one, two, three, four, or five of a partial peptide of AFP, a partial peptide of TGFBI, a partial peptide of SPARC, a partial peptide of HSP105α, and a partial peptide of FOXM1.

Preferably, the partial peptides may include all of a partial peptide of GPC3, a partial peptide of ROBO1, a partial peptide of EPHB4, a partial peptide of CLDN1, a partial peptide of LAT1, a partial peptide of AFP, a partial peptide of TGFBI, a partial peptide of SPARC, a partial peptide of HSP105α, and a partial peptide of FOXM1.

The partial peptides are each an epitope peptide, a peptide that binds to MHC (HLA for humans) to be presented on cell surfaces as antigen presentation, and has antigenicity (recognizable for T cells). Epitope peptides include a CTL epitope peptide, which is an epitope peptide that binds to MHC class I to be presented as antigen presentation and is recognized by CD8-positive T cells, and a helper epitope peptide, which is an epitope peptide that binds to MHC class II to be presented as antigen presentation and recognized by CD4-positive T cells. Each of the partial peptides in the present invention is preferably a CTL epitope peptide, which is an epitope peptide that binds to MHC class I to be presented as antigen presentation and is recognized by CD8-positive T cells.

Each of the partial peptides in the present invention is a peptide derived from a protein specifically expressed in tumor cells, thus being a tumor antigen peptide. Antigen presentation is a phenomenon that a peptide present in a cell binds to MHC and the MHC/antigen peptide complex localizes on the cell surface. The antigen presented on the cell surface is recognized by T cells or the like, and then activates cell-mediated immunity and humoral immunity. Antigens presented by MHC class I not only activate cell-mediated immunity but also are recognized by T-cell receptors of naive T cells to induce the naive T cells into CTLs, which have cytotoxic activity; hence, peptides that bind to MHC class I to be presented as antigen presentation are preferred as tumor antigen peptides that are used for immunotherapy.

Each of the partial peptides in the present invention, being a partial peptide of GPC3, ROBO1, EPHB4, CLDN1, LAT1, AFP, TGFBI, SPARC, HSP105α, or FOXM1 as described above, is a peptide that binds to MHC, in particular, to HLA, preferably a peptide that is presented by MHC, in particular, by HLA as antigen presentation, and more preferably a peptide that is presented by MHC, in particular, by HLA as antigen presentation and capable of inducing CTLs. The partial peptides are preferably capable of binding to HLA class I, and more preferably capable of binding to HLA-A02 and/or HLA-A24.

The amino acid length of each partial peptide is not limited as long as the sequence contains the amino acid sequence of an epitope, and the amino acid length is preferably about 8 to 14 amino acids, more preferably about 8 to 11 amino acids, and particularly preferably about 9 to about 11 amino acids in typical cases.

It is known that epitope peptides that bind to HLA class I, which is human MHC class I, each have a length of about 8 to 14 amino acids, preferably have a length of about 9 to 11 amino acids, and each have a HLA-specific binding motif that binds to a part in the sequence. For example, a peptide that binds to HLA-A02 may have a binding motif such that the second amino acid from the N terminus may be leucine, isoleucine, or methionine and/or the C-terminal amino acid may be valine, leucine, or isoleucine, and a peptide that binds to HLA-A24 may have a binding motif such that the second amino acid from the N terminus may be tyrosine, phenylalanine, methionine, or tryptophan and/or the C-terminal amino acid may be leucine, isoleucine, or phenylalanine; however, the peptides are not limited to these modes.

Accordingly, the partial peptides are preferably each a partial peptide of GPC3, ROBO1, EPHB4, CLDN1, LAT1, AFP, TGFBI, SPARC, HSP105α, or FOXM1, wherein the partial peptide consists of contiguous 8 to 14 amino acids in the amino acid sequence of the protein, and contains an epitope peptide that may be a peptide such that the second amino acid from the N terminus may be leucine, isoleucine, or methionine and/or the C-terminal amino acid may be valine, leucine, or isoleucine. Alternatively, the partial peptides may be preferably each a partial peptide of GPC3, ROBO1, EPHB4, CLDN1, LAT1, AFP, TGFBI, SPARC, HSP105α, or FOXM1, wherein the partial peptide consists of contiguous 8 to 14 amino acids in the amino acid sequence of the protein, and is a peptide such that the second amino acid from the N terminus may be tyrosine, phenylalanine, methionine, or tryptophan and/or the C-terminal amino acid may be leucine, isoleucine, or phenylalanine; however, the partial peptides are not limited to these modes.

The N terminus and/or C terminus of each of the partial peptides may be modified. Specific examples of the modification include N-alkanoylation (e.g., acetylation), N-alkylation (e.g., methylation), C-terminal alkyl ester (e.g., ethyl ester), and C-terminal amide (e.g., carboxamide).

The partial peptides can be synthesized according to a known method that is used in common peptide chemistry.

Peptides each having an amino acid set forth in any one of SEQ ID NOs: 1 to 80 can be used as the partial peptides described above.

Among those peptides, the peptides each having an amino acid sequence set forth in any of SEQ ID NOs: 5, 7 to 10, 14 to 22, 24 to 38, 40, 42, 48, 49, and 52 to 80 are novel peptides.

The partial peptides described above each have CTL-inducing activity, and can serve as a tumor antigen peptide for use as a CTL inducer. Specifically, peripheral blood lymphocytes are isolated from a human positive for an HLA-A02 antigen or HLA-A24 antigen and stimulated *in vitro* with addition of the partial peptides described above; as a result, CTLs that specifically recognize HLA-A02 antigen-positive cells or HLA-A24 antigen-positive cells presenting the partial peptides described above are successfully induced. For example, the presence or absence of induction of CTLs can be confirmed through measurement of the amounts of various cytokines (e.g., IFN-γ) produced by CTLs in response to the antigen peptide-presenting cells by ELISA or the like. Alternatively, it can be confirmed with a method of measuring the cytotoxicity of CTLs to the antigen peptide-presenting cells labeled with 51Cr.

In the present invention, dendritic cells stimulated with the partial peptides can be used as a cancer vaccine. Specifically, antigen-presenting cells each presenting a complex of an HLA-A02 antigen or HLA-A24 antigen and any of the partial peptides on the cell surface can be produced by bringing the partial peptides and dendritic cells into contact *in vitro.* Preferably, isolated dendritic cells derived from a cancer patient can be used. Dendritic cells can be induced, for example, through a process in which lymphocytes are separated from the peripheral blood of a cancer patient with a Ficoll method, non-adherent cells are then removed, and adherent cells are cultured in the presence of GM-CSF and IL-4. Antigen-presenting cells each presenting a complex of an HLA-A02 antigen or HLA-A24 antigen and any of the partial peptides on the cell surface can be produced by bringing dendritic cells isolated from a cancer patient in that manner and the partial peptides of the present invention into contact *in vitro.*

In the present invention, mRNAs encoding the common cancer antigens described above or the partial peptides described above can be used as a cancer vaccine. An mRNA tandemly encoding multiple partial peptides may be used as an mRNA encoding the common cancer antigens or the partial peptides. In this case, multiple partial peptides are encoded in one mRNA molecule.

The cancer vaccine of the present invention is effective for cancer patients, in particular, for cancer patients positive for HLA-A02 or HLA-A24. Specifically, for example, the cancer vaccine of the present invention is effective for patients with hepatocellular carcinoma, colorectal cancer, oropharyngeal cancer, esophageal cancer, uterine cancer, nephroblastoma, lung cancer, breast cancer, tongue cancer, intrahepatic bile duct cancer, renal cancer, neuroblastoma, or choriocarcinoma.

The cancer vaccine of the present invention can be used for prevention or treatment of cancer. The meaning of prevention of cancer includes not only preventing a patient from being affected by cancer but also preventing recurrence in a patient subjected to surgical excision of tumor in the primary lesion, and prevention of the metastasis of tumor incompletely removed through cancer treatment such as surgery, radiotherapy, or drug therapy. The meaning of treatment of cancer includes not only cure of and/or amelioration of symptoms of cancer to cause cancer regression, but also preventing progression to suppress the growth of cancer cells, the enlargement of tumor, or the metastasis of cancer cells from the primary lesion.

The cancer vaccine of the present invention can be provided in the form of a pharmaceutical composition.

The cancer vaccine of the present invention may be in a dosage form of either an oral agent or a parenteral agent, but is preferably in the form of a parenteral agent in typical cases. Examples of the parenteral agent include subcutaneous injections, intramuscular injections, intravenous injections, and suppositories.

In the case that the cancer vaccine of the present invention is in the form of an oral agent, the active ingredients described above can be formulated together with a pharmaceutically acceptable diluent into the cancer vaccine. Examples of the diluent include starch, mannitol, lactose, magnesium stearate, cellulose, polymerized amino acids, and albumin.

In the case that the cancer vaccine of the present invention is in the form of a parenteral agent, the active ingredients described above can be formulated together with a pharmaceutically acceptable carrier into the cancer vaccine. Examples of the carrier include water, sodium chloride, dextrose, ethanol, glycerol, and DMSO.

The cancer vaccine of the present invention may further contain, for example, albumin, a wetting agent, and/or an emulsifying agent, as desired.

The active ingredients described above can be used in combination with an appropriate adjuvant to activate cell-mediated immunity. The cancer vaccine of the present invention may contain the adjuvant.

Adjuvants known in the art are applicable, and specific examples thereof include: adjuvants of gel type such as aluminum hydroxide, aluminum phosphate, and calcium phosphate; adjuvants of bacterial cell type such as CpG, monophosphoryl lipid A, cholera toxin, *Escherichia coli* heat-labile toxin, pertussis toxin, and muramyl dipeptide; adjuvants of oil emulsion type (emulsion formulations) such as incomplete Freund's adjuvants; adjuvants of polymer nanoparticle type such as liposomes, biodegradable microspheres, and QS-21 derived from saponin; adjuvants of synthetic type such as nonionic block copolymers, muramyl peptide analogs, polyphosphazene, and synthetic polynucleotides; and adjuvants of cytokine type such as IFN-y, IL-2, and IL-12.

Examples of the dosage form of the cancer vaccine of the present invention (pharmaceutical composition) include, but are not limited to, an oil emulsion (emulsion formulation), polymer nanoparticles, a liposome formulation, a particulate formulation bound to beads of several micrometers in diameter, a formulation bound to lipid, a microsphere formulation, and a microcapsule formulation.

Examples of methods for administering the cancer vaccine of the present invention (pharmaceutical composition) include intradermal administration, subcutaneous administration, intramuscular administration, and intravenous administration. The dose of the active ingredients of the cancer vaccine can be appropriately adjusted according to the disease to be treated, the age and body weight of the patient, and so on, and is typically 0.0001 mg to 1000 mg, preferably 0.001 mg to 1000 mg, and more preferably 0.1 mg to 10 mg per adult (body weight: 50 kg), and it is preferable to administer the dose once per several days to several months.

In the case that dendritic cells stimulated with the partial peptides are used as the cancer vaccine, it is preferable for the cancer vaccine to contain physiological saline, phosphate-buffered saline (PBS), medium, or the like in order to stably maintain the dendritic cells. Examples of administration methods include intravenous administration, subcutaneous administration, and intradermal administration. If the cancer vaccine containing such dendritic cells stimulated with the partial peptides as an active ingredient is returned into the body of the patient, then CTLs specific to cancer cells expressing any of the common cancer antigens of the present invention are efficiently induced in the body of the patient, who is affected by cancer, and as a result the cancer can be prevented or treated.

### <CAR-T cell therapy agent>

The CAR-T cell therapy agent of the present invention comprises a mixture of T cells with chimeric antigen receptors (CARs) for common cancer antigens comprising three or more selected from GPC3, ROBO1, EPHB4, CLDN1, and LAT1.

It is sufficient for the common cancer antigens to include three or more selected from GPC3, ROBO1, EPHB4, CLDN1, and LAT1, and the common cancer antigens may include three, four, or five selected from GPC3, ROBO1, EPHB4, CLDN1, and LAT1. Preferably, the common cancer antigens may include all of GPC3, ROBO1, EPHB4, CLDN1, and LAT1.

The common cancer antigens may further include one or more of AFP, TGFBI, SPARC, HSP105α, and FOXM1 in addition to those selected from GPC3, ROBO1, EPHB4, CLDN1, and LAT1. That is, the common cancer antigens may further include one, two, three, four, or five of AFP, TGFBI, SPARC, HSP105α, and FOXM1.

Preferably, the common cancer antigens may include all of GPC3, ROBO1, EPHB4, CLDN1, LAT1, AFP, TGFBI, SPARC, HSP105α, and FOXM1.

The CAR-T cell therapy is an immunocytic treatment method in which T cells derived from a patient are transfected with a chimeric antigen receptor (CAR) given through modification by genetically engineering part of a monoclonal antibody specific to a tumor antigen, and the genetically modified T cells are subjected to *ex vivo* amplification culture and then infused into the patient. Specifically, peripheral blood monocytes collected from a patient are cultured in the presence of an anti-CD3 antibody and IL-2 or the like to activate T cells, and a gene encoding a CAR is introduced into the T cells with a transforming vector such as a retroviral vector or a lentiviral vector to produce genetically modified T cells.

Each chimeric antigen receptor is a chimeric protein molecule designed to have a single-chain antibody in which the light chain and heavy chain of an antibody variable region of an antibody that recognizes a molecule present on the cell surfaces of cancer cells are bound in series (scFv) on the N-terminal side and have the CD3ζ chain of the molecules constituting a T-cell receptor (TCR)/CD3 complex on the C-terminal side. Such a chimeric antigen receptor causes T cell activation via the CD3ζ chain on recognizing a specific antigen with the scFv region. In order to enhance the T cell activation, one or two or more costimulatory molecules (e.g., CD28, 4-1BB, ICOS) may be incorporated between the scFv and the ζ chain. CARs can be produced by using a TCR-like antibody (also available is an antibody molecule that can be designed from the TCR-like antibody or a fragment thereof) for the scFv. A CAR that recognizes a complex of a tumor antigen-derived peptide and MHC is capable of recognizing, for example, cancer cells each presenting the tumor antigen peptide that can be targeted by CTLs and dendritic cells presenting the tumor antigen peptide on MHC class I as a result of phagocytosis of such a cancer cell, and hence, like artificial CTLs, genetically modified T cells with the CAR introduced therein are useful as a prophylactic and/or therapeutic for cancers specific to the tumor antigen.

T cells with chimeric antigen receptors (CARs) (CAR-T cells) can be formulated directly or together with a carrier into the CAR-T cell therapy agent.

The CAR-T cell therapy agent may be in a dosage form of either an oral agent or a parenteral agent. The CAR-T cell therapy agent is preferably in the form of a parenteral agent in typical cases. Examples of the parenteral agent include subcutaneous injections, intramuscular injections, intravenous injections, and suppositories.

In the case that the CAR-T cell therapy agent is in the form of an oral agent, CAR-T cells obtained can be formulated together with a pharmaceutically acceptable diluent into the CAR-T cell therapy agent. Examples of the diluent include starch, mannitol, lactose, magnesium stearate, cellulose, polymerized amino acids, and albumin.

In the case that the CAR-T cell therapy agent is in the form of a parenteral agent, CAR-T cells obtained can be formulated together with a pharmaceutically acceptable carrier into the CAR-T cell therapy agent. Examples of the carrier include water, sodium chloride, dextrose, ethanol, glycerol, and DMSO.

### <TCR-T cell therapy drug>

The TCR-T cell therapy drug of the present invention comprises a mixture of T cells with T-cell receptors (TCRs) capable of recognizing MHC class I-binding antigen peptides derived from common cancer antigens comprising three or more of GPC3, ROBO1, EPHB4, CLDN1, and LAT1.

It is sufficient for the common cancer antigens to include three or more selected from GPC3, ROBO1, EPHB4, CLDN1, and LAT1, and the common cancer antigens may include three, four, or five selected from GPC3, ROBO1, EPHB4, CLDN1, and LAT1. Preferably, the common cancer antigens may include all of GPC3, ROBO1, EPHB4, CLDN1, and LAT1.

The common cancer antigens may further include one or more of AFP, TGFBI, SPARC, HSP105α, and FOXM1 in addition to those selected from GPC3, ROBO1, EPHB4, CLDN1, and LAT1. That is, the common cancer antigens may further include one, two, three, four, or five of AFP, TGFBI, SPARC, HSP105α, and FOXM1.

Preferably, the common cancer antigens may include all of GPC3, ROBO1, EPHB4, CLDN1, LAT1, AFP, TGFBI, SPARC, HSP105α, and FOXM1.

The T-cell receptors (TCRs) capable of recognizing MHC class I-binding antigen peptides derived from the common cancer antigens are each capable of recognizing a complex of any of the partial peptides (antigen peptides) of the present invention and

### HLA.

Each T-cell receptor (TCR) is normally a heterodimer of TRA and TRB.

As specific examples, T-cell receptors (TCRs) listed in Tables 2 to 11 in Examples shown later can be used. Specifically, an example of the T-cell receptors (TCRs) can be:
a heterodimer consisting of a combination of a protein of SEQ ID NO: 103 and a protein of SEQ ID NO: 104;
a heterodimer consisting of a combination of a protein of SEQ ID NO: 105 and a protein of SEQ ID NO: 106;
a heterodimer consisting of a combination of a protein of SEQ ID NO: 107 and a protein of SEQ ID NO: 108;
a heterodimer consisting of a combination of a protein of SEQ ID NO: 109 and a protein of SEQ ID NO: 110;
a heterodimer consisting of a combination of a protein of SEQ ID NO: 111 and a protein of SEQ ID NO: 112;
a heterodimer consisting of a combination of a protein of SEQ ID NO: 123 and a protein of SEQ ID NO: 124;
a heterodimer consisting of a combination of a protein of SEQ ID NO: 125 and a protein of SEQ ID NO: 126;
a heterodimer consisting of a combination of a protein of SEQ ID NO: 127 and a protein of SEQ ID NO: 128;
a heterodimer consisting of a combination of a protein of SEQ ID NO: 129 or 130 and a protein of SEQ ID NO: 131;
a heterodimer consisting of a combination of a protein of SEQ ID NO: 132 and a protein of SEQ ID NO: 133;
a heterodimer consisting of a combination of a protein of SEQ ID NO: 134 and a protein of SEQ ID NO: 135;
a heterodimer consisting of a combination of a protein of SEQ ID NO: 136 and a protein of SEQ ID NO: 137;
a heterodimer consisting of a combination of a protein of SEQ ID NO: 138 or 139 and a protein of SEQ ID NO: 140;
a heterodimer consisting of a combination of a protein of SEQ ID NO: 141 and a protein of SEQ ID NO: 142;
a heterodimer consisting of a combination of a protein of SEQ ID NO: 143 and a protein of SEQ ID NO: 144;
a heterodimer consisting of a combination of a protein of SEQ ID NO: 145 and a protein of SEQ ID NO: 146;
a heterodimer consisting of a combination of a protein of SEQ ID NO: 147 and a protein of SEQ ID NO: 148;
a heterodimer consisting of a combination of a protein of SEQ ID NO: 177 and a protein of SEQ ID NO: 178;
a heterodimer consisting of a combination of a protein of SEQ ID NO: 179 and a protein of SEQ ID NO: 180;
a heterodimer consisting of a combination of a protein of SEQ ID NO: 181 and a protein of SEQ ID NO: 182;
a heterodimer consisting of a combination of a protein of SEQ ID NO: 183 and a protein of SEQ ID NO: 184;
a heterodimer consisting of a combination of a protein of SEQ ID NO: 193 and a protein of SEQ ID NO: 194;
a heterodimer consisting of a combination of a protein of SEQ ID NO: 195 and a protein of SEQ ID NO: 196;
a heterodimer consisting of a combination of a protein of SEQ ID NO: 197 and a protein of SEQ ID NO: 198;
a heterodimer consisting of a combination of a protein of SEQ ID NO: 199 or 200 and a protein of SEQ ID NO: 201;
a heterodimer consisting of a combination of a protein of SEQ ID NO: 202 and a protein of SEQ ID NO: 203;
a heterodimer consisting of a combination of a protein of SEQ ID NO: 216 and a protein of SEQ ID NO: 217; or
a heterodimer consisting of a combination of a protein of SEQ ID NO: 218 and a protein of SEQ ID NO: 219.

All of these amino acid sequences of and nucleotide sequences for the T-cell receptors (TCRs) are novel sequences.

The present invention provides a protein having an amino acid sequence set forth in any of SEQ ID NOs: 103 to 112, 123 to 148, 177 to 184, 193 to 203, and 215 to 219.

The present invention provides a gene having a nucleotide sequence set forth in any of SEQ ID NOs: 113 to 122, 149 to 176, 185 to 192, 204 to 214, and 220 to 224.

T cells with T-cell receptors (TCRs) can be induced by bringing the partial peptides of the common cancer antigens of the present invention into contact with peripheral blood lymphocytes *in vitro.* The resulting T cells with T-cell receptors (TCRs) are capable of specifically damaging cells presenting any of the common cancer antigens of the present invention. Preferably, peripheral blood lymphocytes of a patient are stimulated with the partial peptides of the common cancer antigens of the present invention *in vitro* to increase the number of T cells with T-cell receptors (TCRs) (i.e., tumor-specific CTLs), and then the T cells with T-cell receptors (TCRs) can be returned to the patient.

As described above, T cells each having T-cell receptor (TCR) can serve as an active ingredient of a therapeutic or prophylactic for cancer, and can be used as a TCR-T cell therapy drug. It is preferable for the TCR-T cell therapy drug to contain physiological saline, phosphate-buffered saline (PBS), medium, or the like in order to stably maintain the TCR-T cells. Examples of administration methods include intravenous administration, subcutaneous administration, and intradermal administration. If a therapeutic or prophylactic for cancer containing such TCR-T cells as an active ingredient is returned into the body of the patient, the cytotoxic action of TCR-T cells to cancer cells is promoted in the body of the patient, who has been affected by a cancer positive for any of the common cancer antigens of the present invention, to destroy cancer cells; thereby, the cancer can be treated.

The TCR-T cells of the present invention can exert cytotoxic activity targeting a complex of a partial peptide and HLA presented as antigen presentation for tumor cells. That is, the T-cell receptor (TCR) of each of the TCR-T cells of the present invention recognizes a complex of any of the partial peptides of the present invention and HLA.

The T cells with T-cell receptors (TCRs) (TCR-T cells) can be formulated directly or together with a carrier into a TCR-T cell drug.

The TCR-T cell therapy drug may be in a dosage form of either an oral agent or a parenteral agent. The TCR-T cell therapy drug is preferably in the form of a parenteral agent in typical cases. Examples of the parenteral agent include subcutaneous injections, intramuscular injections, intravenous injections, and suppositories.

In the case that the TCR-T cell therapy drug is in the form of an oral agent, TCR-T cell therapy obtained can be formulated together with a pharmaceutically acceptable diluent into the TCR-T cell therapy drug. Examples of the diluent include starch, mannitol, lactose, magnesium stearate, cellulose, polymerized amino acids, and albumin.

In the case that the TCR-T cell therapy drug is in the form of a parenteral agent, TCR-T cell therapy obtained can be formulated together with a pharmaceutically acceptable carrier into the TCR-T cell therapy drug. Examples of the carrier include water, sodium chloride, dextrose, ethanol, glycerol, and DMSO.

### <Companion diagnostic method>

The companion diagnostic method of the present invention comprises: step 1 of simultaneously measuring the presence or absence of expressions of three or more (i.e., three, four, or five) of GPC3, ROBO1, EPHB4, CLDN1, and LAT1 and cell membrane expression of HLA class I in a sample derived from a subject by multiple immunofluorescence staining; and step 2 of determining indication for cancer immunotherapy on the basis of the presence or absence of the expressions.

Preferably, in step 1, the presence or absence of expressions of one or more (i.e., one, two, three, four, or five) of AFP, TGFBI, SPARC, HSP105α, and FOXM1 may be further measured.

Preferably, in step 1, the presence or absence of expressions of all of GPC3, ROBO1, EPHB4, CLDN1, and LAT1 may be measured.

Preferably, in step 1, the presence or absence of expressions of all of GPC3, ROBO1, EPHB4, CLDN1, LAT1, AFP, TGFBI, SPARC, HSP105α, and FOXM1 may be measured.

Examples of the sample derived from a subject can include, but are not limited to, the liver, large intestine, oropharynx, esophagus, uterus, kidney, lung, breast, tongue, intrahepatic bile duct, nerve, and blood.

The presence or absence of expressions of the common cancer antigens of the present invention and cell membrane expression of HLA class I are simultaneously measured by multiple immunofluorescence staining. The multiple immunofluorescence staining may be either by a direct method or an indirect method. In the direct method, multiple primary antibodies directly fluorescence-labeled are used, and no labeled secondary antibody is used. The direct method can avoid cross-reaction by a secondary antibody, and the system is simple and the operation time is short. In the indirect method, multiple primary antibodies are used, and labeled secondary antibodies for the primary antibodies are used. The indirect method has an advantage that the indirect method allows detection with higher sensitivity than the direct method gives, for example, through the use of an amplification method in combination.

Fluorescent dyes can be selected for use in the multiple immunofluorescence staining in view of fluorescence spectra for excitation wavelengths and fluorescence wavelengths.

In the companion diagnostic method of the present invention, the presence or absence of or the degree of amelioration after administering a therapeutic to a patient with cancer for treatment of the cancer can be tested or diagnosed. Moreover, the companion diagnostic method of the present invention can be used for selection of a patient to be treated to whom the cancer vaccine, TCR-T cell therapeutic, or CAR-T cell therapeutic of the present invention can be effectively applied, and prediction and determination of the therapeutic effect of the cancer vaccine, TCR-T cell therapeutic, or CAR-T cell therapeutic of the present invention.

### <Method for diagnosing risk of cancer onset>

The method of the present invention for diagnosing risk of cancer onset comprises analyzing expressions of three or more (i.e., three, four, or five) of GPC3, ROBO1, EPHB4, CLDN1, and LAT1 in cells in a blood sample derived from a patient.

Preferably, expressions of one or more (i.e., one, two, three, four, or five) of AFP, TGFBI, SPARC, HSP105α, and FOXM1 may be analyzed.

Preferably, expressions of all of GPC3, ROBO1, EPHB4, CLDN1, and LAT1 may be analyzed.

Preferably, expressions of all of GPC3, ROBO1, EPHB4, CLDN1, LAT1, AFP, TGFBI, SPARC, HSP105α, and FOXM1 may be analyzed.

In the method of the present invention for diagnosing risk of cancer onset, typically, blood is collected from a subject, and expressions of three or more (i.e., three, four, or five) of GPC3, ROBO1, EPHB4, CLDN1, and LAT1 in cells contained in the blood are analyzed. The presence or absence of or the degree of cancer morbidity can be detected, tested, or diagnosed by detecting or measuring the expression levels of the common cancer antigens described above.

### Examples

### Example 1: Expression analysis for common cancer antigens by immunohistochemical analysis, and development of companion diagnosis method with multiple immunofluorescence staining system

### <Method>

From formalin-fixed paraffin blocks of various human cancer tissues, 4-um paraffin sections were prepared. For microarray slides of normal tissues, commercially available ones (BioChain Institute, Inc.) were used. Deparaffinization was performed with xylene and ethanol, and the endogenous peroxidase was inactivated with 0.3% H₂O₂/methanol. For antigen activation, heat treatment was performed in Target Retrieval Solution, pH 9 (Agilent Technologies) or AR6 (Akoya Biosciences, Inc.) inactivation buffer with microwaves or an autoclave. After blocking with porcine serum, primary antibodies for the antigens were reacted. GPC3 (BIOMOSICS, 300-fold diluted), AFP (Agikent, Ready to Use), ROBO1 (Proteintech Group, Inc., 300-fold diluted), EPHB4 (Cell Signaling Technology, Inc., 300-fold diluted), CLDN1 (Cell Signaling Technology, Inc., 300-fold diluted), FOXM1 (abcam plc., 150-fold diluted), HSP105α (abcam plc., 200-fold diluted), SPARC (Santa Cruz Biotechnology, Inc., 250-fold diluted), TGFBI (abcam plc., 100-fold diluted), HLA-ABC (Hokudo Co., Ltd., 200-fold diluted), LAT1 (abcam plc., 200-fold diluted). For secondary antibodies, polymer reagents (EnVision+ System- HRP Labelled Polymer Anti-Rabbit or EnVision+ System-HRP Labelled Polymer Anti-Mouse) were used. For coloring, a Liquid DAB+ Substrate Chromogen System was used. Counterstaining with hematoxylin was followed by dehydration and clearing treatment with ethanol and xylene. Virtual slides were produced from the preparations with a NanoZoomer (Hamamatsu Photonics K.K.).

In multiple fluorescence immunohistochemical staining, deparaffinization was performed with xylene and ethanol, and the endogenous peroxidase was inactivated with 0.3% H₂O₂/methanol. For antigen activation, heat treatment was performed in Target Retrieval Solution, pH 9 (Agilent Technologies) or AR6 (Akoya Biosciences, Inc.) inactivation buffer with microwaves. After blocking with porcine serum, primary antibodies for the antigens were reacted. GPC3 (BIOMOSICS, 600-fold diluted), ROBO1 (Proteintech Group, Inc., 600-fold diluted), EPHB4 (Cell Signaling Technology, Inc., 300-fold diluted), CLDN1 (Cell Signaling Technology, Inc., 1200-fold diluted), HLA-ABC (Hokudo Co., Ltd., 600-fold diluted), LAT1 (abcam plc., 800-fold diluted). For secondary antibodies, polymer reagents (EnVision+ System- HRP Labelled Polymer Anti-Rabbit or EnVision+ System- HRP Labelled Polymer Anti-Mouse) were used. As fluorescence dyes, Opal 520, 540, 570, 620, 650, and 690 (Akoya Biosciences, Inc.) were used with 100-fold dilution. After nuclear staining with DAPI, mounting was performed with a water-soluble mounting medium, and the preparations were observed with a Vectra 3 (PerkinElmer, Inc.).

### <Results>

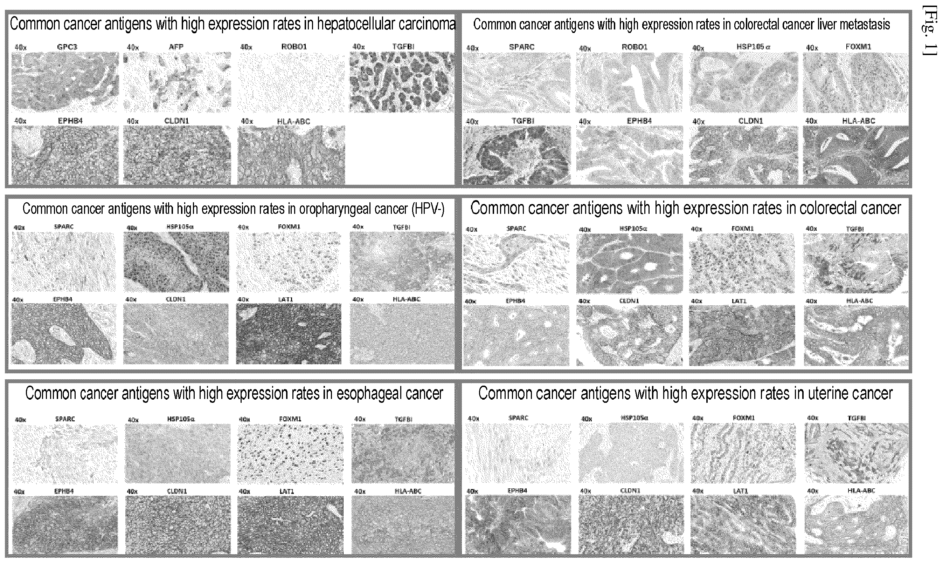

Fig. 1 shows results of immunohistochemical staining of paraffin-embedded tissue sections to confirm expressions of ten common cancer antigens in hepatocellular carcinoma and other cancers. GPC3, AFP, ROBO1, TGFBI, EPHB4, and CLDN1 were found to be expressed to moderate to high degree in cancer parts of hepatocellular carcinoma, and negative or expressed to low degree in non-cancer parts. HLA-Class I was found to be expressed on cell membranes in most cases, indicating the superiority of the common cancer antigens expressed in hepatocellular carcinoma as targets for TCR-T, CAR-T, and cancer vaccine therapy. Similarly, SPARC, HSP105α, FOXM1, TGFBI, EPHB4, CLDN1, and LAT1 were found to be expressed to moderate to high degree in colorectal cancer liver metastasis, oropharyngeal cancer (HPV-), colorectal cancer, esophageal cancer, and uterine cancer.

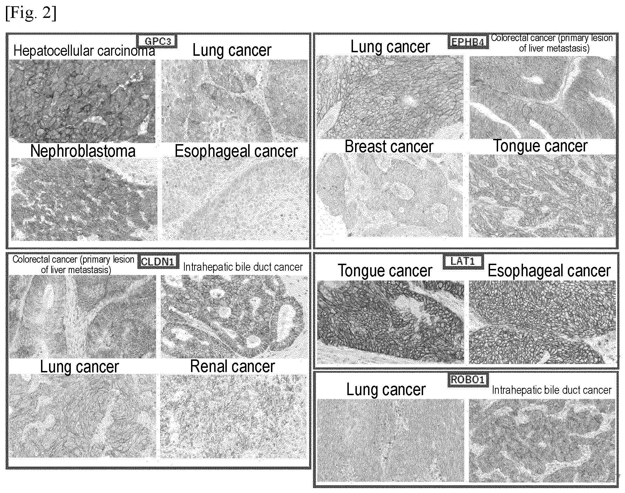

Fig. 2 shows expressions of the cell membrane-expressed common cancer antigens GPC3, EPHB4, CLDN1, LAT1, and ROBO1 in different cancer types. GPC3 was found to be co-expressed in hepatocellular carcinoma and nephroblastoma, which is childhood cancer, and found to be expressed to low to moderate degree in squamous cell cancer such as lung cancer and esophageal cancer. EPHB4, CLDN1, and ROBO1 were found to be expressed to moderate to high degree in lung cancer, colorectal cancer, breast cancer, tongue cancer, intrahepatic bile duct cancer, renal cancer, and esophageal cancer, and LAT1 tended to be highly expressed in squamous cell cancer.

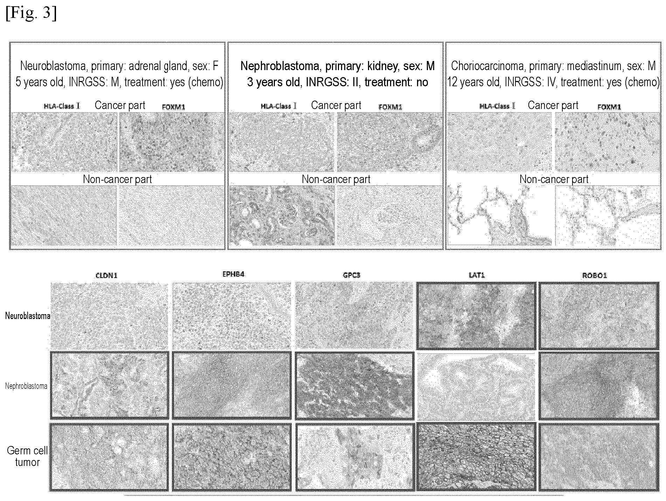

Fig. 3 shows expressions of common cancer antigens and expression of HLA-Class I in neuroblastoma, nephroblastoma, and choriocarcinoma, which are each childhood cancer. The common cancer antigens including nucleus-expressed FOXM1 were found to be expressed to moderate to high degree, but HLA-Class I was not found to be expressed on cell membranes. On the other hand, any of the cell membrane-expressed common cancer antigens GPC3, EPHB4, CLDN1, LAT1, and ROBO1 was expressed to moderate to high degree on the cell membranes of cancer cells of neuroblastoma, nephroblastoma, and choriocarcinoma; thus, the common cancer antigens were revealed to be applicable to CAR-T therapy with the utilization of their HLA-Class I-unrestricted character.

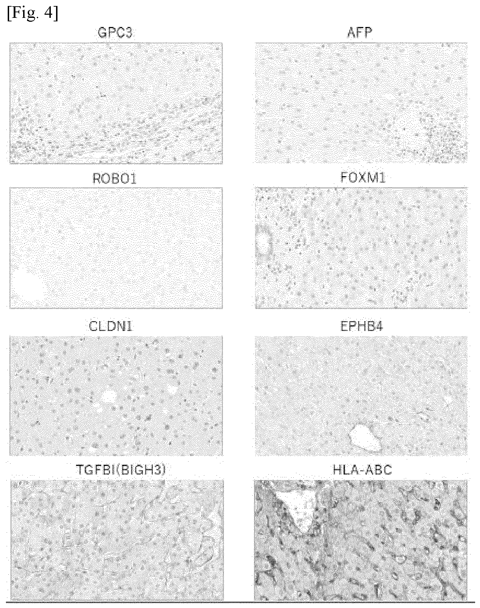

Fig. 4 shows results of confirming expression of each common cancer antigen in non-cancer parts of a hepatocellular carcinoma human clinical specimen. The results for GPC3, AFP, ROBO1, FOXM1, and CLDN1 were negative, and EPHB4 and TGFBI were found to be expressed to low degree in sinusoids of hepatic parenchyma. HLA-Class I are found to be expressed on the cell membranes of hepatic parenchymal cells.

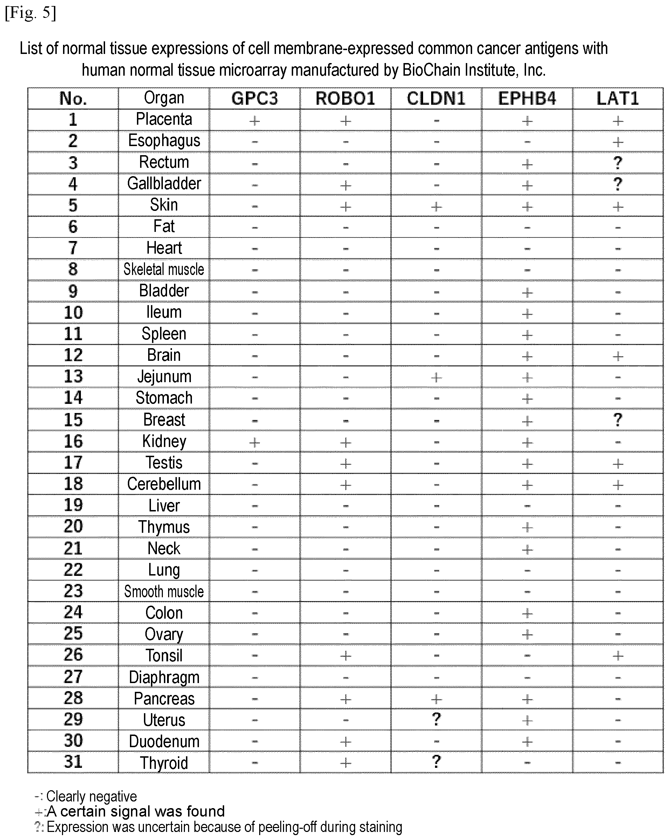

Fig. 5 is a table showing expressions of the cell membrane-expressed common cancer antigens GPC3, EPHB4, CLDN1, LAT1, and ROBO1 in 31 normal tissues with use of a human normal tissue microarray. Although signals from EPHB4 and ROBO1 were detected to low degree in the tissues, no clear cell membrane expression was found for them. GPC3, CLDN1, and LAT1 are found to be clearly expressed only in some of the tissues.

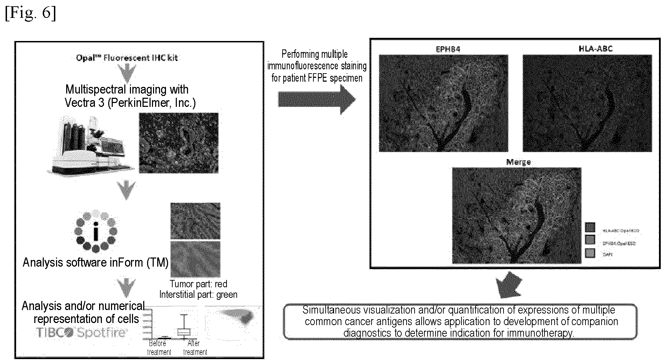

Fig. 6 shows the concept of indication diagnosis system development using multiple immunofluorescence staining to determine which common cancer antigen is effective when being targeted in immunotherapy such as TCR-T, CAR-T, and a cancer vaccine.

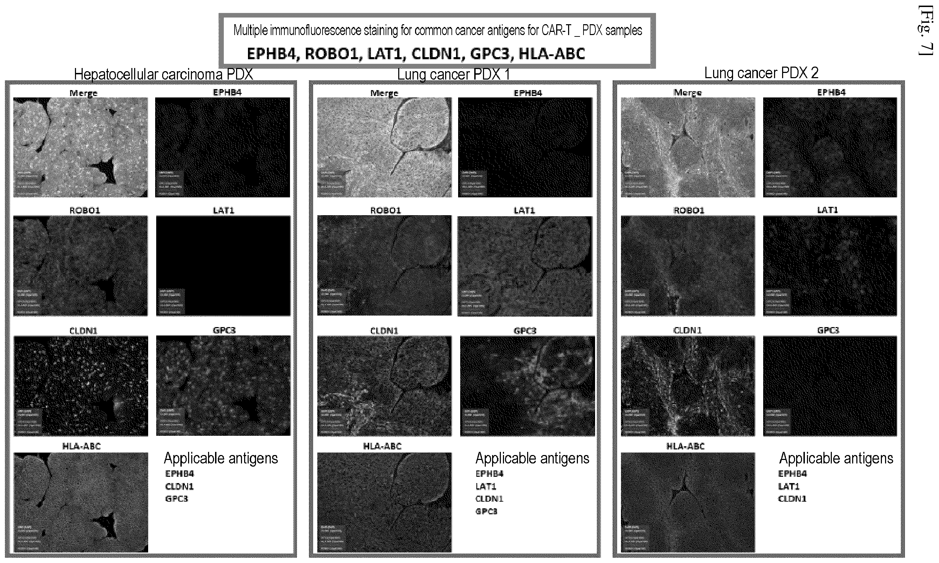

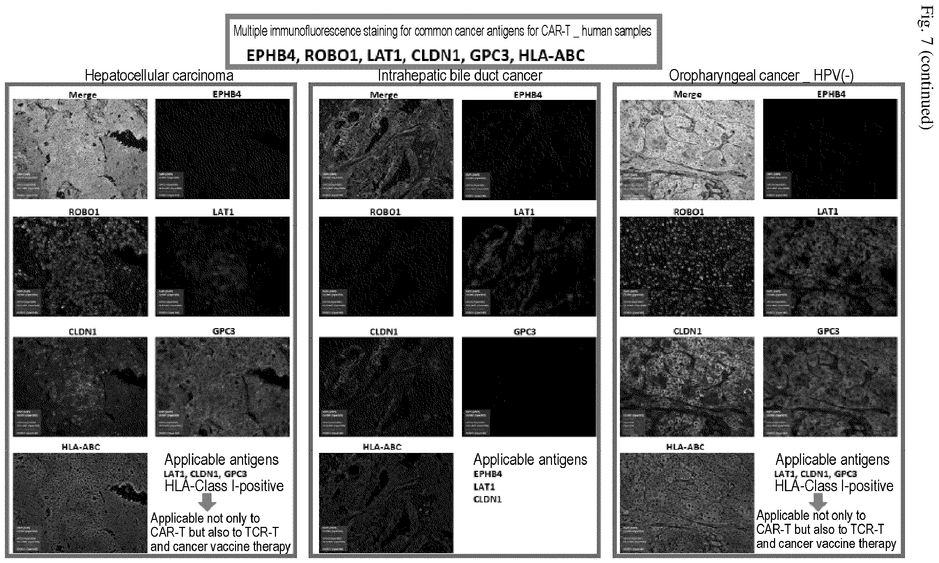

Fig. 7 shows multiple immunofluorescence staining performed for the cell membrane-expressed common cancer antigens GPC3, EPHB4, CLDN1, LAT1, and ROBO1 and HLA-Class I with samples derived from hepatocellular carcinoma and lung cancer patients as PDXs and samples derived from hepatocellular carcinoma, intrahepatic bile duct cancer, and oropharyngeal cancer (HPV-) patients as human clinical specimens. In the hepatocellular carcinoma PDX, GPC3 was highly expressed, followed by CLDN1 and EPHB4, which were also found to be expressed. In the lung cancer PDX, GPC3, EPHB4, CLDN1, and LAT1 were expressed to moderate to high degree in a combination of them. From the human clinical specimens, not only GPC3, CLDN1, and LAT1 but also HLA-Class I was found to be expressed in hepatocellular carcinoma patients and oropharyngeal cancer (HPV-) patients, indicating the possibility that they are applicable not only to CAR-T but also to TCR-T and cancer vaccines. It was found that LAT1 is the most applicable antigen and EPHB4 and CLDN1 are the second most applicable antigens for intrahepatic bile duct cancer patients.

### Example 2: Gene expressions for common cancer antigens and prediction of HLA-binding peptides

### <Method>

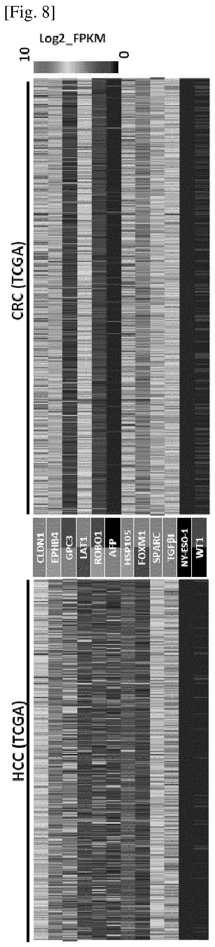

Fig. 8 shows gene expressions for different cancer antigens in hepatocellular carcinoma and primary colorectal cancer. Gene expressions for different cancer antigens in 371 hepatocellular carcinoma cases (left) and 478 primary colorectal cancer cases (right) registered in TCGA (https://www.cancer.gov/ccg/research/genome-sequencing/tcga) were represented as heat maps. The expression levels of genes were normalized to log2 (FPKM+1) and evaluated. The names of cancer antigens with high frequency or high expression were colored red, the names of cancer antigens with moderate frequency or moderate expression were colored green, the names of cancer antigens with low frequency or low expression were colored blue, and the names of cancer antigens for which no expression was found in most cases were colored black.

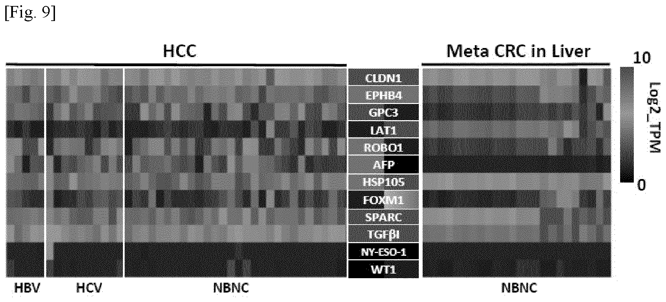

Fig. 9 shows gene expressions for different cancer antigens in hepatocellular carcinoma and primary colorectal cancer for which antigen prediction was performed. Gene expressions for different cancer antigens in 43 hepatocellular carcinoma cases and 24 colorectal cancer-derived metastatic liver cancer cases each involving surgical excision in National Cancer Center Hospital East at any time from 2016 to 2023 were represented as heat maps. The expression levels of genes were normalized to log2 (TPM+1) and evaluated. The hepatocellular carcinoma cases were classified into HBV-positive (5 cases), HCV-positive (10 cases), and nonviral (28 cases) cases, which were presented.

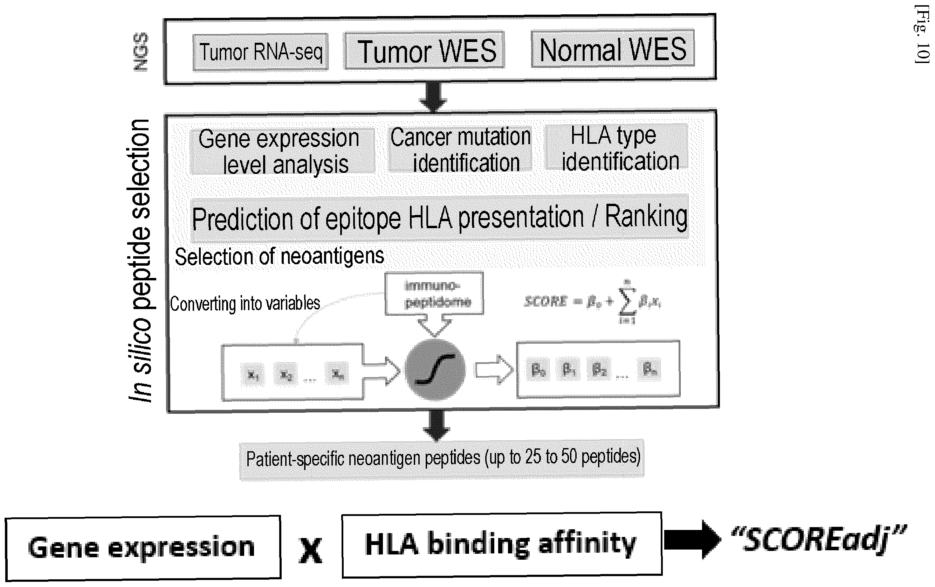

Fig. 10 shows a prediction pipeline for different cancer antigen-derived epitope peptides. Tumor and normal liver tissues were collected from surgically excised specimens in registered cases, DNA and RNA were extracted, and neoantigens and common cancer antigens that were highly expressed in cancer were selected with reference to total exon sequences and RNA sequences. The HLA haplotype in each case was determined through total exon sequence analysis, and peptides having high avidity to MHC Class I (HLA-A, -B, -C) were predicted. The avidity to MHC and the expression levels of the antigens were additionally considered as factors to construct a predictor, and the antigenicity of each cancer antigen-derived peptide was calculated as SCOREadj.

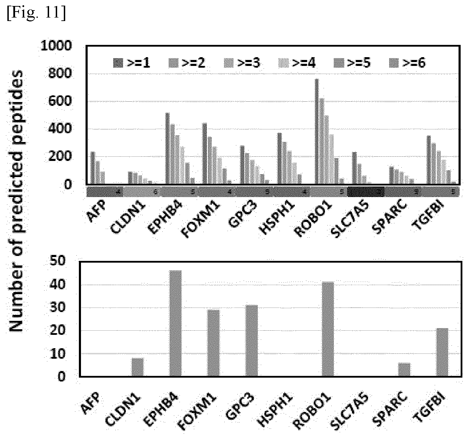

Fig. 11 shows the distribution of SCOREadj of different cancer antigen-derived peptides in hepatocellular carcinoma. Cancer antigen-derived peptides predicted in one case of hepatocellular carcinoma were classified by SCOREadj (1 to 2: light blue, 2 to 3: orange, 3 to 4: gray, 4 to 5: yellow, 5 to 6: blue, and 6 or more: green), and the numbers were shown (top, bar graph). Expressions of the cancer antigen genes were represented with red (Log2_TPM = 10) to blue (Log2_TPM = 1). Only peptides with SCOREadj of 6 or more were extracted from each cancer antigen-derived peptide, and shown in the bottom bar graph.

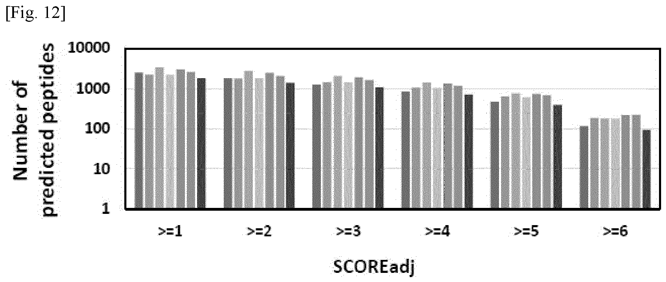

Fig. 12 shows the total numbers of cancer antigen peptides predicted in seven hepatocellular carcinoma cases. The same predictor was used for seven hepatocellular carcinoma cases to predict the antigenicity of each cancer antigen-derived peptide. The total numbers of predicted peptides (the total number of peptides predicted to be cancer antigen-derived and bind to HLA-A, -B, and -C) were classified by SCOREadj, and shown for every case in a bar graph.

### <Results>

Gene expressions for 12 common cancer antigens in hepatocellular carcinoma and colorectal cancer were checked with use of metadata registered in TCGA (hepatocellular carcinoma: TCGA-LIHC, colorectal cancer: TCGA-COAD). The results found CLDN1, SPARC, and TGFβI to be expressed in almost all of the cases. LAT1, FOXM1, EPHB4, and HSP105 were found to be expressed in most of the colorectal cancer cases; although they were expressed at high frequencies in hepatocellular carcinoma, the expressions were lower than those in colorectal cancer. Cases with high expression of GPC3 or AFP were found for hepatocellular carcinoma, but expressions of these cancer antigens were at low levels or not found in colorectal cancer. There were several hepatocellular carcinoma cases with low expression of ROBO1, but the expression was low in most of the colorectal cancer cases. NY-ESO-1 and WT1 were found to be hardly expressed in hepatocellular carcinoma and colorectal cancer. Subsequently, for each of the hepatocellular carcinoma and metastatic colorectal cancer cases collected by us, expressions of neoantigens derived from gene mutation and those common cancer antigens were evaluated through whole exon analysis and RNA sequence analysis with DNA or RNA extracted from tumor and normal tissues to examine whether they were available as candidates for cancer vaccines. As with the case of the analysis with metadata from TCGA, the results showed that CLDN1, GPC3, SPARC, and TGFβI were expressed at high frequencies in hepatocellular carcinoma. For AFP, again, rare cases with high expression thereof were found. In metastatic colorectal cancer, CLDN1, LAT1, HSP105, SPARC, and TGFβI were expressed at high frequencies, but almost no expression was found for cancer antigens specific to hepatocellular carcinoma such as ROBO1, GPC3, and AFP; this tendency was the same as in primary colorectal cancer. On the other hand, expressions of EPHB4 and FOXM1 were only found in some cases for metastatic colorectal cancer, and this suggested the possible difference in expressions of cancer antigens from primary colorectal cancer. In conclusion, those ten cancer antigens with NY-ESO-1 and WT1 excluded are expected to be able to serve as promising targets for revolutionary immunotherapy to overcome the diversity (heterogeneity) of solid cancers in cases of hepatocellular carcinoma or colorectal cancer with metastatic lesions.

Subsequently, we examined to what degree cancer antigen-derived epitopes to serve as targets for CD8+ T cells to damage and eliminate cancer could be present in each case in actually targeting those cancer antigens. Through collaboration with BrightPath Biotherapeutics Co., Ltd., we have previously developed a predictor by combining existing predictions of HLA binding such as netMHCPan (https://services.healthtech.dtu.dk/services/NetMHCpan-4.1/) and MHCFlurry (https://github.com/openvax/mhcflurry) and a prediction algorithm for proteasome cleavage of proteins, NetChop (https://services.healthtech.dtu.dk/services/NetChop-3.1/), with additional consideration of information on expressions of cancer antigens. This predictor optimizes those multiple factors by using a linear prediction function to give SCOREadj for evaluation. We have validated the efficacy of our predictor by using 278 peptides derived from neoantigens predicted from 46 hepatocellular carcinoma or metastatic colorectal cancer cases, and reported the results on an international journal (Cancer Sci. 2022 Apr; 113(4): 1113-1124.). Since gene expression information for each case is available in the series of predictions, expressions not only of neoantigens but also of common cancer antigens can be simultaneously evaluated. In view of this, for cancer antigens that were actually highly expressed, we examined how much peptides predicted to have high antigenicity were found in each case. We made a prediction for one hepatocellular carcinoma case, and succeeded in extracting a group of peptides predicted to be bound by HLA for every antigen. When peptides with high SCOREadj were extracted, again as expected, about 30 to 40 peptides that could be expected to be epitopes were successfully predicted for each of cancer antigens including GPC3, ROBO1, FOXM1, and EPHB4. From a result that peptides that exhibited strong immunogenicity accounted for about 50% of the predicted ones in the examination targeting neoantigens, it was found to be possible to select common cancer antigen-derived peptides in an enough number for using as candidates for cancer vaccines. In addition, as can be seen from a case with high expression but with a small number of predicted peptides such as the case of SPARC, and the contrary cases of ROBO1 and CLDN1 with comparable expressions but with a large number of predicted peptides for the former and rather a small number of predicted peptides for the latter, it was suggested that the number of predicted peptides with high SCOREadj does not depend only on the expression level of the antigen, and largely affected by the binding affinity with HLA. Similarly, six hepatocellular carcinoma cases were examined, and the results led to a prediction that about 100 common cancer antigen-derived peptides were useful as candidates for cancer peptide vaccines in every case.

Because not only of the diversity of expression levels and expression patterns of cancer antigens, but also of the difference in HLA haplotype among different cases, it is desirable to develop a cocktail vaccine targeting multiple cancer antigens as a personalized cancer vaccine that is prescribed as an optimum combination of peptides predicted on a patient-by-patient basis. While personalized medicine often encounters problems on the cost and time required for preparation and test, prediction of peptides for which effects derived from multiple common cancer antigens plus implementation of a peptide library for which immunogenicity derived from those ten common cancer antigens has been confirmed and achievement of providing it as an off-the-shelf product enables a more effective cocktail peptide vaccine to be provided at low cost in a short period of time.

### Example 3: Identification of common cancer antigen-derived CTL-inducing peptides, and development of peptide vaccine and peptide-pulsed dendritic cell vaccine

### <Method>

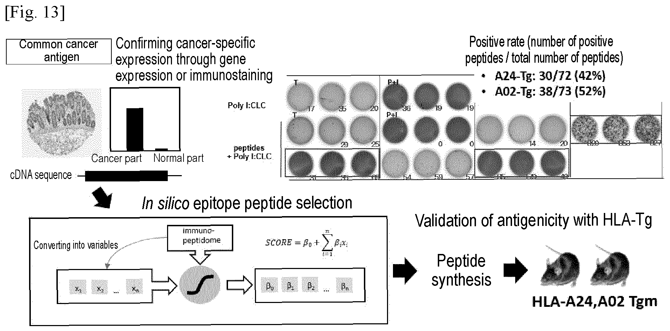

Fig. 13 shows 72 HLA-A*24:02-restricted epitope peptides and 73 HLA-A*02:01-restricted epitope peptides predicted and synthesized from the amino acid sequences of the ten common cancer antigens by using an algorithm established in collaboration with BrightPath Biotherapeutics Co., Ltd. Whole exon analysis and RNA sequence analysis with a next-generation sequencer were performed, and gene mutations given and expression data thereon were used for predicting peptides capable of binding to these HLAs *in silico.* The predicted antigen peptides were evaluated on the capability of inducing CTLs by using transgenic mice with an HLA gene introduced therein. Each peptide was dissolved in dimethyl sulfoxide (DMSO, FUJIFILM Wako Pure Chemical Corporation) at 10 mg/ml, and HLA transgenic mice were immunized with the resultant together with an adjuvant every week, three times in total. One week after that, the spleens were extracted, and the productions of IFN-γ were evaluated through IFN-γ ELISpot assay.

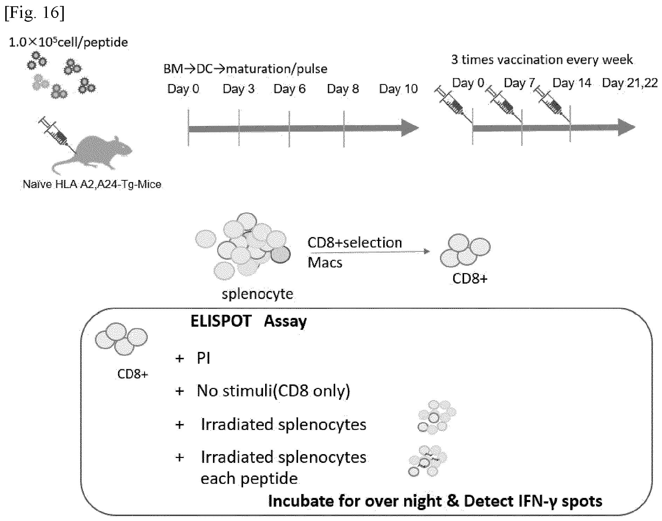

Fig. 16 shows evaluation performed on whether antigen-specific T cells would be introduced through vaccination of transgenic mice with dendritic cells (DCs) sensitized with a common cancer antigen-derived short peptide found to induce CTLs through the use of transgenic mice with different HLA genes introduced therein. Each peptide was dissolved in dimethyl sulfoxide (DMSO, FUJIFILM Wako Pure Chemical Corporation) at 10 mg/ml, 1 × 10^5 peptide-sensitized DCs were prepared for each peptide, the liquid volumes were adjusted with PBS, and HLA-A*02:01 Tgm or HLA-A*24:02 Tgm was immunized therewith at a liquid volume of 100 µl by intradermally applying a 27G injection needle/1-ml syringe (Terumo Corporation) to the base of the tail. Immunization was performed every week three times in total, the spleens were extracted 1 week after the third vaccination, and the productions of IFN-γ were evaluated through IFN-γ ELISpot assay.

The DCs used were those collected from the femora of HLA-A*02:01 Tgm or HLA-A*24:02 Tgm. In Final medium (10% FBS/1% L-glutamine/streptomycin-penicillin/NEAA/10 mM HEPES/Sodium pyruvate/2-ME (Gibco)) + 2-ME (1000-fold diluted) + 20 ng/ml rmGM-CSF (PeproTech, Inc.), 2 × 10^6 DCs were suspended, and seeded on a low-adhesion 10-cm petri dish (AS ONE Corporation) to begin culture. On day 3 after the beginning of culture, 10 ml Final medium + 20 ng/ml rmGM-CSF was added. On day 6, a half of the medium was taken in a conical tube, the supernatant was removed through centrifugation at 1,500 rpm for 5 min, and the pellet was suspended in 10 ml of fresh Final medium and returned in the same dish. On day 8, half-volume medium exchange was performed in the same manner as on day 6. On day 9, 10 mL of the culture medium was collected in a conical tube. With gentle pipetting, non-adherent cells and loosely adhering cells were recovered and collected in the same conical tube. After centrifugation at 1500 rpm for 5 min, the supernatant was discarded. The resultant was suspended in 10 mL of serum-free RPMI, and centrifuged at 1500 rpm for 5 min. This was repeated twice (for removing FBS), the supernatant was discarded, and the resultant was suspended in 10 mL of serum-free RPMI, and cultured overnight with addition of maturation cytokine (20 ng/mL TNFα, 1 µg/mL PGE2, 20 ng/mL IL-4, 0.1 µM Zometa, 10 µg/mL antigen peptide or no peptide for negative control). On day 10, the DCs sensitized with each peptide were washed twice with PBS and suspended at 1 × 10^5/100 µl, and the resultants were administered to mice.

In ELISpot assay, CD8-positive T cells were isolated from splenocytes of immunized mice by means of an MACS, and suspended at 1 × 10^5 cells/well, and the resultant was used as effector cells. Splenocytes of unimmunized mice were subjected to radiation (100 kV, 10 mA, 100 Gy), and suspended at a concentration of 2 × 10^6 cells/well in Final medium supplemented with any one of the peptides at a concentration of 20 ug/ml, and the resultant was used as target cells.

### <Results>

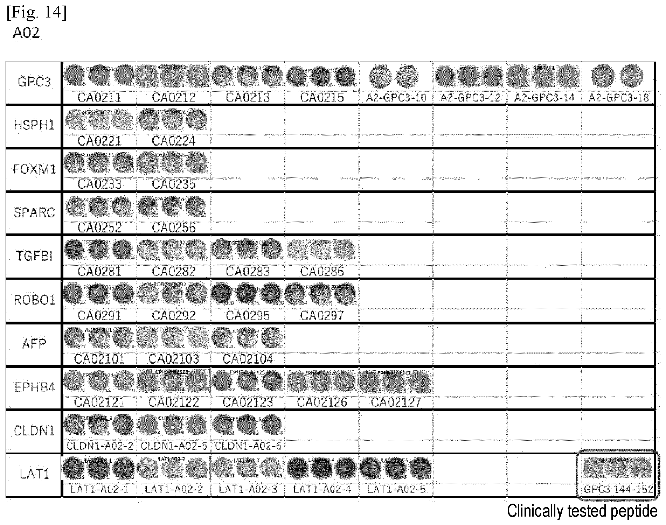

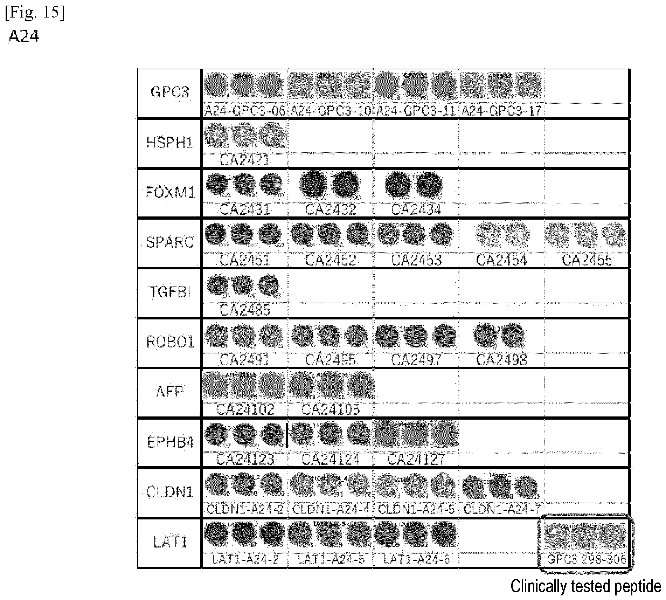

Fig. 14, Fig. 15, and Table 1 show identification of multiple peptides each capable of inducing CD8-positive killer T cells (CTLs) reactive with the peptide in splenocytes of mice systemically expressing human HLA-A*24:02 or HLA-A*02:01 in their cells by screening, wherein a peptide vaccine of peptides predicted to be presented by HLA-A*24:02 or HLA-A*02:01 and synthesized from the amino acid sequences of the ten common cancer antigen full-length proteins was administered once per week, three times in total. The rates of inducing CTLs reactive with those peptides were 30 peptides in the 72 HLA-A*24:02-restricted common cancer antigen short peptides, and 38 peptides in the 73 HLA-A*02:01-restricted common cancer antigen short peptides; 68 peptides in the 145 peptides in total. These peptides are superior in CTL inducibility to peptides of GPC3 peptide vaccines used in clinical trials, and applicable to prevention of recurrence of and prevention of various cancers in the future.

**[Table 1]**

| Restricte d HLA type | Gene name | Peptide-ID | Sequence | Lengt h (mer) | CTL inductio n | Long peptid e reactio n | Sequence (three-letter code) | long peptide sequence |
|---|---|---|---|---|---|---|---|---|
| HLA-A*02:01 | GPC3 | CA02 11 | FLAELAYDL | 9 | Yes | Yes | Phe-Leu-Ala-Glu-Leu-Ala- Tyr-Asp-Leu (SEQ ID NO: 1) | VKNQLR[FLAELAYDL]D VDDAP (SEQ ID NO: 69) |
| HLA-A*02:01 | GPC3 | CA02 12 | ELFDSLFPV | 9 | Yes | - | Glu-Leu-Phe-Asp-Ser- Leu-Phe-Pro-Val (SEQ ID NO: 2) | |
| HLA-A*02:01 | GPC3 | CA02 13 | TIHDSIQYV | 9 | Yes | Yes | Thr-Ile-His-Asp-Ser-Ile- Gln-Tyr-Val (SEQ ID NO: 3) | LLGLFS[TIHDSIQYV]QK NAG (SEQ ID NO: 70) |
| HLA-A*02:01 | GPC3 | CA02 15 | VLLGLFSTI | 9 | Yes | Yes | Val-Leu-Leu-Gly-Leu-Phe- Ser-Thr-Ile (SEQ ID NO: 4) | DMEN[VLLGLFSTII]HDSI QY (SEQ ID NO: 71) |
| HLA-A*02:01 | GPC3 | A02-GPC3- | RIYDMENVLL | 10 | Yes | - | Arg-Ile-Tyr-Asp-Met-Glu- Asn-Val-Leu-Leu (SEQ ID | |
| | | 10 | | | | | NO: 5) | |
| HLA-A*02:01 | GPC3 | A02-GPC3-12 | FLIIQNAAV | 9 | Yes | No | Phe-Leu-Ile-Ile-Gln-Asn- Ala-Ala-Val (SEQ ID NO: 6) | |
| HLA-A*02:01 | GPC3 | A02-GPC3-14 | STIHDSIQYV | 10 | Yes | Yes | Ser-Thr-Ile-His-Asp-Ser- Ile-Gln-Tyr-Val (SEQ ID NO: 7) | LLGLF[STIHDSIQYV]QK NAG (SEQ ID NO: 72) |
| HLA-A*02:01 | GPC3 | A02-GPC3-18 | FFTDVSLYI | 9 | Yes | - | Phe-Phe-Thr-Asp-Val-Ser- Leu-Tyr-Ile (SEQ ID NO: 8) | |
| HLA-A*02:01 | HSPH 1 | CA02 21 | FLRRGPFEL | 9 | Yes | - | [?] Phe-Leu-Arg-Arg-Gly-Pro- Phe-Glu-Leu (SEQ ID NO: 9) | |
| HLA-A*02:01 | HSPH 1 | CA02 24 | LLQKIEVPL | 9 | Yes | - | 9) Leu-Leu-Gln-Lys-Ile-Glu- Val-Pro-Leu (SEQ ID NO: 10) | |
| HLA-A*02:01 | FOXM 1 | CA0233 | KMKPLLPRV | 9 | Yes | - | 10) Lys-Met-Lys-Pro-Leu-Leu- Pro-Arg-Val (SEQ ID NO: 11) | |
| HLA-A*02:01 | FOXM 1 | CA0235 | TMNDSLSKI | 9 | Yes | No | 11) Thr-Met-Asn-Asp-Ser- Leu-Ser-Lys-Ile (SEQ ID NO: 12) | |
| HLA-A*02:01 | SPAR C | CA02 52 | RLEAGDHPV | 9 | Yes | No | Arg-Leu-Glu-Ala-Gly-Asp- His-Pro-Val (SEQ ID NO: 13) | |
| HLA-A*02:01 | SPAR C | CA02 56 | FLLCLAGRAL | 10 | Yes | No | Phe-Leu-Leu-Cys-Leu- Ala-Gly-Arg-Ala-Leu (SEQ ID NO: 14) | |
| HLA-A*02:01 | TGFBI | CA02 81 | VLDSLVSNV | 9 | Yes | - | ID NO: 14) Val-Leu-Asp-Ser-Leu-Val- Ser-Asn-Val (SEQ ID NO: 15) | |
| HLA-A*02:01 | TGFBI | CA02 82 | HLIDKVISTI | 10 | Yes | - | His-Leu-Ile-Asp-Lys-Val- Ile-Ser-Thr-Ile (SEQ ID NO: 16) | |
| HLA-A*02:01 | TGFBI | CA02 83 | SLVSNVNIEL | 10 | Yes | Yes | Ser-Leu-Val-Ser-Asn-Val- Asn-Ile-Glu-Leu (SEQ ID NO: 17) | EVLD[SLVSNVNIEL]LNA L (SEQ ID NO: 73) |
| HLA-A*02:01 | TGFBI | CA02 86 | MLVAAIQSA | 9 | Yes | No | Met-Leu-Val-Ala-Ala-Ile- Gln-Ser-Ala (SEQ ID NO: 18) | |
| HLA-A*02:01 | ROBO 1 | CA02 91 | YIIEAFSHA | 9 | Yes | - | Tyr-Ile-Ile-Glu-Ala-Phe- Ser-His-Ala (SEQ ID NO: 19) | |
| HLA-A*02:01 | ROBO 1 | CA02 92 | TVDGSTFSV | 9 | Yes | - | Thr-Val-Asp-Gly-Ser-Thr- Phe-Ser-Val (SEQ ID NO: 20) | |
| HLA-A*02:01 | ROBO 1 | CA02 95 | ILMVFSIWL | 9 | Yes | Yes | Ile-Leu-Met-Val-Phe-Ser- Ile-Trp-Leu (SEQ ID NO: 21) | I[ILMVFSIWL]YRHRK (SEQ ID NO: 74) |
| HLA-A*02:01 | ROBO 1 | CA02 97 | VIADRPPPV | 9 | Yes | Yes | Val-Ile-Ala-Asp-Arg-Pro- Pro-Pro-Val (SEQ ID NO: 22) | YLEVTD[VIADRPPPV]IR QGP (SEQ ID NO: 75) |
| HLA-A*02:01 | AFP | CA02 101 | FIFHKDLCQA | 10 | Yes | No | Phe-Ile-Phe-His-Lys-Asp- Leu-Cys-Gln-Ala (SEQ ID NO: 23) | |
| HLA-A*02:01 | AFP | CA02 103 | FIFHKDLCQ | 9 | Yes | No | NO: 23) Phe-Ile-Phe-His-Lys-Asp- Leu-Cys-Gln (SEQ ID NO: 24) | |
| HLA-A*02:01 | AFP | CA02 104 | DFSGLLEKC | 9 | Yes | No | Asp-Phe-Ser-Gly-Leu- Leu-Glu-Lys-Cys (SEQ ID NO: 25) | |
| HLA- | EPHB | CA02 | GLDEEQHSV | 9 | Yes | Yes | Gly-Leu-Asp-Glu-Glu-Gln- | LS[GLDEEQHSV]RTYE |
| A*02:01 | 4 | 121 | | | | | His-Ser-Val (SEQ ID NO: 26) | (SEQ ID NO: 76) |
| HLA-A*02:01 | EPHB 4 | CA02 122 | YLIGHGTKV | 9 | Yes | - | Tyr-Leu-Ile-Gly-His-Gly- Thr-Lys-Val (SEQ ID NO: 27) | |
| HLA-A*02:01 | EPHB 4 | CA02 123 | RLNDGQFTV | 9 | Yes | Yes | Arg-Leu-Asn-Asp-Gly-Gln- Phe-Thr-Val (SEQ ID NO: 28) | ALDSFL[RLNDGQFTV]I Q (SEQ ID NO: 77) |
| HLA-A*02:01 | EPHB 4 | CA02 126 | GQFTVIQLV | 9 | Yes | - | Gly-Gln-Phe-Thr-Val-Ile- Gln-Leu-Val (SEQ ID NO: 29) | |
| HLA-A*02:01 | EPHB 4 | CA02 127 | FTYEDPNEA V | 10 | Yes | - | Phe-Thr-Tyr-Glu-Asp-Pro- Asn-Glu-Ala-Val (SEQ ID NO: 30) | |
| HLA-A*02:01 | CLDN 1 | CLDN1-A02-2 | ALFTGWAAA | 9 | Yes | No | Ala-Leu-Phe-Thr-Gly-Trp- Ala-Ala-Ala (SEQ ID NO: 31) | |
| HLA-A*02:01 | CLDN 1 | CLDN1-A02-5 | YSYAGDNIV | 9 | Yes | - | Tyr-Ser-Tyr-Ala-Gly-Asp- Asn-Ile-Val (SEQ ID NO: 32) | |
| HLA-A*02:01 | CLDN 1 | CLDN1-A02-6 | LQLLGFILA | 9 | Yes | - | Leu-Gln-Leu-Leu-Gly- Phe-Ile-Leu-Ala (SEQ ID NO: 33) | |
| HLA-A*02:01 | LAT1 | LAT1-A02-1 | QLLTPVPSL | 9 | Yes | No | Gln-Leu-Leu-Thr-Pro-Val- Pro-Ser-Leu (SEQ ID NO: 34) | |
| HLA-A*02:01 | LAT1 | LAT1-A02-2 | LLTPVPSLV | 9 | Yes | - | Leu-Leu-Thr-Pro-Val-Pro- Ser-Leu-Val (SEQ ID NO: 35) | |
| HLA-A*02:01 | LAT1 | LAT1-A02-3 | KLDVGNIVL | 9 | Yes | - | Lys-Leu-Asp-Val-Gly-Asn- Ile-Val-Leu (SEQ ID NO: 36) | |
| HLA-A*02:01 | LAT1 | LAT1-A02-4 | ILSMIHPQL | 9 | Yes | No | Ile-Leu-Ser-Met-Ile-His- Pro-Gln-Leu (SEQ ID NO: 37) | |
| HLA-A*02:01 | LAT1 | LAT1-A02-5 | YMLEVYGSL | 9 | Yes | No | Tyr-Met-Leu-Glu-Val-Tyr- Gly-Ser-Leu (SEQ ID NO: 38) | |
| HLA-A*24:02 | GPC3 | A24-GPC3-06 | KYWREYILSL | 10 | Yes | No | Lys-Tyr-Trp-Arg-Glu-Tyr- Ile-Leu-Ser-Leu (SEQ ID NO: 39) | |
| HLA-A*24:02 | GPC3 | A24-GPC3-10 | FFTDVSLYI | 9 | Yes | - | Phe-Phe-Thr-Asp-Val-Ser- Leu-Tyr-Ile (SEQ ID NO: 40) | |
| HLA-A*24:02 | GPC3 | A24-GPC3-11 | AYYPEDLFI | 9 | Yes | No | Ala-Tyr-Tyr-Pro-Glu-Asp- Leu-Phe-Ile (SEQ ID NO: 41) | |
| HLA-A*24:02 | GPC3 | A24-GPC3-17 | AFEFVGEFF | 9 | Yes | No | Ala-Phe-Glu-Phe-Val-Gly- Glu-Phe-Phe (SEQ ID NO: 42) | |
| HLA-A*24:02 | HSPH 1 | CA24 21 | HYAKIAADF | 9 | Yes | No | His-Tyr-Ala-Lys-Ile-Ala- Ala-Asp-Phe (SEQ ID NO: 43) | |
| HLA-A*24:02 | FOXM 1 | CA24 31 | RYLTLDQVF | 9 | Yes | No | Arg-Tyr-Leu-Thr-Leu-Asp- Gln-Val-Phe (SEQ ID NO: 44) | |
| HLA-A*24:02 | FOXM 1 | CA24 32 | IYTWIEDHF | 9 | Yes | No | Ile-Tyr-Thr-Trp-Ile-Glu- Asp-His-Phe (SEQ ID NO: 45) | |
| HLA-A*24:02 | FOXM 1 | CA24 34 | SYMAMIQFAI | 10 | Yes | No | Ser-Tyr-Met-Ala-Met-Ile- Gln-Phe-Ala-Ile (SEQ ID NO: 46) | |
| HLA-A*24:02 | SPAR C | CA24 51 | MYIFPVHWQ F | 10 | Yes | No | Met-Tyr-Ile-Phe-Pro-Val- His-Trp-Gln-Phe (SEQ ID NO: 47) | |
| HLA-A*24:02 | SPAR C | CA24 52 | YIFPVHWQF | 9 | Yes | No | Tyr-Ile-Phe-Pro-Val-His- Trp-Gln-Phe (SEQ ID NO: 48) | |
| HLA-A*24:02 | SPAR C | CA24 53 | TFDSSCHFF | 9 | Yes | No | Thr-Phe-Asp-Ser-Ser- Cys-His-Phe-Phe (SEQ ID NO: 49) | |
| HLA-A*24:02 | SPAR C | CA24 54 | DYIGPCKYI | 9 | Yes | - | Asp-Tyr-Ile-Gly-Pro-Cys- Lys-Tyr-Ile (SEQ ID NO: 50) | |
| HLA-A*24:02 | SPAR C | CA24 55 | HFFATKCTL | 9 | Yes | - | His-Phe-Phe-Ala-Thr-Lys- Cys-Thr-Leu (SEQ ID NO: 51) | |
| HLA-A*24:02 | TGFBI | CA24 85 | KYFTNCKQW | 9 | Yes | - | Lys-Tyr-Phe-Thr-Asn-Cys- Lys-Gln-Trp (SEQ ID NO: 52) | |
| HLA-A*24:02 | ROBO 1 | CA24 91 | AYLEVTDVI | 9 | Yes | - | Ala-Tyr-Leu-Glu-Val-Thr- Asp-Val-Ile (SEQ ID NO: 53) | |
| HLA-A*24:02 | ROBO 1 | CA24 95 | KYVCVGTNM | 9 | Yes | - | Lys-Tyr-Val-Cys-Val-Gly- Thr-Asn-Met (SEQ ID NO: 54) | |
| HLA-A*24:02 | ROBO 1 | CA24 97 | YYICQTLNV | 9 | Yes | No | Tyr-Tyr-Ile-Cys-Gln-Thr- Leu-Asn-Val (SEQ ID NO: 55) | |
| HLA-A*24:02 | ROBO 1 | CA24 98 | ILPYCRPTF | 9 | Yes | Yes | Ile-Leu-Pro-Tyr-Cys-Arg- Pro-Thr-Phe (SEQ ID NO: 56) | LIQED[ILPYCRPTF]PTS N (SEQ ID NO: 78) |
| HLA-A*24:02 | AFP | CA24 102 | DFSGLLEKC | 9 | Yes | No | Asp-Phe-Ser-Gly-Leu- Leu-Glu-Lys-Cys (SEQ ID NO: 57) | |
| HLA-A*24:02 | AFP | CA24 105 | ADFSGLLEK C | 10 | Yes | No | Ala-Asp-Phe-Ser-Gly-Leu- Leu-Glu-Lys-Cys (SEQ ID NO: 58) | |
| HLA-A*24:02 | EPHB 4 | CA24 123 | AWSYGIVMW | 9 | Yes | - | Ala-Trp-Ser-Tyr-Gly-Ile- Val-Met-Trp (SEQ ID NO: 59) | |
| HLA-A*24:02 | EPHB 4 | CA24 124 | SYGIVMWEV | 9 | Yes | Yes | Ser-Tyr-Gly-Ile-Val-Met- Trp-Glu-Val (SEQ ID NO: 60) | [SYGIVMWEV]MSFGER (SEQ ID NO: 79) |
| HLA-A*24:02 | EPHB 4 | CA24 127 | IVMWEVMSF | 9 | Yes | - | Ile-Val-Met-Trp-Glu-Val- Met-Ser-Phe (SEQ ID NO: 61) | |
| HLA-A*24:02 | CLDN 1 | CLDN1-A24-2 | WYGNRIVQE F | 10 | Yes | No | Trp-Tyr-Gly-Asn-Arg-Ile- Val-Gln-Glu-Phe (SEQ ID NO: 62) | |
| HLA-A*24:02 | CLDN 1 | CLDN1-A24-4 | YGNRIVQEF | 9 | Yes | No | Tyr-Gly-Asn-Arg-Ile-Val- Gln-Glu-Phe (SEQ ID NO: 63) | |
| HLA-A*24:02 | CLDN 1 | CLDN1-A24-5 | EFGQALFTG W | 10 | Yes | No | Glu-Phe-Gly-Gln-Ala-Leu- Phe-Thr-Gly-Trp (SEQ ID NO: 64) | |
| HLA-A*24:02 | CLDN 1 | CLDN1-A24-7 | GWAAASLCL | 9 | Yes | No | Gly-Trp-Ala-Ala-Ala-Ser- Leu-Cys-Leu (SEQ ID NO: 65) | |
| HLA-A*24:02 | LAT1 | LAT1-A24-2 | CYAELGTTI | 9 | Yes | Yes | Cys-Tyr-Ala-Glu-Leu-Gly- Thr-Thr-Ile (SEQ ID NO: 66) | VGAL[CYAELGTTI]SKS G (SEQ ID NO: 80) |
| HLA-A*24:02 | LAT1 | LAT1-A24-5 | AYMLEVYGS L | 10 | Yes | No | Ala-Tyr-Met-Leu-Glu-Val- Tyr-Gly-Ser-Leu (SEQ ID NO: 67) | |
| HLA-A*24:02 | LAT1 | LAT1-A24-6 | VMSWIIPVF | 9 | Yes | - | Val-Met-Ser-Trp-Ile-Ile- Pro-Val-Phe (SEQ ID NO: 68) | |

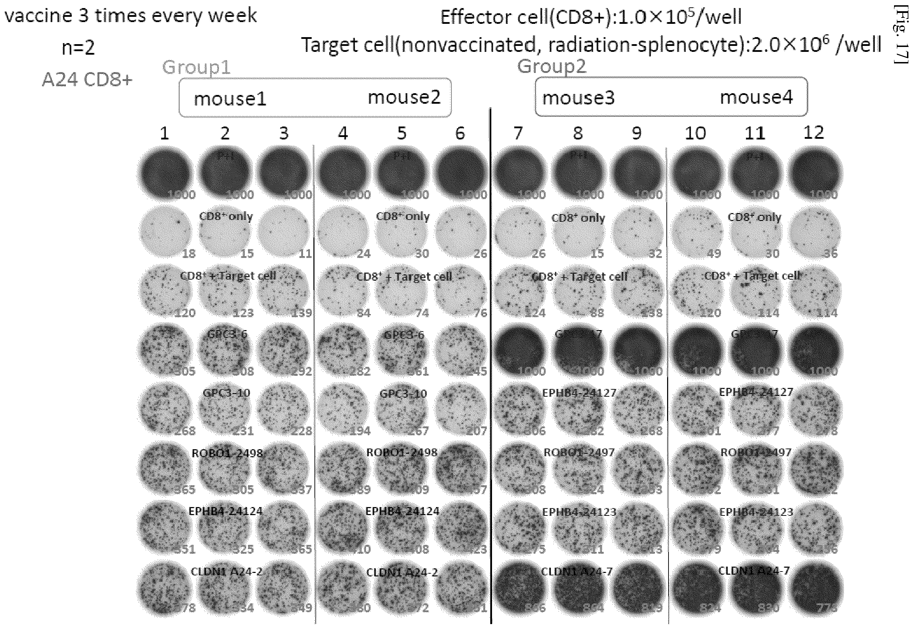

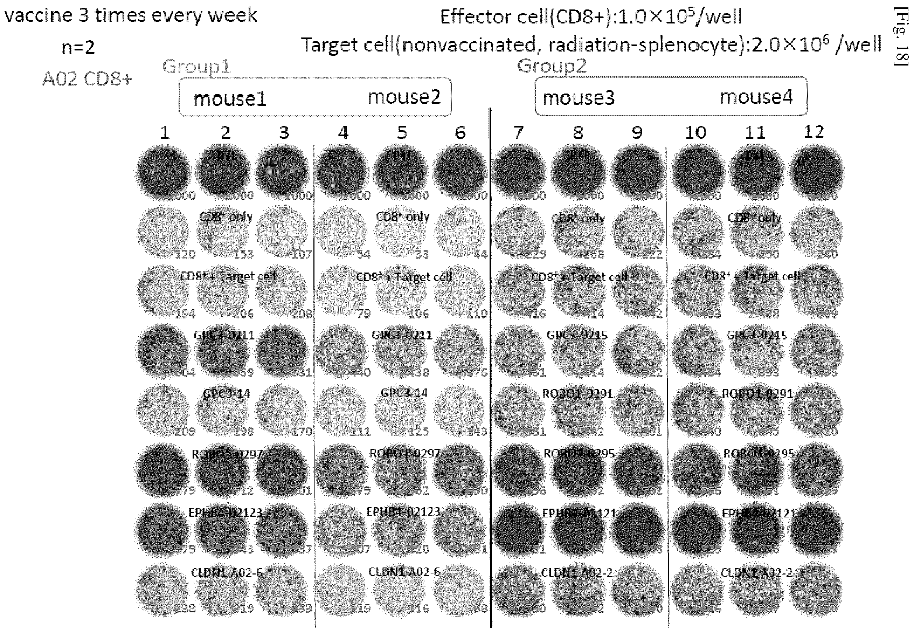

Fig. 17 and Fig. 18 show evaluation on whether antigen-specific T cells are induced through vaccination of HLA-A*24:02 or HLA-A*02:01 transgenic mice with dendritic cells (DCs) sensitized with a common cancer antigen-derived short peptide found to induce CTLs through the use of transgenic mice with different HLA genes introduced therein. This evaluation successfully confirmed induction of antigen-specific CTLs caused by a common cancer antigen peptide-sensitized dendritic cell vaccine for many peptides in an IFN-γ ELISpot assay. For antigens found to be highly expressed primarily in hepatocellular carcinoma, three peptides that caused high induction of CTLs through the use of a peptide vaccine were selected for each antigen for use. DCs were sensitized with the selected peptides, transgenic mice were vaccinated therewith, and the inducibilities for antigen-specific CTLs were evaluated with use of IFN-γ ELISpot assay.

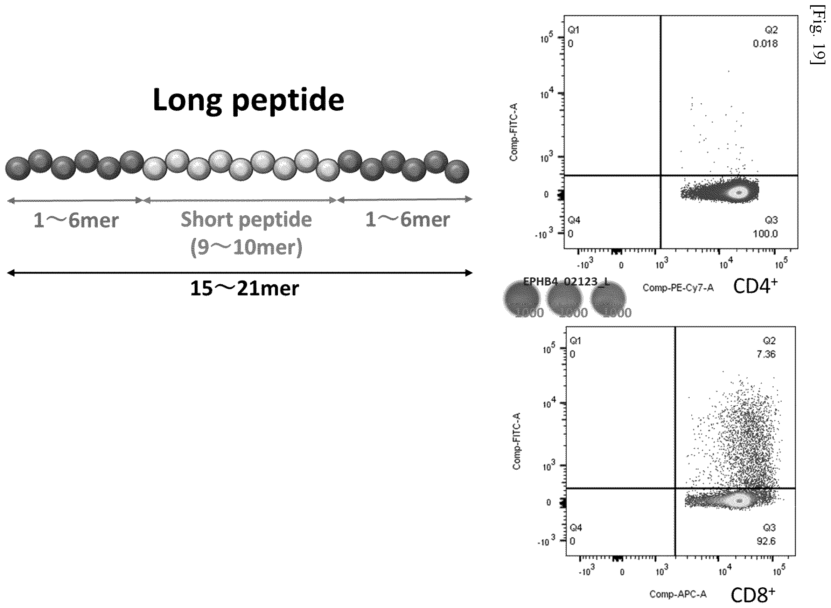

Fig. 19 shows validation performed with HLA-A*02:01 Tgm or HLA-A*24:02 Tgm to check whether a peptide is actually intracellularly processed and presented on HLA by using long peptides each containing the sequence of a common cancer antigen-derived short peptide found to induce CTLs. A long peptide is a long-chain peptide (15 to 21mer) designed to contain the amino acid sequence of a short peptide of 9 or 10mer that successfully induced CTLs reactive with the peptide, and evaluation was performed by using IFN-γ ELISpot assay on whether peptide-specific CTLs would be induced by vaccination with such a long peptide through the process including actual intracellular processing, binding to an MHC molecule, and presentation on HLA. For the HLA-A*24:02-restricted common cancer antigen long peptide vaccines, three of 21 synthesized long peptides were found to successfully induce CTLs reactive with short peptides contained in their sequences. For the HLA-A*02:01-restricted common cancer antigen long peptide vaccines, nine of 21 synthesized long peptides were found to successfully induce CTLs reactive with short peptides contained in their sequences.

### Example 4: Development of common cancer antigen mRNA vaccine

LNP-Covid-19 Spike mRNA was administered and antigen-specific IFN-γ production was evaluated.

An mRNA vaccine in which an mRNA encoding the full length of Covid-19 (SARS-CoV-2) Spike protein was encapsulated in an LNP (lipid nano particle) (Arcturus Therapeutics, Inc., Conventional mRNA described in Non-Patent Document (de Alwis R. et al. A single dose of self-transcribing and replicating RNA-based SARS-CoV-2 vaccine produces protective adaptive immunity in mice. Mol Ther. 2021; 29: 1970-1983.), mRNA vaccines hereinafter were prepared in the same manner) in an amount of 2 µg or 10 µg was administered intramuscularly (i.m.) into the rectus femoris muscle of or intradermally (i.d.) into the base of the tail of HLA-A*02:01 Tg mice and HLA-A*24:02 Tg mice. In a triple administration protocol, administration was performed on day 0, day 7, and day 14, three times in total, and the HLA-A*24:02 Tg mice and the HLA-A*02:01 Tg mice were dissected on day 20 and on day 21, respectively. In a double administration protocol, administration was performed on day 0 and day 28, and the HLA-A*24:02 Tg mice and the HLA-A*02:01 Tg mice were dissected on day 35 and on day 36, respectively. Cells were prepared from their spleens and inguinal lymph nodes (LN), and serums were collected from their blood obtained through heart blood sampling. The antigen-specific IFN-γ productions from T cells were evaluated by using BD ELISPOT Mouse IFN-γ Set and BD ELISPOT AEC Substrate Set (both from Becton, Dickinson and Company) with an ELISPOT method in accordance with the recommended protocol. In a 96-well plate coated with an anti-mIFN-γ antibody, spleen cells and LN cells were seeded at 2 × 10^6 cells/well and at 2 × 10^5 cells/well, respectively, and, with addition of any one of PMA (25 ng/mL) + Ionomycin (1 µg/mL), a mix of peptides covering Covid-19 Spike protein (PepMix S (JPT Peptide Technologies, PM-WCPV-S-1) for the triple administration experiment, PepTivator SARC-CoV-2 Prot_S (Miltenyi Biotech, 130-127-953) for the double administration experiment, 1 µg/mL for each peptide), POOL1, which was a mix of peptides for which peptide-specific IFN-γ production had been found in peptide immunization for HLA-A*02:01 Tg mice or HLA-A*24:02 Tg mice after prediction from the Covid-19 Spike protein sequence with the predictor described in the paragraph [0094] (5 µg/mL for each peptide), bone marrow-derived dendritic cells (BMDCs) (2 × 10^5 cells/well) obtained in such a manner that the bone marrow of a naive mouse of the corresponding HLA Tg mice was induced to differentiate with mouse GM-CSF (20 ng/mL) and the differentiated cells were matured with addition of mouse TNF-α (20 ng/mL), mouse IL-4 (20 ng/mL), and PGE2 (1 µg/mL) on the day before use, and BMDCs (2 × 10^5 cells/well) with 10 µg LNP-Spike mRNA per 1 × 10^6 cells added simultaneously with the same maturation, coculture was performed for 20 hours and the productions of IFN-γ were evaluated. Spots were photographed with an Eliphoto Counter AT-X (Minerva Tech K.K.), and the numbers of spots were counted. On the lower left of each well, the number of spots was written down.

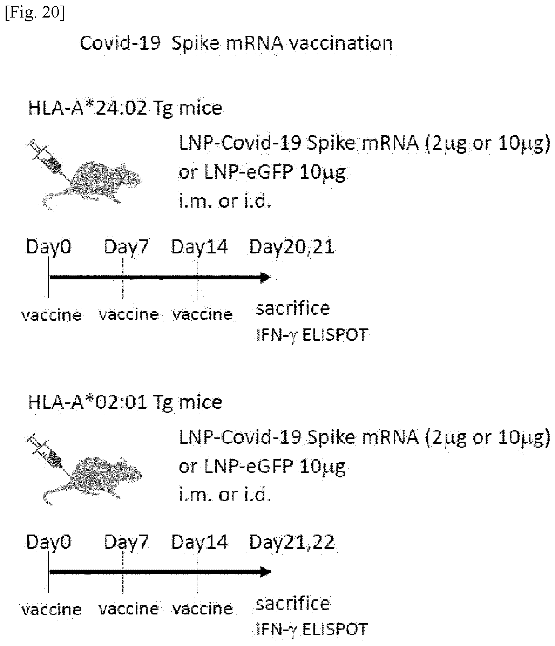

Fig. 20 shows the administration method by triple administration of LNP-Covid-19 Spike mRNA.

An mRNA vaccine in which an mRNA encoding the full length of Covid-19 Spike protein was encapsulated in an LNP (Arcturus Therapeutics, Inc.) in an amount of 2 µg or 10 µg was administered intramuscularly (i.m.) into the rectus femoris muscle of or intradermally (i.d.) into the base of the tail of HLA-A*02:01 Tg mice and HLA-A*24:02 Tg mice on day 0, day 7, and day 14, three times in total, and the HLA-A*24:02 Tg mice and the HLA-A*02:01 Tg mice were dissected on day 20 and on day 21, respectively, and the antigen-specific productions of IFN-γ were evaluated with an ELISPOT method.

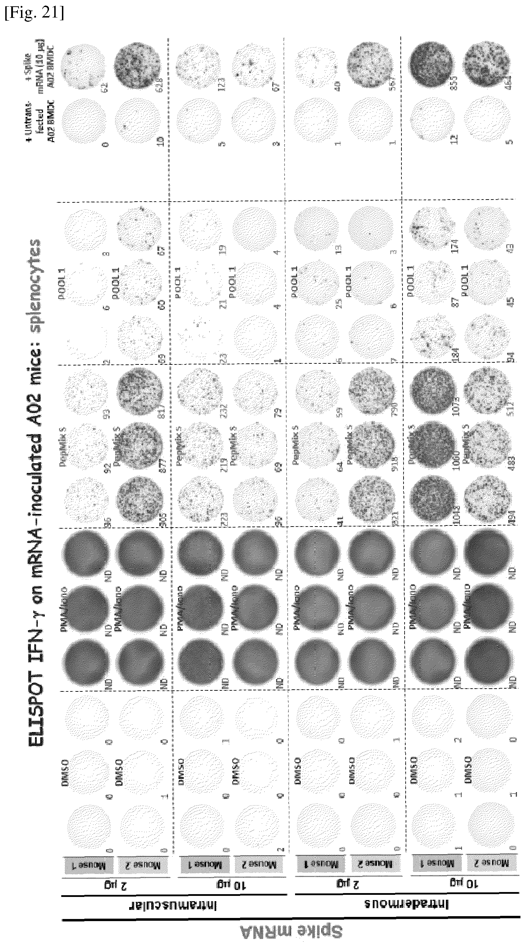

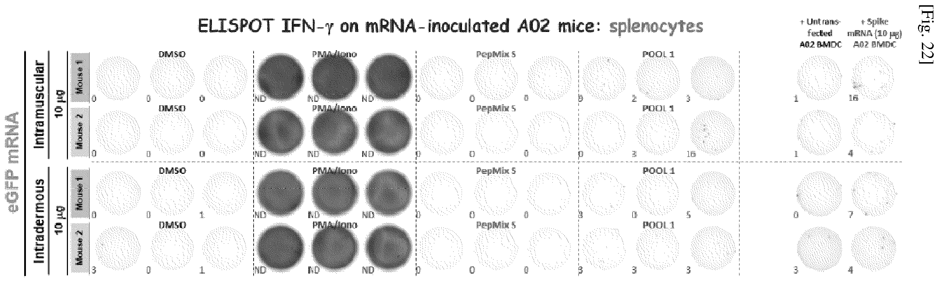

Fig. 21 and Fig. 22 show results of Spike antigen-specific IFN-γ production in spleen cells of the HLA-A*02:01 Tg mice to which LNP-Covid-19 Spike mRNA was administered three times.

In spleen cells of the HLA-A*02:01 Tg mice to which LNP-Covid-19 Spike mRNA had been administered three times, T cells producing IFN-γ were induced against PepMix S, POOL1, and BMDCs with addition of LNP-Covid-19 Spike mRNA in both of the i.m. and i.d. cases. In HLA-A*02:01 Tg mice to which LNP-eGFP mRNA had been similarly administered three times, as a negative control, production of IFN-γ was hardly found for PepMix S, POOL1, and BMDCs with addition of LNP-Covid-19 Spike mRNA; hence, it can be understood that the aforementioned production of IFN-γ was specifically induced by administration of LNP-Covid-19 Spike mRNA.

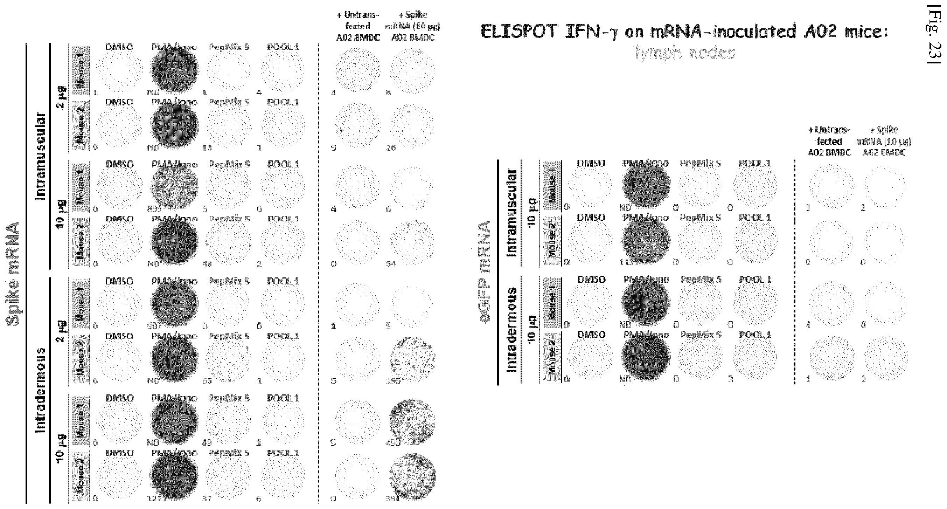

Fig. 23 shows results of Spike antigen-specific IFN-γ production in inguinal lymph node cells of the HLA-A*02:01 Tg mice to which LNP-Covid-19 Spike mRNA was administered three times.

In inguinal lymph node cells of the HLA-A*02:01 Tg mice to which LNP-Covid-19 Spike mRNA had been administered three times, T cells producing IFN-γ were induced against PepMix S and BMDCs with addition of LNP-Covid-19 Spike mRNA in both of the i.m. and i.d. cases. In HLA-A*02:01 Tg mice to which LNP-eGFP mRNA had been similarly administered three times, as a negative control, production of IFN-γ was hardly found for PepMix S and BMDCs with addition of LNP-Covid-19 Spike mRNA; hence, it can be understood that the aforementioned production of IFN-γ was specifically induced by administration of LNP-Covid-19 Spike mRNA.

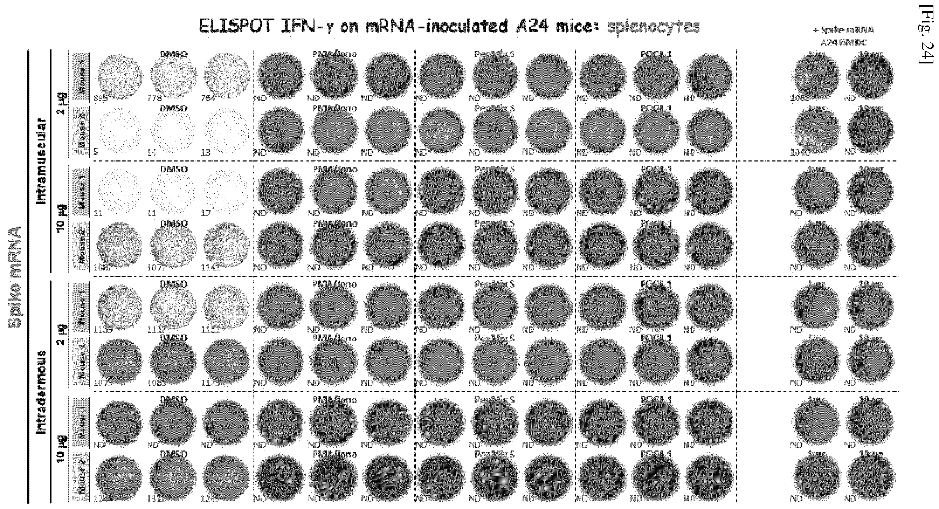

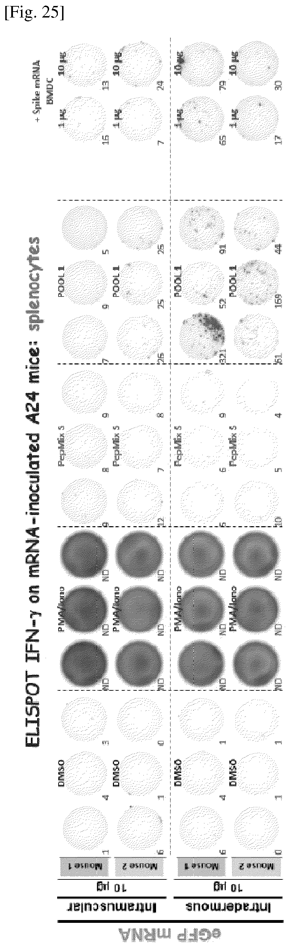

Fig. 24 and Fig. 25 show results of Spike antigen-specific IFN-γ production in spleen cells of the HLA-A*24:02 Tg mice to which LNP-Covid-19 Spike mRNA was administered three times.

In spleen cells of the HLA-A*24:02 Tg mice to which LNP-Covid-19 Spike mRNA had been administered three times, T cells producing IFN-γ were strongly induced against PepMix S, POOL1, and BMDCs with addition of LNP-Covid-19 Spike mRNA in an amount of 1 µg or 10 µg per 1 × 10^6 cells in both of the i.m. and i.d. cases. In HLA-A*24:02 Tg mice to which LNP-eGFP mRNA had been similarly administered three times, as a negative control, production of IFN-γ was low for PepMix S, POOL1, and BMDCs with addition of LNP-Covid-19 Spike mRNA; hence, it can be understood that the aforementioned production of IFN-γ was specifically induced by administration of LNP-Covid-19 Spike mRNA.

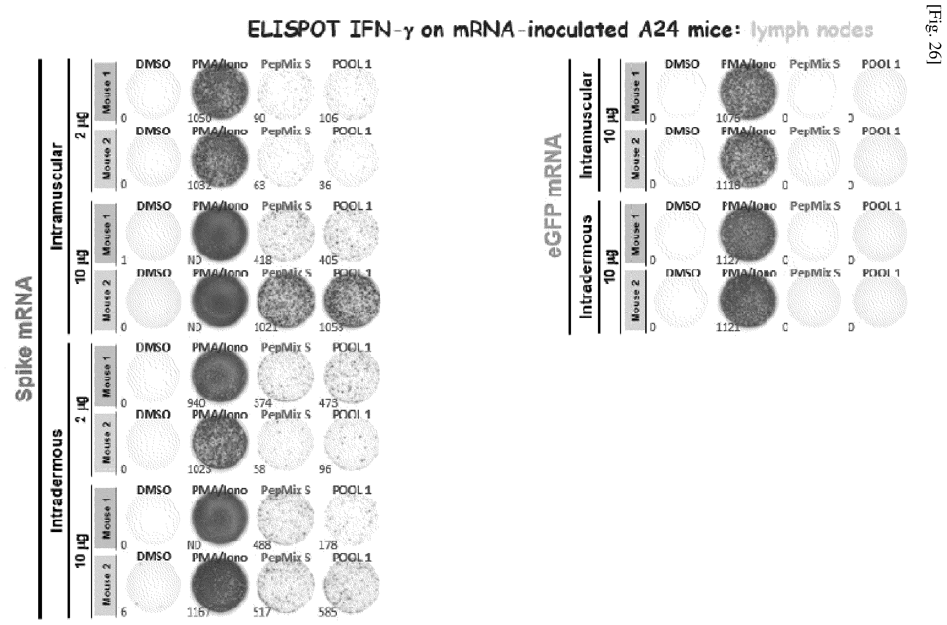

Fig. 26 shows results of Spike antigen-specific IFN-γ production in inguinal lymph node cells of the HLA-A*24:02 Tg mice to which LNP-Covid-19 Spike mRNA was administered three times.

In inguinal lymph node cells of the HLA-A*24:02 Tg mice to which LNP-Covid-19 Spike mRNA had been administered three times, T cells producing IFN-γ were induced against PepMix S and POOL1 in both of the i.m. and i.d. cases. In HLA-A*24:02 Tg mice to which LNP-eGFP mRNA had been similarly administered three times, as a negative control, production of IFN-γ was hardly found for PepMix S and POOL1; hence, it can be understood that the aforementioned production of IFN-γ was specifically induced by administration of LNP-Covid-19 Spike mRNA.

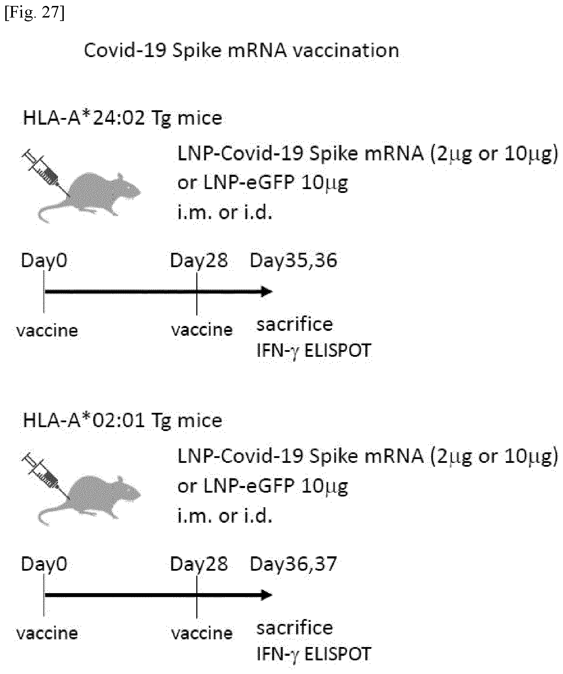

Fig. 27 shows an administration method by double administration of LNP-Covid-19 Spike mRNA.

An mRNA vaccine in which an mRNA encoding the full length of Covid-19 Spike protein was encapsulated in an LNP in an amount of 2 µg or 10 µg was administered intramuscularly (i.m.) into the rectus femoris muscle of or intradermally (i.d.) into the base of the tail of HLA-A*02:01 Tg mice and HLA-A*24:02 Tg mice on day 0 and day 28, and the HLA-A*24:02 Tg mice and the HLA-A*02:01 Tg mice were dissected on day 35 and on day 36, respectively, and antigen-specific productions of IFN-γ were evaluated with an ELISPOT method.

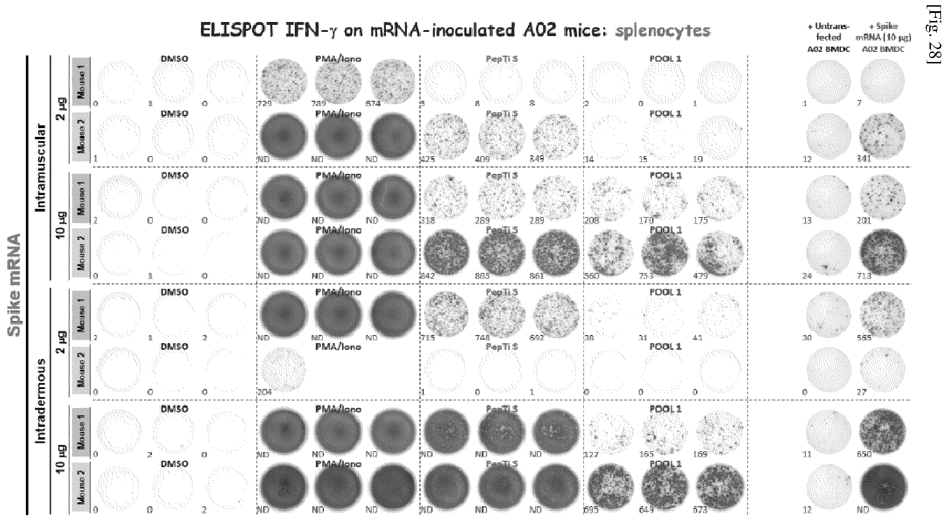

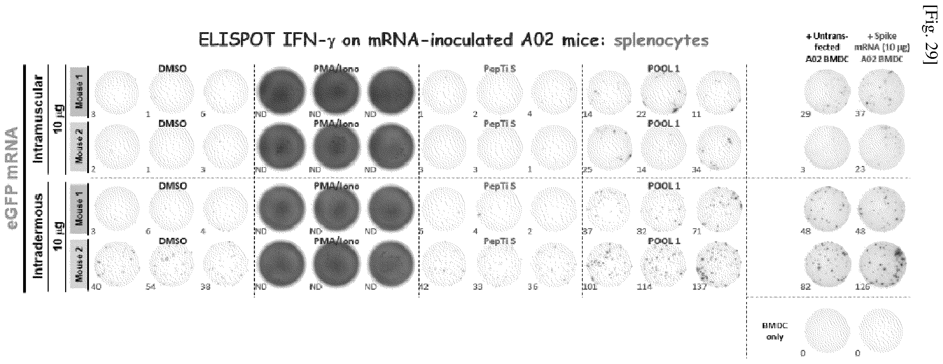

Fig. 28 and Fig. 29 show results of Spike antigen-specific IFN-γ production in spleen cells of the HLA-A*02:01 Tg mice to which LNP-Covid-19 Spike mRNA was administered twice.

In spleen cells of the HLA-A*02:01 Tg mice to which LNP-Covid-19 Spike mRNA had been administered twice, T cells producing IFN-γ were induced against PepTi S, POOL1, and BMDCs with addition of LNP-Covid-19 Spike mRNA in both of the i.m. and i.d. cases. In HLA-A*02:01 Tg mice to which LNP-eGFP mRNA had been similarly administered twice, as a negative control, only marginal production of IFN-γ was found for PepTi S, POOL1, and BMDCs with addition of LNP-Covid-19 Spike mRNA; hence, it can be understood that the aforementioned production of IFN-γ was specifically induced by administration of LNP-Covid-19 Spike mRNA.

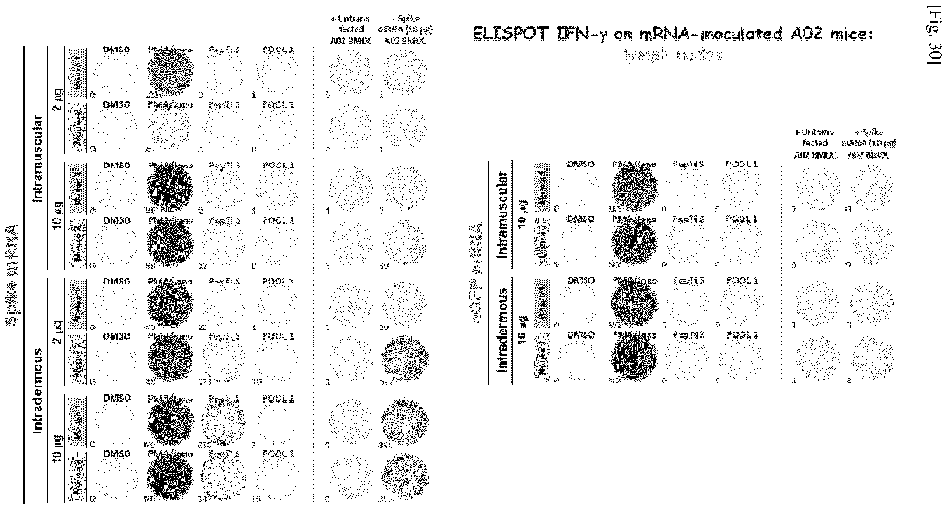

Fig. 30 shows results of Spike antigen-specific IFN-γ production in inguinal lymph node cells of the HLA-A*02:01 Tg mice to which LNP-Covid-19 Spike mRNA was administered twice.

In inguinal lymph node cells of the HLA-A*02:01 Tg mice to which LNP-Covid-19 Spike mRNA had been administered twice, T cells producing IFN-γ were induced against PepTi S and BMDCs with addition of LNP-Covid-19 Spike mRNA in both of the i.m. and i.d. cases. In particular, high induction was found in the i.d. case. In HLA-A*02:01 Tg mice to which LNP-eGFP mRNA had been similarly administered three times, as a negative control, production of IFN-γ was hardly found for PepTi S and BMDCs with addition of LNP-Covid-19 Spike mRNA; hence, it can be understood that the aforementioned production of IFN-γ was specifically induced by administration of LNP-Covid-19 Spike mRNA.

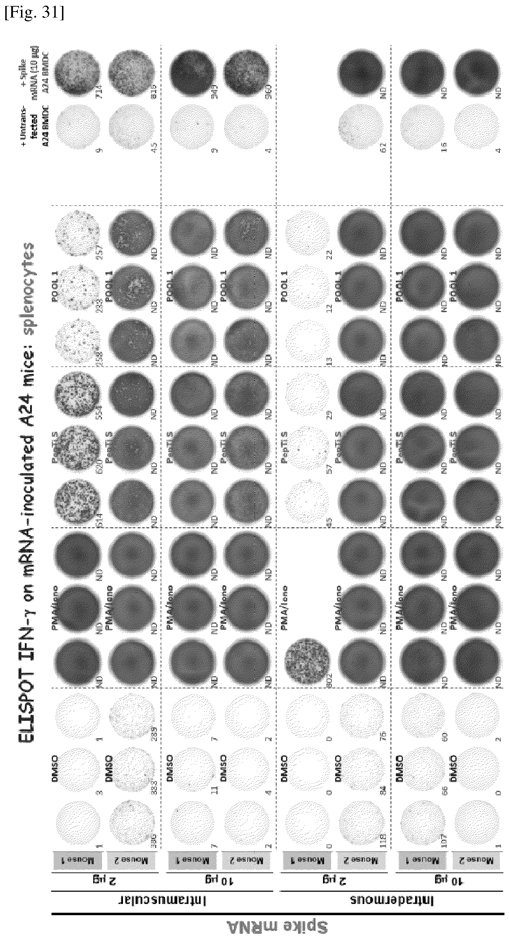

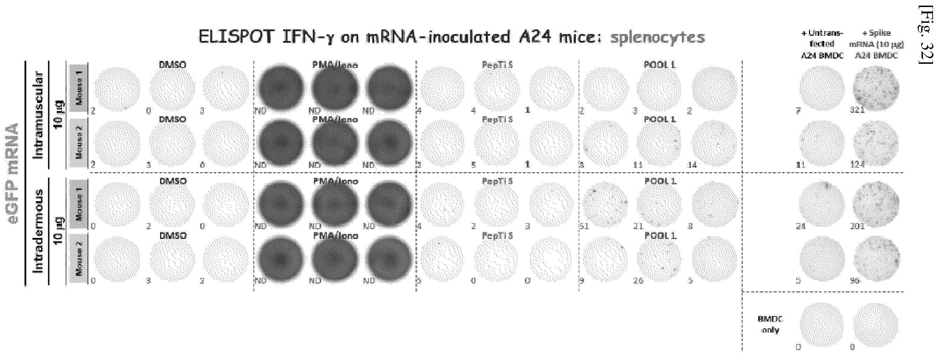

Fig. 31 and Fig. 32 show results of Spike antigen-specific IFN-γ production in spleen cells of the HLA-A*24:02 Tg mice to which LNP-Covid-19 Spike mRNA was administered twice.

In spleen cells of the HLA-A*24:02 Tg mice to which LNP-Covid-19 Spike mRNA had been administered twice, T cells producing IFN-γ were strongly induced against PepTi S, POOL1, and BMDCs with addition of LNP-Covid-19 Spike mRNA in both of the i.m. and i.d. cases. In HLA-A*02:01 Tg mice to which LNP-eGFP mRNA had been similarly administered twice, as a negative control, only marginal production of IFN-γ was found for PepTi S, POOL1, and BMDCs with addition of LNP-Covid-19 Spike mRNA; hence, it can be understood that the aforementioned production of IFN-γ was specifically induced by administration of LNP-Covid-19 Spike mRNA.

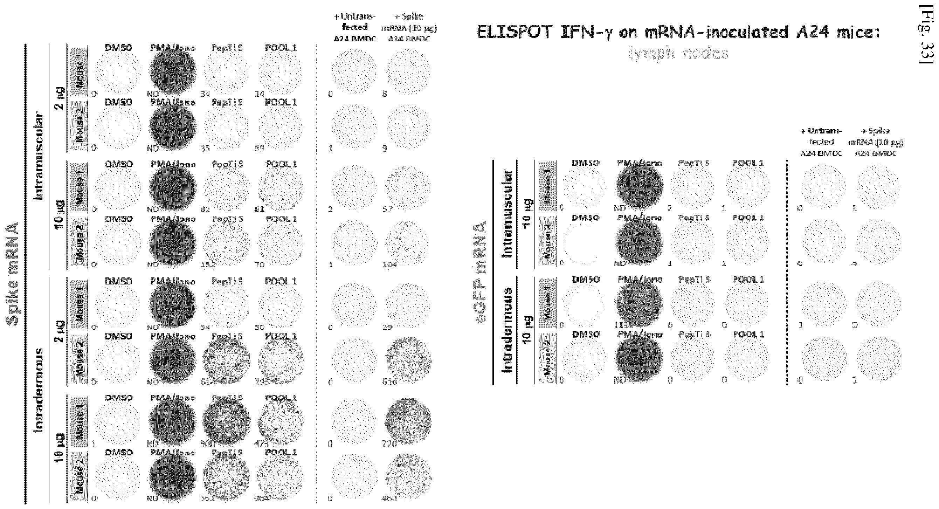

Fig. 33 shows results of Spike antigen-specific IFN-γ production in inguinal lymph node cells of the HLA-A*24:02 Tg mice to which LNP-Covid-19 Spike mRNA was administered twice.

In inguinal lymph node cells of the HLA-A*24:02 Tg mice to which LNP-Covid-19 Spike mRNA had been administered twice, T cells producing IFN-γ were induced against PepTi S and BMDCs with addition of LNP-Covid-19 Spike mRNA in both of the i.m. and i.d. cases. In particular, strong induction was found in the i.d. case. In HLA-A*24:02 Tg mice to which LNP-eGFP mRNA had been similarly administered three times, as a negative control, production of IFN-γ was hardly found for PepTi S and BMDCs with addition of LNP-Covid-19 Spike mRNA; hence, it can be understood that the aforementioned production of IFN-γ was specifically induced by administration of LNP-Covid-19 Spike mRNA.

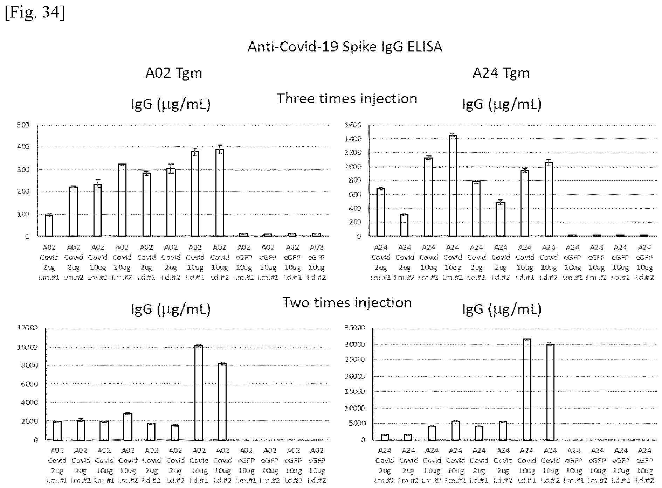

Fig. 34 shows results of antibody measurement for Spike protein.

### <Method>

A MaxiSorp plate (Thermo Fisher Scientific) was coated with Covid-19 Spike recombinant protein (Sino Biological, Inc., 40589-V08B1) at 100 ng/50 µL PBS. After washing once with PBS containing 0.05% Tween 20 (PBS-T), blocking was performed with 3% non-fat milk at room temperature for 1 hour. Serum diluted with PBS containing 1% non-fat milk was added, and incubation was performed at 37°C for 1 hour. Washing was performed three times with PBS-T, and incubation was performed in PBS-T containing 1% non-fat milk with horseradish peroxidase (HRP)-labeled goat anti-mouse IgG (SouthernNiotech, 6000-fold diluted) at room temperature for 1 hour. After washing three times with PBS-T, 3,3',5,5'-tetramethylbenzine substrate solution (Pierce) was added for coloring, the reaction was quenched with 1.3 N H₂SO4, and the absorbance at 450 nm was measured.

### <Results>

While administration of LNP-eGFP mRNA, as a negative control, did not cause induction of any antibody, an IgG antibody to Covid-19 Spike protein was induced with any of 2 µg and 10 µg of LNP-Covid-19 Spike mRNA, in both of the i.m. and i.d. cases in both of the triple administration and the double administration. As a general tendency, induction of the IgG antibody was higher at 10 µg than at 2 µg, and higher in the i.d. case than in the i.m. case. In particular, high antibody induction was found in the case of the double administration in the i.d. case at 10 µg.

### Example 5: Development of common cancer antigen mRNA vaccine

LNP-hGPC3 mRNA, LNP-hROBO1 mRNA, LNP-hCLDN1 mRNA, LNP-hEphB4 mRNA, LNP-hTGFBI mRNA, LNP-hSPARC mRNA, LNP-hAFP mRNA, LNP-hHSP105α mRNA, and LNP-(hCLDN1+hEPHB4) mRNA were administered, and antigen-specific IFN-γ productions were evaluated.

Any one of mRNAs respectively encoding the full-length proteins of hGPC3 isoform 2 (RefSeq: NP_004475), hROBO1 isoform X5 (RefSeq: XP_006713340), hCLDN1 (RefSeq: NP_066924), hEphB4 (RefSeq: NP_004435), TGFBI (RefSeq: NP_000349), hSPARC isoform 1 (NP_003109), hAFP isoform 1 (NP_001125), and hHSPH1 isoform 1 (NP_006635), an equimass mixture of an mRNA encoding the full-length protein of hCLDN1 (RefSeq: NP_066924) and an mRNA encoding the full-length protein of hEphB4 (RefSeq: NP_004435), an mRNA encoding the full-length protein of Luciferase, or an mRNA vaccine (Arcturus Therapeutics, Inc.) in which an mRNA encoding the full-length protein of eGFP in an LNP (lipid nano particle, Arcturus Therapeutics, Inc.) was administered intramuscularly (i.m.) into the rectus femoris muscle of or intradermally (i.d.) into the base of the tail of HLA-A*02:01 Tg mice and HLA-A*24:02 Tg mice. Cells were prepared from their spleens and inguinal lymph nodes (LN). The antigen-specific IFN-γ productions from T cells were evaluated by using BD ELISPOT Mouse IFN-γ Set and BD ELISPOT AEC Substrate Set (both from Becton, Dickinson and Company) with an ELISPOT method in accordance with the recommended protocol. In a 96-well plate coated with an anti-mIFN-γ antibody, spleen cells and LN cells were seeded at 2 × 10^6 cells/well and at 2 × 10^5 cells/well, respectively, and, with addition of any one of PMA (25 ng/mL) + Ionomycin (1 µg/mL), a mix of short-chain peptides for which peptide-specific IFN-γ production had been found in peptide immunization for the corresponding HLA Tg mice (10 µg/mL for each peptide), a mix of long-chain peptides for which peptide-specific IFN-γ production had been found in peptide immunization for the corresponding HLA Tg mice (10 µg/mL for each peptide), mouse cancer cells (MC38 or MCA205, 2 × 10^5 cells/well) expressing HLA-HHD (chimeric protein of hβ2M-linker-[α1+α2 domain of HLA]-[α3 to C-terminal domain of H-2D^{b}]) of corresponding HLA, mouse cancer cells (MC38 or MCA205, 2 × 10^5 cells/well) expressing HLA-HHD of corresponding HLA and the corresponding cancer antigen, bone marrow-derived dendritic cells (BMDCs) (2 × 10^5 cells/well) obtained in such a manner that the bone marrow of a naive mouse of the corresponding HLA Tg mice was induced to differentiate with mouse GM-CSF (20 ng/mL) and the differentiated cells were matured with addition of mouse TNF-α (20 ng/mL), mouse IL-4 (20 ng/mL), and PGE2 (1 µg/mL) on the day before use, and BMDCs (2 × 10^5 cells/mL) transfected with 11 µg of the corresponding cancer antigen mRNA simultaneously with the same maturation, coculture was performed for 20 hours and the productions of IFN-γ were evaluated. Spots were photographed with an Eliphoto Counter AT-X (Minerva Tech K.K.). As the mix of short-chain peptides, the following peptides were added.

hGPC3 HLA-A*02:01: CA02 11, CA02 13, CA02 15, A02-GPC3-10, A02-GPC3-12, A02-GPC3-14, A02-GPC3-18
hGPC3 HLA-A*24:02: A24-GPC3-06, A24-GPC3-10, A24-GPC3-11, A24-GPC3-17
hROBO1 HLA-A*02:01: CA02 91, CA02 92, CA02 95, CA02 97
hROBO1 HLA-A*24:02: CA24 95, CA24 97, CA24 98
hCLDN1 HLA-A*02:01: CLDN1-A02-2, CLDN1-A02-5, CLDN1-A02-6
hCLDN1 HLA-A*24:02: CLDN1-A24-2, CLDN1-A24-4, CLDN1-A24-5, CLDN1-A24-7
hEPHB4 HLA-A*02:01: CA02 121, CA02 122, CA02 123, CA02 126, CA02 127
hEPHB4 HLA-A*24:02: CA24 123, CA24 124, CA24 127
hTGFBI HLA-A*02:01: CA02 81, CA02 82, CA02 83, CA02 86
hTGFBI HLA-A*24:02: CA24 85
hSPARC HLA-A*02:01: CA02 52, CA02 56
hSPARC HLA-A*24:02: CA24 51, CA24 52, CA24 53
hAFP HLA-A*02:01: CA02 101, CA02 103, CA02 104
hAFP HLA-A*24:02: CA24 102, CA24 105
hHSP105α HLA-A*02:01: CA02 21, CA02 24
hHSP105α HLA-A*24:02: CA24 22, CA24 23, CA24 24, CA24 25
hHSP105α HLA-A*24:02 short peptide
CA24 22: PFSKVLTFL
CA24 23: KLCGPYEKF
CA24 24: PYPEAKIGRF
CA24 25: NYGIYKQDL

As the mix of long-chain peptides, the following peptides were added.
hGPC3 HLA-A*02:01: CA02 11_L, CA02 15_L, A02-GPC3-14_L
hGPC3 HLA-A*24:02: A24-GPC3-06_L, A24-GPC3-11_L, A24-GPC3-17_L
hROBO1 HLA-A*02:01: CA02 95_L, CA02 97_L
hROBO1 HLA-A*24:02: CA24 97_L, CA24 98_L
hCLDN1 HLA-A*02:01: CLDN1-A02-2_L, CLDN1-A02-5_L
hCLDN1 HLA-A*24:02: CLDN1-A24-2_L, CLDN1-A24-4_L, CLDN1-A24-5_L, CLDN1-A24-7_L
hEPHB4 HLA-A*02:01: CA02 121_L, CA02 123_L
hEPHB4 HLA-A*24:02: CA24 124_L
hTGFBI HLA-A*02:01: CA02 83_L, CA02 86_L
hSPARC HLA-A*02:01: CA02 56_L
hSPARC HLA-A*24:02: CA24 51_L, CA24 53_L
hAFP HLA-A*02:01: 02 103_L
hAFP HLA-A*24:02: 24 105_L
hHSP105α HLA-A*02:01: A2-7_L
hHSP105α HLA-A*24:02: A24-1_L, A24-7_L
hHSP105α HLA-A*02:01 long peptide
A2-7_L: VGLNCL[RLMNDMTAV]ALNYG
hHSP105α HLA-A*24:02 long peptide
A24-1_L: KNAVE[EYVYEFRDKL]CGPYE
A24-7_L: AVAL[NYGIYKQDL]PSLDEK

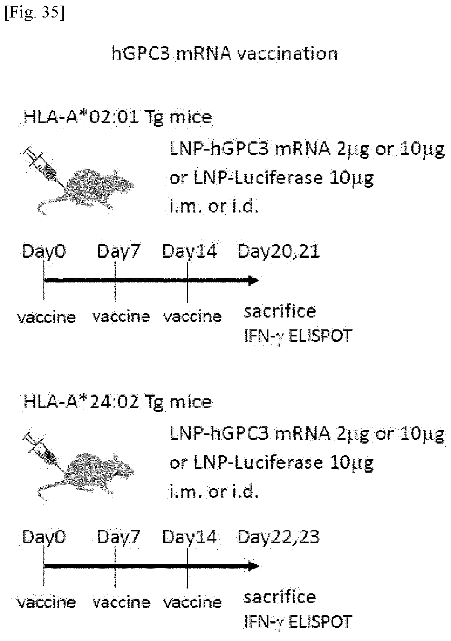

Fig. 35 shows an administration method by triple administration of LNP-hGPC3 mRNA.

LNP-hGPC3 mRNA in an amount of 2 µg or 10 µg or LNP-Luciferase mRNA in an amount of 10 µg was administered i.m. into the rectus femoris muscle of or i.d. into the base of the tail of HLA-A*02:01 Tg mice and HLA-A*24:02 Tg mice on day 0, day 7, and day 14, three times in total, the HLA-A*24:02 Tg mice and the HLA-A*02:01 Tg mice were dissected on day 20 and on day 22, respectively, and antigen-specific productions of IFN-y were evaluated with an ELISPOT method.

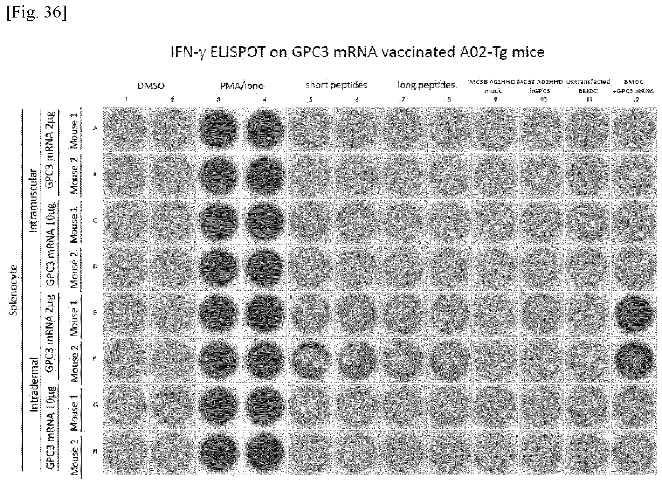

Fig. 36 to Fig. 39 shows the results.

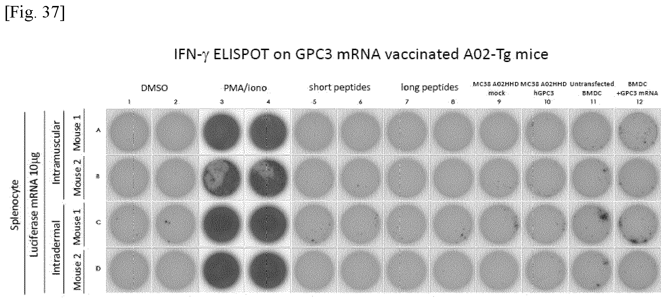

As shown in Fig. 36 and Fig. 37, in spleen cells of the HLA-A*02:01 Tg mice to which LNP-hGPC3 mRNA had been administered three times, T cells producing IFN-γ were induced against the mix of hGPC3 short-chain peptides for which peptide-specific IFN-γ production had been found in peptide immunization for HLA-A*02:01 Tg mice, the mix of hGPC3 long-chain peptides for which peptide-specific IFN-γ production had been found in peptide immunization for HLA-A*02:01 Tg mice, the cancer cells MC38 expressing HLA-A*02:01HHD and hGPC3, and BMDCs transfected with hGPC3 mRNA. More IFN-γ was produced in the i.d. case than in the i.m. case, and in particular strong IFN-γ induction was found in the triple i.d. at 2 µg. The IFN-γ production against the cancer cells MC38 A02fH-ID hGPC3, expressing HLA-A*02:01HHD and hGPC3, was significantly higher than that against the cancer cells MC38 A02HHD mock, into which HLA-A*02:01HHD and an empty vector had been introduced, and hence it can be understood that T cells that specifically recognized and attacked cancer cells expressing hGPC3 were induced. In HLA-A*02:01 Tg mice to which LNP-Luciferase mRNA had been similarly administered three times, as a negative control, only marginal production of IFN-γ was found for any target; hence, it can be understood that the aforementioned production of IFN-γ was specifically induced by administration of LNP-hGPC3 mRNA.

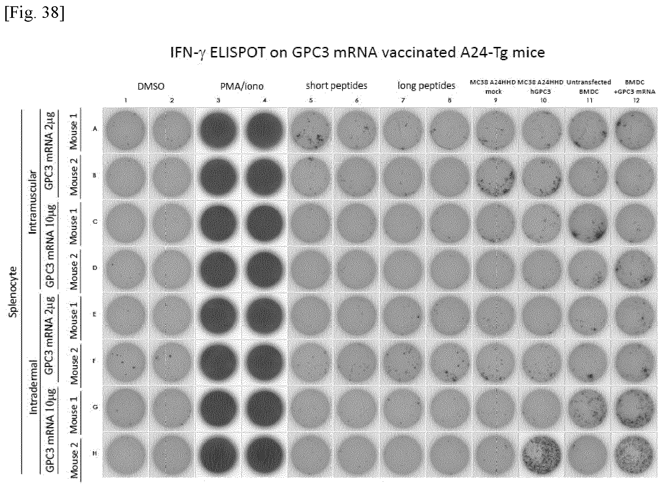

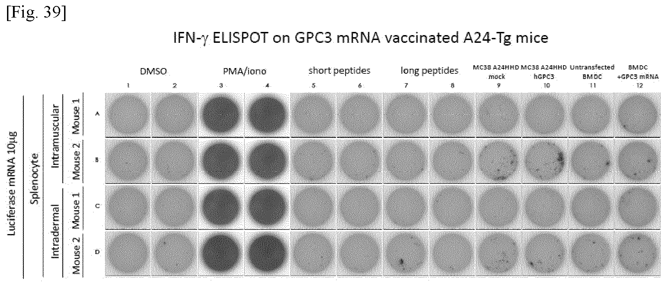

As shown in Fig. 38 and Fig. 39, in spleen cells of the HLA-A*24:02 Tg mice to which LNP-hGPC3 mRNA had been administered three times, T cells producing IFN-γ were induced against the mix of hGPC3 short-chain peptides for which peptide-specific IFN-γ production had been found in peptide immunization for HLA-A*24:02 Tg mice, the mix of hGPC3 long-chain peptides for which peptide-specific IFN-γ production had been found in peptide immunization for HLA-A*24:02 Tg mice, the cancer cells MC38 expressing HLA-A*24:02HHD and hGPC3, and BMDCs transfected with hGPC3 mRNA. More IFN-γ was produced in the i.d. case than in the i.m. case as a tendency, and in particular strong IFN-γ induction was found in the triple i.d. at 10 µg. The IFN-y production against the cancer cells MC38 A24HHD hGPC3, expressing HLA-A*24:02HHD and hGPC3, was significantly higher than that against the cancer cells MC38 A24HHD mock, into which HLA-A*24:02HHD and an empty vector had been introduced, and hence it can be understood that T cells that specifically recognized and attacked cancer cells expressing hGPC3 were induced. In HLA-A*24:02 Tg mice to which LNP-Luciferase mRNA had been similarly administered three times, as a negative control, only marginal production of IFN-γ was found for any target; hence, it can be understood that the aforementioned production of IFN-γ was specifically induced by administration of LNP-hGPC3 mRNA.

### Example 6: Development of common cancer antigen mRNA vaccine

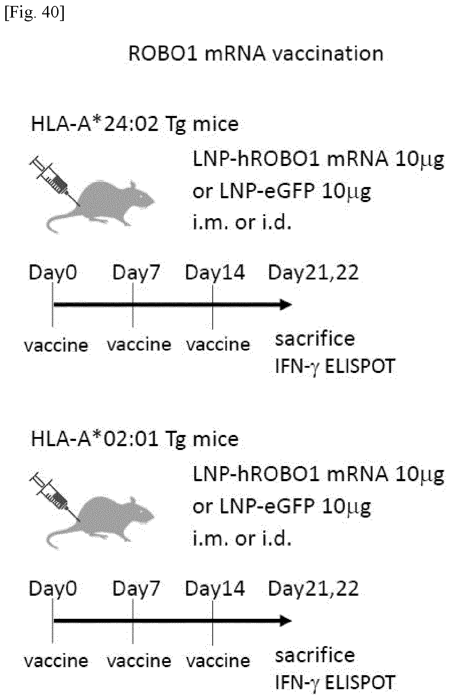

Fig. 40 shows an administration method by triple administration of LNP-hROBO1 mRNA.

LNP-hROBO1 mRNA or LNP-eGFP mRNA in an amount of 10 µg was administered intramuscularly (i.m.) into the rectus femoris muscle of or intradermally (i.d.) into the base of the tail of HLA-A*02:01 Tg mice and HLA-A*24:02 Tg mice on day 0, day 7, and day 14, three times in total, the mice were dissected on day 21, and antigen-specific productions of IFN-γ were evaluated with an ELISPOT method.

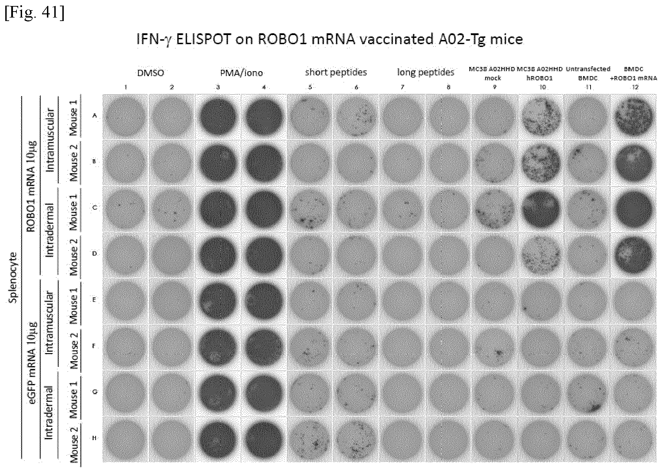

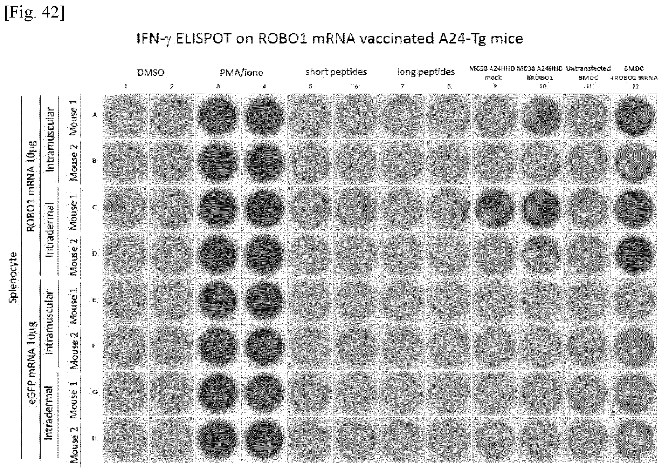

Fig. 41 and Fig. 42 show the results.

As shown in Fig. 41, in spleen cells of the HLA-A*02:01 Tg mice to which LNP-hROBO1 mRNA had been administered three times, T cells producing IFN-γ were induced against the mix of hROBO1 short-chain peptides for which peptide-specific IFN-y production had been found in peptide immunization for HLA-A*02:01 Tg mice, the mix of hROBO1 long-chain peptides for which peptide-specific IFN-γ production had been found in peptide immunization for HLA-A*02:01 Tg mice, the cancer cells MC38 expressing HLA-A*02:01HHD and hROBO1, and BMDCs transfected with hROBO1 mRNA. The IFN-γ production against the cancer cells MC38 A02HHD hROBO1, expressing HLA-A*02:01HHD and hROBO1, was significantly higher than that against the cancer cells MC38 A02HHD mock, into which HLA-A*02:01HHD and an empty vector had been introduced, in both of the i.m. and i.d. cases, and hence it can be understood that T cells that specifically recognized and attacked cancer cells expressing hROBO1 were induced. In HLA-A*02:01 Tg mice to which LNP-eGFP mRNA had been similarly administered three times, as a negative control, only marginal production of IFN-γ was found for any target; hence, it can be understood that the aforementioned production of IFN-γ was specifically induced by administration of LNP-hROBO1 mRNA.

As shown in Fig. 42, in spleen cells of the HLA-A*24:02 Tg mice to which LNP-hROBO1 mRNA had been administered three times, T cells producing IFN-γ were induced against the mix of hROBO1 short-chain peptides for which peptide-specific IFN-y production had been found in peptide immunization for HLA-A*24:02 Tg mice, the mix of hROBO1 long-chain peptides for which peptide-specific IFN-γ production had been found in peptide immunization for HLA-A*24:02 Tg mice, the cancer cells MC38 expressing HLA-A*24:02HHD and hROBO1, and BMDCs transfected with hROBO1 mRNA. The IFN-γ production against the cancer cells MC38 A24HHD hROBO1, expressing HLA-A*24:02HHD and hROBO1, was significantly higher than that against the cancer cells MC38 A24HHD mock, into which HLA-A2402HHD and an empty vector had been introduced, in both of the i.m. and i.d. cases, and hence it can be understood that T cells that specifically recognized and attacked cancer cells expressing hROBO1 were induced. In HLA-A2402 Tg mice to which LNP-eGFP mRNA had been similarly administered three times, as a negative control, only marginal production of IFN-γ was found for any target; hence, it can be understood that the aforementioned production of IFN-γ was specifically induced by administration of LNP-hROBO1 mRNA.

### Example 7: Development of common cancer antigen mRNA vaccine

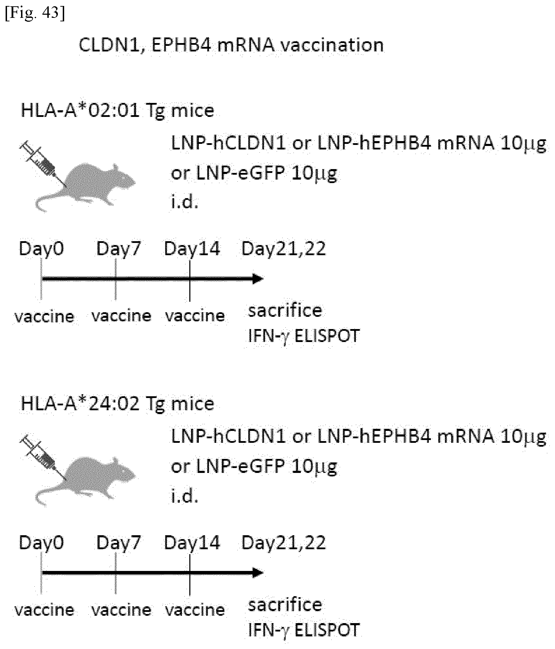

Fig. 43 shows an administration method by triple administration of LNP-hCLDN1 mRNA and LNP-hEPHB4 mRNA.

LNP-hCLDN1 mRNA, LNP-hEPHB4 mRNA, or LNP-eGFP mRNA in an amount of 10 µg was administered intradermally (i.d.) into the base of the tail of HLA-A*02:01 Tg mice and HLA-A*24:02 Tg mice on day 0, day 7, and day 14, three times in total, the mice were dissected on day 21, and antigen-specific productions of IFN-γ were evaluated with an ELISPOT method.

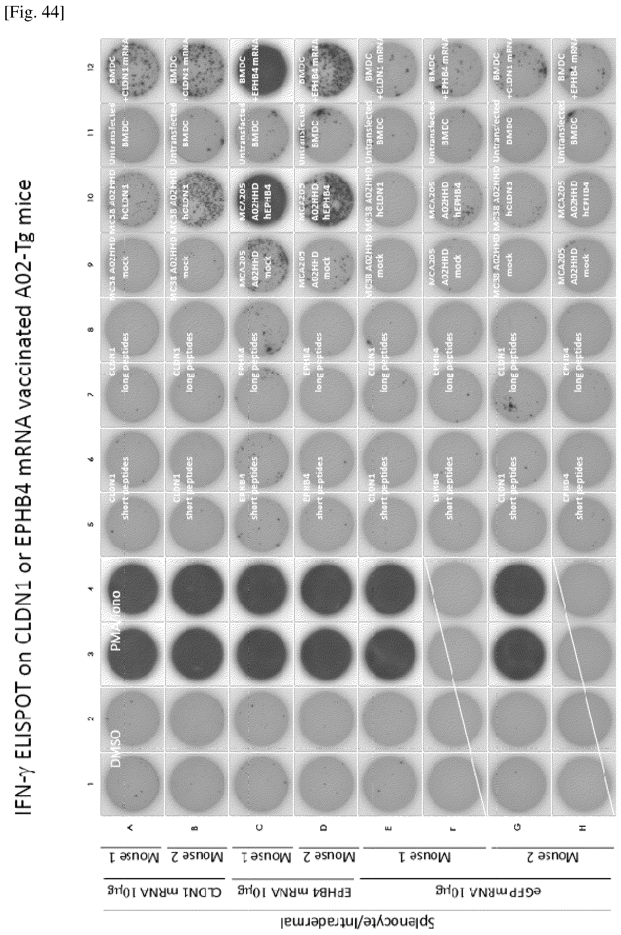

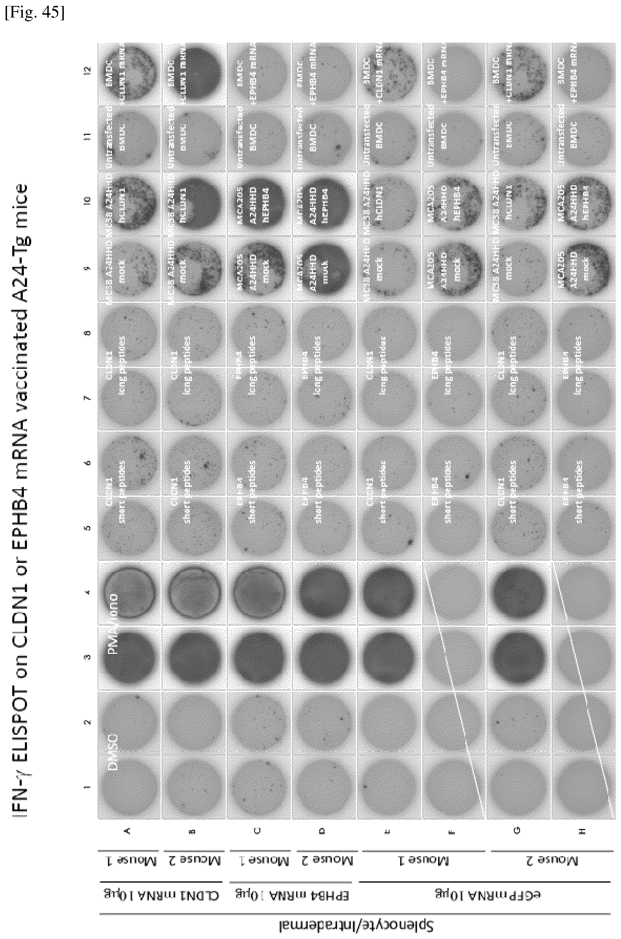

Fig. 44 and Fig. 45 show the results.

As shown in Fig. 44, in spleen cells of the HLA-A*02:01 Tg mice to which LNP-hCLDN1 mRNA or LNP-hEPHB4 mRNA had been administered three times, T cells producing IFN-γ were induced against the mix of hCLDN1 or hEPHB4 short-chain peptides for which peptide-specific IFN-γ production had been found in peptide immunization for HLA-A*02:01 Tg mice, the mix of hCLDN1 or hEPHB4 long-chain peptides for which peptide-specific IFN-γ production had been found in peptide immunization for HLA-A*02:01 Tg mice, the cancer cells MC38 expressing HLA-A*02:01HHD and hCLDN1 or the cancer cells MCA205 expressing HLA-A*02:01HHD and hEPHB4, and BMDCs transfected with hCLDN1 mRNA or hEPHB4 mRNA. The IFN-γ productions against the cancer cells MC38 A02HHD hCLDN1, expressing HLA-A*02:01HHD and hCLDN1, and against the cancer cells MCA205 A02HHD hEPHB4, expressing HLA-A*02:01HHD and hEPHB4, were significantly higher than those against the cancer cells MC38 A02HHD mock and against the cancer cells MCA205 A02HHD mock, into each of which HLA-A*02:01HHD and an empty vector had been introduced, and hence it can be understood that T cells that specifically recognized and attacked cancer cells expressing hCLDN1 or hEPHB4 were induced. In HLA-A*02:01 Tg mice to which LNP-eGFP mRNA had been similarly administered three times, as a negative control, only marginal production of IFN-γ was found for any target; hence, it can be understood that the aforementioned production of IFN-γ was specifically induced by administration of LNP-hCLDN1 mRNA or LNP-hEPHB4 mRNA.

As shown in Fig. 45, in spleen cells of the HLA-A*24:02 Tg mice to which LNP-hCLDN1 mRNA or LNP-hEPHB4 mRNA had been administered three times, T cells producing IFN-γ were induced against the mix of hCLDN1 or hEPHB4 short-chain peptides for which peptide-specific IFN-γ production had been found in peptide immunization for HLA-A*24:02 Tg mice, the mix of hCLDN1 or hEPHB4 long-chain peptides for which peptide-specific IFN-γ production had been found in peptide immunization for HLA-A*24:02 Tg mice, the cancer cells MC38 expressing HLA-A*24:02HHD and hCLDN or the cancer cells MCA205 each expressing HLA-A*24:02HHD and hEPHB4, and BMDCs transfected with hCLDN1 mRNA or hEPHB4 mRNA. The IFN-γ productions against the cancer cells MC38 A24HHD hCLDN1, expressing HLA-A*24:02HHD and hCLDN1, and against the cancer cells MCA205 A24HHD hEPHB4, expressing HLA-A*24:02HHD and hEPHB4, were higher than those against the cancer cells MC38 A24HHD mock and against MCA205 A24HHD mock, into each of which HLA-A2402HHD and an empty vector had been introduced, and hence it can be understood that T cells that specifically recognized and attacked cancer cells expressing hCLDN1 or hEPHB4 were induced. The reason for the IFN-γ production against MC38 A24HHD mock or MCA205 A24HHD mock is probably that a mouse CLDN1- or EPHB4-derived peptide was presented by HLA-A*24:02HHD. In HLA-A*24:02 Tg mice to which LNP-eGFP mRNA had been similarly administered three times, as a negative control, only marginal production of IFN-γ was found for any target; hence, it can be understood that the aforementioned production of IFN-γ was specifically induced by administration of LNP-hCLDN1 mRNA or LNP-hEPHB4 mRNA.

### Example 8: Development of common cancer antigen mRNA vaccine

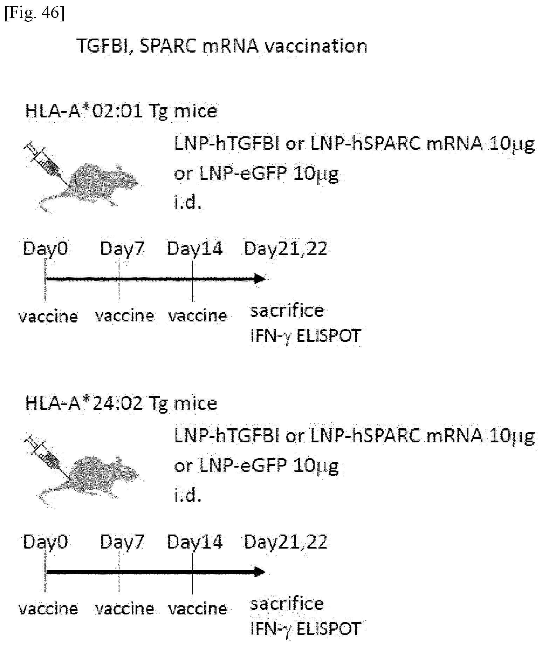

Fig. 46 shows an administration method by triple administration of LNP-hTGFBI mRNA and LNP-hSPARC mRNA.

LNP-hTGFBI mRNA, LNP-hSPARC mRNA, or LNP-eGFP mRNA in an amount of 10 µg was administered intradermally (i.d.) into the base of the tail of HLA-A*02:01 Tg mice and HLA-A*24:02 Tg mice on day 0, day 7, and day 14, three times in total, the mice were dissected on day 21, and antigen-specific productions of IFN-γ were evaluated with an ELISPOT method.

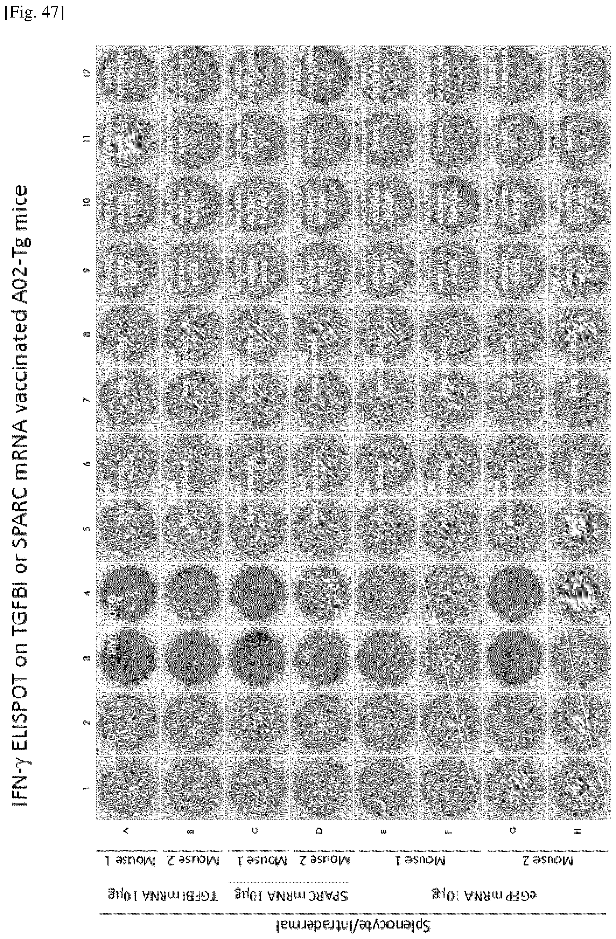

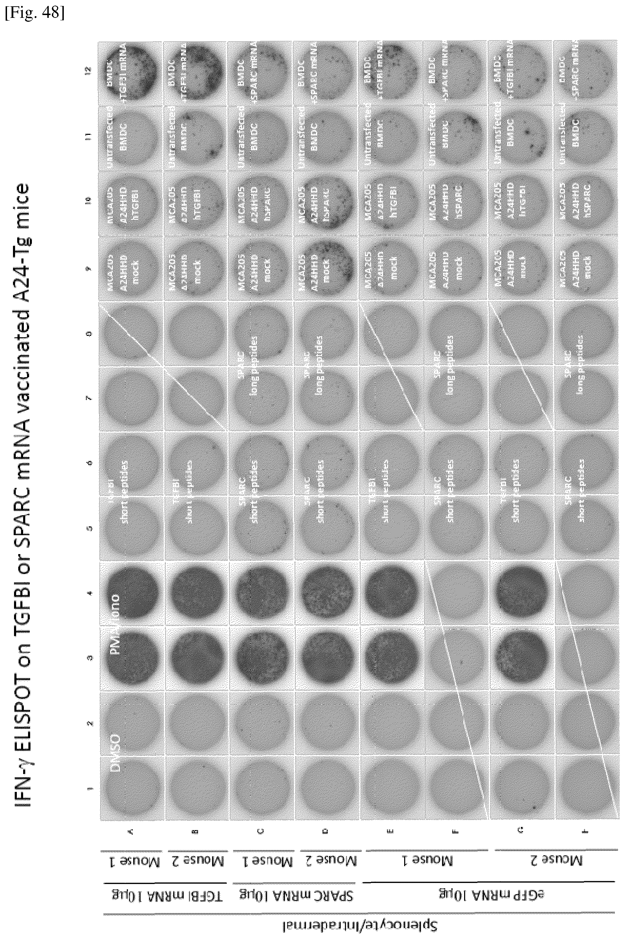

Fig. 47 and Fig. 48 show the results.

As shown in Fig. 47, in spleen cells of the HLA-A*02:01 Tg mice to which LNP-hTGFBI mRNA or LNP-hSPARC mRNA had been administered three times, T cells producing IFN-γ were induced against the mix of hTGFBI or hSPARC short-chain peptides for which peptide-specific IFN-γ production had been found in peptide immunization for HLA-A*02:01 Tg mice, the mix of hTGFBI or hSPARC long-chain peptides for which peptide-specific IFN-γ production had been found in peptide immunization for HLA-A*02:01 Tg mice, the cancer cells MCA205 expressing HLA-A*02:01HHD and hTGFBI or the cancer cells MCA205 expressing HLA-A*02:01HHD and hSPARC, and BMDCs transfected with hTGFBI mRNA or hSPARC mRNA. The IFN-γ productions against the cancer cells MCA205 A02HHD hTGFBI, expressing HLA-A*02:01HHD and hTGFBI, and against the cancer cells MCA205 A02HHD hSPARC, expressing HLA-A*02:01HHD and hSPARC, were significantly higher than that against the cancer cells MCA205 A02HHD mock, to which HLA-A*02:01HHD and an empty vector had been introduced, and hence it can be understood that T cells that specifically recognized and attacked cancer cells expressing hTGFBI or hSPARC were induced. In HLA-A*02:01 Tg mice to which LNP-eGFP mRNA had been similarly administered three times, as a negative control, production of IFN-γ was low for any target; hence, it can be understood that the aforementioned production of IFN-γ was specifically induced by administration of LNP-hTGFBI mRNA or LNP-hSPARC mRNA.

As shown in Fig. 48, in spleen cells of the HLA-A*24:02 Tg mice to which LNP-hTGFBI mRNA or LNP-hSPARC mRNA had been administered three times, T cells producing IFN-γ were induced against the mix of hTGFBI or hSPARC short-chain peptides for which peptide-specific IFN-γ production had been found in peptide immunization for HLA-A*24:02 Tg mice, the mix of hTGFBI or hSPARC long-chain peptides for which peptide-specific IFN-γ production had been found in peptide immunization for HLA-A*24:02 Tg mice, the cancer cells MCA205 expressing HLA-A*24:02HHD and hTGFBI or the cancer cells MCA205 expressing HLA-A*24:02HHD and hSPARC, and BMDCs transfected with hTGFBI mRNA or hSPARC mRNA. The cancer cells MCA205 A02HHD hTGFBI, expressing HLA-A*02:01HHD and hTGFBI, was significantly higher than that to the cancer cells MCA205 A02HHD mock, into which HLA-A*02:01HHD and an empty vector had been introduced, and hence it can be understood that T cells that specifically recognized and attacked cancer cells expressing hTGFBI were induced. The reason for the IFN-γ production against MCA205 A24HHD mock in the mice with administration of LNP-hSPARC mRNA is probably that a mouse SPARC-derived peptide was presented by HLA-A*24:02HHD. In HLA-A*02:01 Tg mice to which LNP-eGFP mRNA had been similarly administered three times, as a negative control, production of IFN-γ was low for any target; hence, it can be understood that the aforementioned production of IFN-γ was specifically induced by administration of LNP-hTGFBI mRNA or LNP-hSPARC mRNA.

### Example 9: Development of common cancer antigen mRNA vaccine

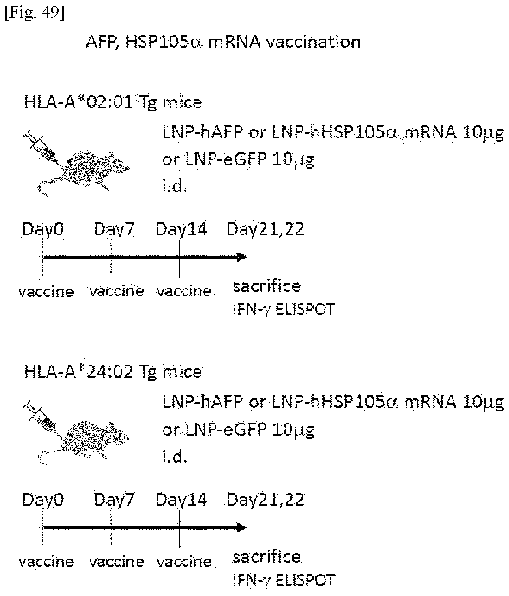

Fig. 49 shows an administration method by triple administration of LNP-hAFP mRNA and LNP-hHSP105α mRNA.

LNP-hAFP mRNA, LNP-hHSP105α mRNA, or LNP-eGFP mRNA in an amount of 10 µg was administered intradermally (i.d.) into the base of the tail of HLA-A*02:01 Tg mice and HLA-A*24:02 Tg mice on day 0, day 7, and day 14, three times in total, the mice were dissected on day 21, and antigen-specific productions of IFN-γ were evaluated with an ELISPOT method.

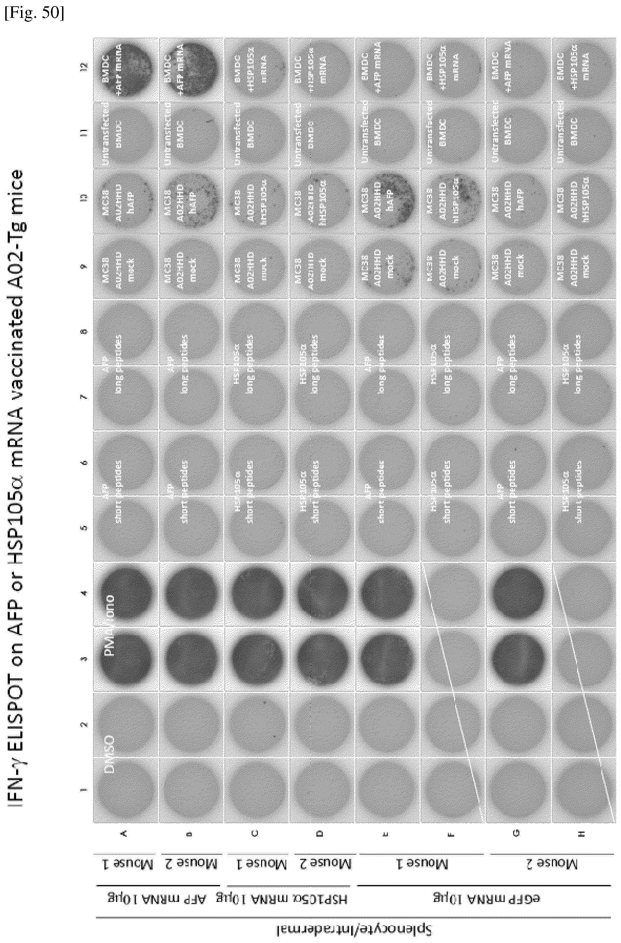

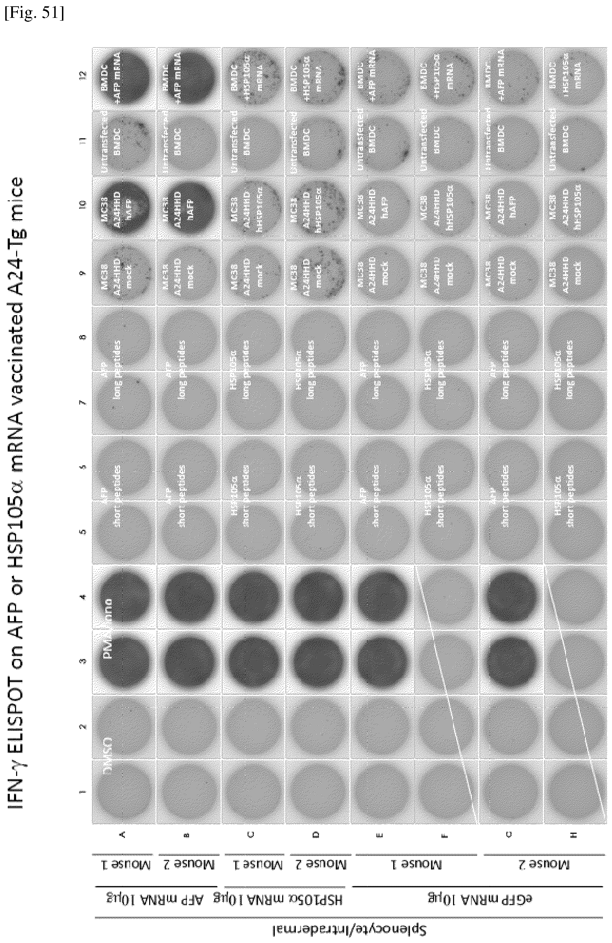

Fig. 50 and Fig. 51 show the results.

As shown in Fig. 50, in spleen cells of the HLA-A*02:01 Tg mice to which LNP-hAFP mRNA or LNP-hHSP105α mRNA had been administered three times, T cells producing IFN-γ were induced against the cancer cells MC38 expressing HLA-A*02:01HHD and hAFP or the cancer cells MC38 expressing HLA-A*02:01HHD and hHSP105α, and BMDCs transfected with hAFP mRNA or hHSP105α mRNA. The IFN-γ productions against the cancer cells MC38 A02HHD hAFP, expressing HLA-A*02:01HHD and hAFP, and the cancer cells MC38 A02HHD hHSP105α, expressing HLA-A*02:01HHD and hHSP105α, were significantly higher than that against the cancer cells MC38 A02HHD mock, into which HLA-A*02:01HHD and an empty vector had been introduced, and hence it can be understood that T cells that specifically recognized and attacked cancer cells expressing hAFP or hHSP105α were induced. In HLA-A*02:01 Tg mice to which LNP-eGFP mRNA had been similarly administered three times, as a negative control, production of IFN-γ was high for MC38 in one individual, but low for BMDCs transfected with any of the antigen mRNAs, and the production of IFN-γ to BMDCs transfected with any of the antigen mRNAs was inferred to be specifically induced by administration of LNP-hAFP mRNA or LNP-hHSP105α mRNA.

As shown in Fig. 51, in spleen cells of the HLA-A*24:02 Tg mice to which LNP-hAFP mRNA or LNP-hHSP105α mRNA had been administered three times, T cells producing IFN-γ were induced against the cancer cells MC38 expressing HLA-A*24:02HHD and hAFP or the cancer cells MC38 expressing HLA-A*24:02HHD and hHSP105α, and BMDCs transfected with hAFP mRNA or hHSP105α mRNA. The IFN-γ production against the cancer cells MC38 A24HHD hAFP, expressing HLA-A*24:02HHD and hAFP, was significantly higher than that against the cancer cells MC38 A24HHD mock, into which HLA-A*24:02HHD and an empty vector had been introduced, and hence it can be understood that T cells that specifically recognized and attacked cancer cells expressing hAFP were induced. The reason why IFN-γ production comparable to that against the cancer cells MC38 A24HHD hHSP105α, expressing HLA-A*24:02HHD and hHSP105α, was found for MC38 A24HHD mock is probably that a mouse HSP105α-derived peptide was presented by HLA-A*24:02HHD. In HLA-A*24:02 Tg mice to which LNP-eGFP mRNA had been similarly administered three times, as a negative control, production of IFN-γ was low for any target; hence, it can be understood that the aforementioned production of IFN-γ was specifically induced by administration of LNP-hAFP mRNA or LNP-hHSP105α mRNA.

### Example 10: Development of common cancer antigen mRNA vaccine

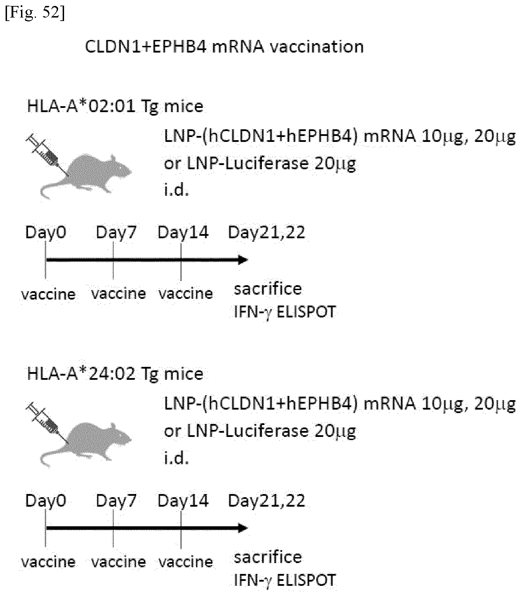

Fig. 52 shows an administration method by triple administration of LNP-(hCLDN1+hEPHB4) mRNA.

LNP-(hCLDN1+hEPHB4) mRNA in an amount of 10 µg or 20 µg or LNP-Luciferase mRNA in an amount of 20 µg was administered intradermally (i.d.) into the base of the tail of HLA-A*02:01 Tg mice and HLA-A*24:02 Tg mice on day 0, day 7, and day 14, three times in total, the mice were dissected on day 21, and antigen-specific productions of IFN-γ were evaluated with an ELISPOT method.

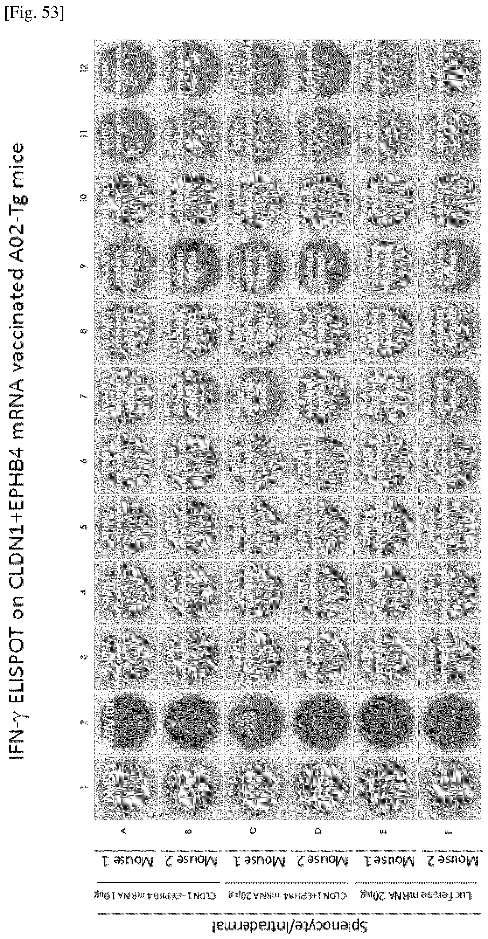

Fig. 53 to Fig. 56 show the results.

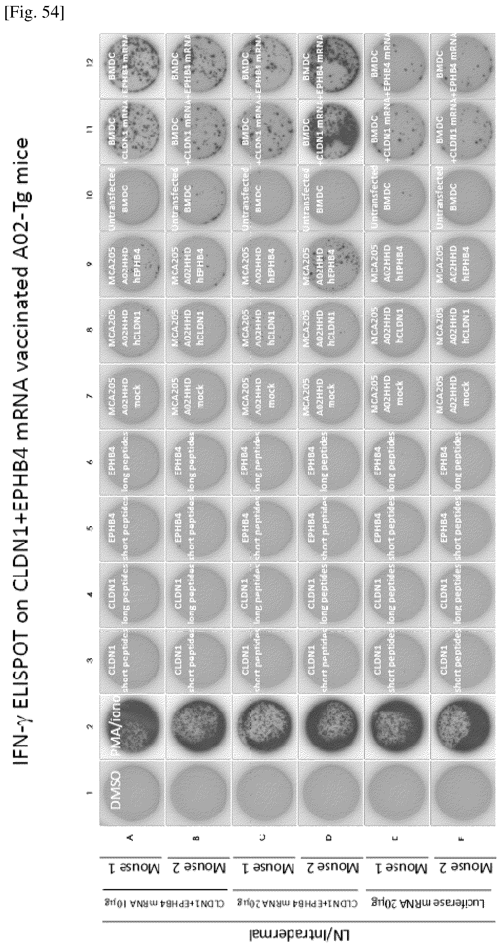

As shown in Fig. 53 and Fig. 54, in spleen cells and inguinal lymph nodes of the HLA-A*02:01 Tg mice to which LNP-(hCLDN1+hEPHB4) mRNA had been administered three times, T cells producing IFN-γ were induced against the cancer cells MCA205 expressing HLA-A*02:01HHD and hCLDN1 or the cancer cells MCA205 expressing HLA-A*02:01HHD and hEPHB4, and BMDCs transfected with hCLDN1 mRNA or hEPHB4 mRNA. The IFN-γ production against the cancer cells MCA205 A02HHD hEPHB4, expressing HLA-A*02:01HHD and hEPHB4, was significantly higher than that against the cancer cells MCA205 A02HHD mock, into which HLA-A*02:01HHD and an empty vector had been introduced, and hence it can be understood that T cells that specifically recognized and attacked cancer cells expressing hEPHB4 were induced. The reason why IFN-γ production comparable to that against the cancer cells MCA205 A02HHD hCLDN1, expressing HLA-A*02:01HHD and hCLDN1, was found for MCA205 A02HHD mock is probably that a mouse CLDN1- or EPHB4-derived peptide was presented by HLA-A*02:01HHD. In HLA-A*02:01 Tg mice to which LNP-Luciferase mRNA had been similarly administered three times, as a negative control, production of IFN-γ was relatively low for any target; and hence, it can be understood that the aforementioned production of IFN-γ was induced by administration of LNP-(hCLDN1+hEPHB4) mRNA.

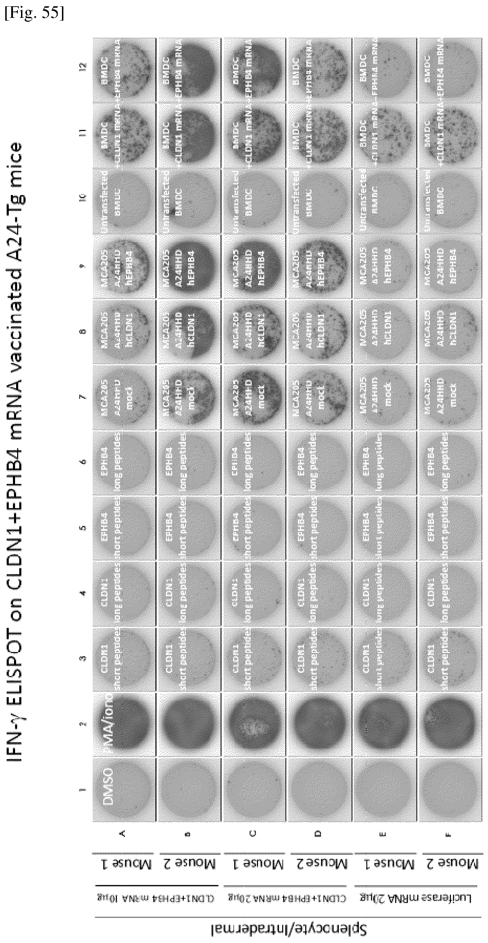

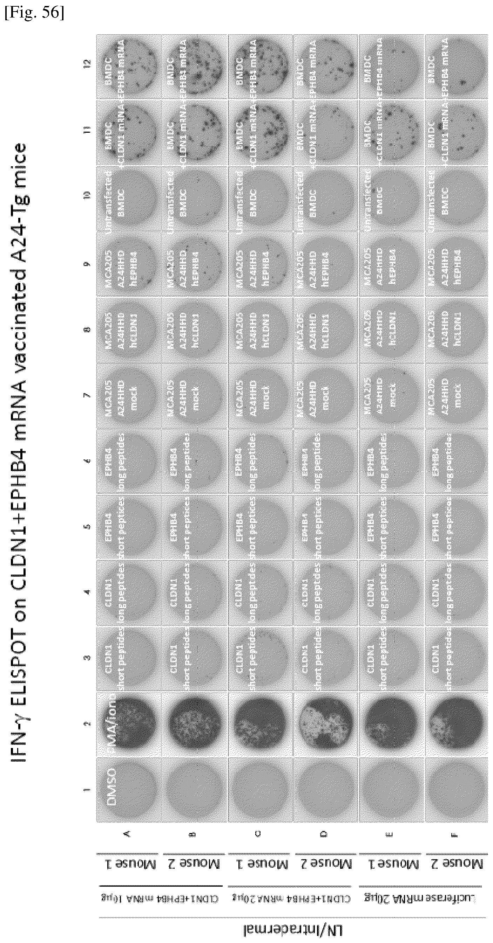

As shown in Fig. 55 and Fig. 56, in spleen cells and inguinal lymph nodes of the HLA-A*24:02 Tg mice to which LNP-(hCLDN1+hEPHB4) mRNA had been administered three times, T cells producing IFN-γ were induced against the cancer cells MCA205 expressing HLA-A*24:02HHD and hCLDN1 or the cancer cells MCA205 expressing HLA-A*24:02HHD and hEPHB4, and BMDCs transfected with hCLDN1 mRNA or hEPHB4 mRNA. The IFN-γ production against the cancer cells MCA205 A24HHD hEPHB4, expressing HLA-A*24:02HHD and hEPHB4, was significantly higher than that against the cancer cells MCA205 A24HHD mock, into which HLA-A*24:02HHD and an empty vector had been introduced, and hence it can be understood that T cells that specifically recognized and attacked cancer cells expressing hEPHB4 were induced. The reason why IFN-γ production comparable to that against the cancer cells MCA205 A24HHD hCLDN1, expressing HLA-A*24:02HHD and hCLDN1, was found for MCA205 A02HHD mock is probably that a mouse CLDN1- or EPHB4-derived peptide was presented by HLA-A*24:02HHD. In HLA-A*02:01 Tg mice to which LNP-Luciferase mRNA had been similarly administered three times, as a negative control, production of IFN-γ was relatively low for any target; hence, it can be understood that the aforementioned production of IFN-γ was specifically induced by administration of LNP-(hCLDN1+hEPHB4) mRNA.

### <Amino acid sequences for mRNAs>

>hGPC3_isoform_2_NP_004475 (SEQ ID No. 81)
>hROBO1_isoform_X5_XP_006713340 (hROBO1 of intracytoplasmic type) (SEQ ID NO: 82) 
MIAEPAHFYLFGLICLCSGSRLRQEDFPPRIVEHPSDLIVSKGEPATLNCKAEGRP
TPTIEWYKGGERVETDKDDPRSHRMLLPSGSLFFLRIVHGRKSRPDEGVYVCVA
RNYLGEAVSHNASLEVAILRDDFRQNPSDVMVAVGEPAVMECQPPRGHPEPTIS
 
WKKDGSPLDDKDERITIRGGKLMITYTRKSDAGKYVCVGTNMVGERESEVAEL
TVLERPSFVKRPSNLAVTVDDSAEFKCEARGDPVPTVRWRKDDGELPKSRYEIR
DDHTLKIRKVTAGDMGSYTCVAENMVGKAEASATLTVQVGSEPPHFVVKPRDQ
VVALGRTVTFQCEATGNPQPAIFWRREGSQNLLFSYQPPQSSSRFSVSQTGDLTIT
NVQRSDVGYYICQTLNVAGSIITKAYLEVTDVIADRPPPVIRQGPVNQTVAVDGT
FVLSCVATGSPVPTILWRKDGVLVSTQDSRIKQLENGVLQIRYAKLGDTGRYTCI
ASTPSGEATWSAYIEVQEFGVPVQPPRPTDPNLIPSAPSKPEVTDVSRNTVTLSW
QPNLNSGATPTSYIIEAFSHASGSSWQTVAENVKTETSAIKGLKPNAIYLFLVRAA
NAYGISDPSQISDPVKTQDVLPTSQGVDHKQVQRELGNAVLHLHNPTVLSSSSIE
VHWTVDQQSQYIQGYKILYRPSGANHGESDWLVFEVRTPAKNSVVIPDLRKGV
NYEIKARPFFNEFQGADSEIKFAKTLEEAPSAPPQGVTVSKNDGNGTAILVSWQP
PPEDTQNGMVQEYKVWCLGNETRYHINKTVDGSTFSVVIPFLVPGIRYSVEVAA
STGAGSGVKSEPQFIQLDAHGNPVSPEDQVSLAQQISDVVKQPAFIAGIGAACWI
ILMVFSIWLYRHRKKRNGLTSTYAGIRKVPSFTFTPTVTYQRGGEAVSSGGRPGL
LNISEPAAQPWLADTWPNTGNNHNDCSISCCTAGNGNSDSNLTTYSRPADCIAN
YNNQLDNKQTNLMLPESTVYGDVDLSNKINEMKTFNSPNLKDGRFVNPSGQPT
PYATTQLIQSNLSNNMNNGSGDSGEKHWKPLGQQKQEVAPVQYNIVEQNKLNK
DYRANDTVPPTIPYNQSYDQNTGGSYNSSDRGSSTSGSQGHKKGARTPKVPKQ
GGMNWADLLPPPPAHPPPHSNSEEYNISVDESYDQEMPCPVPPARMYLQQDELE
EEEDERGPTPPVRGAASSPAAVSYSHQSTATLTPSPQEELQPMLQDCPEETGHMQ
HQPDRRRQPVSPPPPPRPISPPHTYGYISGPLVSDMDTDAPEEEEDEADMEVAKM
QTRRLLLRGLEQTPASSVGDLESSVTGSMINGWGSASEEDNISSGRSSVSSSDGS
FFTDADFAQAVAAAAEYAGLKVARRQMQDAAGRRHFHASQCPRPTSPVSTDSN
MSAAVMQKTRPAKKLKHQPGHLRRETYTDDLPPPPVPPPAIKSPTAQSKTQLEV
RPVVVPKLPSMDARTDRSSDRKGSSYKGREVLDGRQVVDMRTNPGDPREAQE
QQNDGKGRGNKAAKRDLPPAKTHLIQEDILPYCRPTFPTSNNPRDPSSSSSMSSR
GSGSRQREQANVGRRNIAEMQVLGGYERGEDNNEELEETES
>hROBO1_isoform_a_NP_002932 (hROBO1 of membrane-delimited type) (SEQ ID NO: 83)
>hEPHB4_NP_004435 (SEQ ID No. 84)
>hCLDN1_NP_066924 (SEQ ID No. 85) 
MANAGLQLLGFILAFLGWIGAIVSTALPQWRIYSYAGDNIVTAQAMYEGLWMS
CVSQSTGQIQCKVFDSLLNLSSTLQATRALMVVGILLGVIAIFVATVGMKCMKC
LEDDEVQKMRMAVIGGAIFLLAGLAILVATAWYGNRIVQEFYDPMTPVNARYEF
GQALFTGWAAASLCLLGGALLCCSCPRKTTSYPTPRPYPKPAPSSGKDYV
>hSLC7A5_NP_003477 (SEQ ID No. 86) 
MAGAGPKRRALAAPAAEEKEEAREKMLAAKSADGSAPAGEGEGVTLQRNITLL
NGVAIIVGTIIGSGIFVTPTGVLKEAGSPGLALVVWAACGVFSIVGALCYAELGTT
ISKSGGDYAYMLEVYGSLPAFLKLWIELLIIRPSSQYIVALVFATYLLKPLFPTCPV
PEEAAKLVACLCVLLLTAVNCYSVKAATRVQDAFAAAKLLALALIILLGFVQIGK
GDVSNLDPNFSFEGTKLDVGNIVLALYSGLFAYGGWNYLNFVTEEMINPYRNLP
LAIIISLPIVTLVYVLTNLAYFTTLSTEQMLSSEAVAVDFGNYHLGVMSWIIPVFVG
LSCFGSVNGSLFTSSRLFFVGSREGHLPSILSMIHPQLLTPVPSLVFTCVMTLLYAF
SKDIFSVINFFSFFNWLCVALAIIGMIWLRHRKPELERPIKVNLALPVFFILACLFLI
AVSFWKTPVECGIGFTIILSGLPVYFFGVWWKNKPKWLLQGIFSTTVLCQKLMQ
VVPQET
>hAFP_isoform_1_NP_001125 (SEQ ID No. 87)
>hTGFBI_NP_000349 (SEQ ID No. 88) 
MALFVRLLALALALALALGPAATLAGPAKSPYQLVLQHSRLRGRQHGPNVCAVQK
VIGTNRKYFTNCKQWYQRKICGKSTVISYECCPGYEKVPGEKGCPAALPLSNLY
ETLGVVGSTTTQLYTDRTEKLRPEMEGPGSFTIFAPSNEAWASLPAEVLDSLVSN
VNIELLNALRYHMVGRRVLTDELKHGMTLTSMYQNSNIQIHHYPNGIVTVNCA
RLLKADHHATNGVVHLIDKVISTITNNIQQIIEIEDTFETLRAAVAASGLNTMLEG
NGQYTLLAPTNEAFEKIPSETLNRILGDPEALRDLLNNHILKSAMCAEAIVAGLS
VETLEGTTLEVGCSGDMLTINGKAIISNKDILATNGVIHYIDELLIPDSAKTLFELA
AESDVSTAIDLFRQAGLGNHLSGSERLTLLAPLNSVFKDGTPPIDAHTRNLLRNH
IIKDQLASKYLYHGQTLETLGGKKLRVFVYRNSLCIENSCIAAHDKRGRYGTLFT
MDRVLTPPMGTVMDVLKGDNRFSMLVAAIQSAGLTETLNREGVYTVFAPTNEA
FRALPPRERSRLLGDAKELANILKYHIGDEILVSGGIGALVRLKSLQGDKLEVSL
KNNVVSVNKEPVAEPDIMATNGVVHVITNVLQPPANRPQERGDELADSALEIFK
QASAFSRASQRSVRLAPVYQKLLERMKH
>hSPARC_isoform_1_NP_003109 (SEQ ID No. 89) 
MRAWIFFLLCLAGRALAAPQQEALPDETEVVEETVAEVTEVSVGANPVQVEVG
EFDDGAEETEEEVVAENPCQNHHCKHGKVCELDENNTPMCVCQDPTSCPAPIG
EFEKVCSNDNKTFDSSCHFFATKCTLEGTKKGHKLHLDYIGPCKYIPPCLDSELT
EFPLRMRDWLKNVLVTLYERDEDNNLLTEKQKLRVKKIHENEKRLEAGDHPVE
LLARDFEKNYNMYIFPVHWQFGQLDQHPIDGYLSHTELAPLRAPLIPMEHCTTR
FFETCDLDNDKYIALDEWAGCFGIKQKDIDKDLVI
>hFOXM1_isoform_2_NP_068772 (SEQ ID No. 90) 
MKTSPRRPLILKRRRLPLPVQNAPSETSEEEPKRSPAQQESNQAEASKEVAESNS
CKFPAGIKIINHPTMPNTQVVAIPNNANIHSIITALTAKGKESGSSGPNKFILISCGG
APTQPPGLRPQTQTSYDAKRTEVTLETLGPKPAARDVNLPRPPGALCEQKRETC
ADGEAAGCTINNSLSNIQWLRKMSSDGLGSRSIKQEMEEKENCHLEQRQVKVE
EPSRPSASWQNSVSERPPYSYMAMIQFAINSTERKRMTLKDIYTWIEDHFPYFKH
 
IAKPGWKNSIRHNLSLHDMFVRETSANGKVSFWTIHPSANRYLTLDQVFKPLDP
GSPQLPEHLESQQKRPNPELRRNMTIKTELPLGARRKMKPLLPRVSSYLVPIQFP
VNQSLVLQPSVKVPLPLAASLMSSELARHSKRVRIAPKVLLAEEGIAPLSSAGPG
KEEKLLFGEGFSPLLPVQTIKEEEIQPGEEMPHLARPIKVESPPLEEWPSPAPSFKE
ESSHSWEDSSQSPTPRPKKSYSGLRSPTRCVSEMLVIQHRERRERSRSRRKQHLL
PPCVDEPELLFSEGPSTSRWAAELPFPADSSDPASQLSYSQEVGGPFKTPIKETLPI
SSTPSKSVLPRTPESWRLTPPAKVGGLDFSPVQTSQGASDPLPDPLGLMDLSTTP
LQSAPPLESPQRLLSSEPLDLISVPFGNSSPSDIDVPKPGSPEPQVSGLAANRSLTE
GLVLDTMNDSLSKILLDISFPGLDEDPLGPDNINWSQFIPELQ
>hHSPH1_isoform_1_NP_006635 (SEQ ID No. 91)

MSVVGLDVGSQSCYIAVARAGGIETIANEFSDRCTPSVISFGSKNRTIGVAAKNQ
QITHANNTVSNFKRFHGRAFNDPFIQKEKENLSYDLVPLKNGGVGIKVMYMGE
EHLFSVEQITAMLLTKLKETAENSLKKPVTDCVISVPSFFTDAERRSVLDAAQIV
GLNCLRLMNDMTAVALNYGIYKQDLPSLDEKPRIVVFVDMGHSAFQVSACAFN
KGKLKVLGTAFDPFLGGKNFDEKLVEHFCAEFKTKYKLDAKSKIRALLRLYQEC
EKLKKLMSSNSTDLPLNIECFMNDKDVSGKMNRSQFEELCAELLQKIEVPLYSL
LEQTHLKVEDVSAVEIVGGATRIPAVKERIAKFFGKDISTTLNADEAVARGCALQ
CAILSPAFKVREFSVTDAVPFPISLIWNHDSEDTEGVHEVFSRNHAAPFSKVLTFL
RRGPFELEAFYSDPQGVPYPEAKIGRFVVQNVSAQKDGEKSRVKVKVRVNTHG
IFTISTASMVEKVPTEENEMSSEADMECLNQRPPENPDTDKNVQQDNSEAGTQP
QVQTDAQQTSQSPPSPELTSEENKIPDADKANEKKVDQPPEAKKPKIKVVNVEL
PIEANLVWQLGKDLLNMYIETEGKMIMQDKLEKERNDAKNAVEEYVYEFRDK
LCGPYEKFICEQDHQNFLRLLTETEDWLYEEGEDQAKQAYVDKLEELMKIGTPV
KVRFQEAEERPKMFEELGQRLQHYAKIAADFRNKDEKYNHIDESEMKKVEKSV
NEVMEWMNNVMNAQAKKSLDQDPVVRAQEIKTKIKELNNTCEPVVTQPKPKI
ESPKLERTPNGPNIDKKEEDLEDKNNFGAEPPHQNGECYPNEKNSVNMDLD

### <Original sequence of mRNA vaccine before codon optimization>

>hGPC3_var2_NM_004484 (SEQ ID No. 92) 
ATGGCCGGGACCGTGCGCACCGCGTGCTTGGTGGTGGCGATGCTGCTCAGCT
TGGACTTCCCGGGACAGGCGCAGCCCCCGCCGCCGCCGGACGCCACCT
GTCACCAAGTCCGCTCCTTCTTCCAGAGACTGCAGCCCGGACTCAAGTGGGT
GCCAGAAACTCCCGTGCCAGGATCAGATTTGCAAGTATGTCTCCCTAAGGGC
CCAACATGCTGCTCAAGAAAGATGGAAGAAAAATACCAACTAACAGCACGA
TTGAACATGGAACAGCTGCTTCAGTCTGCAAGTATGGAGCTCAAGTTCTTAA
 
TTATTCAGAATGCTGCGGTTTTCCAAGAGGCCTTTGAAATTGTTGTTCGCCAT
GCCAAGAACTACACCAATGCCATGTTCAAGAACAACTACCCAAGCCTGACTC
CACAAGCTTTTGAGTTTGTGGGTGAATTTTTCACAGATGTGTCTCTCTACATC
TTGGGTTCTGACATCAATGTAGATGACATGGTCAATGAATTGTTTGACAGCCT
GTTTCCAGTCATCTATACCCAGCTAATGAACCCAGGCCTGCCTGATTCAGCCT
TGGACATCAATGAGTGCCTCCGAGGAGCAAGACGTGACCTGAAAGTATTTGG
GAATTTCCCCAAGCTTATTATGACCCAGGTTTCCAAGTCACTGCAAGTCACTA
GGATCTTCCTTCAGGCTCTGAATCTTGGAATTGAAGTGATCAACACAACTGAT
CACCTGAAGTTCAGTAAGGACTGTGGCCGAATGCTCACCAGAATGTGGTACT
GCTCTTACTGCCAGGGACTGATGATGGTTAAACCCTGTGGCGGTTACTGCAAT
GTGGTCATGCAAGGCTGTATGGCAGGTGTGGTGGAGATTGACAAGTACTGGA
GAGAATACATTCTGTCCCTTGAAGAACTTGTGAATGGCATGTACAGAATCTAT
GACATGGAGAACGTACTGCTTGGTCTCTTTTCAACAATCCATGATTCTATCCA
GTATGTCCAGAAGAATGCAGGAAAGCTGACCACCACTATTGGCAAGTTATGT
GCCCATTCTCAACAACGCCAATATAGATCTGCTTATTATCCTGAAGATCTCTTT
ATTGACAAGAAAGTATTAAAAGTTGCTCATGTAGAACATGAAGAAACCTTAT
CCAGCCGAAGAAGGGAACTAATTCAGAAGTTGAAGTCTTTCATCAGCTTCTA
TAGTGCTTTGCCTGGCTACATCTGCAGCCATAGCCCTGTGGCGGAAAACGAC
ACCCTTTGCTGGAATGGACAAGAACTCGTGGAGAGATACAGCCAAAAGGCA
GCAAGGAATGGAATGAAAAACCAGTTCAATCTCCATGAGCTGAAAATGAAG
GGCCCTGAGCCAGTGGTCAGTCAAATTATTGACAAACTGAAGCACATTAACC
AGCTCCTGAGAACCATGTCTATGCCCAAAGGTAGAGTTCTGGATAAAAACCT
GGATGAGGAAGGGTTTGAAAGTGGAGACTGCGGTGATGATGAAGATGAGTG
CATTGGAGGCTCTGGTGATGGAATGATAAAAGTGAAGAATCAGCTCCGCTTC
CTTGCAGAACTGGCCTATGATCTGGATGTGGATGATGCGCCTGGAAACAGTC
AGCAGGCAACTCCGAAGGACAACGAGATAAGCACCTTTCACAACCTCGGGA
ACGTTCATTCCCCGCTGAAGCTTCTCACCAGCATGGCCATCTCGGTGGTGTGC
TTCTTCTTCCTGGTGCACTGA
>hROBO1_varX6_cloned_from_SW480 (intracytoplasmic type) (SEQ ID NO: 93) 
ATGATTGCGGAGCCCGCTCACTTTTACCTGTTTGGATTAATATGTCTCTGTTCA
GGCTCCCGTCTTCGTCAGGAAGATTTTCCACCTCGCATTGTTGAACACCCTTC
AGACCTGATTGTCTCAAAAGGAGAACCTGCAACTTTGAACTGCAAAGCTGA
AGGCCGCCCCACACCCACTATTGAATGGTACAAAGGGGGAGAGAGAGTGGA
GACAGACAAAGATGACCCTCGCTCACACCGAATGTTGCTGCCGAGTGGATCT
TTATTTTTCTTACGTATAGTACATGGACGGAAAAGTAGACCTGATGAAGGAGT
 
CTATGTCTGTGTAGCAAGGAATTACCTTGGAGAGGCTGTGAGCCACAATGCA
TCGCTGGAAGTAGCCATACTTCGGGATGACTTCAGACAAAACCCTTCGGATG
TCATGGTTGCAGTAGGAGAGCCTGCAGTAATGGAATGCCAACCTCCACGAGG
CCATCCTGAGCCCACCATTTCATGGAAGAAAGATGGCTCTCCACTGGATGATA
AAGATGAAAGAATAACTATACGAGGAGGAAAGCTCATGATCACTTACACCCG
TAAAAGTGACGCTGGCAAATATGTTTGTGTTGGTACCAATATGGTTGGGGAAC
GTGAGAGTGAAGTAGCCGAGCTGACTGTCTTAGAGAGACCATCATTTGTGAA
GAGACCCAGTAACTTGGCAGTAACTGTGGATGACAGTGCAGAATTTAAATGT
GAGGCCCGAGGTGACCCTGTACCTACAGTACGATGGAGGAAAGATGATGGA
GAGCTGCCCAAATCCAGATATGAAATCCGAGATGATCATACCTTGAAAATTAG
GAAGGTGACAGCTGGTGACATGGGTTCATACACTTGTGTTGCAGAAAATATG
GTGGGCAAAGCTGAAGCATCTGCTACTCTGACTGTTCAAGTTGGGTCTGAAC
CTCCACATTTTGTTGTGAAACCCCGTGACCAGGTTGTTGCTTTGGGACGGAC
TGTAACTTTTCAGTGTGAAGCAACCGGAAATCCTCAACCAGCTATTTTCTGG
AGGAGAGAAGGGAGTCAGAATCTACTTTTCTCATATCAACCACCACAGTCAT
CCAGCCGATTTTCAGTCTCCCAGACTGGCGACCTCACAATTACTAATGTCCAG
CGATCTGATGTTGGTTATTACATCTGCCAGACTTTAAATGTTGCTGGAAGCATC
ATCACAAAGGCATATTTGGAAGTTACAGATGTGATTGCAGATCGGCCTCCCCC
AGTTATTCGACAAGGTCCTGTGAATCAGACTGTAGCCGTGGATGGCACTTTC
GTCCTCAGCTGTGTGGCCACAGGCAGTCCAGTGCCCACCATTCTGTGGAGAA
AGGATGGAGTCCTCGTTTCAACCCAAGACTCTCGAATCAAACAGTTGGAGA
ATGGAGTACTGCAGATCCGATATGCTAAGCTGGGTGATACTGGTCGGTACACC
TGCATTGCATCAACCCCCAGTGGTGAAGCAACATGGAGTGCTTACATTGAAG
TTCAAGAATTTGGAGTTCCAGTTCAGCCTCCAAGACCTACTGACCCAAATTT
AATCCCTAGTGCCCCATCAAAACCTGAAGTGACAGATGTCAGCAGAAATACA
GTCACATTATCGTGGCAACCAAATTTGAATTCAGGAGCAACTCCAACATCTTA
TATTATAGAAGCCTTCAGCCATGCATCTGGTAGCAGCTGGCAGACCGTAGCAG
AGAATGTGAAAACAGAAACATCTGCCATTAAAGGACTCAAACCTAATGCAAT
TTACCTTTTCCTTGTGAGGGCAGCTAATGCATATGGAATTAGTGATCCAAGCC
AAATATCAGATCCAGTGAAAACACAAGATGTCCTACCAACAAGTCAGGGGGT
GGACCACAAGCAGGTCCAGAGAGAGCTGGGAAATGCTGTTCTGCACCTCCA
CAACCCCACCGTCCTTTCTTCCTCTTCCATCGAAGTGCACTGGACAGTAGATC
AACAGTCTCAGTATATACAAGGATATAAAATTCTCTATCGGCCATCTGGAGCC
AACCACGGAGAATCAGACTGGTTAGTTTTTGAAGTGAGGACGCCAGCCAAA
AACAGTGTGGTAATCCCTGATCTCAGAAAGGGAGTCAACTATGAAATTAAGG
CTCGCCCTTTTTTTAATGAATTTCAAGGAGCAGATAGTGAAATCAAGTTTGCC
 
AAAACCCTGGAAGAAGCACCCAGTGCCCCACCCCAAGGTGTAACTGTATCC
AAGAATGATGGAAACGGAACTGCAATTCTAGTTAGTTGGCAGCCACCTCCAG
AAGACACTCAAAATGGAATGGTCCAAGAGTATAAGGTTTGGTGTCTGGGCAA
TGAAACTCGATACCACATCAACAAAACAGTGGATGGTTCCACCTTTTCCGTG
GTCATTCCCTTTCTTGTTCCTGGAATCCGATACAGTGTGGAAGTGGCAGCCAG
CACTGGGGCTGGGTCTGGGGTAAAGAGTGAGCCTCAGTTCATCCAGCTGGAT
GCCCATGGAAACCCTGTGTCACCTGAGGACCAAGTCAGCCTCGCTCAGCAG
ATTTCAGATGTGGTGAAGCAGCCGGCCTTCATAGCAGGTATTGGAGCAGCCT
GTTGGATCATCCTCATGGTCTTCAGCATCTGGCTTTATCGACACCGCAAGAAG
AGAAACGGACTTACTAGTACCTACGCGGGTATCAGAAAAGTCCCGTCTTTTA
CCTTCACACCAACAGTAACTTACCAGAGAGGAGGCGAAGCTGTCAGCAGTG
GAGGGAGGCCTGGACTTCTCAACATCAGTGAACCTGCCGCGCAGCCATGGC
TGGCAGACACGTGGCCTAATACTGGCAACAACCACAATGACTGCTCTATCAG
CTGCTGCACGGCAGGCAATGGAAACAGCGACAGCAACCTCACTACCTACAG
TCGCCCAGCTGATTGTATAGCAAATTATAACAACCAACTGGATAACAAACAA
ACAAATCTGATGCTCCCTGAGTCAACTGTTTATGGTGATGTGGACCTTAGTAA
CAAAATCAATGAGATGAAAACCTTCAATAGCCCAAATCTGAAGGATGGGCGT
TTTGTCAATCCATCAGGGCAGCCTACTCCTTACGCCACCACTCAGCTCATCCA
GTCAAACCTCAGCAACAACATGAACAATGGCAGCGGGGACTCTGGCGAGAA
GCACTGGAAACCACTGGGACAGCAGAAACAAGAAGTGGCACCAGTTCAGTA
CAACATCGTGGAGCAAAACAAGCTGAACAAAGATTATCGAGCAAATGACAC
AGTTCCTCCAACTATCCCATACAACCAATCATACGACCAGAACACAGGAGGA
TCCTACAACAGCTCAGACCGGGGCAGTAGTACATCTGGGAGTCAGGGGCAC
AAGAAAGGGGCAAGAACACCCAAGGTACCAAAACAGGGTGGCATGAACTG
GGCAGACCTGCTTCCTCCTCCCCCAGCACATCCTCCTCCACACAGCAATAGC
GAAGAGTACAACATTTCTGTAGATGAAAGCTATGACCAAGAAATGCCATGTC
CCGTGCCACCAGCAAGGATGTATTTGCAACAAGATGAATTAGAAGAGGAGG
AAGATGAACGAGGCCCCACTCCCCCTGTTCGGGGAGCAGCTTCTTCTCCAGC
TGCCGTGTCCTATAGCCATCAGTCCACTGCCACTCTGACTCCCTCCCCACAGG
AAGAACTCCAGCCCATGTTACAGGATTGTCCAGAGGAGACTGGCCACATGCA
GCACCAGCCCGACAGGAGACGGCAGCCTGTGAGTCCTCCTCCACCACCACG
GCCGATCTCCCCTCCACATACCTATGGCTACATTTCAGGACCCCTGGTCTCAG
ATATGGATACGGATGCGCCAGAAGAGGAAGAAGACGAAGCCGACATGGAGG
TAGCCAAGATGCAAACCAGAAGGCTTTTGTTACGTGGGCTTGAGCAGACACC
TGCCTCCAGTGTTGGGGACCTGGAGAGCTCTGTCACGGGGTCCATGATCAAC
GGCTGGGGCTCAGCCTCAGAGGAGGACAACATTTCCAGCGGACGCTCCAGT
 
GTTAGTTCTTCGGACGGCTCCTTTTTCACTGATGCTGACTTTGCCCAGGCAGT
CGCAGCAGCGGCAGAGTATGCTGGTCTGAAAGTAGCACGACGGCAAATGCA
GGATGCTGCTGGTCGTCGACATTTTCATGCGTCTCAGTGCCCTAGGCCCACAA
GTCCCGTGTCTACAGACAGCAACATGAGTGCCGCCGTAATGCAGAAAACCA
GACCAGCCAAGAAACTGAAACACCAGCCAGGACATCTGCGCAGAGAAACCT
ACACAGATGATCTTCCACCACCTCCTGTGCCGCCACCTGCTATAAAGTCACCT
ACTGCCCAATCCAAGACACAGCTGGAAGTACGACCTGTAGTGGTGCCAAAA
CTCCCTTCTATGGATGCAAGAACAGACAGATCATCAGACAGAAAAGGAAGC
AGTTACAAGGGGAGAGAAGTGTTGGATGGAAGACAGGTTGTTGACATGCGA
ACAAATCCAGGTGATCCCAGAGAAGCACAGGAACAGCAAAATGACGGGAA
AGGACGTGGAAACAAGGCAGCAAAACGAGACCTTCCACCAGCAAAGACTC
ATCTCATCCAAGAGGATATTCTACCTTATTGTAGACCTACTTTTCCAACATCAA
ATAATCCCAGAGATCCCAGTTCCTCAAGCTCAATGTCATCAAGAGGATCAGG
AAGCAGACAAAGAGAACAAGCAAATGTAGGTCGAAGAAATATTGCAGAAAT
GCAGGTACTTGGAGGATATGAAAGAGGAGAAGATAATAATGAAGAATTAGAG
GAAACTGAAAGCTGA
>hROBO1_var1_NM_002941 (membrane-delimited type) (SEQ ID NO: 94) 
ATGAAATGGAAACATGTTCCTTTTTTGGTCATGATATCACTCCTCAGCTTATCC
CCAAATCACCTGTTTCTGGCCCAGCTTATTCCAGACCCTGAAGATGTAGAGA
GGGGGAACGACCACGGGACGCCAATCCCCACCTCTGATAACGATGACAATTC
GCTGGGCTATACAGGCTCCCGTCTTCGTCAGGAAGATTTTCCACCTCGCATTG
TTGAACACCCTTCAGACCTGATTGTCTCAAAAGGAGAACCTGCAACTTTGAA
CTGCAAAGCTGAAGGCCGCCCCACACCCACTATTGAATGGTACAAAGGGGG
AGAGAGAGTGGAGACAGACAAAGATGACCCTCGCTCACACCGAATGTTGCT
GCCGAGTGGATCTTTATTTTTCTTACGTATAGTACATGGACGGAAAAGTAGAC
CTGATGAAGGAGTCTATGTCTGTGTAGCAAGGAATTACCTTGGAGAGGCTGT
GAGCCACAATGCATCGCTGGAAGTAGCCATACTTCGGGATGACTTCAGACAA
AACCCTTCGGATGTCATGGTTGCAGTAGGAGAGCCTGCAGTAATGGAATGCC
AACCTCCACGAGGCCATCCTGAGCCCACCATTTCATGGAAGAAAGATGGCTC
TCCACTGGATGATAAAGATGAAAGAATAACTATACGAGGAGGAAAGCTCATG
ATCACTTACACCCGTAAAAGTGACGCTGGCAAATATGTTTGTGTTGGTACCAA
TATGGTTGGGGAACGTGAGAGTGAAGTAGCCGAGCTGACTGTCTTAGAGAG
ACCATCATTTGTGAAGAGACCCAGTAACTTGGCAGTAACTGTGGATGACAGT
GCAGAATTTAAATGTGAGGCCCGAGGTGACCCTGTACCTACAGTACGATGGA
GGAAAGATGATGGAGAGCTGCCCAAATCCAGATATGAAATCCGAGATGATCA
 
TACCTTGAAAATTAGGAAGGTGACAGCTGGTGACATGGGTTCATACACTTGT
GTTGCAGAAAATATGGTGGGCAAAGCTGAAGCATCTGCTACTCTGACTGTTC
AAGAACCTCCACATTTTGTTGTGAAACCCCGTGACCAGGTTGTTGCTTTGGG
ACGGACTGTAACTTTTCAGTGTGAAGCAACCGGAAATCCTCAACCAGCTATT
TTCTGGAGGAGAGAAGGGAGTCAGAATCTACTTTTCTCATATCAACCACCAC
AGTCATCCAGCCGATTTTCAGTCTCCCAGACTGGCGACCTCACAATTACTAAT
GTCCAGCGATCTGATGTTGGTTATTACATCTGCCAGACTTTAAATGTTGCTGG
AAGCATCATCACAAAGGCATATTTGGAAGTTACAGATGTGATTGCAGATCGGC
CTCCCCCAGTTATTCGACAAGGTCCTGTGAATCAGACTGTAGCCGTGGATGG
CACTTTCGTCCTCAGCTGTGTGGCCACAGGCAGTCCAGTGCCCACCATTCTG
TGGAGAAAGGATGGAGTCCTCGTTTCAACCCAAGACTCTCGAATCAAACAG
TTGGAGAATGGAGTACTGCAGATCCGATATGCTAAGCTGGGTGATACTGGTCG
GTACACCTGCATTGCATCAACCCCCAGTGGTGAAGCAACATGGAGTGCTTAC
ATTGAAGTTCAAGAATTTGGAGTTCCAGTTCAGCCTCCAAGACCTACTGACC
CAAATTTAATCCCTAGTGCCCCATCAAAACCTGAAGTGACAGATGTCAGCAG
AAATACAGTCACATTATCGTGGCAACCAAATTTGAATTCAGGAGCAACTCCA
ACATCTTATATTATAGAAGCCTTCAGCCATGCATCTGGTAGCAGCTGGCAGAC
CGTAGCAGAGAATGTGAAAACAGAAACATCTGCCATTAAAGGACTCAAACC
TAATGCAATTTACCTTTTCCTTGTGAGGGCAGCTAATGCATATGGAATTAGTGA
TCCAAGCCAAATATCAGATCCAGTGAAAACACAAGATGTCCTACCAACAAGT
CAGGGGGTGGACCACAAGCAGGTCCAGAGAGAGCTGGGAAATGCTGTTCTG
CACCTCCACAACCCCACCGTCCTTTCTTCCTCTTCCATCGAAGTGCACTGGAC
AGTAGATCAACAGTCTCAGTATATACAAGGATATAAAATTCTCTATCGGCCATC
TGGAGCCAACCACGGAGAATCAGACTGGTTAGTTTTTGAAGTGAGGACGCC
AGCCAAAAACAGTGTGGTAATCCCTGATCTCAGAAAGGGAGTCAACTATGAA
ATTAAGGCTCGCCCTTTTTTTAATGAATTTCAAGGAGCAGATAGTGAAATCAA
GTTTGCCAAAACCCTGGAAGAAGCACCCAGTGCCCCACCCCAAGGTGTAAC
TGTATCCAAGAATGATGGAAACGGAACTGCAATTCTAGTTAGTTGGCAGCCA
CCTCCAGAAGACACTCAAAATGGAATGGTCCAAGAGTATAAGGTTTGGTGTC
TGGGCAATGAAACTCGATACCACATCAACAAAACAGTGGATGGTTCCACCTT
TTCCGTGGTCATTCCCTTTCTTGTTCCTGGAATCCGATACAGTGTGGAAGTGG
CAGCCAGCACTGGGGCTGGGTCTGGGGTAAAGAGTGAGCCTCAGTTCATCC
AGCTGGATGCCCATGGAAACCCTGTGTCACCTGAGGACCAAGTCAGCCTCGC
TCAGCAGATTTCAGATGTGGTGAAGCAGCCGGCCTTCATAGCAGGTATTGGA
GCAGCCTGTTGGATCATCCTCATGGTCTTCAGCATCTGGCTTTATCGACACCG
CAAGAAGAGAAACGGACTTACTAGTACCTACGCGGGTATCAGAAAAGTCCC
 
GTCTTTTACCTTCACACCAACAGTAACTTACCAGAGAGGAGGCGAAGCTGTC
AGCAGTGGAGGGAGGCCTGGACTTCTCAACATCAGTGAACCTGCCGCGCAG
CCATGGCTGGCAGACACGTGGCCTAATACTGGCAACAACCACAATGACTGCT
CCATCAGCTGCTGCACGGCAGGCAATGGAAACAGCGACAGCAACCTCACTA
CCTACAGTCGCCCAGCTGATTGTATAGCAAATTATAACAACCAACTGGATAAC
AAACAAACAAATCTGATGCTCCCTGAGTCAACTGTTTATGGTGATGTGGACC
TTAGTAACAAAATCAATGAGATGAAAACCTTCAATAGCCCAAATCTGAAGGA
TGGGCGTTTTGTCAATCCATCAGGGCAGCCTACTCCTTACGCCACCACTCAGC
TCATCCAGTCAAACCTCAGCAACAACATGAACAATGGCAGCGGGGACTCTG
GCGAGAAGCACTGGAAACCACTGGGACAGCAGAAACAAGAAGTGGCACCA
GTTCAGTACAACATCGTGGAGCAAAACAAGCTGAACAAAGATTATCGAGCA
AATGACACAGTTCCTCCAACTATCCCATACAACCAATCATACGACCAGAACAC
AGGAGGATCCTACAACAGCTCAGACCGGGGCAGTAGTACATCTGGGAGTCA
GGGGCACAAGAAAGGGGCAAGAACACCCAAGGTACCAAAACAGGGTGGCA
TGAACTGGGCAGACCTGCTTCCTCCTCCCCCAGCACATCCTCCTCCACACAG
CAATAGCGAAGAGTACAACATTTCTGTAGATGAAAGCTATGACCAAGAAATG
CCATGTCCCGTGCCACCAGCAAGGATGTATTTGCAACAAGATGAATTAGAAG
AGGAGGAAGATGAACGAGGCCCCACTCCCCCTGTTCGGGGAGCAGCTTCTT
CTCCAGCTGCCGTGTCCTATAGCCATCAGTCCACTGCCACTCTGACTCCCTCC
CCACAGGAAGAACTCCAGCCCATGTTACAGGATTGTCCAGAGGAGACTGGC
CACATGCAGCACCAGCCCGACAGGAGACGGCAGCCTGTGAGTCCTCCTCCA
CCACCACGGCCGATCTCCCCTCCACATACCTATGGCTACATTTCAGGACCCCT
GGTCTCAGATATGGATACGGATGCGCCAGAAGAGGAAGAAGACGAAGCCGA
CATGGAGGTAGCCAAGATGCAAACCAGAAGGCTTTTGTTACGTGGGCTTGAG
CAGACACCTGCCTCCAGTGTTGGGGACCTGGAGAGCTCTGTCACGGGGTCC
ATGATCAACGGCTGGGGCTCAGCCTCAGAGGAGGACAACATTTCCAGCGGA
CGCTCCAGTGTTAGTTCTTCGGACGGCTCCTTTTTCACTGATGCTGACTTTGC
CCAGGCAGTCGCAGCAGCGGCAGAGTATGCTGGTCTGAAAGTAGCACGACG
GCAAATGCAGGATGCTGCTGGCCGTCGACATTTTCATGCGTCTCAGTGCCCTA
GGCCCACAAGTCCCGTGTCTACAGACAGCAACATGAGTGCCGCCGTAATGCA
GAAAACCAGACCAGCCAAGAAACTGAAACACCAGCCAGGACATCTGCGCA
GAGAAACCTACACAGATGATCTTCCACCACCTCCTGTGCCGCCACCTGCTATA
AAGTCACCTACTGCCCAATCCAAGACACAGCTGGAAGTACGACCTGTAGTGG
TGCCAAAACTCCCTTCTATGGATGCAAGAACAGACAGATCATCAGACAGAAA
AGGAAGCAGTTACAAGGGGAGAGAAGTGTTGGATGGAAGACAGGTTGTTGA
CATGCGAACAAATCCAGGTGATCCCAGAGAAGCACAGGAACAGCAAAATGA
 
CGGGAAAGGACGTGGAAACAAGGCAGCAAAACGAGACCTTCCACCAGCAA
AGACTCATCTCATCCAAGAGGATATTCTACCTTATTGTAGACCTACTTTTCCAA
CATCAAATAATCCCAGAGATCCCAGTTCCTCAAGCTCAATGTCATCAAGAGG
ATCAGGAAGCAGACAAAGAGAACAAGCAAATGTAGGTCGAAGAAATATTGC
AGAAATGCAGGTACTTGGAGGATATGAAAGAGGAGAAGATAATAATGAAGA
ATTAGAGGAAACTGAAAGCTGA
>hEPHB4_cloned_from_SKmel23 (SEQ ID No. 95) 
ATGGAGCTCCGGGTGCTGCTCTGCTGGGCTTCGTTGGCCGCAGCTTTGGAAG
AGACCCTGCTGAACACAAAATTGGAAACTGCTGATCTGAAGTGGGTGACATT
CCCTCAGGTGGACGGGCAGTGGGAGGAACTGAGCGGCCTGGATGAGGAAC
AGCACAGCGTGCGCACCTACGAAGTGTGTGACGTGCAGCGTGCCCCGGGCC
AGGCCCACTGGCTTCGCACAGGTTGGGTCCCACGGCGGGGCGCCGTCCACG
TGTACGCCACGCTGCGCTTCACCATGCTCGAGTGCCTGTCCCTGCCTCGGGC
TGGGCGCTCCTGCAAGGAGACCTTCACCGTCTTCTACTATGAGAGCGATGCG
GACACGGCCACGGCCCTCACGCCAGCCTGGATGGAGAACCCCTACATCAAG
GTGGACACGGTGGCCGCGGAGCATCTCACCCGGAAGCGCCCTGGGGCCGAG
GCCACCGGGAAGGTGAATGTCAAGACGCTGCGTCTGGGACCGCTCAGCAAG
GCTGGCTTCTACCTGGCCTTCCAGGACCAGGGTGCCTGCATGGCCCTGCTATC
CCTGCACCTCTTCTACAAAAAGTGCGCCCAGCTGACTGTGAACCTGACTCGA
TTCCCGGAGACTGTGCCTCGGGAGCTGGTTGTGCCCGTGGCCGGTAGCTGCG
TGGTGGATGCCGTCCCCGCCCCTGGCCCCAGCCCCAGCCTCTACTGCCGTGA
GGATGGCCAGTGGGCCGAACAGCCGGTCACGGGCTGCAGCTGTGCTCCGGG
GTTCGAGGCAGCTGAGGGGAACACCAAGTGCCGAGCCTGTGCCCAGGGCAC
CTTCAAGCCCCTGTCAGGAGAAGGGTCCTGCCAGCCATGCCCAGCCAATAGC
CACTCTAACACCATTGGATCAGCCGTCTGCCAGTGCCGCGTCGGGTACTTCC
GGGCACGCACAGACCCCCGGGGTGCACCCTGCACCACCCCTCCTTCGGCTC
CGCGGAGCGTGGTTTCCCGCCTGAACGGCTCCTCCCTGCACCTGGAATGGAG
TGCCCCCCTGGAGTCTGGTGGCCGAGAGGACCTCACCTACGCCCTCCGCTGC
CGGGAGTGCCGACCCGGAGGCTCCTGTGCGCCCTGCGGGGGAGACCTGACT
TTTGACCCCGGCCCCCGGGACCTGGTGGAGCCCTGGGTGGTGGTTCGAGGG
CTACGTCCTGACTTCACCTATACCTTTGAGGTCACTGCATTGAACGGGGTATC
CTCCTTAGCCACGGGGCCCGTCCCATTTGAGCCTGTCAATGTCACCACTGAC
CGAGAGGTACCTCCTGCAGTGTCCGACATCCGGGTGACGCGGTCCTCACCCA
GCAGCTTGAGCCTGGCCTGGGCTGTTCCCCGGGCACCCAGTGGGGCTGTGCT
GGACTACGAGGTCAAATACCATGAGAAGGGCGCCGAGGGTCCCAGCAGCGT
 
GCGGTTCCTGAAGACGTCAGAAAACCGGGCAGAGCTGCGGGGGCTGAAGC
GGGGAGCCAGCTACCTGGTGCAGGTACGGGCGCGCTCTGAGGCCGGCTACG
GGCCCTTCGGCCAGGAACATCACAGCCAGACCCAACTGGATGAGAGCGAGG
GCTGGCGGGAGCAGCTGGCCCTGATTGCGGGCACGGCAGTCGTGGGTGTGG
TCCTGGTCCTGGTGGTCATTGTGGTCGCAGTTCTCTGCCTCAGGAAGCAGAG
CAATGGGAGAGAAGCAGAATATTCGGACAAACACGGACAGTATCTCATCGGG
CATGGTACTAAGGTCTACATCGACCCCTTCACTTATGAAGACCCTAATGAGGC
TGTGAGGGAATTTGCAAAAGAGATCGATGTCTCCTACGTCAAGATTGAAGAG
GTGATTGGTGCAGGTGAGTTTGGCGAGGTGTGCCGGGGGCGGCTCAAGGCC
CCAGGGAAGAAGGAGAGCTGTGTGGCAATCAAGACCCTGAAGGGTGGCTAC
ACGGAGCGGCAGCGGCGTGAGTTTCTGAGCGAGGCCTCCATCATGGGCCAG
TTCGAGCACCCCAATATCATCCGCCTGGAGGGCGTGGTCACCAACAGCATGC
CCGTCATGATTCTCACAGAGTTCATGGAGAACGGCGCCCTGGACTCCTTCCT
GCGGCTAAACGACGGACAGTTCACAGTCATCCAGCTCGTGGGCATGCTGCGG
GGCATCGCCTCGGGCATGCGGTACCTTGCCGAGATGAGCTACGTCCACCGAG
ACCTGGCTGCTCGCAACATCCTAGTCAACAGCAACCTCGTCTGCAAAGTGTC
TGACTTTGGCCTTTCCCGATTCCTGGAGGAGAACTCTTCCGATCCCACCTACA
CGAGCTCCCTGGGAGGAAAGATTCCCATCCGATGGACTGCCCCGGAGGCCAT
TGCCTTCCGGAAGTTCACTTCCGCCAGTGATGCCTGGAGTTACGGGATTGTG
ATGTGGGAGGTGATGTCATTTGGGGAGAGGCCGTACTGGGACATGAGCAATC
AGGACGTGATCAATGCCATTGAACAGGACTACCGGCTGCCCCCGCCCCCAGA
CTGTCCCACCTCCCTCCACCAGCTCATGCTGGACTGTTGGCAGAAAGACCGG
AATGCCCGGCCCCGCTTCCCCCAGGTGGTCAGCGCCCTGGACAAGATGATCC
GGAACCCCGCCAGCCTCAAAATCGTGGCCCGGGAGAATGGCGGGGCCTCAC
ACCCTCTCCTGGACCAGCGGCAGCCTCACTACTCAGCTTTTGGCTCTGTGGG
CGAGTGGCTTCGGGCCATCAAAATGGGAAGATACGAAGAAAGTTTCGCAGC
CGCTGGCTTTGGCTCCTTCGAGCTGGTCAGCCAGATCTCTGCTGAGGACCTG
CTCCGAATCGGAGTCACTCTGGCGGGACACCAGAAGAAAATCTTGGCCAGT
GTCCAGCACATGAAGTCCCAGGCCAAGCCGGGAACCCCGGGTGGGACAGGA
GGACCGGCCCCGCAGTACTGA
>HCLDN1_NM_021101 (SEQ ID No. 96) 
ATGGCCAACGCGGGGCTGCAGCTGTTGGGCTTCATTCTCGCCTTCCTGGGAT
GGATCGGCGCCATCGTCAGCACTGCCCTGCCCCAGTGGAGGATTTACTCCTAT
GCCGGCGACAACATCGTGACCGCCCAGGCCATGTACGAGGGGCTGTGGATGT
CCTGCGTGTCGCAGAGCACCGGGCAGATCCAGTGCAAAGTCTTTGACTCCTT
 
GCTGAATCTGAGCAGCACATTGCAAGCAACCCGTGCCTTGATGGTGGTTGGC
ATCCTCCTGGGAGTGATAGCAATCTTTGTGGCCACCGTTGGCATGAAGTGTAT
GAAGTGCTTGGAAGACGATGAGGTGCAGAAGATGAGGATGGCTGTCATTGG
GGGTGCGATATTTCTTCTTGCAGGTCTGGCTATTTTAGTTGCCACAGCATGGT
ATGGCAATAGAATCGTTCAAGAATTCTATGACCCTATGACCCCAGTCAATGCC
AGGTACGAATTTGGTCAGGCTCTCTTCACTGGCTGGGCTGCTGCTTCTCTCTG
CCTTCTGGGAGGTGCCCTACTTTGCTGTTCCTGTCCCCGAAAAACAACCTCTT
ACCCAACACCAAGGCCCTATCCAAAACCTGCACCTTCCAGCGGGAAAGACT
ACGTGTGA
>hSLC7A5_NM_003486 (SEQ ID No. 97) 
ATGGCGGGTGCGGGCCCGAAGCGGCGCGCGCTAGCGGCGCCGGCGGCCGAG
GAGAAGGAAGAGGCGCGGGAGAAGATGCTGGCCGCCAAGAGCGCGGACGG
CTCGGCGCCGGCAGGCGAGGGCGAGGGCGTGACCCTGCAGCGGAACATCAC
GCTGCTCAACGGCGTGGCCATCATCGTGGGGACCATTATCGGCTCGGGCATCT
TCGTGACGCCCACGGGCGTGCTCAAGGAGGCAGGCTCGCCGGGGCTGGCGC
TGGTGGTGTGGGCCGCGTGCGGCGTCTTCTCCATCGTGGGCGCGCTCTGCTA
CGCGGAGCTCGGCACCACCATCTCCAAATCGGGCGGCGACTACGCCTACATG
CTGGAGGTCTACGGCTCGCTGCCCGCCTTCCTCAAGCTCTGGATCGAGCTGC
TCATCATCCGGCCTTCATCGCAGTACATCGTGGCCCTGGTCTTCGCCACCTAC
CTGCTCAAGCCGCTCTTCCCCACCTGCCCGGTGCCCGAGGAGGCAGCCAAG
CTCGTGGCCTGCCTCTGCGTGCTGCTGCTCACGGCCGTGAACTGCTACAGCG
TGAAGGCCGCCACCCGGGTCCAGGATGCCTTTGCCGCCGCCAAGCTCCTGGC
CCTGGCCCTGATCATCCTGCTGGGCTTCGTCCAGATCGGGAAGGGTGATGTG
TCCAATCTAGATCCCAACTTCTCATTTGAAGGCACCAAACTGGATGTGGGGA
ACATTGTGCTGGCATTATACAGCGGCCTCTTTGCCTATGGAGGATGGAATTAC
TTGAATTTCGTCACAGAGGAAATGATCAACCCCTACAGAAACCTGCCCCTGG
CCATCATCATCTCCCTGCCCATCGTGACGCTGGTGTACGTGCTGACCAACCTG
GCCTACTTCACCACCCTGTCCACCGAGCAGATGCTGTCGTCCGAGGCCGTGG
CCGTGGACTTCGGGAACTATCACCTGGGCGTCATGTCCTGGATCATCCCCGTC
TTCGTGGGCCTGTCCTGCTTCGGCTCCGTCAATGGGTCCCTGTTCACATCCTC
CAGGCTCTTCTTCGTGGGGTCCCGGGAAGGCCACCTGCCCTCCATCCTCTCC
ATGATCCACCCACAGCTCCTCACCCCCGTGCCGTCCCTCGTGTTCACGTGTGT
GATGACGCTGCTCTACGCCTTCTCCAAGGACATCTTCTCCGTCATCAACTTCT
TCAGCTTCTTCAACTGGCTCTGCGTGGCCCTGGCCATCATCGGCATGATCTGG
CTGCGCCACAGAAAGCCTGAGCTTGAGCGGCCCATCAAGGTGAACCTGGCC
 
CTGCCTGTGTTCTTCATCCTGGCCTGCCTCTTCCTGATCGCCGTCTCCTTCTGG
AAGACACCCGTGGAGTGTGGCATCGGCTTCACCATCATCCTCAGCGGGCTGC
CCGTCTACTTCTTCGGGGTCTGGTGGAAAAACAAGCCCAAGTGGCTCCTCCA
GGGCATCTTCTCCACGACCGTCCTGTGTCAGAAGCTCATGCAGGTGGTCCCC
CAGGAGACATAG
>hAFP_var1_BC027881 (SEQ ID No. 98) 
ATGAAGTGGGTGGAATCAATTTTTTTAATTTTCCTACTAAATTTTACTGAATCC
AGAACACTGCATAGAAATGAATATGGAATAGCTTCCATATTGGATTCTTACCA
ATGTACTGCAGAGATAAGTTTAGCTGACCTGGCTACCATATTTTTTGCCCAGTT
TGTTCAAGAAGCCACTTACAAGGAAGTAAGCAAAATGGTGAAAGATGCATT
GACTGCAATTGAGAAACCCACTGGAGATGAACAGTCTTCAGGGTGTTTAGAA
AACCAGCTACCTGCCTTTCTGGAAGAACTTTGCCATGAGAAAGAAATTTTGG
AGAAGTACGGACATTCAGACTGCTGCAGCCAAAGTGAAGAGGGAAGACATA
ACTGTTTTCTTGCACACAAAAAGCCCACTCCAGCATCGATCCCACTTTTCCA
AGTTCCAGAACCTGTCACAAGCTGTGAAGCATATGAAGAAGACAGGGAGAC
ATTCATGAACAAATTCATTTATGAGATAGCAAGAAGGCATCCCTTCCTGTATG
CACCTACAATTCTTCTTTGGGCTGCTCGCTATGACAAAATAATTCCATCTTGCT
GCAAAGCTGAAAATGCAGTTGAATGCTTCCAAACAAAGGCAGCAACAGTTA
CAAAAGAATTAAGAGAAAGCAGCTTGTTAAATCAACATGCATGTGCAGTAAT
GAAAAATTTTGGGACCCGAACTTTCCAAGCCATAACTGTTACTAAACTGAGT
CAGAAGTTTACCAAAGTTAATTTTACTGAAATCCAGAAACTAGTCCTGGATGT
GGCCCATGTACATGAGCACTGTTGCAGAGGAGATGTGCTGGATTGTCTGCAG
GATGGGGAAAAAATCATGTCCTACATATGTTCTCAACAAGACACTCTGTCAA
ACAAAATAACAGAATGCTGCAAACTGACCACGCTGGAACGTGGTCAATGTAT
AATTCATGCAGAAAATGATGAAAAACCTGAAGGTCTATCTCCAAATCTAAAC
AGGTTTTTAGGAGATAGAGATTTTAACCAATTTTCTTCAGGGGAAAAAAATAT
CTTCTTGGCAAGTTTTGTTCATGAATATTCAAGAAGACATCCTCAGCTTGCTG
TCTCAGTAATTCTAAGAGTTGCTAAAGGATACCAGGAGTTATTGGAGAAGTGT
TTCCAGACTGAAAACCCTCTTGAATGCCAAGATAAAGGAGAAGAAGAATTAC
AGAAATACATCCAGGAGAGCCAAGCATTGGCAAAGCGAAGCTGCGGCCTCT
TCCAGAAACTAGGAGAATATTACTTACAAAATGCGTTTCTCGTTGCTTACACA
AAGAAAGCCCCCCAGCTGACCTCGTCGGAGCTGATGGCCATCACCAGAAAA
ATGGCAGCCACAGCAGCCACTTGTTGCCAACTCAGTGAGGACAAACTATTGG
CCTGTGGCGAGGGAGCGGCTGACATTATTATCGGACACTTATGTATCAGACAT
GAAATGACTCCAGTAAACCCTGGTGTTGGCCAGTGCTGCACTTCTTCATATGC
 
CAACAGGAGGCCATGCTTCAGCAGCTTGGTGGTGGATGAAACATATGTCCCT
CCTGCATTCTCTGATGACAAGTTCATTTTCCATAAGGATCTGTGCCAAGCTCA
GGGTGTAGCGCTGCAAACGATGAAGCAAGAGTTTCTCATTAACCTTGTGAAG
CAAAAGCCACAAATAACAGAGGAACAACTTGAGGCTGTCATTGCAGATTTCT
CAGGCCTGTTGGAGAAATGCTGCCAAGGCCAGGAACAGGAAGTCTGCTTTG
CTGAAGAGGGACAAAAACTGATTTCAAAAACTCGTGCTGCTTTGGGAGTTTA
A
>hTGFBI_M77349 (SEQ ID No. 99) 
ATGGCGCTCTTCGTGCGGCTGCTGGCTCTCGCCCTGGCTCTGGCCCTGGGCC
CCGCCGCGACCCTGGCGGGTCCCGCCAAGTCGCCCTACCAGCTGGTGCTGCA
GCACAGCAGGCTCCGGGGCCGCCAGCACGGCCCCAACGTGTGTGCTGTGCA
GAAGGTTATTGGCACTAATAGGAAGTACTTCACCAACTGCAAGCAGTGGTAC
CAAAGGAAAATCTGTGGCAAATCAACAGTCATCAGCTACGAGTGCTGTCCTG
GATATGAAAAGGTCCCTGGGGAGAAGGGCTGTCCAGCAGCCCTACCACTCTC
AAACCTTTACGAGACCCTGGGAGTCGTTGGATCCACCACCACTCAGCTGTAC
ACGGACCGCACGGAGAAGCTGAGGCCTGAGATGGAGGGGCCCGGCAGCTTC
ACCATCTTCGCCCCTAGCAACGAGGCCTGGGCCTCCTTGCCAGCTGAAGTGC
TGGACTCCCTGGTCAGCAATGTCAACATTGAGCTGCTCAATGCCCTCCGCTAC
CATATGGTGGGCAGGCGAGTCCTGACTGATGAGCTGAAACACGGCATGACCC
TCACCTCTATGTACCAGAATTCCAACATCCAGATCCACCACTATCCTAATGGG
ATTGTAACTGTGAACTGTGCCCGGCTCCTGAAAGCCGACCACCATGCAACCA
ACGGGGTGGTGCACCTCATCGATAAGGTCATCTCCACCATCACCAACAACAT
CCAGCAGATCATTGAGATCGAGGACACCTTTGAGACCCTTCGGGCTGCTGTG
GCTGCATCAGGGCTCAACACGATGCTTGAAGGTAACGGCCAGTACACGCTTT
TGGCCCCGACCAATGAGGCCTTCGAGAAGATCCCTAGTGAGACTTTGAACCG
TATCCTGGGCGACCCAGAAGCCCTGAGAGACCTGCTGAACAACCACATCTTG
AAGTCAGCTATGTGTGCTGAAGCCATCGTTGCGGGGCTGTCTGTAGAGACCC
TGGAGGGCACGACACTGGAGGTGGGCTGCAGCGGGGACATGCTCACTATCA
ACGGGAAGGCGATCATCTCCAATAAAGACATCCTAGCCACCAACGGGGTGAT
CCACTACATTGATGAGCTACTCATCCCAGACTCAGCCAAGACACTATTTGAAT
TGGCTGCAGAGTCTGATGTGTCCACAGCCATTGACCTTTTCAGACAAGCCGG
CCTCGGCAATCATCTCTCTGGAAGTGAGCGGTTGACCCTCCTGGCTCCCCTG
AATTCTGTATTCAAAGATGGAACCCCTCCAATTGATGCCCATACAAGGAATTT
GCTTCGGAACCACATAATTAAAGACCAGCTGGCCTCTAAGTATCTGTACCATG
GACAGACCCTGGAAACTCTGGGCGGCAAAAAACTGAGAGTTTTTGTTTATCG
 
TAATAGCCTCTGCATTGAGAACAGCTGCATCGCGGCCCACGACAAGAGGGGG
AGGTACGGGACCCTGTTCACGATGGACCGGGTGCTGACCCCCCCAATGGGG
ACTGTCATGGATGTCCTGAAGGGAGACAATCGCTTTAGCATGCTGGTAGCTG
CCATCCAGTCTGCAGGACTGACGGAGACCCTCAACCGGGAAGGAGTCTACA
CAGTCTTTGCTCCCACAAATGAAGCCTTCCGAGCCCTGCCACCAAGAGAACG
GAGCAGACTCTTGGGAGATGCCAAGGAACTTGCCAACATCCTGAAATACCAC
ATTGGTGATGAAATCCTGGTTAGCGGAGGCATCGGGGCCCTGGTGCGGCTAA
AGTCTCTCCAAGGTGACAAGCTGGAAGTCAGCTTGAAAAACAATGTGGTGA
GTGTCAACAAGGAGCCTGTTGCCGAGCCTGACATCATGGCCACAAATGGCGT
GGTCCATGTCATCACCAATGTTCTGCAGCCTCCAGCCAACAGACCTCAGGAA
AGAGGGGATGAACTTGCAGACTCTGCGCTTGAGATCTTCAAACAAGCATCAG
CGTTTTCCAGGGCTTCCCAGAGGTCTGTGCGACTAGCCCCTGTCTATCAAAA
GTTATTAGAGAGGATGAAGCATTAG
>hSPARC_var1_NM_003118 (SEQ ID No. 100) 
ATGAGGGCCTGGATCTTCTTTCTCCTTTGCCTGGCCGGGAGGGCCTTGGCAG
CCCCTCAGCAAGAAGCCCTGCCTGATGAGACAGAGGTGGTGGAAGAAACTG
TGGCAGAGGTGACTGAGGTATCTGTGGGAGCTAATCCTGTCCAGGTGGAAGT
AGGAGAATTTGATGATGGTGCAGAGGAAACCGAAGAGGAGGTGGTGGCGGA
AAATCCCTGCCAGAACCACCACTGCAAACACGGCAAGGTGTGCGAGCTGGA
TGAGAACAACACCCCCATGTGCGTGTGCCAGGACCCCACCAGCTGCCCAGC
CCCCATTGGCGAGTTTGAGAAGGTGTGCAGCAATGACAACAAGACCTTCGA
CTCTTCCTGCCACTTCTTTGCCACAAAGTGCACCCTGGAGGGCACCAAGAAG
GGCCACAAGCTCCACCTGGACTACATCGGGCCTTGCAAATACATCCCCCCTT
GCCTGGACTCTGAGCTGACCGAATTCCCCCTGCGCATGCGGGACTGGCTCAA
GAACGTCCTGGTCACCCTGTATGAGAGGGATGAGGACAACAACCTTCTGACT
GAGAAGCAGAAGCTGCGGGTGAAGAAGATCCATGAGAATGAGAAGCGCCTG
GAGGCAGGAGACCACCCCGTGGAGCTGCTGGCCCGGGACTTCGAGAAGAA
CTATAACATGTACATCTTCCCTGTACACTGGCAGTTCGGCCAGCTGGACCAGC
ACCCCATTGACGGGTACCTCTCCCACACCGAGCTGGCTCCACTGCGTGCTCC
CCTCATCCCCATGGAGCATTGCACCACCCGCTTTTTCGAGACCTGTGACCTGG
ACAATGACAAGTACATCGCCCTGGATGAGTGGGCCGGCTGCTTCGGCATCAA
GCAGAAGGATATCGACAAGGATCTTGTGATCTAA
>hFOXM1_var2_cloned_from_AsPC1 (SEQ ID No. 101) ATGAAAACTAGCCCCCGTCGGCCACTGATTCTCAAAAGACGGAGGCTGCCCC 
TTCCTGTTCAAAATGCCCCAAGTGAAACATCAGAGGAGGAACCTAAGAGATC
CCCTGCCCAACAGGAGTCTAATCAAGCAGAGGCCTCCAAGGAAGTGGCAGA
GTCCAACTCTTGCAAGTTTCCAGCTGGGATCAAGATTATTAACCACCCCACCA
TGCCCAACACGCAAGTAGTGGCCATCCCCAACAATGCTAATATTCACAGCATC
ATCACAGCACTGACTGCCAAGGGAAAAGAGAGTGGCAGTAGTGGGCCCAAC
AAATTCATCCTCATCAGCTGTGGGGGAGCCCCAACTCAGCCTCCAGGACTCC
GGCCTCAAACCCAAACCAGCTATGATGCCAAAAGGACAGAAGTGACCCTGG
AGACCTTGGGACCAAAACCTGCAGCTAGGGATGTGAATCTTCCTAGACCACC
TGGAGCCCTTTGCGAGCAGAAACGGGAGACCTGTGCAGATGGTGAGGCAGC
AGGCTGCACTATCAACAATAGCCTATCCAACATCCAGTGGCTTCGAAAGATG
AGTTCTGATGGACTGGGCTCCCGCAGCATCAAGCAAGAGATGGAGGAAAAG
GAGAATTGTCACCTGGAGCAGCGACAGGTTAAGGTTGAGGAGCCTTCGAGA
CCATCAGCGTCCTGGCAGAACTCTGTGTCTGAGCGGCCACCCTACTCTTACAT
GGCCATGATACAATTCGCCATCAACAGCACTGAGAGGAAGCGCATGACTTTG
AAAGACATCTATACGTGGATTGAGGACCACTTTCCCTACTTTAAGCACATTGC
CAAGCCAGGCTGGAAGAACTCCATCCGCCACAACCTTTCCCTGCACGACATG
TTTGTCCGGGAGACGTCTGCCAATGGCAAGGTCTCCTTCTGGACCATTCACC
CCAGTGCCAACCGCTACTTGACATTGGACCAGGTGTTTAAGCCACTGGACCC
AGGGTCTCCACAATTGCCCGAGCACTTGGAATCACAGCAGAAACGACCGAA
TCCAGAGCTCCGCCGGAACATGACCATCAAAACCGAACTCCCCCTGGGCGC
ACGGCGGAAGATGAAGCCACTGCTACCACGGGTCAGCTCATACCTGGTACCT
ATCCAGTTCCCGGTGAACCAGTCACTGGTGTTGCAGCCCTCGGTGAAGGTGC
CATTGCCCCTGGCGGCTTCCCTCATGAGCTCAGAGCTTGCCCGCCATAGCAA
GCGAGTCCGCATTGCCCCCAAGGTGCTGCTAGCTGAGGAGGGGATAGCTCCT
CTTTCTTCTGCAGGACCAGGGAAAGAGGAGAAACTCCTGTTTGGAGAAGGG
TTTTCTCCTTTGCTTCCAGTTCAGACTATCAAGGAGGAAGAAATCCAGCCTG
GGGAGGAAATGCCACACTTAGCGAGACCCATCAAAGTGGAGAGCCCTCCCT
TGGAAGAGTGGCCCTCCCCGGCCCCATCTTTCAAAGAGGAATCATCTCACTC
CTGGGAGGATTCGTCCCAATCTCCCACCCCAAGACCCAAGAAGTCCTACAGT
GGGCTTAGGTCCCCAACCCGGTGTGTCTCGGAAATGCTTGTGATTCAACACA
GGGAGAGGAGGGAGAGGAGCCGGTCTCGGAGGAAACAGCATCTACTGCCTC
CCTGTGTGGATGAGCCGGAGCTGCTCTTCTCAGAGGGGCCCAGTACTTCCCG
CTGGGCCGCAGAGCTCCCGTTCCCAGCAGACTCCTCTGACCCTGCCTCCCAG
CTCAGCTACTCCCAGGAAGTGGGAGGACCTTTTAAGACACCCATTAAGGAAA
CGCTGCCCATCTCCTCCACCCCGAGCAAATCTGTCCTCCCCAGAACCCCTGA
ATCCTGGAGGCTCACGCCCCCAGCCAAAGTAGGGGGACTGGATTTCAGCCCA
 
GTACAAACCCCCCAGGGTGCCTCTGACCCCTTGCCTGACCCCCTGGGGCTGA
TGGATCTCAGCACCACTCCCTTGCAAAGTGCTCCCCCCCTTGAATCACCGCA
AAGGCTCCTCAGTTCAGAACCCTTAGACCTCATCTCCGTCCCCTTTGGCAACT
CTTCTCCCTCAGATATAGACGTCCCCAAGCCAGGCTCCCCGGAGCCACAGGT
TTCTGGCCTTGCAGCCAATCGTTCTCTGACAGAAGGCCTGGTCCTGGACACA
ATGAATGACAGCCTCAGCAAGATCCTGCTGGACATCAGCTTTCCTGGCCTGG
ACGAGGACCCACTGGGCCCTGACAACATCAACTGGTCCCAGTTTATTCCTGA
GCTACAGTAG
>hHSPH1_var1_NM_006644 (SEQ ID No. 102)

ATGTCGGTGGTGGGGTTGGACGTGGGCTCGCAGAGCTGCTACATCGCGGTAG
CCCGGGCCGGGGGCATCGAGACCATCGCCAATGAGTTCAGCGACCGGTGCA
CCCCGTCAGTCATATCATTTGGATCAAAAAATAGAACAATCGGAGTTGCAGCC
AAAAATCAGCAAATCACTCATGCAAACAATACGGTGTCTAACTTCAAAAGAT
TTCATGGCCGAGCATTCAATGACCCCTTCATTCAAAAGGAGAAGGAAAACTT
GAGTTACGATTTGGTTCCATTGAAAAATGGTGGAGTTGGAATAAAGGTAATGT
ACATGGGTGAAGAACATCTATTTAGTGTGGAGCAGATAACAGCCATGTTGTTG
ACTAAGCTGAAGGAAACTGCTGAAAACAGCCTCAAGAAACCAGTAACAGAT
TGTGTTATTTCAGTCCCCTCCTTCTTTACAGATGCTGAGAGGCGATCTGTGTTA
GATGCTGCACAGATTGTTGGCCTAAACTGTTTAAGACTTATGAATGACATGAC
AGCTGTTGCTTTGAATTACGGAATTTATAAGCAGGATCTCCCAAGCCTGGATG
AGAAACCTCGGATAGTGGTTTTTGTTGATATGGGACATTCAGCTTTTCAAGTG
TCTGCTTGTGCTTTTAACAAGGGAAAATTGAAGGTACTGGGAACAGCTTTTG
ATCCTTTCTTAGGAGGAAAAAACTTCGATGAAAAGTTAGTGGAACATTTTTG
TGCAGAATTTAAAACTAAGTACAAGTTGGATGCAAAATCCAAAATACGAGCA
CTCCTACGTCTGTATCAGGAATGTGAAAAACTGAAAAAGCTAATGAGCTCTA
ACAGCACAGACCTTCCACTGAATATCGAATGCTTTATGAATGATAAAGATGTT
TCCGGAAAGATGAACAGGTCACAATTTGAAGAACTCTGTGCTGAACTTCTGC
AAAAGATAGAAGTACCCCTTTATTCACTGTTGGAACAAACTCATCTCAAAGT
AGAAGATGTGAGTGCAGTTGAGATTGTTGGAGGCGCTACACGAATTCCAGCT
GTGAAGGAAAGAATTGCCAAATTCTTTGGAAAAGATATTAGCACAACACTCA
ATGCAGATGAAGCAGTAGCCAGAGGATGTGCATTACAGTGTGCAATACTTTC
CCCGGCATTTAAAGTTAGAGAATTTTCCGTCACAGATGCAGTTCCTTTTCCAA
TATCTCTGATCTGGAACCATGATTCAGAAGATACTGAAGGTGTTCATGAAGTC
TTTAGTCGAAACCATGCTGCTCCTTTCTCCAAAGTTCTCACCTTTCTGAGAAG
GGGGCCTTTTGAGCTAGAAGCTTTCTATTCTGATCCCCAAGGAGTTCCATATC

CAGAAGCAAAAATAGGCCGCTTTGTAGTTCAGAATGTTTCTGCACAGAAAGA
TGGAGAAAAATCTAGAGTAAAAGTCAAAGTGCGAGTCAACACCCATGGCAT
TTTCACCATCTCTACGGCATCTATGGTGGAGAAAGTCCCAACTGAGGAGAAT
GAAATGTCTTCTGAAGCTGACATGGAGTGTCTGAATCAGAGACCACCAGAA
AACCCAGACACTGATAAAAATGTCCAGCAAGACAACAGTGAAGCTGGAACA
CAGCCCCAGGTACAAACTGATGCTCAACAAACCTCACAGTCTCCCCCTTCAC
CTGAACTTACCTCAGAAGAAAACAAAATCCCAGATGCTGACAAAGCAAATG
AAAAAAAAGTTGACCAGCCTCCAGAAGCTAAAAAGCCCAAAATAAAGGTGG
TGAATGTTGAGCTGCCTATTGAAGCCAACTTGGTCTGGCAGTTAGGGAAAGA
CCTTCTTAACATGTATATTGAGACAGAGGGTAAGATGATAATGCAAGATAAAT
TGGAAAAAGAAAGGAATGATGCTAAAAATGCAGTTGAGGAATATGTGTATGA
GTTCAGAGACAAGCTGTGTGGACCATATGAAAAATTTATATGTGAGCAGGATC
ATCAAAATTTTTTGAGACTCCTCACAGAAACTGAAGACTGGCTGTATGAAGA
AGGAGAGGACCAAGCTAAACAAGCATATGTTGACAAGTTGGAAGAATTAATG
AAAATTGGCACTCCAGTTAAAGTTCGGTTTCAGGAAGCTGAAGAACGGCCA
AAAATGTTTGAAGAACTAGGACAGAGGCTGCAGCATTATGCCAAGATAGCAG
CTGACTTCAGAAATAAGGATGAGAAATACAACCATATTGATGAGTCTGAAATG
AAAAAAGTGGAGAAGTCTGTTAATGAAGTGATGGAATGGATGAATAATGTCA
TGAATGCTCAGGCTAAAAAGAGTCTTGATCAGGATCCAGTTGTACGTGCTCA
GGAAATTAAAACAAAAATCAAGGAATTGAACAACACATGTGAACCCGTTGTA
ACACAACCGAAACCAAAAATTGAATCACCCAAACTGGAAAGAACTCCAAAT
GGCCCAAATATTGATAAAAAGGAAGAAGATTTAGAAGACAAAAACAATTTTG
GTGCTGAACCTCCACATCAGAATGGTGAATGTTACCCTAATGAGAAAAATTCT
GTTAATATGGACTTGGACTAG

### Example 11: Development of TCR-T cell therapy and cocktail TCR-T cell therapy targeting common cancer antigen-derived peptides

### <Method>

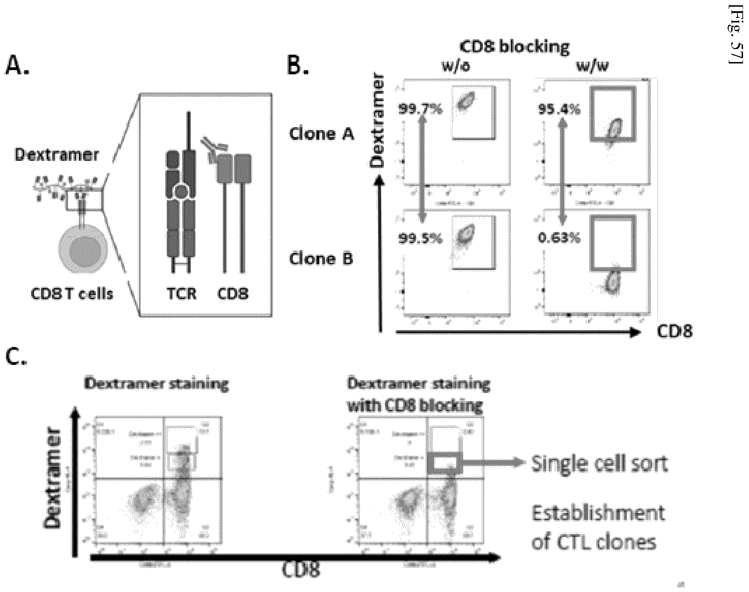

Fig. 57 shows a method for selecting high-affinity T cell clones through inhibition of binding of CD8 molecules to MHC.

A of Fig. 57 shows the interaction between Dextramer and a TCR molecule in the presence of an anti-CD8 antibody. When an MHC molecule/cancer antigen peptide complex bound to Dextramer binds to a TCR molecule, an anti-CD8 antibody added in advance prevents the peptide-nonspecific binding between a CD8 molecule and an MHC molecule, and only T cell clones with TCR molecules having stronger avidity are stained.

B of Fig. 57 shows comparison of Dextramer staining between cases with and without CD8 blocking. T cell clones A and B exhibited stainability to Dextramer to almost the same degree without CD8 blocking, but clone A exhibited higher stainability than clone B with inhibition of the binding of CD8 molecules.

C of Fig. 57 shows selection of high-affinity clones on the basis of CD8 blocking. Through purification of T cells that recognized a cancer antigen peptide by Dextramer staining with the utilization of the aforementioned principle, a T cell clone having a TCR gene with higher affinity with the cancer antigen peptide can be established.

### <Results>

It is known that a CD8 molecule on a T cell assists in binding between an MHC-peptide complex and a TCR to enhance the binding. With the present method, we inhibited the binding of CD8 molecules to MHC molecules by using an anti-CD8 antibody before Dextramer staining, and thereby succeeded in attenuating nonspecific staining depending on the binding of CD8 molecules and in detecting clones with TCR genes each individually having strong avidity. In fact, when two T cell clones reactive with a GPC3 peptide were subjected to Dextramer staining under conditions without CD8 inhibition, the staining intensities of Clone A and Clone B were comparable, but clear difference in stainability was found under conditions with CD8 inhibition. In the present task, in order to select T cells each expressing a peptide-reactive TCR gene having anti-affinity from the peripheral blood of a patient on the basis of the same principle, a staining method with Dextramer in combination with CD8 inhibition was used.

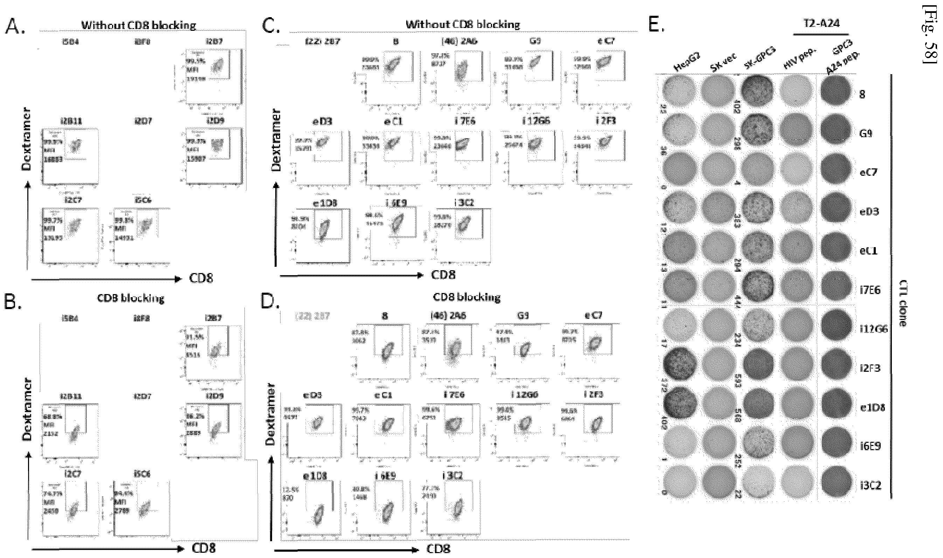

Fig. 58 shows establishment of peptide-specific CTL clones from the peripheral blood of a patient inoculated with a GPC3 peptide vaccine. Dextramer-positive T cells were selected by single sorting under conditions with CD8 blocking from the peripheral blood of a patient inoculated with a cancer peptide vaccine with a GPC3-derived peptide, and cultured on allofeeder cells to establish T cell clones.

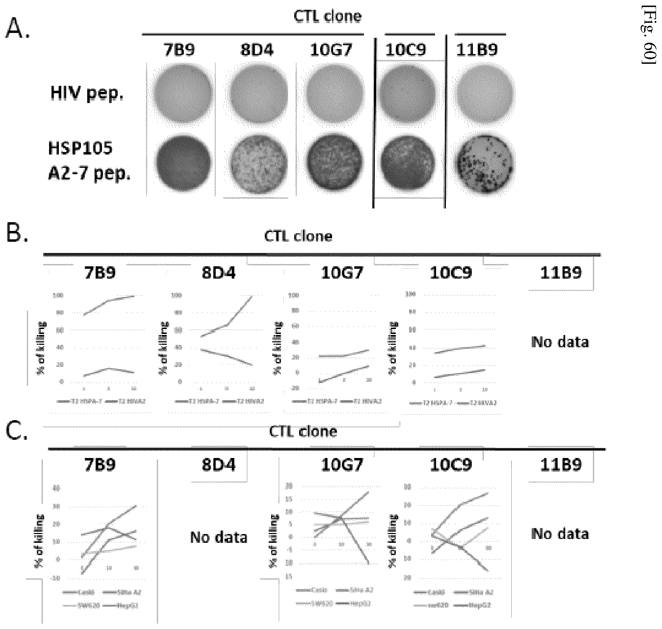

A and B of Fig. 60 show evaluation of the avidities of TCRs by Dextramer staining for HLA-A2-restricted GPC3-specific T cell clones. Comparison and examination were performed between A. without CD8 blocking and B. with CD8 blocking.

C and D of Fig. 60 show evaluation of the avidities of TCRs by Dextramer staining for HLA-A24-restricted GPC3-specific T cell clones. Comparison and examination were performed between C. without CD8 blocking and D. with CD8 blocking.

E of Fig. 60 shows evaluation of the antigen-specific IFN-γ production capabilities of HLA-A24-restricted GPC3-specific T cell clones. Established T cell clones were each cocultured with each of a GPC3-positive HepG2 liver cancer cell line, a GPC3-negative SK-Hep cell line (SK-vec), and a GPC3 forced-expression cell line (SK-GPC3) for 20 hours, and their IFN-γ productions were evaluated with an ELISPOT method. The antigen specificities were evaluated by using T2-A24 cells, a cell line lacking TAP, to which the GPC3-derived peptide (GPC3 A24 prp.) used for the peptide vaccine had been exogenously bound as a positive control, and those to which an HIV-derived peptide had been bound as a negative control.

### <Results>

For the purpose of isolating GPC3 peptide-specific TCR genes, peripheral blood lymphocytes provided by a patient of HLA-A2 haplotype and a patient of HLA-A24 haplotype, each having been inoculated with the GPC3 peptide vaccine, were subjected to stimulation culture with the peptide, and anti-affinity T cells were then selected with the staining method using Dextramer in combination with CD8 inhibition to establish T cell clones. Comparison on bindability to Dextramer between the established clones with CD8 inhibition and those without CD8 inhibition showed that A2-restricted clones had generally lower staining intensity but all maintained the stainability; on the other hand, the comparison found clones with largely lower stainability, as can be found in e1D8 clones, from A24-restricted clones. These results suggest that selecting T cell clones that exhibit higher stainability to Dextramer without being affected by the presence or absence of CD8 inhibition allows efficient identification of TCR genes with high affinity. Subsequently, the IFN-γ production capabilities of the established HLA-A24-restricted T cell clones were evaluated. All the T cell clones exhibited strong IFN-γ production when cocultured with T2-A24 cells to which the peptide had been exogenously bound. In the cases of coculture with the forced-expression cell line endogenously expressing GPC3 (SK-GPC3) and coculture with the HepG2 cell line, by contrast, large difference in reactivity was found among the clones. The IFN-γ production capabilities did not necessarily match the Dextramer staining, and the results showed that the aforementioned e1D8 exhibited low stainability to Dextramer but strong IFN-γ production against endogenous GPC3-expressing cells, whereas the eC7 clone had high Dextramer staining intensity but exhibited low IFN-γ production capability when cocultured with the endogenous GPC3-expressing cell. This is probably because the IFN-γ production capabilities of T cells are determined not only by the avidities of TCRs but also by clone-to-clone difference in various functions in the cells. It is needed for selection of TCR genes capable of recognizing endogenous antigens and excluding tumors to construct TCR-T cells for use in treatment and reevaluate their functions. The amino acid sequences for and gene sequences of TCR genes isolated from the clones are shown in tables.

### [Table 2]

**Table 2: Amino acid sequences of HLA-A2-restricted T-cell receptors that recognize GPC3-derived peptide (FVGEFFTDV)**

| Clone | Sequence TRA | Sequence TRB | TRA (bp) | TRB (bp) |
|---|---|---|---|---|
| ⑤2B11 | MMKSLRVLLVILWLQLSWV WSQQKEVEQDPGPLSVPEGA IVSLNCTYSNSAFQYFMWYR QYSRKGPELLMYTYSSGNKE DGRFTAQVDKSSKYISLFIRD SQPSDSATYLCAQRIVMEYG NKLVFGAGTILRVKS (SEQ ID NO: 103) | MDTWLVCWAIFSLLKAGLTE PEVTQTPSHQVTQMGQEVIL RCVPISNHLYFYWYRQILGQK VEFLVSFYNNEISEKSEIFDDQ FSVERPDGSNFTLKIRSTKLE DSAMYFCASSEGAEAFFGQG TRLTVV (SEQ ID NO: 104) | 135 | 130 |
| ⑧2D9 | MKSLRVLLVILWLQLSWVWS QQKEVEQNSGPLSVPEGAIAS LNCTYSDRGSQSFFWYRQYS GKSPELIMFIYSNGDKEDGRF TAQLNKASQYVSLLIRDSQPS DSATYLCAVNMDGNNRLAFG KGNQVVVIP (SEQ ID NO: 105) | MRSWPGPEMGTRLFFYVALC LLWTGHMDAGITQSPRHKVT ETGTPVTLRCHQTENHRYMY WYRQDPGHGLRLIHYSYGVK DTDKGEVSDGYSVSRSKTED FLLTLESATSSQTSVYFCASV YGSEAFFGQGTRLTVV (SEQ ID NO: 106) | 132 | 137 |
| i2B7 | MASAPISMLAMLFTLSGLRA QSVAQPEDQVNVAEGNPLTV KCTYSVSGNPYLFWYVQYPN RGLQFLLKYITGDNLVKGSY GFEAEFNKSQTSFHLKKPSAL VSDSALYFCAVNPTGGGNKL TFGTGTQLKVEL (SEQ ID NO: 107) | MATRLLCCVVLCLLGEELIDA RVTQTPRDKVTEMGQEVTM RCQPILGHNTVFWYRQTMM QGLELLAYFRNRAPLDDSGM PKDRFSAEMPDATLATLKIQP SEPRDSAVYFCASGLGGNQP QHFGDGTRLSIL (SEQ ID NO: 108) | 133 | 132 |
| ⑥2C7 | MKSLRVLLVILWLQLSWVWS QQKEVEQNSGPLSVPEGAIAS LNCTYSDRGSQSFFWYRQYS GKSPELIMFIYSNGDKEDGRF TAQLNKASQYVSLLIRDSQPS DSATYLCAVKGTGRRALTFGS GTRLQVQP (SEQ ID NO: 109) | MGCRLLCCAVLCLLGAVPIDT EVTQTPKHLVMGMTNKKSL KCEQHMGHRAMYWYKQKA KKPPELMFVYSYEKLSINESV PSRFSPECPNSSLLNLHLHAL QPEDSALYLCASSQSGVGNTE AFFGQGTRLTVV (SEQ ID NO: 110) | 132 | 133 |
| ⑨5C6 | MLLELIPLLGIHFVLRTARAQS VTQPDIHITVSEGASLELRCN YSYGATPYLFWYVQSPGQGL | MGPQLLGYVVLCLLGAGPLE AQVTQNPRYLITVTGKKLTVT CSQNMNHEYMSWYRQDPGL | 133 | 131 |
| | QLLLKYFSGDTLVQGIKGFEA EFKRSQSSFNLRKPSVHWSD AAEYFCAVGSPSGYSTLTFGK GTMLLVSP (SEQ ID NO: 111) | GLRQIYYSMNVEVTDKGDVP EGYKVSRKEKRNFPLILESPS PNQTSLYFCASSPTRFYEQYF GPGTRLTVT (SEQ ID NO: 112) | | |

### [Table 3]

**Table 3: Gene sequences for HLA-A2-restricted T-cell receptors that recognize GPC3-derived peptide (FVGEFFTDV)**

| clone | Sequence TRA | Sequence TRB | TRA (bp) | TRB (bp) |
|---|---|---|---|---|
| ⑤2B11 | ATGATGAAATCCTTGAGAG TTTTACTGGTGATCCTGTG GCTTCAGTTAAGCTGGGTT TGGAGCCAACAGAAGGAG GTGGAGCAGGATCCTGGA CCACTCAGTGTTCCAGAG GGAGCCATTGTTTCTCTCA ACTGCACTTACAGCAACA GTGCTTTTCAATACTTCAT GTGGTACAGACAGTATTCC AGAAAAGGCCCTGAGTTG CTGATGTACACATACTCCA GTGGTAACAAAGAAGATG GAAGGTTTACAGCACAGG TCGATAAATCCAGCAAGTA TATCTCCTTGTTCATCAGA GACTCACAGCCCAGTGAT TCAGCCACCTACCTCTGTG CCCAAAGAATTGTCATGG AATATGGAAACAAGCTGG TCTTTGGCGCAGGAACCA TTCTGAGAGTCAAGTCC (SEQ ID NO: 113) | ATGGATACCTGGCTCGTATG CTGGGCAATTTTTAGTCTCT TGAAAGCAGGACTCACAGA ACCTGAAGTCACCCAGACT CCCAGCCATCAGGTCACAC AGATGGGACAGGAAGTGAT CTTGCGCTGTGTCCCCATCT CTAATCACTTATACTTCTATT GGTACAGACAAATCTTGGG GCAGAAAGTCGAGTTTCTG GTTTCCTTTTATAATAATGA AATCTCAGAGAAGTCTGAA ATATTCGATGATCAATTCTC AGTTGAAAGGCCTGATGGA TCAAATTTCACTCTGAAGAT CCGGTCCACAAAGCTGGAG GACTCAGCCATGTACTTCTG TGCCAGCAGTGAGGGGGCT GAAGCTTTCTTTGGACAAG GCACCAGACTCACAGTTGT A (SEQ ID NO: 114) | 405 | 390 |
| ⑧2D9 | ATGAAATCCTTGAGAGTTT TACTAGTGATCCTGTGGCT TCAGTTGAGCTGGGTTTG GAGCCAACAGAAGGAGGT GGAGCAGAATTCTGGACC CCTCAGTGTTCCAGAGGG AGCCATTGCCTCTCTCAAC TGCACTTACAGTGACCGA GGTTCCCAGTCCTTCTTCT GGTACAGACAATATTCTGG GAAAAGCCCTGAGTTGAT AATGTTCATATACTCCAAT GGTGACAAAGAAGATGGA AGGTTTACAGCACAGCTC AATAAAGCCAGCCAGTAT | ATGAGATCCTGGCCTGGAC CTGAAATGGGCACAAGGTT GTTCTTCTATGTGGCCCTTT GTCTCCTGTGGACAGGACA CATGGATGCTGGAATCACCC AGAGCCCAAGACACAAGGT CACAGAGACAGGAACACC AGTGACTCTGAGATGTCATC AGACTGAGAACCACCGCTA TATGTACTGGTATCGACAAG ACCCGGGGCATGGGCTGAG GCTGATCCATTACTCATATG GTGTTAAAGATACTGACAA AGGAGAAGTCTCAGATGGC TATAGTGTCTCTAGATCAAA | 396 | 411 |
| | GTTTCTCTGCTCATCAGAG ACTCCCAGCCCAGTGATTC AGCCACCTACCTCTGTGCC GTGAACATGGACGGGAAC AACAGACTCGCTTTTGGG AAGGGGAACCAAGTGGTG GTCATACCA (SEQ ID NO: 115) | GACAGAGGATTTCCTCCTC ACTCTGGAGTCCGCTACCA GCTCCCAGACATCTGTGTAC TTCTGTGCCAGCGTTTACGG GTCTGAAGCTTTCTTTGGAC AAGGCACCAGACTCACAGT TGTA (SEQ ID NO: 116) | | |
| i2B7 | ATGGCCTCTGCACCCATCT CGATGCTTGCGATGCTCTT CACATTGAGTGGGCTGAG AGCTCAGTCAGTGGCTCA GCCGGAAGATCAGGTCAA CGTTGCTGAAGGGAATCC TCTGACTGTGAAATGCAC CTATTCAGTCTCTGGAAAC CCTTATCTTTTTTGGTATGT TCAATACCCCAACCGAGG CCTCCAGTTCCTTCTGAAA TACATCACAGGGGATAACC TGGTTAAAGGCAGCTATG GCTTTGAAGCTGAATTTAA CAAGAGCCAAACCTCCTT CCACCTGAAGAAACCATC TGCCCTTGTGAGCGACTC CGCTTTGTACTTCTGTGCT GTGAACCCCACGGGAGGA GGAAACAAACTCACCTTT GGGACAGGCACTCAGCTA AAAGTGGAACTC (SEQ ID NO: 117) | ATGGCCACCAGGCTCCTCT GCTGTGTGGTTCTTTGTCTC CTGGGAGAAGAGCTTATAG ATGCTAGAGTCACCCAGAC ACCAAGGGACAAGGTGAC AGAGATGGGACAAGAAGTA ACAATGAGATGTCAGCCAA TTTTAGGCCACAATACTGTT TTCTGGTACAGACAGACCA TGATGCAAGGACTGGAGTT GCTGGCTTACTTCCGCAAC CGGGCTCCTCTAGATGATTC GGGGATGCCGAAGGATCGA TTCTCAGCAGAGATGCCTG ATGCAACTTTAGCCACTCTG AAGATCCAGCCCTCAGAAC CCAGGGACTCAGCTGTGTA TTTTTGTGCTAGTGGTTTGG GGGGAAATCAGCCCCAGCA TTTTGGTGATGGGACTCGA CTCTCCATCCTA (SEQ ID NO: 118) | 399 | 396 |
| ⑥2C7 | ATGAAATCCTTGAGAGTTT TACTAGTGATCCTGTGGCT TCAGTTGAGCTGGGTTTG GAGCCAACAGAAGGAGGT GGAGCAGAATTCTGGACC CCTCAGTGTTCCAGAGGG AGCCATTGCCTCTCTCAAC TGCACTTACAGTGACCGA GGTTCCCAGTCCTTCTTCT GGTACAGACAATATTCTGG GAAAAGCCCTGAGTTGAT AATGTTCATATACTCCAAT GGTGACAAAGAAGATGGA AGGTTTACAGCACAGCTC AATAAAGCCAGCCAGTAT GTTTCTCTGCTCATCAGAG ACTCCCAGCCCAGTGATTC AGCCACCTACCTCTGTGCC GTGAAGGGGACGGGCAG GAGAGCACTTACTTTTGG | ATGGGCTGCAGGCTGCTCT GCTGTGCGGTTCTCTGTCTC CTGGGAGCAGTTCCCATAG ACACTGAAGTTACCCAGAC ACCAAAACACCTGGTCATG GGAATGACAAATAAGAAGT CTTTGAAATGTGAACAACA TATGGGGCACAGGGCTATGT ATTGGTACAAGCAGAAAGC TAAGAAGCCACCGGAGCTC ATGTTTGTCTACAGCTATGA GAAACTCTCTATAAATGAAA GTGTGCCAAGTCGCTTCTC ACCTGAATGCCCCAACAGC TCTCTCTTAAACCTTCACCT ACACGCCCTGCAGCCAGAA GACTCAGCCCTGTATCTCTG CGCCAGCAGCCAAAGTGGG GTCGGGAACACTGAAGCTT TCTTTGGACAAGGCACCAG | 396 | 399 |
| | GAGTGGAACAAGACTCCA AGTGCAACCA (SEQ ID NO: 119) | ACTCACAGTTGTA (SEQ ID NO: 120) | | |
| ⑨5C6 | ATGCTCCTGGAGCTTATCC CACTGCTGGGGATACATTT TGTCCTGAGAACTGCCAG AGCCCAGTCAGTGACCCA GCCTGACATCCACATCACT GTCTCTGAAGGAGCCTCA CTGGAGTTGAGATGTAAC TATTCCTATGGGGCAACAC CTTATCTCTTCTGGTATGTC CAGTCCCCCGGCCAAGGC CTCCAGCTGCTCCTGAAG TACTTTTCAGGAGACACTC TGGTTCAAGGCATTAAAG GCTTTGAGGCTGAATTTAA GAGGAGTCAATCTTCCTTC AATCTGAGGAAACCCTCT GTGCATTGGAGTGATGCTG CTGAGTACTTCTGTGCTGT GGGTTCGCCTTCAGGATAC AGCACCCTCACCTTTGGG AAGGGGACTATGCTTCTAG TCTCTCCA (SEQ ID NO: 121) | ATGGGCCCCCAGCTCCTTG GCTATGTGGTCCTTTGCCTT CTAGGAGCAGGCCCCCTGG AAGCCCAAGTGACCCAGAA CCCAAGATACCTCATCACA GTGACTGGAAAGAAGTTAA CAGTGACTTGTTCTCAGAAT ATGAACCATGAGTATATGTC CTGGTATCGACAAGACCCA GGGCTGGGCTTAAGGCAGA TCTACTATTCAATGAATGTT GAGGTGACTGATAAGGGAG ATGTTCCTGAAGGGTACAA AGTCTCTCGAAAAGAGAAG AGGAATTTCCCCCTGATCCT GGAGTCGCCCAGCCCCAAC CAGACCTCTCTGTACTTCTG TGCCAGCAGCCCCACTAGG TTCTACGAGCAGTACTTCGG GCCGGGCACCAGGCTCACG GTCACA (SEQ ID NO: 122) | 399 | 393 |

**[Table 4]**

| Table 4: Amino acid sequences of HLA-A24-restricted T-cell receptors that recognize GPC3-derived peptide (EYILSLEEL) | | | | |
|---|---|---|---|---|
| clone | Sequence TRA | Sequence TRB | TRA (bp) | TRB (bp) |
| 8 | MVLKFSVSILWIQLAWVST QLLEQSPQFLSIQEGENLTV YCNSSSVFSSLQWYRQEP GEGPVLLVTVVTGGEVKK LKRLTFQFGDARKDSSLHI TAAQPGDTGLYLCAGRYS SASKIIFGSGTRLSIRP (SEQ ID NO: 123) | MGFRLLCCVAFCLLGAGPV DSGVTQTPKHLITATGQRVT LRCSPRSGDLSVYWYQQSL DQGLQFLIQYYNGEERAKG NILERFSAQQFPDLHSELNL SSLELGDSALYFCASSVGGG APNEQFFGPGTRLTVL (SEQ ID NO: 124) | 129 | 133 |
| G9 | METLLGLLILWLQLQWVS SKQEVTQIPAALSVPEGEN LVLNCSFTDSAIYNLQWFR QDPGKGLTSLLLIQSSQRE QTSGRLNASLDKSSGRSTL YIAASQPGDSATYLCAVTR RALTFGSGTRLQVQP (SEQ ID NO: 125) | MGSRLLCWVLLCLLGAGPV KAGVTQTPRYLIKTRGQQV TLSCSPISGHRSVSWYQQTP GQGLQFLFEYFSETQRNKG NFPGRFSGRQFSNSRSEMNV STLELGDSALYLCASSVTSG RTHTDTQYFGPGTRLTVL (SEQ ID NO: 126) | 128 | 135 |
| (46)2A | MMISLRVLLVILWLQLSW | MGFRLLCCVAFCLLGAGPV | 131 | 133 |
| 6 | VWSQRKEVEQDPGPFNVP EGATVAFNCTYSNSASQSF FWYRQDCRKEPKLLMSVY SSGNEDGRFTAQLNRASQ YISLLIRDSKLSDSATYLCK ISGGYNKLIFGAGTRLAVH P (SEQ ID NO: 127) | DSGVTQTPKHLITATGQRVT LRCSPRSGDLSVYWYQQSL DQGLQFLIQYYNGEERAKG NILERFSAQQFPDLHSELNL SSLELGDSALYFCASSVGGG LGTEAFFGQGTRLTVV (SEQ ID NO: 128) | | |
| i12G6 | MRQVARVIVFLTLSTLSLA KTTQPISMDSYEGQEVNIT CSHNNIATNDYITWYQQFP SQGPRFIIQGYKTKVTNEV ASLFIPADRKSSTLSLPRVS LSDTAVYYCLVGHSGNTPL VFGKGTRLSVIA (SEQ ID NO: 129) | MGFRLLCCVAFCLLGAGPV DSGVTQTPKHLITATGQRVT LRCSPRSGDLSVYWYQQSL DQGLQFLIQYYNGEERAKG NILERFSAQQFPDLHSELNL SSLELGDSALYFCASSVGGG AGNTIYFGEGSWLTVV (SEQ ID NO: 131) | 127 | 133 |
| | MISLRVLLVILWLQLSWV WSQRKEVEQDPGPFNVPE GATVAFNCTYSNSASQSFF WYRQDCRKEPKLLMSVYS SGNEDGRFTAQLNRASQYI SLLIRDSKLSDSATYLCVV NTGRRALTFGSGTRLQVQP (SEQ ID NO: 130) | | 130 | |
| eC1 | MAGIRALFMYLWLQLDW VSRGESVGLHLPTLSVQEG DNSIINCAYSNSASDYFIW YKQESGKGPQFIIDIRSNM DKRQGQRVTVLLNKTVKH LSLQIAATQPGDSAVYFCA GRTGFQKLVFGTGTRLLVS P (SEQ ID NO: 132) | MGFRLLCCVAFCLLGAGPV DSGVTQTPKHLITATGQRVT LRCSPRSGDLSVYWYQQSL DQGLQFLIQYYNGEERAKG NILERFSAQQFPDLHSELNL SSLELGDSALYFCASSVGGG LYNEQFFGPGTRLTVL (SEQ ID NO: 133) | 131 | 133 |
| eC7 | METLLGVSLVILWLQLARV NSQQGEEDPQALSIQEGEN ATMNCSYKTSINNLQWYR QNSGRGLVHLILIRSNERE KHSGRLRVTLDTSKKSSSL LITASRAADTASYFCAVRY SSASKIIFGSGTRLSIRP (SEQ ID NO: 134) | MGFRLLCCVAFCLLGAGPV DSGVTQTPKHLITATGQRVT LRCSPRSGDLSVYWYQQSL DQGLQFLIQYYNGEERAKG NILERFSAQQFPDLHSELNL SSLELGDSALYFCASSAGGG SNNEQFFGPGTRLTVL (SEQ ID NO: 135) | 131 | 133 |
| eD3 | MISLRVLLVILWLQLSWV WSQRKEVEQDPGPFNVPE GATVAFNCTYSNSASQSFF WYRQDCRKEPKLLMSVYS SGNEDGRFTAQLNRASQYI SLLIRDSKLSDSATYLCVV KGGNNFNKFYFGSGTKLN VKP (SEQ ID NO: 136) | MSIGLLCCVAFSLLWASPVN AGVTQTPKFQVLKTGQSMT LQCAQDMNHNSMYWYRQ DPGMGLRLIYYSASEGTTD KGEVPNGYNVSRLNKREFS LRLESAAPSQTSVYFCASSH RVNSNQPQHFGDGTRLSIL (SEQ ID NO: 137) | 132 | 133 |
| i7E6 | MKPTLISVLVIIFILRGTRA QRVTQPEKLLSVFKGAPVE LKCNYSYSGSPELFWYVQ YSRQRLQLLLRHISRESIKG | MGFRLLCCVAFCLLGAGPV DSGVTQTPKHLITATGQRVT LRCSPRSGDLSVYWYQQSL DQGLQFLIQYYNGEERAKG | 128 | 133 |
| | FTADLNKGETSFHLKKPFA QEEDSAMYYCALANQAG TALIFGKGTTLSVSS (SEQ ID NO: 138) | NILERFSAQQFPDLHSELNL SSLELGDSALYFCASSPGGG ASSPLHFGNGTRLTVT (SEQ ID NO: 140) | | |
| | METLLGLLILWLQLQWVS SKQEVTQIPAALSVPEGEN LVLNCSFTDSAIYNLQWFR QDPGKGLTSLLLIQSSQRE QTSGRLNASLDKSSGRSTL YIAASQPGDSATYLCAVKR RAAGNKLTFGGGTRVLVK P (SEQ ID NO: 139) | | 132 | |
| i2F3 | MISLRVLLVILWLQLSWV WSQRKEVEQDPGPFNVPE GATVAFNCTYSNSASQSFF WYRQDCRKEPKLLMSVYS SGNEDGRFTAQLNRASQYI SLLIRDSKLSDSATYLCVV NKSWGKLQFGAGTQVVV TP (SEQ ID NO: 141) | MGFRLLCCVAFCLLGAGPV DSGVTQTPKHLITATGQRVT LRCSPRSGDLSVYWYQQSL DQGLQFLIQYYNGEERAKG NILERFSAQQFPDLHSELNL SSLELGDSALYFCASSAGTS GNEQFFGPGTRLTVL (SEQ ID NO: 142) | 130 | 132 |
| i3C2 | MWGVFLLYVSMKMGGTT GQNIDQPTEMTATEGAIVQ INCTYQTSGFNGLFWYQQ HAGEAPTFLSYNVLDGLE EKGRFSSFLSRSKGYSYLL LKELQMKDSASYLCAVRV WKAAGNKLTFGGGTRVLV KP (SEQ ID NO: 143) | MLLLLLLLGPGSGLGAVVS QHPSWVICKSGTSVKIECRS LDFQATTMFWYRQFPKQSL MLMATSNEGSKATYEQGVE KDKFLINHASLTLSTLTVTS AHPEDSSFYICSARRLAGGG NEQFFGPGTRLTVL (SEQ ID NO: 144) | 129 | 131 |
| i6E9 | MISLRVLLVILWLQLSWV WSQRKEVEQDPGPFNVPE GATVAFNCTYSNSASQSFF WYRQDCRKEPKLLMSVYS SGNEDGRFTAQLNRASQYI SLLIRDSKLSDSATYLCVV NLGNYGQNFVFGPGTRLS VLP (SEQ ID NO: 145) | MGFRLLCCVAFCLLGAGPV DSGVTQTPKHLITATGQRVT LRCSPRSGDLSVYWYQQSL DQGLQFLIQYYNGEERAKG NILERFSAQQFPDLHSELNL SSLELGDSALYFCASSVGGG LSNEQFFGPGTRLTVL (SEQ ID NO: 146) | 132 | 133 |
| e1D8 | MKTFAGFSFLFLWLQLDC MSRGEDVEQSLFLSVREG DSSVINCTYTDSSSTYLYW YKQEPGAGLQLLTYIFSNM DMKQDQRLTVLLNKKDK HLSLRIADTQTGDSAIYFC AENHSSASKIIFRCGCGPAE (SEQ ID NO: 147) | MGFRLLCCVAFCLLGAGPV DSGVTQTPKHLITATGQRVT LRCSPRSGDLSVYWYQQSL DQGLQFLIQYYNGEERAKG NILERFSAQQFPDLHSELNL SSLELGDSALYFCASSVGSG RNYEQYFGPGTRLTVT (SEQ ID NO: 148) | 130 | 133 |

**[Table 5]**

| Table 5: Gene sequences for HLA-A24-restricted T-cell receptors that recognize GPC3-derived peptide (EYILSLEEL) | | | | |
|---|---|---|---|---|
| clone | Sequence TRA | Sequence TRB | TRA (bp) | TRB (bp) |
| 8 | ATGGTCCTGAAATTCTCCG TGTCCATTCTTTGGATTCAG TTGGCATGGGTGAGCACCC AGCTGCTGGAGCAGAGCC CTCAGTTTCTAAGCATCCA AGAGGGAGAAAATCTCAC TGTGTACTGCAACTCCTCA AGTGTTTTTTCCAGCTTAC AATGGTACAGACAGGAGC CTGGGGAAGGTCCTGTCCT CCTGGTGACAGTAGTTACG GGTGGAGAAGTGAAGAAG CTGAAGAGACTAACCTTTC AGTTTGGTGATGCAAGAAA GGACAGTTCTCTCCACATC ACTGCAGCCCAGCCTGGTG ATACAGGCCTCTACCTCTG TGCAGGCCGGTACAGCAG TGCTTCCAAGATAATCTTT GGATCAGGGACCAGACTC AGCATCCGGCCAA (SEQ ID NO: 149) | ATGGGCTTCAGGCTCCTCT GCTGTGTGGCCTTTTGTCT CCTGGGAGCAGGCCCAGT GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC GTAGGCGGGGGGGCACCC AATGAGCAGTTCTTCGGGC CAGGGACACGGCTCACCG TGCTA (SEQ ID NO: 150) | 388 | 399 |
| G9 | ATGGAGACCCTCTTGGGCC TGCTTATCCTTTGGCTGCA GCTGCAATGGGTGAGCAG CAAACAGGAGGTGACACA GATTCCTGCAGCTCTGAGT GTCCCAGAAGGAGAAAAC TTGGTTCTCAACTGCAGTT TCACTGATAGCGCTATTTAC AACCTCCAGTGGTTTAGGC AGGACCCTGGGAAAGGTC TCACATCTCTGTTGCTTATT CAGTCAAGTCAGAGAGAG CAAACAAGTGGAAGACTT AATGCCTCGCTGGATAAAT CATCAGGACGTAGTACTTT ATACATTGCAGCTTCTCAG CCTGGTGACTCAGCCACCT ACCTCTGTGCTGTTACCAG GAGAGCACTTACTTTTGGG AGTGGAACAAGACTCCAA GTGCAACCAA (SEQ ID NO: 151) | ATGGGCTCCAGGCTGCTCT GTTGGGTGCTGCTTTGTCT CCTGGGAGCAGGCCCAGT AAAGGCTGGAGTCACTCA AACTCCAAGATATCTGATC AAAACGAGAGGACAGCAA GTGACACTGAGCTGCTCCC CTATCTCTGGGCATAGGAG TGTATCCTGGTACCAACAG ACCCCAGGACAGGGCCTT CAGTTCCTCTTTGAATACTT CAGTGAGACACAGAGAAA CAAAGGAAACTTCCCTGGT CGATTCTCAGGGCGCCAGT TCTCTAACTCTCGCTCTGA GATGAATGTGAGCACCTTG GAGCTGGGGGACTCGGCC CTTTATCTTTGCGCCAGCA GCGTAACTAGCGGGAGAA CACACACAGATACGCAGTA TTTTGGCCCAGGCACCCGG CTGACAGTGCTC (SEQ ID NO: 152) | 385 | 405 |
| (46)2 A6 | ATGATGATATCCTTGAGAG TTTTACTGGTGATCCTGTG GCTTCAGTTAAGCTGGGTT TGGAGCCAACGGAAGGAG GTGGAGCAGGATCCTGGA | ATGGGCTTCAGGCTCCTCT GCTGTGTGGCCTTTTGTCT CCTGGGAGCAGGCCCAGT GGATTCTGGAGTCACACAA ACCCCAAAGCACCTGATCA | 394 | 399 |
| | CCCTTCAATGTTCCAGAGG GAGCCACTGTCGCTTTCAA CTGTACTTACAGCAACAGT GCTTCTCAGTCTTTCTTCTG GTACAGACAGGATTGCAG GAAAGAACCTAAGTTGCT GATGTCCGTATACTCCAGT GGTAATGAAGATGGAAGGT TTACAGCACAGCTCAATAG AGCCAGCCAGTATATTTCC CTGCTCATCAGAGACTCCA AGCTCAGTGATTCAGCCAC CTACCTCTGTAAGATTTCTG GTGGCTACAATAAGCTGAT TTTTGGAGCAGGGACCAG GCTGGCTGTACACCCAT (SEQ ID NO: 153) | CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC GTAGGTGGGGGTTTAGGGA CTGAAGCTTTCTTTGGACA AGGCACCAGACTCACAGT TGTA (SEQ ID NO: 154) | | |
| i12Gb -1 | ATGAGGCAAGTGGCGAGA GTGATCGTGTTCCTGACCC TGAGTACTTTGAGCCTTGC TAAGACCACCCAGCCCATC TCCATGGACTCATATGAAG GACAAGAAGTGAACATAA CCTGTAGCCACAACAACAT TGCTACAAATGATTATATCA CGTGGTACCAACAGTTTCC CAGCCAAGGACCACGATTT ATTATTCAAGGATACAAGA CAAAAGTTACAAACGAAG TGGCCTCCCTGTTTATCCCT GCCGACAGAAAGTCCAGC ACTCTGAGCCTGCCCCGGG TTTCCCTGAGCGACACTGC TGTGTACTACTGCCTCGTG GGACATTCAGGAAACACA CCTCTTGTCTTTGGAAAGG GCACAAGACTTTCTGTGAT TGCAA (SEQ ID NO: 155) | ATGGGCTTCAGGCTCCTCT GCTGTGTGGCCTTTTGTCT CCTGGGAGCAGGCCCAGT GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC GTAGGAGGGGGAGCTGGA AACACCATATATTTTGGAG AGGGAAGTTGGCTCACTGT TGTA (SEQ ID NO: 156) | 382 | 399 |
| i12Gb -2 | ATGATATCCTTGAGAGTTTT ACTGGTGATCCTGTGGCTT CAGTTAAGCTGGGTTTGGA GCCAACGGAAGGAGGTGG AGCAGGATCCTGGACCCTT CAATGTTCCAGAGGGAGCC ACTGTCGCTTTCAACTGTA CTTACAGCAACAGTGCTTC TCAGTCTTTCTTCTGGTAC AGACAGGATTGCAGGAAA GAACCTAAGTTGCTGATGT | ATGGGCTTCAGGCTCCTCT GCTGTGTGGCCTTTTGTCT CCTGGGAGCAGGCCCAGT GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT | 390 | 399 |
| | CCGTATACTCCAGTGGTAA TGAAGATGGAAGGTTTACA GCACAGCTCAATAGAGCCA GCCAGTATATTTCCCTGCTC ATCAGAGACTCCAAGCTCA GTGATTCAGCCACCTACCT CTGTGTGGTGAACACGGG CAGGAGAGCACTTACTTTT GGGAGTGGAACAAGACTC CAAGTGCAACCA (SEQ ID NO: 157) | AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC GTAGGAGGGGGAGCTGGA AACACCATATATTTTGGAG AGGGAAGTTGGCTCACTGT TGTA (SEQ ID NO: 158) | | |
| eC1 | ATGGCAGGCATTCGAGCTT TATTTATGTACTTGTGGCTG CAGCTGGACTGGGTGAGC AGAGGAGAGAGTGTGGGG CTGCATCTTCCTACCCTGA GTGTCCAGGAGGGTGACA ACTCTATTATCAACTGTGCT TATTCAAACAGCGCCTCAG ACTACTTCATTTGGTACAA GCAAGAATCTGGAAAAGG TCCTCAATTCATTATAGACA TTCGTTCAAATATGGACAA AAGGCAAGGCCAAAGAGT CACCGTTTTATTGAATAAG ACAGTGAAACATCTCTCTC TGCAAATTGCAGCTACTCA ACCTGGAGACTCAGCTGTC TACTTTTGTGCAGGGCGAA CAGGCTTTCAGAAACTTGT ATTTGGAACTGGCACCCGA CTTCTGGTCAGTCCA (SEQ ID NO: 159) | ATGGGCTTCAGGCTCCTCT GCTGTGTGGCCTTTTGTCT CCTGGGAGCAGGCCCAGT GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC GTAGGAGGGGGGCTCTAC AATGAGCAGTTCTTCGGGC CAGGGACACGGCTCACCG TGCTA (SEQ ID NO: 160) | 393 | 399 |
| eC7 | ATGGAAACTCTCCTGGGAG TGTCTTTGGTGATTCTATGG CTTCAACTGGCTAGGGTGA ACAGTCAACAGGGAGAAG AGGATCCTCAGGCCTTGAG CATCCAGGAGGGTGAAAAT GCCACCATGAACTGCAGTT ACAAAACTAGTATAAACAA TTTACAGTGGTATAGACAA AATTCAGGTAGAGGCCTTG TCCACCTAATTTTAATACGT TCAAATGAAAGAGAGAAA CACAGTGGAAGATTAAGA GTCACGCTTGACACTTCCA AGAAAAGCAGTTCCTTGTT GATCACGGCTTCCCGGGCA GCAGACACTGCTTCTTACT TCTGTGCTGTGAGGTACAG | ATGGGCTTCAGGCTCCTCT GCTGTGTGGCCTTTTGTCT CCTGGGAGCAGGCCCAGT GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC | 394 | 399 |
| | CAGTGCTTCCAAGATAATC TTTGGATCAGGGACCAGAC TCAGCATCCGGCCAA (SEQ ID NO: 161) | GCAGGAGGGGGTAGTAAC AATGAGCAGTTCTTCGGGC CAGGGACACGGCTCACCG TGCTA (SEQ ID NO: 162) | | |
| eD3 | ATGATATCCTTGAGAGTTTT ACTGGTGATCCTGTGGCTT CAGTTAAGCTGGGTTTGGA GCCAACGGAAGGAGGTGG AGCAGGATCCTGGACCCTT CAATGTTCCAGAGGGAGCC ACTGTCGCTTTCAACTGTA CTTACAGCAACAGTGCTTC TCAGTCTTTCTTCTGGTAC AGACAGGATTGCAGGAAA GAACCTAAGTTGCTGATGT CCGTATACTCCAGTGGTAA TGAAGATGGAAGGTTTACA GCACAGCTCAATAGAGCCA GCCAGTATATTTCCCTGCTC ATCAGAGACTCCAAGCTCA GTGATTCAGCCACCTACCT CTGTGTGGTGAAAGGTGG GAACAACTTCAACAAATTT TACTTTGGATCTGGGACCA AACTCAATGTAAAACCAA (SEQ ID NO: 163) | ATGAGCATCGGGCTCCTGT GCTGTGTGGCCTTTTCTCT CCTGTGGGCAAGTCCAGTG AATGCTGGTGTCACTCAGA CCCCAAAATTCCAGGTCCT GAAGACAGGACAGAGCAT GACACTGCAGTGTGCCCA GGATATGAACCATAACTCC ATGTACTGGTATCGACAAG ACCCAGGCATGGGACTGA GGCTGATTTATTACTCAGCT TCTGAGGGTACCACTGACA AAGGAGAAGTCCCCAATG GCTACAATGTCTCCAGATT AAACAAACGGGAGTTCTC GCTCAGGCTGGAGTCGGCT GCTCCCTCCCAGACATCTG TGTACTTCTGTGCCAGCAG CCACAGGGTTAATAGCAAT CAGCCCCAGCATTTTGGTG ATGGGACTCGACTCTCCAT CCTA (SEQ ID NO: 164) | 397 | 399 |
| i7E6-1 | ATGAAGCCCACCCTCATCT CAGTGCTTGTGATAATATTT ATACTCAGAGGAACAAGA GCCCAGAGAGTGACTCAG CCCGAGAAGCTCCTCTCTG TCTTTAAAGGGGCCCCAGT GGAGCTGAAGTGCAACTAT TCCTATTCTGGGAGTCCTG AACTCTTCTGGTATGTCCA GTACTCCAGACAACGCCTC CAGTTACTCTTGAGACACA TCTCTAGAGAGAGCATCAA AGGCTTCACTGCTGACCTT AACAAAGGCGAGACATCT TTCCACCTGAAGAAACCAT TTGCTCAAGAGGAAGACT CAGCCATGTATTACTGTGCT CTTGCCAACCAGGCAGGA ACTGCTCTGATCTTTGGGA AGGGAACCACCTTATCAGT GAGTTCCA (SEQ ID NO: 165) | ATGGGCTTCAGGCTCCTCT GCTGTGTGGCCTTTTGTCT CCTGGGAGCAGGCCCAGT GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC CCGGGTGGAGGGGCGAGT TCACCCCTCCACTTTGGGA ATGGGACCAGGCTCACTGT GACA (SEQ ID NO: 166) | 385 | 399 |
| i7E6-2 | ATGGAGACCCTCTTGGGCC TGCTTATCCTTTGGCTGCA GCTGCAATGGGTGAGCAG | ATGGGCTTCAGGCTCCTCT GCTGTGTGGCCTTTTGTCT CCTGGGAGCAGGCCCAGT | 397 | 399 |
| | CAAACAGGAGGTGACACA GATTCCTGCAGCTCTGAGT GTCCCAGAAGGAGAAAAC TTGGTTCTCAACTGCAGTT TCACTGATAGCGCTATTTAC AACCTCCAGTGGTTTAGGC AGGACCCTGGGAAAGGTC TCACATCTCTGTTGCTTATT CAGTCAAGTCAGAGAGAG CAAACAAGTGGAAGACTT AATGCCTCGCTGGATAAAT CATCAGGACGTAGTACTTT ATACATTGCAGCTTCTCAG CCTGGTGACTCAGCCACCT ACCTCTGTGCTGTGAAACG ACGAGCTGCAGGCAACAA GCTAACTTTTGGAGGAGGA ACCAGGGTGCTAGTTAAAC CAA (SEQ ID NO: 167) | GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC CCGGGTGGAGGGGCGAGT TCACCCCTCCACTTTGGGA ATGGGACCAGGCTCACTGT GACA (SEQ ID NO: 168) | | |
| i2F3 | ATGATATCCTTGAGAGTTTT ACTGGTGATCCTGTGGCTT CAGTTAAGCTGGGTTTGGA GCCAACGGAAGGAGGTGG AGCAGGATCCTGGACCCTT CAATGTTCCAGAGGGAGCC ACTGTCGCTTTCAACTGTA CTTACAGCAACAGTGCTTC TCAGTCTTTCTTCTGGTAC AGACAGGATTGCAGGAAA GAACCTAAGTTGCTGATGT CCGTATACTCCAGTGGTAA TGAAGATGGAAGGTTTACA GCACAGCTCAATAGAGCCA GCCAGTATATTTCCCTGCTC ATCAGAGACTCCAAGCTCA GTGATTCAGCCACCTACCT CTGTGTGGTGAACAAGAG CTGGGGGAAATTGCAGTTT GGAGCAGGGACCCAGGTT GTGGTCACCCCAG (SEQ ID NO: 169) | ATGGGCTTCAGGCTCCTCT GCTGTGTGGCCTTTGTCT CCTGGGAGCAGGCCCAGT GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC GCCGGGACTAGCGGAAAT GAGCAGTTCTTCGGGCCAG GGACACGGCTCACCGTGCT A (SEQ ID NO: 170) | 391 | 396 |
| i3C2 | ATGTGGGGAGTTTTCCTTC TTTATGTTTCCATGAAGATG GGAGGCACTACAGGACAA AACATTGACCAGCCCACTG AGATGACAGCTACGGAAG GTGCCATTGTCCAGATCAA CTGCACGTACCAGACATCT GGGTTCAACGGGCTGTTCT GGTACCAGCAACATGCTGG | ATGCTGCTGCTTCTGCTGC TTCTGGGGCCAGGCTCCGG GCTTGGTGCTGTCGTCTCT CAACATCCGAGCTGGGTTA TCTGTAAGAGTGGAACCTC TGTGAAGATCGAGTGCCGT TCCCTGGACTTTCAGGCCA CAACTATGTTTTGGTATCGT CAGTTCCCGAAACAGAGT | 388 | 393 |
| | CGAAGCACCCACATTTCTG TCTTACAATGTTCTGGATG GTTTGGAGGAGAAAGGTC GTTTTTCTTCATTCCTTAGT CGGTCTAAAGGGTACAGTT ACCTCCTTTTGAAGGAGCT CCAGATGAAAGACTCTGCC TCTTACCTCTGTGCTGTGA GAGTGTGGAAAGCTGCAG GCAACAAGCTAACTTTTGG AGGAGGAACCAGGGTGCT AGTTAAACCAA (SEQ ID NO: 171) | CTCATGCTGATGGCAACTT CCAATGAGGGCTCCAAGG CCACATACGAGCAAGGCGT CGAGAAGGACAAGTTTCT CATCAACCATGCAAGCCTG ACCTTGTCCACTCTGACAG TGACCAGTGCCCATCCTGA AGACAGCAGCTTCTACATC TGCAGTGCTAGAAGGCTAG CGGGAGGAGGGAATGAGC AGTTCTTCGGGCCAGGGAC ACGGCTCACCGTGCTA (SEQ ID NO: 172) | | |
| i6E9 | ATGATATCCTTGAGAGTTTT ACTGGTGATCCTGTGGCTT CAGTTAAGCTGGGTTTGGA GCCAACGGAAGGAGGTGG AGCAGGATCCTGGACCCTT CAATGTTCCAGAGGGAGCC ACTGTCGCTTTCAACTGTA CTTACAGCAACAGTGCTTC TCAGTCTTTCTTCTGGTAC AGACAGGATTGCAGGAAA GAACCTAAGTTGCTGATGT CCGTATACTCCAGTGGTAA TGAAGATGGAAGGTTTACA GCACAGCTCAATAGAGCCA GCCAGTATATTTCCCTGCTC ATCAGAGACTCCAAGCTCA GTGATTCAGCCACCTACCT CTGTGTGGTGAACCTCGGG AACTATGGTCAGAATTTTG TCTTTGGTCCCGGAACCAG ATTGTCCGTGCTGCCCT (SEQ ID NO: 173) | ATGGGCTTCAGGCTCCTCT GCTGTGTGGCCTTTTGTCT CCTGGGAGCAGGCCCAGT GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG AGCTGGGGGACTCAGCTTT GTATTTCTGTGCCAGCAGC GTAGGGGGGGGCCTAAGC AATGAGCAGTTCTTCGGGC CAGGGACACGGCTCACCG TGCTA (SEQ ID NO: 174) | 397 | 399 |
| E1D8 | ATGAAGACATTTGCTGGAT TTTCGTTCCTGTTTTTGTGG CTGCAGCTGGACTGTATGA GTAGAGGAGAGGATGTGG AGCAGAGTCTTTTCCTGAG TGTCCGAGAGGGAGACAG CTCCGTTATAAACTGCACT TACACAGACAGCTCCTCCA CCTACTTATACTGGTATAAG CAAGAACCTGGAGCAGGT CTCCAGTTGCTGACGTATAT TTTTTCAAATATGGACATG AAACAAGACCAAAGACTC ACTGTTCTATTGAATAAAA AGGATAAACATCTGTCTCT GCGCATTGCAGACACCCAG | ATGGGCTTCAGGCTCCTCT GCTGTGTGGCCTTTTGTCT CCTGGGAGCAGGCCCAGT GGATTCTGGAGTCACACAA ACCCCAAAGCACCTGATCA CAGCAACTGGACAGCGAG TGACGCTGAGATGCTCCCC TAGGTCTGGAGACCTCTCT GTGTACTGGTACCAACAGA GCCTGGACCAGGGCCTCC AGTTCCTCATTCAGTATTAT AATGGAGAAGAGAGAGCA AAAGGAAACATTCTTGAAC GATTCTCCGCACAACAGTT CCCTGACTTGCACTCTGAA CTAAACCTGAGCTCTCTGG | 390 | 399 |
| | ACTGGGGACTCAGCTATCT ACTTCTGTGCAGAGAACCA CAGCAGTGCTTCCAAGATA ATCTTTCGCTGCGGCTGTG GTCCAGCTGAA (SEQ ID NO: 175) | AGCTGGGGGACTCAGCTTT GTATTTCTGTGCCAGCAGC GTAGGGTCGGGACGAAATT ACGAGCAGTACTTCGGGCC GGGCACCAGGCTCACGGT CACA (SEQ ID NO: 176) | | |

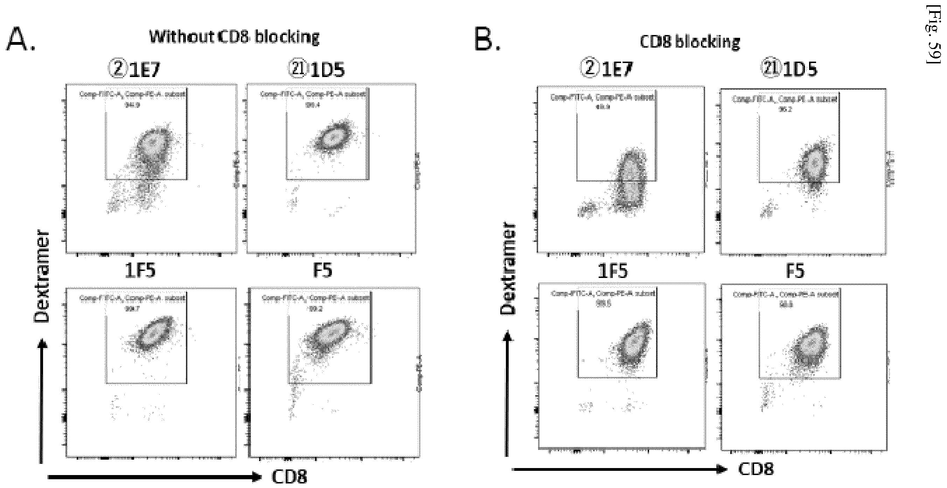

Fig. 59 shows establishment of peptide-specific CTL clones from the peripheral blood of a patient inoculated with a FOXM1 peptide vaccine. Dextramer-positive T cells were selected by single sorting under conditions with CD8 blocking from the peripheral blood of a patient inoculated with a cancer peptide vaccine with a FOXM1-derived peptide, and cultured on allofeeder cells to establish T cell clones.

A and B of Fig. 59 show evaluation of the avidities of TCRs by Dextramer staining for HLA-A24-restricted FOXM1-specific T cell clones. Comparison and examination were performed between A. without CD8 blocking and B. with CD8 blocking.

### <Results>

The peripheral blood of a patient inoculated with the FOXM1 peptide vaccine was subjected to stimulation culture in the presence of the peptide, and reactive clones were established through HLA-A24 Dextramer staining. Evaluation of the avidities of the TCRs of the established FOXM1-specific reactive T cell clones through Dextramer staining found that three of four clones had good stainability even under conditions with CD8 inhibition, but the 21E7 clone had largely lower stainability due to CD8 inhibition. The amino acid sequences for and gene sequences of TCR genes isolated from the clones are shown in tables.

**[Table 6]**

| Table 6: Amino acid sequences of HLA-A24-restricted T-cell receptors that recognize FOXM1-derived peptide (IYTWIEDHF) | | | | |
|---|---|---|---|---|
| Clone | SequenCe TRA | SequenCe TRB | TRA (bp) | TRB (bp) |
| F5 | MALQSTLGAVWLGLLLNSL WKVAESKDQVFQPSTVASSE GAVVEIFCNHSVSNAYNFFW YLHFPGCAPRLLVKGSKPSQ QGRYNMTYERFSSSLLILQVR EADAAVYYCAVNQAGTALIF | MGCRLLCCAVLCLLGAVPIDT EVTQTPKHLVMGMTNKKSL KCEQHMGHRAMYWYKQKA KKPPELMFVYSYEKLSINESV PSRFSPECPNSSLLNLHLHAL QPEDSALYLCASSQERTSGRS | 130 | 137 |
| | GKGTTLSVSS (SEQ ID NO: 177) | ADTQYFGPGTRLTVLE (SEQ ID NO: 178) | | |
| ②1E7 | MLLLLIPVLGMIFALRDARAQ SVSQHNHHVILSEAASLELGC NYSYGGTVNLFWYVQYPGQ HLQLLLKYFSGDPLVKGIKGF EAEFIKSKFSFNLRKPSVQWS DTAEYFCAVRHGGTGTASKL TFGTGTRLQVTL (SEQ ID NO: 179) | MLLLLLLLGPGISLLLPGSLA GSGLGAVVSQHPSWVICKSG TSVKIECRSLDFQATTMFWY RQFPKQSLMLMATSNEGSKA TYEQGVEKDKFLINHASLTLS TLTVTSAHPEDSSFYICSARA LGGYTFGSGTRLTVVE (SEQ ID NO: 180) | 135 | 139 |
| (21)1 D5 | MMKSLRVLLVILWLQLSWV WSQQKEVEQDPGPLSVPEGA IVSLNCTYSNSAFQYFMWYR QYSRKGPELLMYTYSSGNKE DGRFTAQVDKSSKYISLFIRD SQPSDSATYLCAMSGHFNKF YFGSGTKLNVKP (SEQ ID NO: 181) | MGSRLLCWVLLCLLGAGPV KAGVTQTPRYLIKTRGQQVT LSCSPISGHRSVSWYQQTPGQ GLQFLFEYFSETQRNKGNFPG RFSGRQFSNSRSEMNVSTLEL GDSALYLCASSLRAGELFFGE GSRLTVLE (SEQ ID NO: 182) | 132 | 131 |
| 1F5 | MEKMLECAFIVLWLQLGWLS GEDQVTQSPEALRLQEGESSS LNCSYTVSGLRGLFWYRQDP GKGPEFLFTLYSAGEEKEKER LKATLTKKESFLHITAPKPEDS ATYLCAASNSGGSNYKLTFG KGTLLTVNP (SEQ ID NO: 183) | MGFRLLCCVAFCLLGAGPVD SGVTQTPKHLITATGQRVTLR CSPRSGDLSVYWYQQSLDQG LQFLIQYYNGEERAKGNILER FSAQQFPDLHSELNLSSLELG DSALYFCASSVDGKGTQYFG PGTRLLVLE (SEQ ID NO: 184) | 133 | 132 |

### [Table 7]

**Table 7: Gene sequencs for HLA-A24-restricted T-cell receptors that recognize FOXM1-derived peptide (IYTWIEDHF)**

| Clone | SequenCe TRA | SequenCe TRB | TRA (bp) | TRB (bp) |
|---|---|---|---|---|
| F5 | ATGGCTTTGCAGAGCACTC TGGGGGCGGTGTGGCTAG GGCTTCTCCTCAACTCTCT CTGGAAGGTTGCAGAAAG CAAGGACCAAGTGTTTCA GCCTTCCACAGTGGCATCT TCAGAGGGAGCTGTGGTG GAAATCTTCTGTAATCACT CTGTGTCCAATGCTTACAA CTTCTTCTGGTACCTTCACT TCCCGGGATGTGCACCAAG ACTCCTTGTTAAAGGCTCA AAGCCTTCTCAGCAGGGA CGATACAACATGACCTATG AACGGTTCTCTTCATCGCT GCTCATCCTCCAGGTGCGG GAGGCAGATGCTGCTGTTT | ATGGGCTGCAGGCTGCTCTGC TGTGCGGTTCTCTGTCTCCTG GGAGCAGTTCCCATAGACACT GAAGTTACCCAGACACCAAA ACACCTGGTCATGGGAATGAC AAATAAGAAGTCTTTGAAATG TGAACAACATATGGGGCACA GGGCTATGTATTGGTACAAGC AGAAAGCTAAGAAGCCACCG GAGCTCATGTTTGTCTACAGC TATGAGAAACTCTCTATAAAT GAAAGTGTGCCAAGTCGCTT CTCACCTGAATGCCCCAACA GCTCTCTCTTAAACCTTCACC TACACGCCCTGCAGCCAGAA GACTCAGCCCTGTATCTCTGC GCCAGCAGCCAAGAGCGGAC | 391 | 408 |
| | ACTACTGTGCTGTTAACCA GGCAGGAACTGCTCTGATC TTTGGGAAGGGAACCACC TTATCAGTGAGTTCCA (SEQ ID NO: 185) | TAGCGGGAGGTCGGCAGATA CGCAGTATTTTGGCCCAGGCA CCCGGCTGACAGTGCTC (SEQ ID NO: 186) | | |
| ②1E7 | ATGCTCCTGTTGCTCATACC AGTGCTGGGGATGATTTTT GCCCTGAGAGATGCCAGA GCCCAGTCTGTGAGCCAGC ATAACCACCACGTAATTCT CTCTGAAGCAGCCTCACTG GAGTTGGGATGCAACTATT CCTATGGTGGAACTGTTAA TCTCTTCTGGTATGTCCAGT ACCCTGGTCAACACCTTCA GCTTCTCCTCAAGTACTTT TCAGGGGATCCACTGGTTA AAGGCATCAAGGGCTTTGA GGCTGAATTTATAAAGAGT AAATTCTCCTTTAATCTGA GGAAACCCTCTGTGCAGTG GAGTGACACAGCTGAGTA CTTCTGTGCCGTGAGGCAC GGCGGTACCGGCACTGCC AGTAAACTCACCTTTGGGA CTGGAACAAGACTTCAGG TCACGCTCG (SEQ ID NO: 187) | ATGCTGCTGCTTCTGCTGCTT CTGGGGCCAGGTATAAGCCTC CTTCTACCTGGGAGCTTGGCA GGCTCCGGGCTTGGTGCTGTC GTCTCTCAACATCCGAGCTGG GTTATCTGTAAGAGTGGAACC TCTGTGAAGATCGAGTGCCGT TCCCTGGACTTTCAGGCCACA ACTATGTTTTGGTATCGTCAG TTCCCGAAACAGAGTCTCATG CTGATGGCAACTTCCAATGAG GGCTCCAAGGCCACATACGA GCAAGGCGTCGAGAAGGACA AGTTTCTCATCAACCATGCAA GCCTGACCTTGTCCACTCTGA CAGTGACCAGTGCCCATCCTG AAGACAGCAGCTTCTACATCT GCAGTGCTAGAGCTTTAGGA GGCTACACCTTCGGTTCGGGG ACCAGGTTAACCGTTGTA (SEQ ID NO: 188) | 406 | 414 |
| (21)1 D5 | ATGATGAAATCCTTGAGAG TTTTACTGGTGATCCTGTG GCTTCAGTTAAGCTGGGTT TGGAGCCAACAGAAGGAG GTGGAGCAGGATCCTGGA CCACTCAGTGTTCCAGAGG GAGCCATTGTTTCTCTCAA CTGCACTTACAGCAACAGT GCTTTTCAATACTTCATGTG GTACAGACAGTATTCCAGA AAAGGCCCTGAGTTGCTG ATGTACACATACTCCAGTG GTAACAAAGAAGATGGAA GGTTTACAGCACAGGTCGA TAAATCCAGCAAGTATATCT CCTTGTTCATCAGAGACTC ACAGCCCAGTGATTCAGCC ACCTACCTCTGTGCAATGA GCGGTCACTTCAACAAATT TTACTTTGGATCTGGGACC AAACTCAATGTAAAACCAA (SEQ ID NO: 189) | ATGGGCTCCAGGCTGCTCTGT TGGGTGCTGCTTTGTCTCCTG GGAGCAGGCCCAGTAAAGGC TGGAGTCACTCAAACTCCAA GATATCTGATCAAAACGAGAG GACAGCAAGTGACACTGAGC TGCTCCCCTATCTCTGGGCAT AGGAGTGTATCCTGGTACCAA CAGACCCCAGGACAGGGCCT TCAGTTCCTCTTTGAATACTT CAGTGAGACACAGAGAAACA AAGGAAACTTCCCTGGTCGAT TCTCAGGGCGCCAGTTCTCTA ACTCTCGCTCTGAGATGAATG TGAGCACCTTGGAGCTGGGG GACTCGGCCCTTTATCTTTGC GCCAGCAGCTTGCGGGCCGG GGAGCTGTTTTTTGGAGAAG GCTCTAGGCTGACCGTACTG (SEQ ID NO: 190) | 397 | 390 |
| 1F5 | ATGGAGAAAATGTTGGAGT | ATGGGCTTCAGGCTCCTCTGC | 400 | 393 |
| | GTGCATTCATAGTCTTGTG GCTTCAGCTTGGCTGGTTG AGTGGAGAAGACCAGGTG ACGCAGAGTCCCGAGGCC CTGAGACTCCAGGAGGGA GAGAGTAGCAGTCTCAACT GCAGTTACACAGTCAGCG GTTTAAGAGGGCTGTTCTG GTATAGGCAAGATCCTGGG AAAGGCCCTGAATTCCTCT TCACCCTGTATTCAGCTGG GGAAGAAAAGGAGAAAGA AAGGCTAAAAGCCACATTA ACAAAGAAGGAAAGCTTT CTGCACATCACAGCCCCTA AACCTGAAGACTCAGCCA CTTATCTCTGTGCTGCCTCC AATAGTGGAGGTAGCAACT ATAAACTGACATTTGGAAA AGGAACTCTCTTAACCGTG AATCCAA (SEQ ID NO: 191) | TGTGTGGCCTTTTGTCTCCTG GGAGCAGGCCCAGTGGATTC TGGAGTCACACAAACCCCAA AGCACCTGATCACAGCAACT GGACAGCGAGTGACGCTGAG ATGCTCCCCTAGGTCTGGAGA CCTCTCTGTGTACTGGTACCA ACAGAGCCTGGACCAGGGCC TCCAGTTCCTCATTCAGTATTA TAATGGAGAAGAGAGAGCAA AAGGAAACATTCTTGAACGAT TCTCCGCACAACAGTTCCCTG ACTTGCACTCTGAACTAAACC TGAGCTCTCTGGAGCTGGGG GACTCAGCTTTGTATTTCTGT GCCAGCAGCGTAGACGGGAA GGGGACCCAGTACTTCGGGC CAGGCACGCGGCTCCTGGTG CTC (SEQ ID NO: 192) | | |

Fig. 60 shows establishment of peptide-specific CTL clones from the peripheral blood of a patient inoculated with an HSP105 peptide vaccine. HSP105-HLA-A2 Dextramer was not capable of being sufficiently stained, thus being unavailable for detection of binding T cells. For this reason, the peripheral blood of a patient inoculated with a cancer peptide vaccine with an HSP105-derived peptide was stimulated in the presence of the antigen, and CD8 T cells that exhibited degranulation response were detected through CD107a assay. CD107a-positive CD8 T cells were selected by single sorting, and cultured on allofeeder cells to establish T cell clones.

A of Fig. 60 shows reactivity evaluation for the IFN-γ productions of HLA-A2-restricted HSP105-specific T cell clones with an ELISPOT method. The established T cell clones were cocultured with target cells (T2 cells) to which an HSP105-derived peptide had been exogenously bound for 20 hours, and the antigen stimulation-dependent IFN-γ productions were evaluated. The antigen specificities were evaluated by using cells to which a HIV-derived peptide had been bound as a negative control.

B and C of Fig. 60 show the cytotoxic activities of HLA-A2-restricted HSP105-specific T cell clones. Target cells were labeled with a fluorescent dye (calcein) and cocultured with each T cell clone for 4 hours, and the cytotoxic activities against target cells were evaluated on the basis of attenuation of fluorescence as an indicator. B. T2 cells to which the peptide had been exogenously bound were targeted. The antigen specificities were evaluated by using T2 cells, a TAP-lacking cell line, to which the HSP105-derived peptide (HSPA-7) used for the peptide vaccine had been exogenously bound as a positive control, and those to which an HIV-derived peptide (HIVA2) had been bound as a negative control. C. The cytotoxic activities were evaluated with cancer cell lines endogenously expressing HSP105 as targets. Caski cells and SiHa-A2 cells were used as HSP105-positive cell lines, and an SW620 cell line and HepG2 cell line were used as HSP105-negative cell lines. The cytotoxic activity of the 11B9 clone was not evaluated.

### <Results>

T cell clones with HSP105 peptide reactivity were established from a patient of HLA-A2 haplotype who had been inoculated with an HSP105 peptide vaccine. HLA-A2-HSP105 Dextramer was not capable of being sufficiently stained, and it was difficult to detect reactive clones through Dextramer staining; accordingly, in the present examination, peripheral blood monocytes of the patient that had been subjected in advance to stimulation culture with the peptide were cocultured again with an A2-expressing K562 cell line in the presence of the peptide, and T cells that recognized the antigen and exhibited degranulation (with a degranulation evaluation method utilizing the phenomenon that when degranulation is exhibited, a CD107a molecule present in a cell is typically exposed on the cell surface) were separated by sorting to establish reactive T cell clones. Evaluation of the antigen-specific IFN-γ production capabilities of the five established T cell clones found IFN-γ production due to coculture with HLA-A2 T2 cells in the presence of the peptide in all the clones, and that 7B9, 10G7, and 10C9 exhibited strong IFN-γ production capability, in particular. The 11B9 clone was found to have largely lower reactivity than the other clones, and hence cytotoxicity test was subsequently carried out for 7B9, 8D4, 10G7, and 10C9. All of the four clones exhibited damaging activity to T2 cells to which the peptide had been exogenously bound, and the 7B9 and 8D4 clones exhibited strong damaging activity, in particular. Results of additional evaluation of the cytotoxic activities of those clones against cancer cell lines endogenously expressing HSP105 showed that each of the 7B9, 10G7, and 10C9 clones exhibited significant cytotoxic activity against the CasKi cell line. No significant cytotoxic activity was found against the SiHaA2 and SW620 cell lines as compared with the HSP105-negative HepG2 cell line. The 8D4 and 11B9 clones were not examined, and we will reevaluate them in the future. The amino acid sequences and the gene sequences of TCR genes isolated from the clones were shown in tables.

**[Table 8]**

| Table 8: Amino acid sequences of HLA-A2-restricted T-cell receptors that recognize HSP105-derived peptide (RLMNDMTAV or KLMSSNSTDL) | | | | |
|---|---|---|---|---|
| Clone | Sequence TRA | Sequence TRB | TRA (bp) | TRB (bp) |
| 11B9 | MACPGFLWALVISTCLEFSM AQTVTQSQPEMSVQEAETVT LSCTYDTSESDYYLFWYKQP PSRQMILVIRQEAYKQQNATE NRFSVNFQKAAKSFSLKISDS QLGDAAMYFCAYRIPGAGS YQLTFGKGTKLSVIP (SEQ ID NO: 193) | MGTRLLCWVVLGFLGTDHTG AGVSQSPRYKVAKRGQDVAL RCDPISGHVSLFWYQQALGQ GPEFLTYFQNEAQLDKSGLPS DRFFAERPEGSVSTLKIQRTQQ EDSAVYLCASSPGLAGGAQET QYFGPGTRLLVL (SEQ ID NO: 194) | 136 | 136 |
| 7B9 | METLLGVSLVILWLQLARVN SQQGEEDPQALSIQEGENAT MNCSYKTSINNLQWYRQNS GRGLVHLILIRSNEREKHSGR LRVTLDTSKKSSSLLITASRA ADTASYFCATDTGGAQKLVF GQGTRLTINP (SEQ ID NO: 195) | MDSWTLCCVSLCILVAKHTDA GVIQSPRHEVTEMGQEVTLRC KPISGHDYLFWYRQTMMRGL ELLIYFNNNVPIDDSGMPEDRF SAKMPNASFSTLKIQPSEPRDS AVYFCASSPDRGETQYFGPGT RLLVL (SEQ ID NO: 196) | 131 | 132 |
| 10G7 | MWGVFLLYVSMKMGGTTG QNIDQPTEMTATEGAIVQINC TYQTSGFNGLFWYQQHAGE APTFLSYNVLDGLEEKGRFS SFLSRSKGYSYLLLKELQMK DSASYLCAVRALREAGTALIF GKGTTLSVSS (SEQ ID NO: 197) | MDTWLVCWAIFSLLKAGLTEP EVTQTPSHQVTQMGQEVILRC VPISNHLYFYWYRQILGQKVE FLVSFYNNEISEKSEIFDDQFSV ERPDGSNFTLKIRSTKLEDSA MYFCASSQNREAFFGQGTRLT VV (SEQ ID NO: 198) | 129 | 131 |
| 8D4 | MISLRVLLVILWLQLSWVWS QRKEVEQDPGPFNVPEGATV AFNCTYSNSASQSFFWYRQD CRKEPKLLMSVYSSGNEDGR FTAQLNRASQYISLLIRDSKL SDSATYLCVVSARVFGSGTR LSIRP (SEQ ID NO: 199) | MGTSLLCWMALCLLGADHA DTGVSQDPRHKITKRGQNVTF RCDPISEHNRLYWYRQTLGQG PEFLTYFQNEAQLEKSRLLSDR FSAERPKGSFSTLEIQRTEQGD SAMYLCASSPLAGVRYEQYF GPGTRLTVT (SEQ ID NO: 201) | 126 | 134 |
| | MMKSLRVLLVILWLQLSWV WSQQKEVEQDPGPLSVPEGA IVSLNCTYSNSAFQYFMWYR QYSRKGPELLMYTYSSGNKE DGRFTAQVDKSSKYISLFIRD SQPSDSATYLCAMSVRGSGA GSYQLTFGKGTKLSVIP (SEQ ID NO: 200) | | 137 | |
| 10C9 | MSLSSLLKVVTASLWLGPGI AQKITQTQPGMFVQEKEAVT LDCTYDTSDPSYGLFWYKQP | MGFRLLCCVAFCLLGAGPVDS GVTQTPKHLITATGQRVTLRCS PRSGDLSVYWYOOSLDOGLO | 135 | 131 |
| | SSGEMIFLIYQGSYDQQNATE GRYSLNFQKARKSANLVISA SQLGDSAMYFCAMRDRNTG NQFYFGTGTSLTVIP (SEQ ID NO: 202) | FLIQYYNGEERAKGNILERFSA QQFPDLHSELNLSSLELGDSA LYFCASSVEVYNEQFFGPGTR LTVL (SEQ ID NO: 203) | | |

**[Table 9]**

| Table 9: Gene sequences for HLA-A2-restricted T-cell receptors that recognize HSP105-derived peptide (RLMNDMTAV or KLMSSNSTDL) | | | | |
|---|---|---|---|---|
| clone | Sequence TRA | Sequence TRB | TRA (bp) | TRB (bp) |
| 11B9 | ATGGCATGCCCTGGCTTCCT GTGGGCACTTGTGATCTCCA CCTGTCTTGAATTTAGCATG GCTCAGACAGTCACTCAGT CTCAACCAGAGATGTCTGT GCAGGAGGCAGAGACCGTG ACCCTGAGCTGCACATATGA CACCAGTGAGAGTGATTATT ATTTATTCTGGTACAAGCAG CCTCCCAGCAGGCAGATGA TTCTCGTTATTCGCCAAGAA GCTTATAAGCAACAGAATG CAACAGAGAATCGTTTCTCT GTGAACTTCCAGAAAGCAG CCAAATCCTTCAGTCTCAAG ATCTCAGACTCACAGCTGG GGGATGCCGCGATGTATTTC TGTGCTTATAGGATCCCGGG TGCTGGGAGTTACCAACTC ACTTTCGGGAAGGGGACCA AACTCTCGGTCATACCAA (SEQ ID NO: 204) | ATGGGCACCAGGCTCCTCTG CTGGGTGGTCCTGGGTTTCC TAGGGACAGATCACACAGGT GCTGGAGTCTCCCAGTCCCC TAGGTACAAAGTCGCAAAGA GAGGACAGGATGTAGCTCTC AGGTGTGATCCAATTTCGGG TCATGTATCCCTTTTTTGGTA CCAACAGGCCCTGGGGCAGG GGCCAGAGTTTCTGACTTATT TCCAGAATGAAGCTCAACTA GACAAATCGGGGCTGCCCAG TGATCGCTTCTTTGCAGAAA GGCCTGAGGGATCCGTCTCC ACTCTGAAGATCCAGCGCAC ACAGCAGGAGGACTCCGCCG TGTATCTCTGTGCCAGCAGCC CGGGTTTAGCGGGAGGGGCC CAAGAGACCCAGTACTTCGG GCCAGGCACGCGGCTCCTGG TGCTC (SEQ ID NO: 205) | 409 | 408 |
| 7B9 | ATGGAAACTCTCCTGGGAG TGTCTTTGGTGATTCTATGG CTTCAACTGGCTAGGGTGA ACAGTCAACAGGGAGAAG AGGATCCTCAGGCCTTGAG CATCCAGGAGGGTGAAAAT GCCACCATGAACTGCAGTT ACAAAACTAGTATAAACAAT TTACAGTGGTATAGACAAA ATTCAGGTAGAGGCCTTGTC CACCTAATTTTAATACGTTC AAATGAAAGAGAGAAACAC AGTGGAAGATTAAGAGTCA CGCTTGACACTTCCAAGAA AAGCAGTTCCTTGTTGATCA | ATGGACTCCTGGACCCTCTG CTGTGTGTCCCTTTGCATCCT GGTAGCAAAGCACACAGATG CTGGAGTTATCCAGTCACCCC GGCACGAGGTGACAGAGATG GGACAAGAAGTGACTCTGAG ATGTAAACCAATTTCAGGAC ACGACTACCTTTTCTGGTACA GACAGACCATGATGCGGGGA CTGGAGTTGCTCATTTACTTT AACAACAACGTTCCGATAGA TGATTCAGGGATGCCCGAGG ATCGATTCTCAGCTAAGATGC CTAATGCATCATTCTCCACTC TGAAGATCCAGCCCTCAGAA | 394 | 396 |
| | CGGCTTCCCGGGCAGCAGA CACTGCTTCTTACTTCTGTG CTACGGACACGGGGGGAGC CCAGAAGCTGGTATTTGGC CAAGGAACCAGGCTGACTA TCAACCCAA (SEQ ID NO: 206) | CCCAGGGACTCAGCTGTGTA CTTCTGTGCCAGCAGCCCGG ACAGGGGGGAGACCCAGTAC TTCGGGCCAGGCACGCGGCT CCTGGTGCTC (SEQ ID NO: 207) | | |
| 10G7 | ATGTGGGGAGTTTTCCTTCT TTATGTTTCCATGAAGATGG GAGGCACTACAGGACAAAA CATTGACCAGCCCACTGAG ATGACAGCTACGGAAGGTG CCATTGTCCAGATCAACTGC ACGTACCAGACATCTGGGT TCAACGGGCTGTTCTGGTA CCAGCAACATGCTGGCGAA GCACCCACATTTCTGTCTTA CAATGTTCTGGATGGTTTGG AGGAGAAAGGTCGTTTTTC TTCATTCCTTAGTCGGTCTA AAGGGTACAGTTACCTCCTT TTGAAGGAGCTCCAGATGA AAGACTCTGCCTCTTACCTC TGTGCTGTGAGAGCCCTCC GGGAGGCAGGAACTGCTCT GATCTTTGGGAAGGGAACC ACCTTATCAGTGAGTTCCA (SEQ ID NO: 208) | ATGGATACCTGGCTCGTATGC TGGGCAATTTTTAGTCTCTTG AAAGCAGGACTCACAGAAC CTGAAGTCACCCAGACTCCC AGCCATCAGGTCACACAGAT GGGACAGGAAGTGATCTTGC GCTGTGTCCCCATCTCTAATC ACTTATACTTCTATTGGTACA GACAAATCTTGGGGCAGAAA GTCGAGTTTCTGGTTTCCTTT TATAATAATGAAATCTCAGAG AAGTCTGAAATATTCGATGAT CAATTCTCAGTTGAAAGGCC TGATGGATCAAATTTCACTCT GAAGATCCGGTCCACAAAGC TGGAGGACTCAGCCATGTAC TTCTGTGCCAGCAGTCAAAA TCGGGAAGCTTTCTTTGGAC AAGGCACCAGACTCACAGTT GTA (SEQ ID NO: 209) | 388 | 391 |
| 8D4 | ATGATATCCTTGAGAGTTTT ACTGGTGATCCTGTGGCTTC AGTTAAGCTGGGTTTGGAG CCAACGGAAGGAGGTGGA GCAGGATCCTGGACCCTTC AATGTTCCAGAGGGAGCCA CTGTCGCTTTCAACTGTACT TACAGCAACAGTGCTTCTC AGTCTTTCTTCTGGTACAGA CAGGATTGCAGGAAAGAAC CTAAGTTGCTGATGTCCGTA TACTCCAGTGGTAATGAAG ATGGAAGGTTTACAGCACA GCTCAATAGAGCCAGCCAG TATATTTCCCTGCTCATCAG AGACTCCAAGCTCAGTGAT TCAGCCACCTACCTCTGTGT GGTGAGCGCCCGGGTCTTT GGATCAGGGACCAGACTCA GCATCCGGCCAA (SEQ ID NO: 210) | ATGGGCACCAGCCTCCTCTG CTGGATGGCCCTGTGTCTCCT GGGGGCAGATCACGCAGATA CTGGAGTCTCCCAGGACCCC AGACACAAGATCACAAAGA GGGGACAGAATGTAACTTTC AGGTGTGATCCAATTTCTGA ACACAACCGCCTTTATTGGTA CCGACAGACCCTGGGGCAGG GCCCAGAGTTTCTGACTTACT TCCAGAATGAAGCTCAACTA GAAAAATCAAGGCTGCTCAG TGATCGGTTCTCTGCAGAGA GGCCTAAGGGATCTTTCTCCA CCTTGGAGATCCAGCGCACA GAGCAGGGGGACTCGGCCAT GTATCTCTGTGCCAGCAGCCC CCTAGCGGGGGTCAGGTACG AGCAGTACTTCGGGCCGGGC ACCAGGCTCACGGTCACA (SEQ ID NO: 212) | 379 | 402 |
| | ATGATGAAATCCTTGAGAGT TTTACTGGTGATCCTGTGGC | | 412 | |
| | TTCAGTTAAGCTGGGTTTGG AGCCAACAGAAGGAGGTG GAGCAGGATCCTGGACCAC TCAGTGTTCCAGAGGGAGC CATTGTTTCTCTCAACTGCA CTTACAGCAACAGTGCTTTT CAATACTTCATGTGGTACAG ACAGTATTCCAGAAAAGGC CCTGAGTTGCTGATGTACAC ATACTCCAGTGGTAACAAA GAAGATGGAAGGTTTACAG CACAGGTCGATAAATCCAG CAAGTATATCTCCTTGTTCA TCAGAGACTCACAGCCCAG TGATTCAGCCACCTACCTCT GTGCAATGAGCGTCAGAGG CTCTGGGGCTGGGAGTTAC CAACTCACTTTCGGGAAGG GGACCAAACTCTCGGTCAT ACCAA (SEQ ID NO: 211) | | | |
| 10C9 | ATGTCACTTTCTAGCCTGCT GAAGGTGGTCACAGCTTCA CTGTGGCTAGGACCTGGCA TTGCCCAGAAGATAACTCA AACCCAACCAGGAATGTTC GTGCAGGAAAAGGAGGCTG TGACTCTGGACTGCACATAT GACACCAGTGATCCAAGTT ATGGTCTATTCTGGTACAAG CAGCCCAGCAGTGGGGAAA TGATTTTTCTTATTTATCAGG GGTCTTATGACCAGCAAAAT GCAACAGAAGGTCGCTACT CATTGAATTTCCAGAAGGC AAGAAAATCCGCCAACCTT GTCATCTCCGCTTCACAACT GGGGGACTCAGCAATGTAC TTCTGTGCAATGAGAGATAG GAACACCGGTAACCAGTTC TATTTTGGGACAGGGACAA GTTTGACGGTCATTCCAA (SEQ ID NO: 213) | ATGGGCTTCAGGCTCCTCTG CTGTGTGGCCTTTTGTCTCCT GGGAGCAGGCCCAGTGGATT CTGGAGTCACACAAACCCCA AAGCACCTGATCACAGCAAC TGGACAGCGAGTGACGCTGA GATGCTCCCCTAGGTCTGGA GACCTCTCTGTGTACTGGTAC CAACAGAGCCTGGACCAGG GCCTCCAGTTCCTCATTCAGT ATTATAATGGAGAAGAGAGA GCAAAAGGAAACATTCTTGA ACGATTCTCCGCACAACAGT TCCCTGACTTGCACTCTGAA CTAAACCTGAGCTCTCTGGA GCTGGGGGACTCAGCTTTGT ATTTCTGTGCCAGCAGCGTA GAGGTTTACAATGAGCAGTT CTTCGGGCCAGGGACACGGC TCACCGTGCTA (SEQ ID NO: 214) | 406 | 393 |

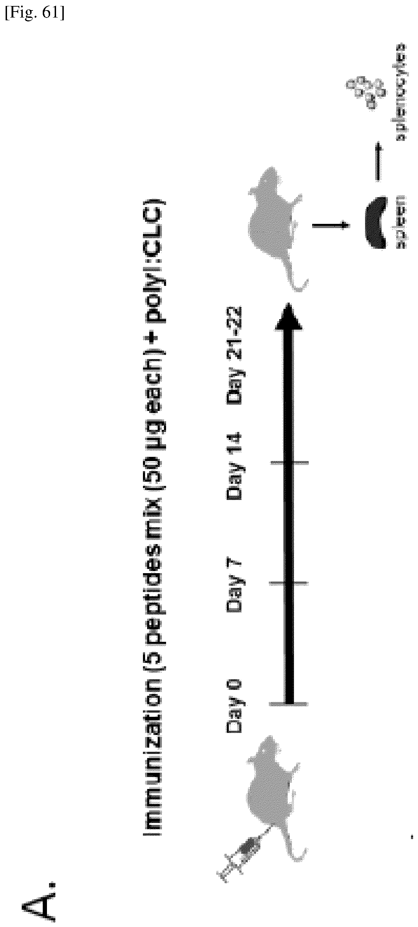

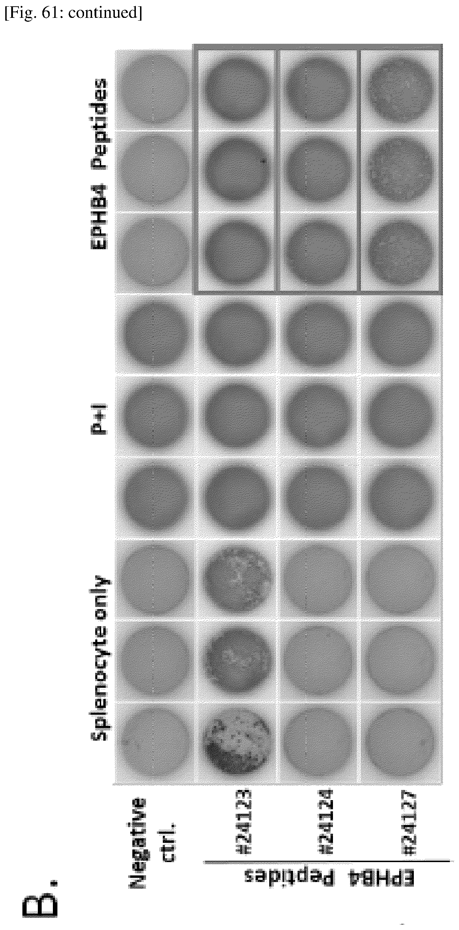

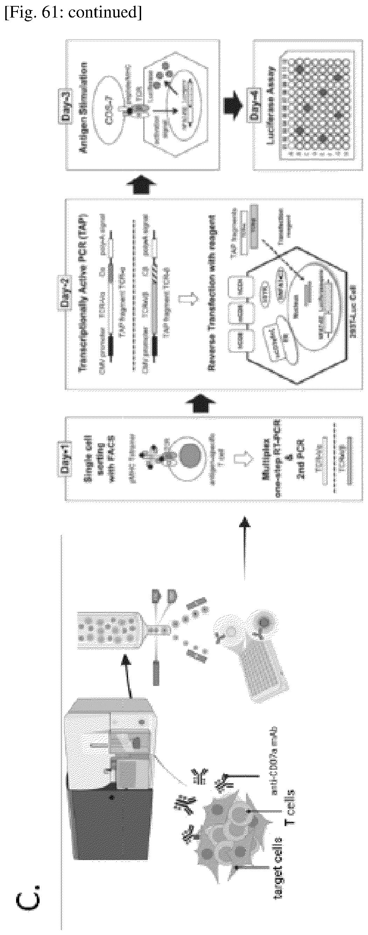

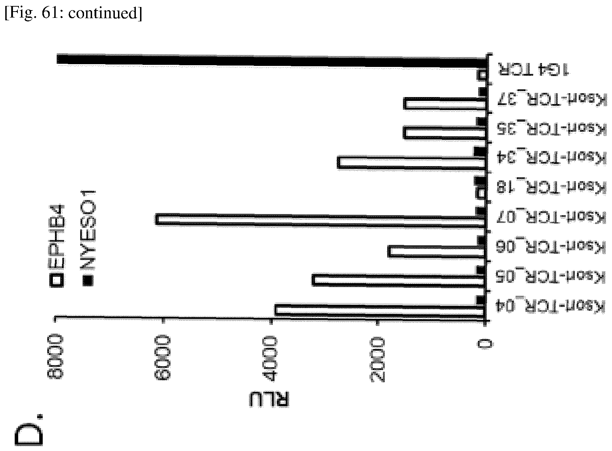

Fig. 61 shows identification of peptide-reactive TCR genes by using mice with human HLA genes introduced therein.

A. and B of Fig. 61 show peptide vaccination to mice with human HLA genes introduced therein and evaluation of the peptide reactivity in their spleens. Candidate cancer antigen-derived peptides each in an amount of 50 ug were each mixed with polyI:CLC as an adjuvant, each of the mixtures was intradermally administered to the base of the tail of the mice every week, three times in total. Thereafter, the spleens were extracted, each of them was cultured for 20 hours *in vitro* with addition of the peptide used for vaccination, and antigen-specific IFN-γ production was detected with an ELISPOT method.

C of Fig. 61 shows isolation of peptide-reactive TCR genes and evaluation of antigen reactivity by reporter assay. Each of the spleens of the vaccinated mice was cultured in the presence of the antigen peptide and an anti-CD107a antibody for 4 hours, and degranulated cells (CD107a-positive cells) were selected by single-cell sorting and collected in a 96-well plate. From the collected cells, TCR genes were amplified with use of reverse transcription and TCR-specific primers, and TAP fragments each incorporating any one of the TCR genes were constructed. Jurkat cells were transfected with each of the constructed TAP fragments with the TCR genes and an NFAT transcription factor-reactive luciferase-expressing vector. After culturing for 16 hours, the transfected Jurkat cells and HLA-A24-expressing COS-7 cells to which the corresponding antigen peptide had been exogenously bound were cocultured, and expressions of luciferase depending on stimulation by the reconstructed TCRs were measured with luciferase assay. The figure was excerpted from an article by Hamana et al., the University of Toyama (Biochem Biophys Res Commun. 2016, 10; 474(4): 709-714.).

D of Fig. 61 shows reactivity evaluation for EPHB4 peptide-reactive TCRs isolated from the mice with human HLA genes introduced therein. With the TCR-expressing Jurnakt cells prepared, each reactivity to the EPHB4-derived peptide was represented as luciferase activity (RLU). A known NY-ESO-1 peptide-specific TCR (1G4 TCR) was used as a positive control. Data for coculture with Cos-7 cells to which the EPHB4 peptide had been bound were represented as open bars, and data for coculture with Cos-7 cells to which the NY-ESO-1 peptide had been bound as solid bars.

### <Results>

In the present task, the above description has demonstrated that use of mice with human HLA genes introduced therein allows high-throughput identification of epitope peptides even for the novel cancer antigens. For the purpose of examining whether use of mice with human HLA genes introduced therein could similarly allow identification of TCR genes that recognize the novel cancer antigens, HLA-A24Tg mice were repeatedly immunized with the EPHB4-derived peptide together with an adjuvant to induce peptide-responsive CD8 T cells, and EPHB4-reactive TCR genes were then isolated from the spleens through single-cell sorting. Splenocytes of the vaccinated mice were restimulated *in vitro* in the presence of the peptide, and T cells that underwent degranulation and became CD107a-positive were collected through sorting. In conventional establishment of T cell clones, reculture in the presence of feeder cells is used for establishing T cell clones, but it takes much time for the growth to become stable, and T cell clones are not necessarily established successfully from all of the cells given by sorting. In view of this, we employed high-throughput methods of detection of TCR genes from single cells and functional evaluation of them, each developed by Hamana et al., the University of Toyama. In the methods by Hamana et al., TCR genes are directly amplified and isolated with a PCR method, not through reculture of cells given by sorting, TCR genes isolated with Gibson Assembly are each linked to a linear DNA containing a gene expression promoter to construct TCR gene expression vectors, cells of a Jurkat cell line derived from T-cell lymphoma are transfected with the constructed TCR gene expression vectors to reconstruct TCR molecules, and the reactivities are evaluated. Avoidance of culture enables short-time evaluation without any loss of cells given by sorting. Twenty-one of 39 CD107a-positive T cells isolated from splenocytes of the vaccinated mice through sorting allowed identification of their TCR genes, the TCR genes of eight T cells with duplicate TCR genes excluded were reconstructed, ant their antigen reactivities were evaluated. In each cell of the Jurkat cell line subjected to TCR reconstruction, a reporter gene from which luciferase gene expression is induced by the transcription factor NFAT, which is activated in the downstream of TCR signaling, had been incorporated in advance; and when the reconstructed TCR molecule recognized the antigen, the reactivity can be quantitatively evaluated by measuring light emission caused by luciferin-luciferase reaction depending on the intensity of the activation signal. In evaluation of the reactivities of the Jurkat cells subjected to TCR reconstruction in the presence of the EPHB4-derived peptide or the NY-ESO-1-derived peptide, seven of the eight TCR genes were found to be reactive with the EPHB4 peptide, and the TCR_07 gene was found to have the strongest antigen-specific reactivity. The amino acid sequences and the gene sequences of TCR genes isolated from the clones are shown in tables.

**[Table 10]**

| Table 10: Amino acid sequences of HLA-A2-restricted T-cell receptors that recognize EPHB4-derived peptide (RLNDGQFTV) | | | | |
|---|---|---|---|---|
| Clone | SequenCe TRA | SequenCe TRB | TRA (bp) | TRB (bp) |
| 5,6 | Not Determined | MRVRLISAVVLCFLGTGLVDMK VTQMPRYLIKRMGENVLLECGQ DMSHETMYWYRQDPGLGLQLI YISYDVDSNSEGDIPKGYRVSRK KREHFSLILDSAKTNQTSVYFCA SSPRQTNTEVFFGKGTRLTVVE (SEQ ID NO: 215) | - | 133 |
| 4,7,34 | MKTVTGPLFLCFWLQLNC VSRGEQVEQRPPHLSVRE GDSAFIICTYTDSATAYFY WYKQEPGAGLQLLMSVFS NVDRKEEQGLTVLLNKKD KRLSLNLTAAHPGDSAVYF CAVGNSNNRIFFGDGTQLV VKP (SEQ ID NO: 216) | MNTKITQSPRYLILGRANKSLEC EQHLGHNAMYWYKQSAEKPPE LMFLYNLKQLIRNETVPSRFIPE CPDSSKLLLHISAVDPEDSAVYF CASSQLGSSAETLYFGSGTRLTV LE (SEQ ID NO: 217) | 132 | 115 |
| 35, 37 | MRPVTCSVLVLLLMLRRS NGDSVTQTEGLVTVTEGLP VKLNCTYQTTYLTIAFFWY VQYLNEAPQVLLKSSTDN KRTEHQGFHATLHKSSSSF HLQKSSAQLSDSALYYCA LSDYSNNRLTLGKGTQVV VLP (SEQ ID NO: 218) | MGSRLFLVLSLLCTKHMEAAVT QSPRNKVTVTGGNVTLSCRQTN SHNYMYWYRQDTGHGLRLIHY SYGAGNLQIGDVPDGYKATRTT QEDFFLLLELASPSQTSLYFCASS DLGGYNSPLYFAAGTRLTVTE (SEQ ID NO: 219) | 132 | 132 |

**[Table 11]**

| Table 11: Gene sequences for HLA-A2-restricted T-cell receptors that recognize EPHB4-derived peptide (RLNDGQFTV) | | | | |
|---|---|---|---|---|
| Clone | SequenCe TRA | SequenCe TRB | TRA (bp) | TRB (bp) |
| 5,6 | Not Determined | ATGAACACTAAAATTACTCAGT CACCAAGATATCTAATCCTGGG AAGAGCAAATAAGTCTTTGGA ATGTGAGCAACATCTGGGACAT AATGCTATGTACTGGTATAAAC AGAGCGCTGAGAAGCCGCCAG AGCTCATGTTTCTCTACAATCT TAAACAGTTGATTCGAAATGAG ACGGTGCCCAGTCGTTTTATAC CTGAATGCCCAGACAGCTCCA AGCTACTTTTACATATATCTGCC GTGGATCCAGAAGACTCAGCT GTCTATTTTTGTGCCAGCAGCC AACTGGGGTCTAGTGCAGAAA CGCTGTATTTTGGCTCAGGAAC CAGACTGACTGTTCTCG (SEQ ID NO: 220) | - | 344 |
| 4,7,34 | ATGCGTCCTGTCACCTGC TCAGTTCTCGTGCTCCTC CTAATGCTCAGAAGGAGC AATGGAGACTCCGTGACC CAGACAGAAGGCCTGGT CACTGTCACCGAGGGGTT GCCTGTGAAGCTGAACTG CACCTATCAGACTACTTAT TTAACTATTGCCTTTTTCT GGTATGTGCAATATCTCAA CGAAGCCCCTCAGGTACT CCTGAAGAGCTCCACAG ACAACAAGAGGACCGAG CACCAAGGGTTCCACGCC ACTCTCCATAAGAGCAGC AGCTCCTTCCATCTGCAG AAGTCCTCAGCGCAGCTG TCAGACTCTGCCCTGTAC TACTGTGCTCTGAGTGAC TACAGCAACAACAGACTT ACTTTGGGGAAGGGAAC CCAGGTGGTGGTGTTACC AA (SEQ ID NO: 221) | ATGGGCTCCAGGCTCTTTCTGG TCTTGAGCCTCCTGTGTACAAA ACACATGGAGGCTGCAGTCAC CCAAAGCCCTAGAAACAAGGT GACAGTAACAGGAGGAAACGT GACATTGAGCTGTCGCCAGAC TAATAGCCACAACTACATGTAC TGGTATCGGCAGGACACTGGG CATGGGCTGAGGCTGATCCATT ACTCATATGGTGCTGGCAACCT TCAAATAGGAGATGTCCCTGAT GGGTACAAGGCCACCAGAACA ACGCAAGAAGACTTCTTCCTC CTGCTGGAATTGGCTTCTCCCT CTCAGACATCTTTGTACTTCTG TGCCAGCAGTGATCTGGGGGG CTATAATTCGCCCCTCTACTTTG CGGCAGGCACCCGGCTCACTG TGACAG (SEQ ID NO: 222) | 397 | 394 |
| 35, 37 | ATGCGTCCTGACACCTGC TCAGTTCTTGTGCTCCTCT TAATGCTCAGAAGGAACA ATGGAGACTCTGTGACCC AGACAGAAGGCCTGGTC ACTCTCACCGAGGGGTTG CCTGTGATGCTGAACTGC ACCTATCAGAGTACTTAC TCACCTTTCCTTTTCTGGT ATGTGCAACATCTCAACG AAGCCCCTAAGCTACTTT TGAAGAGCTTCACAGAC AACAAGAGGCCCGAGCA CCAAGGGTTCCACGCCAC TCTCCATAAGAGCAGCAG CTCCTTCCATCTGCAGAA GTCCTCAGCGCAGCTGTC AGACTCTGCCCTGTACTA CTGTGCTTTGAGTGATCG GTCGGGAAATGAGAAAAT AACTTTTGGGGCTGGAAC CAAACTCACCATTAAACC CA (SEQ ID NO: 223) | ATGAGAGTTAGGCTCATCTCTG CTGTGGTGCTGTGTTTCCTAGG AACAGGCCTTGTGGACATGAA AGTAACCCAGATGCCAAGATA CCTGATCAAAAGAATGGGAGA GAATGTTTTGCTGGAATGTGGA CAGGACATGAGCCATGAAACA ATGTACTGGTATCGACAAGACC CTGGTCTGGGGCTACAGCTGAT TTATATCTCATACGATGTTGATA GTAACAGCGAAGGAGACATCC CTAAAGGATACAGGGTCTCAC GGAAGAAGCGGGAGCATTTCT CCCTGATTCTGGATTCTGCTAA AACAAACCAGACATCTGTGTA CTTCTGTGCTAGCAGTCCAAGA CAAACAAACACAGAAGTCTTC TTTGGTAAAGGAACCAGACTC ACAGTTGTAG (SEQ ID NO: 224) | 397 | 397 |

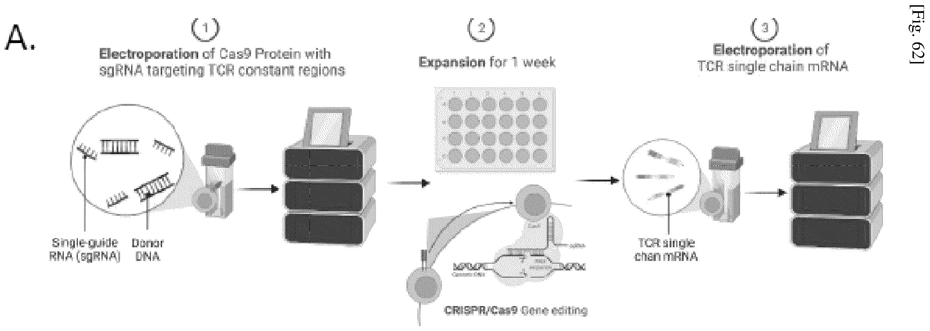

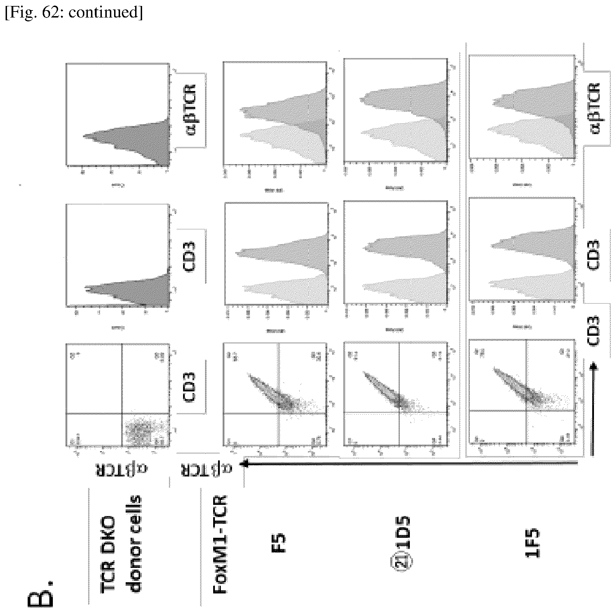

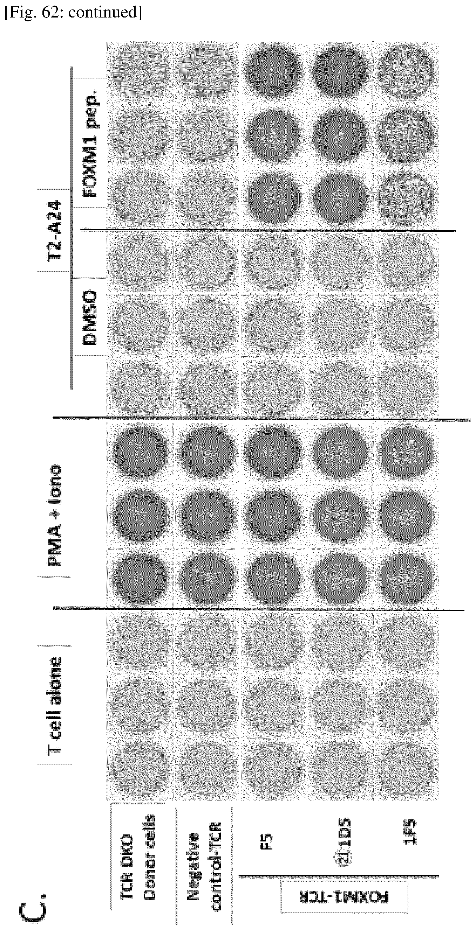

Fig. 62 shows preparation of TCR-T cells that recognize a common cancer antigen (FOXM1) by an electroporation method.

A of Fig. 62 shows preparation of TCR-T cells by an electroporation method. After peripheral blood monocytes were cultured in the presence of an anti-CD3 antibody and IL2, sgRNA specific to the constant region of a TCR was introduced together with Cas9 protein by an electroporation method to prepare T cells lacking endogenous TCRs (TCR DKO donor cells). After culturing, an mRNA of a single-stranded TCR gene in which a TCR α chain gene and a TCR β chain gene were linked via an P2A sequence was introduced by an electroporation method to prepare TCR-T cells.

B of Fig. 62 shows expressions of TCR molecules on the cell surfaces of the prepared TCR-T cells. The prepared TCR-T cells were stained with an anti-CD3 antibody and an anti-TCRαβ antibody, and expressions of TCR molecules on the cell surfaces were evaluated. The expressions of CD3 and TCRαβ molecules on the cell surfaces were represented as histograms (red: TCR-T cells, blue: Donor cells).

C of Fig. 62 shows IFN-y productions caused by antigen stimulation in the prepared TCR-T cells. The prepared TCR-T cells were cocultured with T2-A24 cells to which a cancer antigen peptide (FOXM1 pep.) had been exogenously bound for 20 hours, and IFN-y production was detected with an ELISPOT method.

### <Results>

TCR-T cells with the identified cancer antigen-reactive TCR genes reconstructed were constructed. In the present task, artificial single-stranded TCR genes in each of which the α chain and β chain of a TCR were linked via a P2A sequence were synthesized, and TCR mRNAs prepared from the synthesized artificial genes through *in vitro* transcription were introduced with an electroporation method into peripheral blood-derived CD8-positive T cells subjected to stimulation culture with an anti-CD3 antibody. Knocking-out endogenous TCR molecules in advance with a CRISPR-Cas9 system results in such high efficiency that only TCR molecules exogenously introduced and reconstructed are expressed on cell surfaces. Examination of previously identified FOXM1-specific TCR genes confirmed that all of the TCRs of the F5, (21)1D5, and 1F5 clones were highly expressed on cell surfaces. In evaluation of the antigen reactivities of the prepared TCR-T cells on the basis of IFN-y production as an indicator, all of the TCR-T cells exhibited strong reactivity with the FOXM1-derived peptide, as expected. The TCR of the 1F5 clone was sufficiently expressed, but the reactivity was lower than the other two, and (21)1D5 exhibited the highest reactivity; thus, the TCR gene of the (21)1D5 clone was found to be the most promising among those FOXM1-reactive TCRs. We will evaluate the antigen specificities of the obtained TCR genes and their utility in TCR-T cell therapy in the future.

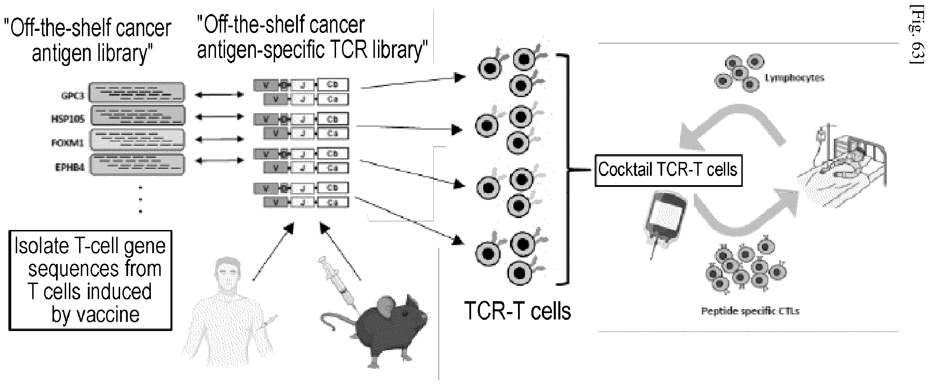

Fig. 63 shows a development scheme for cocktail TCR-T cells to overcome the diversity of cancers. A library of epitope peptides of the ten cancer antigens is constructed, and HLA-A2 or -A24-restricted TCR genes that specifically recognize those peptides are isolated from the peripheral blood of a cancer patient inoculated with a cancer vaccine with those peptides or mice with human HLA genes introduced therein repeatedly vaccinated with the peptides to construct a cancer antigen-specific TCR library. Expressions of the cancer antigens are screened to test the reactivities with peptide vaccines, and TCR genes each specific to any one of the multiple cancer antigen peptides are then selected to prepare TCR-T cells. The prepared TCR-T cells specific to the multiple cancer antigens are mixed together, and the resultant is administered as a "cocktail TCR-T cell" formulation; in this way, T cell therapy effective to the diversity of cancers is successfully established.

### <Results>

Identification of epitope peptides of the ten cancer antigens and construction of a cancer antigen peptide library enable not only achievement of off-the-shelf cancer peptide vaccines, but also implementation of "cocktail TCR-T cell" therapy to break through the immune evasion due to the diversity of cancers by targeting multiple cancer antigens when combined with identification of potent TCRs that recognize those peptides and preparation of a cancer antigen-specific TCR library.

### Example 12: Development of CAR-T cell therapy and cocktail CAR-T cell therapy targeting membrane protein common cancer antigens

### <Method>

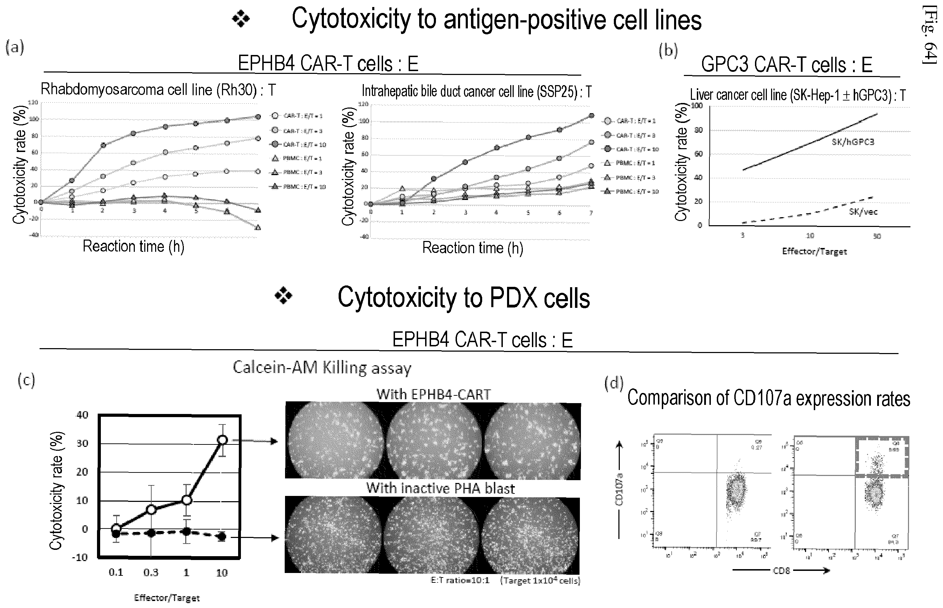

Fig. 64(a): In *in vitro* cytotoxicity evaluation for CAR-T cells, the rhabdomyosarcoma cell line Rh30 and the intrahepatic bile duct carcinoma cell line SSP25 were used as target cells. To each cell line, Calcein-AM (DOJINDO LABORATORIES) with a concentration of 10 µg/ml was added, and the cell lines were left to stand at 37°C for 30 minutes for staining. EPHB4 CAR-T cells to serve as effector cells and the stained target cells were cocultured at a ratio of E/T = 1, E/T = 3, E/T = 10, or E/T = 30 in a 96-well Half Area, Flat Bottom plate (Corning Incorporated) for 4 to 6 hours, and fluorescence values before and after the culture were acquired through measurement with a Terascan VPC2 (Minerva Tech K.K.). For natural liberation of Calcein, inactivated PBMCs (heated on a heat block set at 80°C for 15 minutes or more) were cocultured in place of effector cells. Similarly, for maximum liberation, inactivated PBMCs with addition of IGEPAL CA-630 (MP Biomedicals) (Nonidet P-40 equivalent) with a concentration of 6% were used. Cytotoxicity rates were calculated with the following calculation method. Cytotoxicity rate (%) = Amount of liberation in experimental group - Amount of natural liberation in experimental group) / (Maximum amount of liberation in experimental group - Amount of natural liberation in experimental group) × 100

(b): For GPC3 CAR-T cells, prepared GC33 CAR γδ T cells were used as effector cells. For the cells, a retroviral vector to express a CAR derived from the GPC3-specific antibody GC33 developed by the Chugai-Roche Group was used to introduce the CAR into PBMCs of a healthy individual on day 6 of stimulation culture with zoledronic acid, and a clone of CAR-expressing Vg9-positive γδ T cells was prepared through single-cell sorting for positive cells. The cell line SK/hGPC3 obtained by forcing the cell line SK-Hep-1, a liver carcinoma cell line without expression of GPC3, to express a human GPC3 full-length sequence gene (hGPC3) was used as target cells, and a cell line transformed with an empty vector (SK/vec) was used as a negative control. Calcein staining for the target cells and calculation of damaging rates were performed in the same manner as described above.

(c): Cells were stained and subjected to measurement in the same manner as in (a) and (b). A PDX maintained and swollen in an immunodeficient mouse was extracted, the tissue was cut with scissors as finely as possible, then dispersed with a Tumor Dissociation Kit, human (Miltenyi Biotec), and subjected to erythrocyte removal treatment thereafter, and the resultant was used as target cells.

(d): PDX-derived cells prepared in the same manner as in (c) and EPHB4 CAR-T cells or blast cells activated in advance by PHA stimulation were cocultured, and CD107a-positive cells in CD8-positive cells were then subjected to flow cytometry analysis.

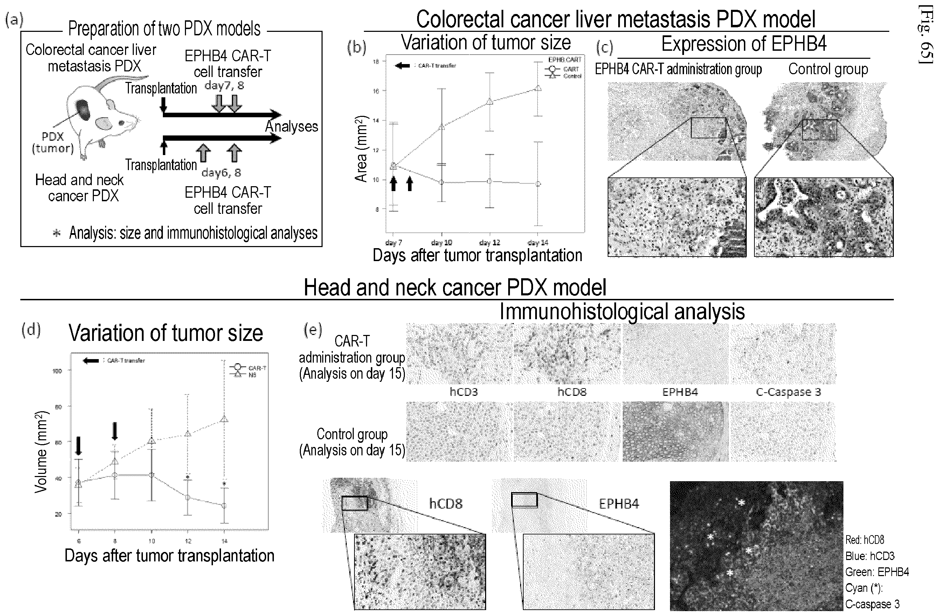

Fig. 65(a): For preparation of PDX models, SCID/Beige mice (colorectal cancer liver metastasis PDX) and NSG mice (head and neck cancer PDX model) were each subjected to transplantation with the corresponding PDX sized to a 5-mm cube. The major axis and minor axis of each tumor were measured with vernier calipers, and the size was calculated as area (b) or volume (d). (c), (e): For immunohistological analysis, FFPE thin section samples were prepared as described above. In short, after obtaining organs, they were soaked in 4% PFA for 18 to 24 h, and the PFA was then washed off with PBS(-). After soaking in 70% ethanol overnight or longer, the organs were embedded in paraffin to prepare paraffin blocks. Each block was sliced into 4-µm sections, which were attached to microscope slides, dried, and subjected to stainings. For DAB staining, polymer reagent suitable as primary antibodies (polymer reagent: EnVision+ System- HRP Labelled Polymer Anti-Rabbit or EnVision+ System- HRP Labelled Polymer Anti-Mouse) and a coloring reagent (Liquid DAB+ Substrate Chromogen System) were used. After counterstaining with hematoxylin, virtual slides were produced from the samples with a NanoZoomer (Hamamatsu Photonics K.K.). In multiple fluorescence immunohistochemical staining, which was also in accordance with the above method, antibodies to the antigens EPHB4 (Cell Signaling Technology, Inc.), human CD8 (Leica Biosystems Nussloch GmbH), CD3 (abcam plc.), and Cleaved Caspase-3 (Cell Signaling Technology, Inc.), polymer reagents (EnVision+ System- HRP Labelled Polymer Anti-Rabbit or EnVision+ System- HRP Labelled Polymer Anti-Mouse) as secondary antibodies, and Opal 520, 540, 570, 620, 650, and 690 (Akoya Biosciences, Inc.) as fluorescent dyes were used, and observation was performed with a Vectra 3 (PerkinElmer, Inc.).

### <Results>

Fig. 64 shows *in vitro* cytotoxicity evaluation for CAR-T cells.
(a): The cytotoxicity of EPHB4 CAR-T cells to EPHB4-positive tumor cell lines. Increase in cytotoxicity in an E:T ratio-dependent manner was found for both a rhabdomyosarcoma cell line (Rh30) and intrahepatic bile duct cancer cell line (SSP25), each expressing EPHB4.
(b): Evaluation of the cytotoxicity of GPC3 CAR-T. GPC3 CAR-T cells exhibited increase in cytotoxicity in an E:T ratio-dependent manner to the cell line SK/hGPC3 forced to express a human GPC3 full-length sequence gene (hGPC3).
(c): The cytotoxicity of EPHB4 CAR-T cells to EPHB4-positive PDX cells. Cytotoxicity rates after reaction for 8 hours were compared with use of EPHB4 CAR-T cells as effector cells and PDX cells as target cells at different E:T ratios. Increased E:T ratios resulted in increased cytotoxicity rates, and the highest cytotoxicity rate was found at E/T = 10. The right panels show microscopic photographs at E/T = 10.
(d): Confirmation of the cytotoxic response of EPHB4 CAR-T cells to EPHB4-positive PDX cells. EPHB4 CAR-T cells and blast cells activated with PHA stimulation were each cocultured with EPHB4-positive PDX cells, and the exposure of CD107a molecules that recognized the antigen and exhibited degranulation on cell surfaces was determined. The exposure of CD107a was found only in EPHB4 CAR-T cells, indicating the occurrence of antigen-specific induction of cytotoxic activity.

Fig. 65 shows *in vivo* cytotoxicity evaluation for CAR-T cells.
(a): Preparation of model animals by EPHB4-positive PDX transplantation (colorectal cancer liver metastasis and head and neck cancer). On day 7 after PDX transplantation for the models, EPHB4 CAR-T cells were transferred, and additionally transferred the next day (colorectal cancer liver metastasis model) or 2 days later (head and neck cancer model). For a control group, physiological saline was transferred.
(b): Variation in tumor size (area) in the colorectal cancer liver metastasis PDX model. The swelling of tumor was suppressed in the CAR-T cell transfer group. (c): Confirmation of expression of EPHB4 in the PDX region in the colorectal cancer liver metastasis PDX model. A tumor part (PDX) was sampled on day 6 after CAR-T cell transfer, and expression of EPHB4 was then confirmed for FFPE thin section samples by DAB staining. The disappearance of EPHB4 was found in the CAR-T cell transfer group. (d): Variation in tumor size (area) in the head and neck cancer PDX model. The swelling of tumor was suppressed in the CAR-T cell transfer group. (e): Immunohistological analysis of the PDX region in the head and neck cancer PDX model. Evaluation with DAB staining and hematoxylin staining except for the lower right panel. hCD3 and hCD8 were found in the CAR-T cell transfer group even on day 15, suggesting the presence of CAR-T cells. The disappearance of expression of EPHB4 was found in the transfer group, and expression of activated caspase 3 (C-Caspase 3), a marker for apoptosis, was found to be stronger than that in the control (top panels). In the lower left panel, reduced expression of EPHB4 was found in the region in which hCD8 was found, and expression of C-Caspase 3 was found in the lower right panel with multiple immunohistological staining (* parts).

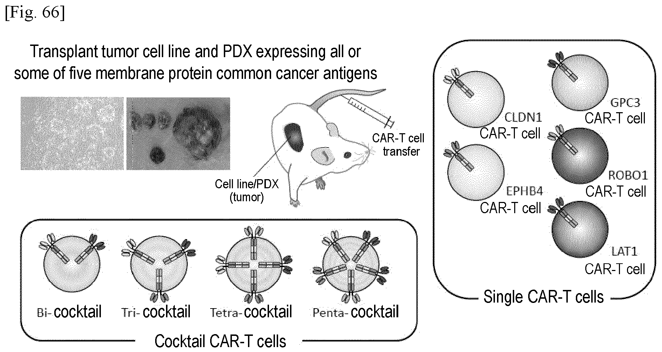

Fig. 66 shows development of cocktail CAR-T treatment.

Utilizing the fact that any of five membrane protein common cancer antigens is expressed in most cancer tissues, we will prepare cocktail CAR-T cells to express multiple types of CAR molecules, and aim to develop cocktail CAR-T treatment with an optimized combination and ratio. This will enable multifaceted handling of problems on cancer such as heterogeneity and patient-to-patient difference, specifically, induction of effective and efficient cancer growth suppression and infiltration of T cells.

### Example 13: Expression analysis for membrane protein common cancer antigens in various cancer cell lines and development of method for diagnosing risk of cancer onset by detecting circulating tumor cells with use of antibodies against membrane protein common cancer antigens

### <Method>

Hepatocellular carcinoma cell lines (HepG2, Hep3B, HuH-7, PLC/PRF/5, JHH-2, JHH-4, JHH-5, JHH-6, JHH-7), intrahepatic bile duct cancer cell lines (RBE, SSP-25), colorectal cancer cell lines (HCT116, Lovo, colo201, HT29, WiDr, SW480, CaCO2), pancreatic cancer cell lines (Panc1, AsPC1, PK1, PK9), lung cancer cell lines (RERF-LC-AL, LK-2, II-18, A549, LU99, H1975, Lu-135, NCI-H446), breast cancer cell lines (MDA-MB-231, MCF7), a cervical cancer cell line (HeLa), ovarian cancer cell lines (KOC-7C, NOY1, NOY2, RMG-II, RMUG-S), renal cell cancer cell lines (Caki-1, ACHN, RCC7), malignant melanoma cell lines (A375ml, SKmel23), mouth cancer cell lines (SAS, Ca9-22, HSC2, HSC3), gastric cancer cell lines (MKN74, MKN45, KATO-3), and lymphoma cell lines (Raji, K562) were peeled off with 0.05% Trypsin-EDTA, then treated with Fc block (BD Biosciences, 10-fold diluted) in FACS buffer (PBS + 2% FCS) for 20 minutes, further stained with a PE-labeled anti-EpCAM antibody (Miltenyi Biotech, 100-fold diluted), a PE-labeled anti-Cell-Surface Vimentin (CSV) antibody (Abnova Corporation, clone: 84-1, 100-fold diluted), an APC-labeled anti-human GPC3 antibody (Sino Biological, Inc., clone: 024, 10-fold diluted), an AF647-labeled anti-human ROBO1 antibody (R&D Systems, Inc., clone: 770502, 50-fold diluted), an AF647-labeled anti-human Claudin-1 antibody (R&D Systems, Inc., clone: 421203, 25-fold diluted), and an AF647-labeled anti-human EphB4 antibody (Miltenyi Biotech, clone: REA923, 50-fold diluted) for 20 minutes, and analyzed with a FACS Canto (BD Biosciences).

### <Results>

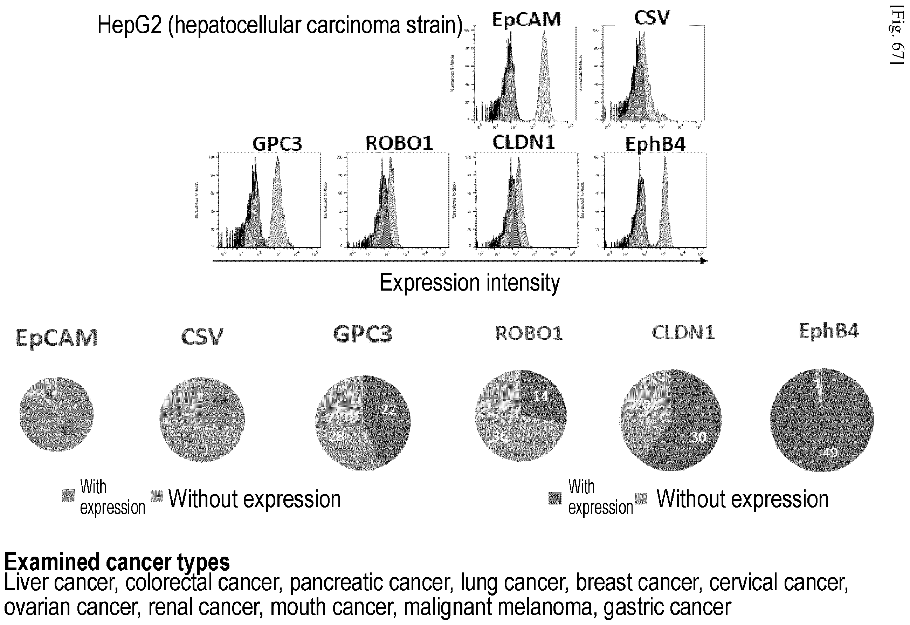

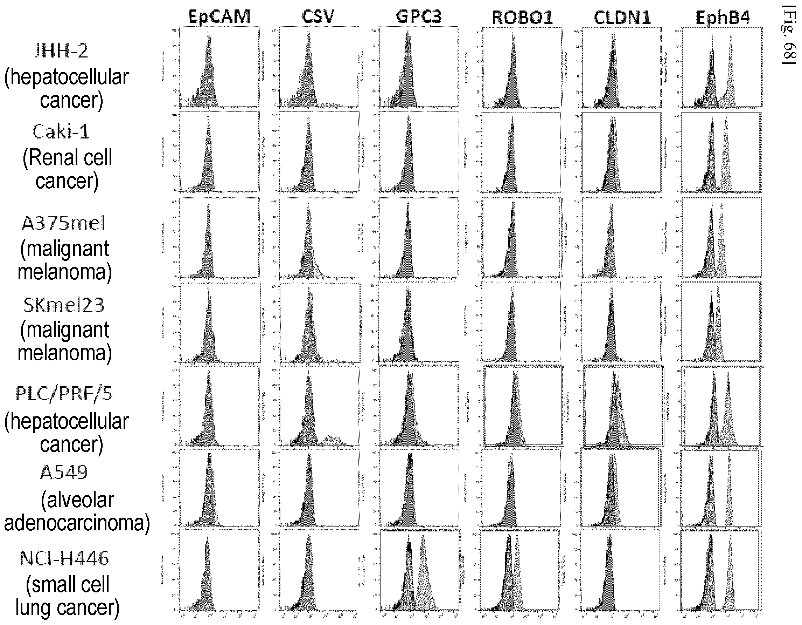

The results are shown in Fig. 67 and Fig. 68.

The hepatocellular carcinoma cell line HepG2 was expressing all of the four membrane protein common cancer antigens GPC3, ROBO1, CLDN1, and EPHB4 in addition to EpCAM and CSV.

The 50 cell lines derived from various cancer types were stained with antibodies against those antigens, and it was found that seven cell lines that were hardly expressing the conventional CTC markers EpCAM and CSV were expressing any of the four membrane protein common cancer antigens GPC3, ROBO1, CLDN1, and EPHB4 on the cell membranes. This finding suggests the possibility that CTCs that are missed with conventional CTC markers are successfully captured by using the four membrane protein common cancer antigens in combination.

### <Method>

From a hepatocellular carcinoma patient 3 days after tumor extirpation, approximately 5 mL of peripheral blood was collected with an EDTA-2K blood collection tube. To each of the hepatocellular carcinoma cell line HepG2 and the colorectal cancer cell line HCT116 each suspended in PBS, a 1/2 volume of Calcein-AM (DOJINDO LABORATORIES) diluted with PBS to 0.1 ug/mL was added, and the cell lines were labeled at 37°C for 15 minutes. To a half of the peripheral blood of the patient, 1 × 10^3 cells of HepG2 and 1 × 10^3 cells of HCT116, each labeled with Calcein, were added. The peripheral blood to which the cancer cell lines had been added and the untreated peripheral blood were subjected to hemolysis at room temperature for 10 minutes with addition of a triple amount of RBC lysis buffer (G-Biosciences). Residual cells were suspended in 1 mL of MACS Buffer (PBS + 0.5% BSA + 2 mM EDTA), and stirred at 4°C for 1 hour with addition of 250 uL of CD45 Dynabeads and 250 uL of CD15 Dynabeads, and CD45-positive cells and CD15-positive cells were then removed with a magnet. Residual cells were stained with zombie-NIR 1200-fold diluted with PBS at room temperature for 15 minutes, then treated with Fc block (BD Biosciences, 10-fold diluted) in FACS buffer for 20 minutes, further stained with a PerCP-labeled anti-CD45 antibody (Miltenyi Biotech, 50-fold diluted), a PE-labeled anti-EpCAM antibody (Miltenyi Biotech, 100-fold diluted), a PE-labeled anti-Cell-Surface Vimentin (CSV) antibody (Abnova Corporation, 100-fold diluted), an APC-labeled anti-human GPC3 antibody (Sino Biological, Inc., 10-fold diluted), an AF647-labeled anti-human ROBO1 antibody (R&D Systems, Inc., 50-fold diluted), an AF647-labeled anti-human Claudin-1 antibody (R&D Systems, Inc., 50-fold diluted), an AF647-labeled anti-human EphB4 antibody (Miltenyi Biotech, 50-fold diluted), and Hoechst 33342 (DOJINDO LABORATORIES, 30 ug/mL) for 20 minutes, and analyzed with a FACS Melody (BD Biosciences).

### <Results>

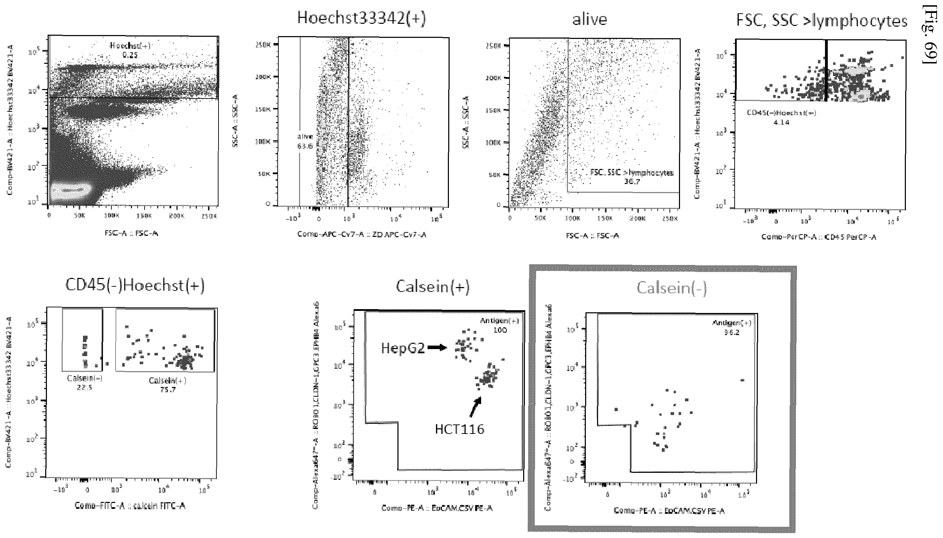

The results are shown in Fig. 69.

In a plurality of cases of hepatocellular carcinoma and colorectal cancer, CTC-like cells expressing any of the four membrane protein common cancer antigens GPC3, ROBO1, CLDN1, and EPHB4 without expressing any of the conventional CTC markers EpCAM and CSV were detected in 5 to 10 mL of a blood collection specimen before or after (on day 1 or day 3) excision operation; thus, it was demonstrated that adding GPC3, ROBO1, CLDN1, and EPHB4 in addition to the conventional CTC markers results in increased detection sensitivity for CTC-like cells.

## Claims

1. A cancer vaccine comprising:
(1) common cancer antigens comprising three or more selected from GPC3, ROBO1, EPHB4, CLDN1, and LAT1;
(2) partial peptides of the three or more common cancer antigens with CTL inducibility;
(3) a dendritic cell stimulated with the partial peptides; or
(4) mRNAs encoding the common cancer antigens or the partial peptides.

2. The cancer vaccine according to claim 1, wherein the common cancer antigens further comprise one or more of AFP, TGFBI, SPARC, HSP105α, and FOXM1.

3. The cancer vaccine according to claim 1, wherein the common cancer antigens comprise all of GPC3, ROBO1, EPHB4, CLDN1, and LAT1.

4. The cancer vaccine according to claim 1, wherein the common cancer antigens comprise all of GPC3, ROBO1, EPHB4, CLDN1, LAT1, AFP, TGFBI, SPARC, HSP105α, and FOXM1.

5. The cancer vaccine according to any one of claims 1 to 4, wherein the partial peptides are each a peptide having an amino acid sequence set forth in any of SEQ ID NOs: 1 to 80.

6. A cancer vaccine comprising:
(1) common cancer antigens comprising three or more selected from GPC3, ROBO1, EPHB4, CLDN1, LAT1, AFP, TGFBI, SPARC, HSP105α, and FOXM1;
(2) partial peptides of the three or more common cancer antigens with CTL inducibility;
(3) a dendritic cell stimulated with the partial peptides; or
(4) mRNAs encoding the common cancer antigens or the partial peptides.

7. A peptide having an amino acid sequence set forth in any of SEQ ID NOs: 5, 7 to 10, 14 to 22, 24 to 38, 40, 42, 48, 49, and 52 to 80.

8. A CAR-T cell therapy agent comprising a mixture of T cells with chimeric antigen receptors (CARs) for common cancer antigens comprising three or more selected from GPC3, ROBO1, EPHB4, CLDN1, and LAT1.

9. The CAR-T cell therapy agent according to claim 8, wherein the common cancer antigens further comprise one or more of AFP, TGFBI, SPARC, HSP105α, and FOXM1.

10. The CAR-T cell therapy agent according to claim 8, wherein the common cancer antigens comprise all of GPC3, ROBO1, EPHB4, CLDN1, and LAT1.

11. The CAR-T cell therapy agent according to claim 8, wherein the common cancer antigens comprise all of GPC3, ROBO1, EPHB4, CLDN1, LAT1, AFP, TGFBI, SPARC, HSP105α, and FOXM1.

12. A CAR-T cell therapy agent comprising a mixture of T cells with chimeric antigen receptors (CARs) for common cancer antigens comprising three or more selected from GPC3, ROBO1, EPHB4, CLDN1, LAT1, AFP, TGFBI, SPARC, HSP105α, and FOXM1.

13. A TCR-T cell therapy drug comprising a mixture of T cells with T-cell receptors (TCRs) capable of recognizing MHC class I-binding antigen peptides derived from common cancer antigens comprising three or more of GPC3, ROBO1, EPHB4, CLDN1, and LAT1.

14. The TCR-T cell therapy agent according to claim 13, wherein the common cancer antigens further comprise one or more of AFP, TGFBI, SPARC, HSP105α, and FOXM1.

15. The TCR-T cell therapy agent according to claim 13, wherein the common cancer antigens comprise all of GPC3, ROBO1, EPHB4, CLDN1, and LAT1.

16. The TCR-T cell therapy agent according to claim 13, wherein the common cancer antigens comprise all of GPC3, ROBO1, EPHB4, CLDN1, LAT1, AFP, TGFBI, SPARC, HSP105α, and FOXM1.

17. A TCR-T cell therapy drug comprising a mixture of T cells with T-cell receptors (TCRs) capable of recognizing MHC class I-binding antigen peptides derived from common cancer antigens comprising three or more of GPC3, ROBO1, EPHB4, CLDN1, LAT1, AFP, TGFBI, SPARC, HSP105α, and FOXM1.

18. The TCR-T cell therapy agent according to any one of claims 13 to 17, wherein each T-cell receptor (TCR) is any of:
a heterodimer consisting of a combination of a protein of SEQ ID NO: 103 and a protein of SEQ ID NO: 104;
a heterodimer consisting of a combination of a protein of SEQ ID NO: 105 and a protein of SEQ ID NO: 106;
a heterodimer consisting of a combination of a protein of SEQ ID NO: 107 and a protein of SEQ ID NO: 108;
a heterodimer consisting of a combination of a protein of SEQ ID NO: 109 and a protein of SEQ ID NO: 110;
a heterodimer consisting of a combination of a protein of SEQ ID NO: 111 and a protein of SEQ ID NO: 112;
a heterodimer consisting of a combination of a protein of SEQ ID NO: 123 and a protein of SEQ ID NO: 124;
a heterodimer consisting of a combination of a protein of SEQ ID NO: 125 and a protein of SEQ ID NO: 126;
a heterodimer consisting of a combination of a protein of SEQ ID NO: 127 and a protein of SEQ ID NO: 128;
a heterodimer consisting of a combination of a protein of SEQ ID NO: 129 or 130 and a protein of SEQ ID NO: 131;
a heterodimer consisting of a combination of a protein of SEQ ID NO: 132 and a protein of SEQ ID NO: 133;
a heterodimer consisting of a combination of a protein of SEQ ID NO: 134 and a protein of SEQ ID NO: 135;
a heterodimer consisting of a combination of a protein of SEQ ID NO: 136 and a protein of SEQ ID NO: 137;
a heterodimer consisting of a combination of a protein of SEQ ID NO: 138 or 139 and a protein of SEQ ID NO: 140;
a heterodimer consisting of a combination of a protein of SEQ ID NO: 141 and a protein of SEQ ID NO: 142;
a heterodimer consisting of a combination of a protein of SEQ ID NO: 143 and a protein of SEQ ID NO: 144;
a heterodimer consisting of a combination of a protein of SEQ ID NO: 145 and a protein of SEQ ID NO: 146;
a heterodimer consisting of a combination of a protein of SEQ ID NO: 147 and a protein of SEQ ID NO: 148;
a heterodimer consisting of a combination of a protein of SEQ ID NO: 177 and a protein of SEQ ID NO: 178;
a heterodimer consisting of a combination of a protein of SEQ ID NO: 179 and a protein of SEQ ID NO: 180;
a heterodimer consisting of a combination of a protein of SEQ ID NO: 181 and a protein of SEQ ID NO: 182;
a heterodimer consisting of a combination of a protein of SEQ ID NO: 183 and a protein of SEQ ID NO: 184;
a heterodimer consisting of a combination of a protein of SEQ ID NO: 193 and a protein of SEQ ID NO: 194;
a heterodimer consisting of a combination of a protein of SEQ ID NO: 195 and a protein of SEQ ID NO: 196;
a heterodimer consisting of a combination of a protein of SEQ ID NO: 197 and a protein of SEQ ID NO: 198;
a heterodimer consisting of a combination of a protein of SEQ ID NO: 199 or 200 and a protein of SEQ ID NO: 201;
a heterodimer consisting of a combination of a protein of SEQ ID NO: 202 and a protein of SEQ ID NO: 203;
a heterodimer consisting of a combination of a protein of SEQ ID NO: 216 and a protein of SEQ ID NO: 217; and
a heterodimer consisting of a combination of a protein of SEQ ID NO: 218 and a protein of SEQ ID NO: 219.

19. A protein having an amino acid sequence set forth in any of SEQ ID NOs: 103 to 112, 123 to 148, 177 to 184, 193 to 203, and 215 to 219.

20. A T-cell receptor (TCR) being any of:
a heterodimer consisting of a combination of a protein of SEQ ID NO: 103 and a protein of SEQ ID NO: 104;
a heterodimer consisting of a combination of a protein of SEQ ID NO: 105 and a protein of SEQ ID NO: 106;
a heterodimer consisting of a combination of a protein of SEQ ID NO: 107 and a protein of SEQ ID NO: 108;
a heterodimer consisting of a combination of a protein of SEQ ID NO: 109 and a protein of SEQ ID NO: 110;
a heterodimer consisting of a combination of a protein of SEQ ID NO: 111 and a protein of SEQ ID NO: 112;
a heterodimer consisting of a combination of a protein of SEQ ID NO: 123 and a protein of SEQ ID NO: 124;
a heterodimer consisting of a combination of a protein of SEQ ID NO: 125 and a protein of SEQ ID NO: 126;
a heterodimer consisting of a combination of a protein of SEQ ID NO: 127 and a protein of SEQ ID NO: 128;
a heterodimer consisting of a combination of a protein of SEQ ID NO: 129 or 130 and a protein of SEQ ID NO: 131;
a heterodimer consisting of a combination of a protein of SEQ ID NO: 132 and a protein of SEQ ID NO: 133;
a heterodimer consisting of a combination of a protein of SEQ ID NO: 134 and a protein of SEQ ID NO: 135;
a heterodimer consisting of a combination of a protein of SEQ ID NO: 136 and a protein of SEQ ID NO: 137;
a heterodimer consisting of a combination of a protein of SEQ ID NO: 138 or 139 and a protein of SEQ ID NO: 140;
a heterodimer consisting of a combination of a protein of SEQ ID NO: 141 and a protein of SEQ ID NO: 142;
a heterodimer consisting of a combination of a protein of SEQ ID NO: 143 and a protein of SEQ ID NO: 144;
a heterodimer consisting of a combination of a protein of SEQ ID NO: 145 and a protein of SEQ ID NO: 146;
a heterodimer consisting of a combination of a protein of SEQ ID NO: 147 and a protein of SEQ ID NO: 148;
a heterodimer consisting of a combination of a protein of SEQ ID NO: 177 and a protein of SEQ ID NO: 178;
a heterodimer consisting of a combination of a protein of SEQ ID NO: 179 and a protein of SEQ ID NO: 180;
a heterodimer consisting of a combination of a protein of SEQ ID NO: 181 and a protein of SEQ ID NO: 182;
a heterodimer consisting of a combination of a protein of SEQ ID NO: 183 and a protein of SEQ ID NO: 184;
a heterodimer consisting of a combination of a protein of SEQ ID NO: 193 and a protein of SEQ ID NO: 194;
a heterodimer consisting of a combination of a protein of SEQ ID NO: 195 and a protein of SEQ ID NO: 196;
a heterodimer consisting of a combination of a protein of SEQ ID NO: 197 and a protein of SEQ ID NO: 198;
a heterodimer consisting of a combination of a protein of SEQ ID NO: 199 or 200 and a protein of SEQ ID NO: 201;
a heterodimer consisting of a combination of a protein of SEQ ID NO: 202 and a protein of SEQ ID NO: 203;
a heterodimer consisting of a combination of a protein of SEQ ID NO: 216 and a protein of SEQ ID NO: 217; and
a heterodimer consisting of a combination of a protein of SEQ ID NO: 218 and a protein of SEQ ID NO: 219.

21. A gene having a nucleotide sequence of any of SEQ ID NOs: 113 to 122, 149 to 176, 185 to 192, 204 to 214, and 220 to 224.

22. A companion diagnostic method comprising: step 1 of simultaneously measuring the presence or absence of expressions of three or more of GPC3, ROBO1, EPHB4, CLDN1, and LAT1 and cell membrane expression of HLA class I in a sample derived from a subject by multiple immunofluorescence staining; and step 2 of determining indication for cancer immunotherapy on the basis of the presence or absence of the expressions.

23. The companion diagnostic method according to claim 22, wherein, in step 1, the presence or absence of expressions of one or more of AFP, TGFBI, SPARC, HSP105α, and FOXM1 is measured.

24. The companion diagnostic method according to claim 22, wherein, in step 1, the presence or absence of expressions of all of GPC3, ROBO1, EPHB4, CLDN1, and LAT1 is measured.

25. The companion diagnostic method according to claim 22, wherein, in step 1, the presence or absence of expressions of all of GPC3, ROBO1, EPHB4, CLDN1, LAT1, AFP, TGFBI, SPARC, HSP105α, and FOXM1 is measured.

26. A method for diagnosing risk of cancer onset, comprising analyzing expressions of three or more of GPC3, ROBO1, EPHB4, CLDN1, and LAT1 in cells in a blood sample derived from a patient.

27. The method for diagnosing risk of cancer onset according to claim 26, further comprising analyzing expressions of one or more of AFP, TGFBI, SPARC, HSP105α, and FOXM1.

28. The method for diagnosing risk of cancer onset according to claim 26, wherein expressions of all of GPC3, ROBO1, EPHB4, CLDN1, and LAT1 are analyzed.

29. The method for diagnosing risk of cancer onset according to claim 26, wherein expressions of all of GPC3, ROBO1, EPHB4, CLDN1, LAT1, AFP, TGFBI, SPARC, HSP105α, and FOXM1 are analyzed.
